# EUROPEAN PATENT APPLICATION

(11) **EP 2 284 159 A1**
(43) Date of publication of application: **16.02.2011**
(21) Application number: 09733735.6
(22) Date of filing: 21.04.2009
(51) Int. Cl.: C07D 239/34, A61K 31/505, A61K 31/506, A61P 7/06, A61P 43/00, C07D 239/56, C07D 401/06, C07D 405/06

(54) **5-HYDROXYPYRIMIDINE-4-CARBOXAMIDE COMPOUND**

(30) Priority: 22.04.2008 JP 2008111562
(71) Applicant: Daiichi Sankyo Company, Limited, Chuo-ku Tokyo 103-8426 (JP)
(72) Inventor: The designation of the inventor has not yet been filed
(74) Representative: Smyth, Gyles Darren
(86) International application number: PCT/JP2009/057937
(87) International publication number: WO 2009/131129

(57) **Abstract**

The present invention provides compounds which promote erythropoietin production. Compounds represented by the following general formula (1) or pharmacologically acceptable salts thereof are provided: 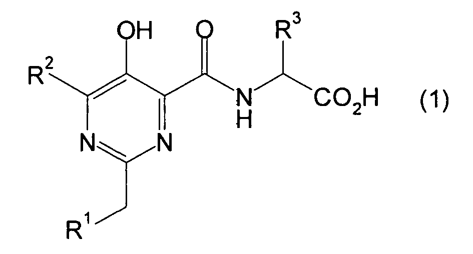 [wherein, R¹ represents a group -X-Q¹, X-Q¹-Y-Q² or -X-Q¹-Y-Q²-Z-Q³, X represents a single bond, -CH₂- or the like, Q¹ represents a monocyclic or bicyclic heterocyclic group which may have substituent(s), Y represents a single bond, -CH₂-, or the like, Q² represents a monocyclic or bicyclic hydrocarbon ring group which may have substituent(s) or a monocyclic or bicyclic heterocyclic group which may have substituent(s), Z represents a single bond, -CR¹¹R¹²- or the like, R¹¹ and R¹² each independently represents a hydrogen atom, a halogen atom or the like, Q³ represents a phenyl group which may have substituent(s), a C₃-C₇ cycloalkyl group which may have substituent(s), a C₃-C₇ cycloalkenyl group which may have substituent(s) or a monocyclic or bicyclic heterocyclic group which may have substituent(s), R² represents a C₁-C₃ alkyl group or the like, and R³ represents a hydrogen atom or a methyl group].

## Description

### Field of the art

The present invention relates to low-molecular-weight compounds that induce erythropoietin production.

### Background of the art

Erythropoietin (hereinafter abbreviated as EPO) is glycoprotein hormone that is essential for erythrocyte hematopoiesis. It is normally secreted from the kidneys and promotes the production of erythrocytes by acting on erythrocyte stem cells present in bone marrow. In diseases presenting with a decrease in intrinsic EPO production (such as chronic renal failure) and the like, since erythrocyte production decreases and symptoms of anemia are exhibited, treatment is provided in the form of replacement therapy using gene-recombinant human EPO. However, this treatment has been indicated as having shortcomings such as being a biological formulation, being associated with expensive medical care costs, having poor convenience due to being an injection, and having antigenicity.

On the other hand, compounds such as pyridine derivatives, cinnoline derivatives, quinoline derivatives, isoquinoline derivatives (see Patent Documents 1 to 6 or 8) or 6-hydroxy-2,4-dioxo-tetrahydropyrimidine derivatives (see Patent Document 7) are known to be low-molecular-weight EPO inducers.

### Prior art references

Patent references
- Patent reference 1: International Publication No. WO2003/049686
- Patent reference 2: International Publication No. WO2003/053997
- Patent reference 3: International Publication No. WO2004/108681
- Patent reference 4: International Publication No. WO2006/133391
- Patent reference 5: International Publication No. WO2007/038571
- Patent reference 6: International Publication No. WO2007/136990
- Patent reference 7: International Publication No. WO2007/15001
- Patent reference 8: International Publication No. WO2008/002576

### Disclosure of the invention

### Object of the invention

The inventors of the present invention conducted studies for the purpose of providing novel low molecular weight compounds that have superior EPO production activity and are useful for the treatment of diseases caused by decreased EPO, and for the purpose of providing pharmaceuticals containing such compounds.

### Means for achieving the object

The inventors of the present invention found that novel compounds having a 5-hydroxypyrimidine-4-carboxamide structure have superior EPO production activity and are effective for treating diseases caused by decreased levels of erythropoietin, thereby leading to completion of the present invention.

According to the present invention, novel 5-hydroxypyrimidine-4-carboxamide compounds represented by the following general formula (1), or pharmacologically acceptable salts thereof (hereinafter collectively referred to as compounds of the present invention), are provided.

Namely, the present invention relates to the following:
(1) a compound represented by the following formula (1) or a pharmacologically acceptable salt thereof: 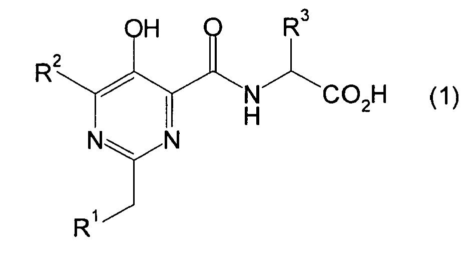 [wherein,
   R¹ represents a group -X-Q¹ X-Q¹-Y Q² or -X-Q¹-Y Q²-Z-Q³,
   X represents a single bond, -CH₂-, -(CH₂)₂-, -(CH₂)₃-, -CH₂O-, -OCH₂-, -O-, - S- or -CO-,
   Q¹ represents a monocyclic or bicyclic heterocyclic group which may have 1 or 2 substituents selected from substituent group α (wherein the heterocyclic group includes 4- to 10-membered aromatic heterocycles and non-aromatic heterocycles, and contains 1 or 2 atoms selected from the group consisting of a nitrogen atom, a sulfur atom and an oxygen atom),
   substituent group α represents a group consisting of a halogen atom, an oxo group, a hydroxyl group, a sulfanyl group, an amino group, a cyano group, a C₁-C₆ alkyl group and a halogenated C₁-C₆ alkyl group,
   Y represents a single bond, -CH₂-, -(CH₂)₂-, -(CH₂)₃-, -CH₂O-, -OCH₂-, -O-, - S-, -NH-, -NCH₃- or -CO-,
   Q² represents a monocyclic or bicyclic hydrocarbon ring group which may have 1 or 2 substituents selected from substituent group β (wherein the hydrocarbon ring group includes 3- to 10-membered aromatic hydrocarbon rings and non-aromatic hydrocarbon rings), or represents a monocyclic or bicyclic heterocyclic group which may have 1 or 2 substituents selected from substituent group β (wherein the heterocyclic group includes 4-to 10-membered aromatic heterocycles and non-aromatic heterocycles, and contains 1 or 2 atoms selected from the group consisting of a nitrogen atom, a sulfur atom and an oxygen atom),
   substituent group β represents a group consisting of a halogen atom, an oxo group, a hydroxyl group, a sulfanyl group, an amino group, a cyano group, a nitro group, a formyl group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a C₁-C₆ alkylamino group, a (C₁-C₆ alkyl)(C₁-C₆ alkyl)amino group and a C₂-C₇ alkanoyl group,
   Z represents a single bond, -CR¹¹R¹², -(CH₂)₂-, -(CH₂)₃-, -CH₂O- -OCH₂-, -O-, -S-, -NH-, -NCH₃- or -CO-,
   R¹¹ and R¹² each independently represents a hydrogen atom, a halogen atom, a hydroxyl group, a cyano group, a C₁-C₆ alkyl group, a halogenated C₁-C₆ alkyl group, a C₁-C₆ alkoxy group or a halogenated C₁-C₆ alkoxy group,
   Q³ represents a phenyl group which may have 1 or 2 substituents selected from substituent group γ, a C₃-C₇ cycloalkyl group which may have 1 or 2 substituents selected from substituent group γ, a C₃-C₇ cycloalkenyl group which may have 1 or 2 substituents selected from substituent group γ, or a monocyclic or bicyclic heterocyclic group which may have 1 or 2 substituents selected from substituent group γ (wherein the heterocyclic group includes 4- to 10-membered aromatic heterocycles and non-aromatic heterocycles, and contains 1 or 2 atoms selected from the group consisting of a nitrogen atom, a sulfur atom and an oxygen atom),
   substituent group γ represents a group consisting of a halogen atom, an oxo group, a hydroxyl group, a nitro group, a sulfanyl group, an amino group, a cyano group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a C₁-C₆ alkylamino group, a (C₁-C₆ alkyl)(C₁-C₆ alkyl)amino group and a C₂-C₇ alkanoyl group,
   R² represents a C₁-C₃ alkyl group, a methylsulfanyl group, a fluoromethyl group or a difluoromethyl group, and
   R³ represents a hydrogen atom or a methyl group];
(2) the compound or pharmacologically acceptable salt thereof according to (1) above, wherein X represents a single bond, -CH₂-, -(CH₂)₂- or -(CH₂)₃-,
(3) the compound or pharmacologically acceptable salt thereof according to (1) above, wherein X represents a single bond or -CH₂-,
(4) the compound or pharmacologically acceptable salt thereof according to any one of (1) to (3) above, wherein Q¹ represents a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a piperazinyl group, a dihydrooxazolyl group, a dihydrothiazolyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a tropinyl group, a chromanyl group, an isochromanyl group, a quinolyl group, a tetrahydroquinolyl group, an isoquinolyl group or a tetrahydroisoquinolyl group, each of which may have 1 or 2 substituents selected from substituent group α,
(5) the compound or pharmacologically acceptable salt thereof according to any one of (1) to (3) above, wherein Q¹ represents a dioxanyl group, a dioxepanyl group, a pyrrolidinyl group, a piperidyl group, an azepanyl group, a pyrrolyl group, a pyridyl group, a dihydropyridyl group, a tetrahydropyridyl group, a pyrimidinyl group, a thiazolyl group, a tropinyl group or a tetrahydroisoquinolyl group, each of which may have 1 or 2 substituents selected from substituent group α,
(6) the compound or pharmacologically acceptable salt thereof according to any one of (1) to (3) above, wherein Q¹ represents a piperidyl group or a pyridyl group, each of which may have 1 or 2 substituents selected from substituent group α,
(7) the compound or pharmacologically acceptable salt thereof according to any one of (1) to (6) above, wherein substituent group α represents a group consisting of a methyl group and an oxo group,
(8) the compound or pharmacologically acceptable salt thereof according to any one of (1) to (7) above, wherein R¹ represents a group X-Q¹-Y-Q² or -X-Q¹-Y Q²-Z-Q³, and Y represents a single bond, -CH₂-, -(CH₂)₂- or -(-CH2)3-,
(9) the compound or pharmacologically acceptable salt thereof according to (8) above, wherein Y represents a single bond,
(10) the compound or pharmacologically acceptable salt thereof according to any one of (1) to (9) above, wherein R¹ represents a group X-Q¹-Y Q² or -X-Q¹-Y Q²-Z-Q³, and Q² represents a cyclopentyl group, a cyclohexyl group, a cycloheptyl group, a cyclopentenyl group, a cyclohexenyl group, a cycloheptenyl group, a phenyl group, a naphthalenyl group, a tetrahydronaphthalenyl group, a decahydronaphthalenyl group, an indanyl group, an indenyl group, a norboranyl group, a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a piperazinyl group, a dihydrooxazolyl group, a dihydrothiazolyl group, a morpholinyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a tetrahydroquinolyl group, an isoquinolyl group, a tetrahydroisoquinolyl group, a benzoxazolyl group, a quinoxalyl group, a benzothiazolyl group, a benzodioxanyl group, an indolidinyl group, a thienopyridyl group, a dihydrothienopyridyl group, a furopyridyl group or a dihydrofuropyridyl group, each of which may have 1 or 2 substituents selected from substituent group β,
(11) the compound or pharmacologically acceptable salt thereof according to (10) above, wherein Q² represents a phenyl group, a naphthalenyl group, a pyridyl group, a thienyl group, a pyrimidinyl group, a pyridazinyl group; a pyrazinyl group, a thiazolyl group, an oxazolyl group, a dihydrobenzofuranyl group, a benzothiophenyl group, an indolyl group, a quinolyl group, an isoquinolyl group, a benzoxazolyl group, a quinoxalyl group or a benzothiazolyl group, each of which may have 1 or 2 substituents selected from substituent group β,
(12) the compound or pharmacologically acceptable salt thereof according to (10) above, wherein Q² represents a phenyl group, a pyridyl group, a thienyl group, a pyrimidinyl group, a pyrazinyl group or a thiazolyl group, each of which may have 1 or 2 substituents selected from substituent group β,
(13) the compound or pharmacologically acceptable salt thereof according to any one of (1) to (12) above, wherein R¹ represents a group X-Q¹-Y-Q² or -X-Q¹-Y-Q²-Z-Q³, and substituent group β represents a group consisting of a halogen atom, a hydroxyl group, a cyano group, a nitro group, a formyl group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a (C₁-C₆ alkyl)(C₁-C₆ alkyl)amino group and a C₂-C₇ alkanoyl group,
(14) the compound or pharmacologically acceptable salt thereof according to (13) above, wherein substituent group β represents a group consisting of a fluorine atom, a methyl group, a n-butyl group, a tert-butyl group and a trifluoromethyl group,
(15) the compound or pharmacologically acceptable salt thereof according to any one of (1) to (14) above, wherein R¹ represents a group -X-Q¹-Y Q²-Z-Q³, Z represents a single bond, -CR¹¹R¹²-, -(CH₂)₂-, -CH₂O- -OCH₂-, -O-, -CO- or -NCH₃-, and R¹¹ and R¹² each independently represents a hydrogen atom, a methyl group or a hydroxyl group,
(16) the compound or pharmacologically acceptable salt thereof according to (15) above, wherein Z represents a single bond or -CR¹¹R¹²-, and R¹¹ and R¹² each independently represents a hydrogen atom, a methyl group or a hydroxyl group,
(17) the compound or pharmacologically acceptable salt thereof according to any one of (1) to (16) above, wherein R¹ represents a group -X-Q¹-Y-Q²-Z-Q³, and Q³ represents a cyclopropyl group, a cyclobutyl group, a cyclopentyl group, a cyclohexyl group, a cycloheptyl group, a cyclopentenyl group, a cyclohexenyl group, a cycloheptenyl group, a phenyl group, a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a dihydrooxazolyl group, a dihydrothiazolyl group, a morpholinyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a tetrahydroquinolyl group, an isoquinolyl group, a tetrahydroisoquinolyl group, an imidazopyridyl group, a benzoxazolyl group, a quinoxalyl group, a benzothiazolyl group, a benzodioxanyl group, an indolidinyl group, a thienopyridyl group, a dihydrothienopyridyl group, a furopyridyl group or a dihydrofuropyridyl group, each of which may have 1 or 2 substituents selected from substituent group γ,
(18) the compound or pharmacologically acceptable salt thereof according to (17) above, wherein Q³ represents a cyclopropyl group, a cyclohexyl group, a cyclohexenyl group, a phenyl group, a dioxanyl group, a piperidyl group, a pyrrolyl group, a pyridyl group, a thienyl group, a pyrazolyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, a quinolyl group, a tetrahydroquinolyl group, an isoquinolyl group, an imidazopyridyl group or a benzodioxanyl group, each of which may have 1 or 2 substituents selected from substituent group γ,
(19) the compound or pharmacologically acceptable salt thereof according to (17) above, wherein Q³ represents a cyclopropyl group, a phenyl group, a pyridyl group, a thienyl group or a thiazolyl group, each of which may have 1 or 2 substituents selected from substituent group γ,
(20) the compound or pharmacologically acceptable salt thereof according to any one of (1) to (19) above, wherein R¹ represents a group -X-Q¹-Y-Q²-Z-Q³, and substituent group γ represents a group consisting of a halogen atom, a hydroxyl group, a nitro group, an amino group, a cyano group, a C₁-C₆ alkyl group, a halogenated C₁-C₆ alkyl group and a C₁-C₆ alkoxy group,
(21) the compound or pharmacologically acceptable salt thereof according to (20) above, wherein substituent group γ represents a group consisting of a fluorine atom, a methyl group and a methoxy group,
(22) the compound or pharmacologically acceptable salt thereof according to any one of (1) to (21), wherein R² represents a methyl group, a methylsulfanyl group or a difluoromethyl group,
(23) the compound or pharmacologically acceptable salt thereof according to any one of (1) to (21) above, wherein R² represents a methyl group or a methylsulfanyl group, and,
(24) the compound or pharmacologically acceptable salt thereof according to any one of (1) to (23), wherein R³ represents a hydrogen atom.
   Moreover, the present invention relates to the following:
(25) the compound or pharmacologically acceptable salt thereof according to (1) above, wherein
   R¹ represents a group X-Q¹-Y-Q²,
   X represents a single bond, -CH₂-, -(CH₂)₂- or -(CH₂)₃-,
   Q¹ represents a piperidyl group or a pyridyl group, each of which may have 1 or 2 substituents selected from substituent group α,
   substituent group α represents a group consisting of a methyl group,
   Y represents a single bond,
   Q² represents a phenyl group, a naphthalenyl group, a pyridyl group, a thienyl group, a thiazolyl group, a benzothiophenyl group, a quinolyl group, an isoquinolyl group, a benzoxazolyl group, a dihydrobenzofuranyl group, a benzothiazolyl group or a quinoxalyl group, each of which may have 1 or 2 substituents selected from substituent group β, and
   substituent group β represents a group consisting of a fluorine atom, a methyl group, a tert-butyl group and a trifluoromethyl group,
(26) the compound or pharmacologically acceptable salt thereof according to (25) above, wherein Q² represents a phenyl group, a pyridyl group, a thienyl group or a thiazolyl group, each of which may have 1 or 2 substituents selected from substituent group β,
(27) the compound or pharmacologically acceptable salt thereof according to (1) above, wherein
   R¹ represents a group -X-Q¹-Y-Q²-Z-Q³,
   X represents a single bond,
   Q¹ represents a piperidyl group or a pyridyl group, each of which may have 1 or 2 substituents selected from substituent group α,
   substituent group α represents a group consisting of a methyl group,
   Y represents a single bond,
   Q² represents a phenyl group, a pyridyl group, a thienyl group, a pyrimidinyl group, a pyridazinyl group, a pyrazinyl group, a thiazolyl group, an oxazolyl group or an indolyl group, each of which may have 1 or 2 substituents selected from substituent group β,
   substituent group β represents a group consisting of a fluorine atom or a methyl group,
   Z represents a single bond, -CR¹¹R¹²-, -(CH₂)₂-, -CH₂O- -OCH₂-, -O-, -CO- or -NCH₃-,
   R¹¹ and R¹² each represents a hydrogen atom, a methyl group or a hydroxyl group,
   Q³ represents a cyclopropyl group, a cyclohexyl group, a cyclohexenyl group, a phenyl group, a dioxanyl group, a piperidyl group, a pyrrolyl group, a pyrazolyl group, a pyridyl group, a thienyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, an imidazopyridyl group, a quinolyl group, an isoquinolyl group, a tetrahydroquinolyl group or a benzodioxanyl group, each of which may have 1 or 2 substituents selected from substituent group γ, and
   substituent group γ represents a group consisting of a fluorine atom, a methyl group and a methoxy group,
(28) the compound or pharmacologically acceptable salt thereof according to (27) above, wherein Q² represents a phenyl group, a pyridyl group, a thienyl group, a pyrimidinyl group, a pyrazinyl group or a thiazolyl group, each of which may have 1 or 2 substituents selected from substituent group β, and Q³ represents a cyclopropyl group, a phenyl group, a pyridyl group, a thienyl group or a thiazolyl group, each of which may have 1 or 2 substituents selected from substituent group γ,
(29) the compound or pharmacologically acceptable salt thereof according to (1) above that is selected from the following:
   {[(2-{[1-(4-tert-butylphenyl)piperidin-4-yl]methyl}-5-hydroxy-6-methylpyrimidin-4-yl)carbonyl]amino}acetic acid,
   [({5-hydroxy-6-methyl-2-[(1-phenylpiperidin-4-yl)methyl]pyrimidin-4-yl}carbonyl)amino]acetic acid,
   {[(2-{[1-(4-fluorophenyl)piperidin-4-yl]methyl}-5-hydroxy-6-methylpyrimidin-4-yl)carbonyl]amino acetic acid,
   ({[5-hydroxy-6-methyl-2-({1-[4-(trifluoromethyl)phenyl]piperidin-4-yl}methyl)pyrimidin-4-yl]carbonyl}amino)acetic acid,
   {[(2-{1-(biphenyl-4-yl)piperidin-4-yl]methyl}-5-hydroxy-6-methylpyrimidin-4-yl)carbonyl]amino}acetic acid,
   {[(2-{[1-(2'-fluorobiphenyl-4-yl)piperidin-4-yl]methyl}-5-hydroxy-6-methylpyrimidin-4-yl)carbonyl]amino}acetic acid,
   {[(2-{[1-(3'-fluorobiphenyl-4-yl)piperidin-4-yl]methyl}-5-hydroxy-6-methylpyrimidin-4-yl)carbonyl]amino}acetic acid,
   {[(2-{[1-(4'-nuorobiphenyl-4-yl)piperidin-4-yl]methyl}-5-hydroxy-6-methylpyrimidin-4-yl)carbonyl]amino}acetic acid,
   {[(2-{[1-(4'-cyanobiphenyl-4-yl)piperidin-4-yl]methyl}-5-hydroxy-6-methylpyrimidin-4-yl)carbonyl]amino}acetic acid,
   {[(5-hydroxy-2-{[1-(4'-methoxybiphenyl-4-yl)piperidin-4-yl]methyl} -6-methylpyrimidin-4-yl)carbonyl]amino}acetic acid,
   {[(2-{[1-(2',4'-difluorobiphenyl-4-yl)piperidin-4-yl]methyl}-5-hydroxy-6-methylpyrimidin-4-yl)carbonyl]amino}acetic acid,
   {[(2-[1-(4'-fluoro-2-methylbiphenyl-4-yl)piperidin-4-yl]methyl}-5-hydroxy-6-methylpyrimidin-4-yl)carbonyl]amino}acetic acid,
   {[(2-{[1-(4-benzylphenyl)piperidin-4-yl]methyl}-5-hydroxy-6-methylpyrimidin-4-yl)carbonyl]amino}acetic acid,
   ({[2-({1-[4-(4-fluorobenzyl)phenyl]piperidin-4-yl}methyl)-5-hydroxy-6-methylpyrimidin-4-yl]carbonyl}amino)acetic acid,
   ({[5-hydroxy-6-methyl-2-({1-[4-(3-methylbenzyl)phenyl]piperidin-4-yl}methyl)pyrimidin-4-yl]carbonyl}amino)acetic acid,
   ({[5-hydroxy-6-methyl-2-({1-[4-(4-methylbenzyl)phenyl]piperidin-4-yl}methyl)pyrimidin-4-yl]carbonyl}amino)acetic acid,
   ({[5-hydroxy-2-({1-[4-(4-methoxybenzyl)phenyl]piperidin-4-yl}methyl)-6-methylpyrimidin-4-yl]carbonyl}amino)acetic acid,
   {[(2-{[1-(4-benzyl-3-fluorophenyl)piperidin-4-yl]methyl}-5-hydroxy-6-methylpyrimidin-4-yl)carbonyl]amino}acetic acid,
   ({[5-hydroxy-6-methyl-2-({1-[5-(4-methylphenyl)pyrimidin-2-yl]piperidin-4-yl}methyl)pyrimidin-4-yl]carbonyl}amino)acetic acid,
   ({[5-hydroxy-6-methyl-2-({1-[5-(4-methylphenyl)pyrazin-2-yl]piperidin-4-yl}methyl)pyrimidin-4-yl]carbonyl}amino)acetic acid,
   {[(5-hydroxy-6-methyl-2-{[1-(5-phenylpyridin-2-yl)piperidin-4-yl]methyl}pyrimidin-4-yl)carbonyl]amino}acetic acid,
   ({[5-hydroxy-6-methyl-2-({1-[6-(4-methylphenyl)pyridin-3-yl]piperidin-4-yl}methyl)pyrimidin-4-yl]carbonyl}amino)acetic acid,
   ({[5-hydroxy-6-methyl-2-({1-[5-(4-methylphenyl)thiophen-2-yl]piperidin-4-yl}methyl)pyrimidin-4-yl]carbonyl}amino)acetic acid,
   ({[5-hydroxy-6-methyl-2-({1-[5-(4-methylphenyl)-1,3-thiazol-2-yl]piperidin-4-yl}methyl)pyrimidin-4-yl]carbonyl}amino)acetic acid,
   ({[5-hydroxy-6-methyl-2-({1-[4-(pyridin-2-yl)phenyl]piperidin-4-yl}methyl)pyrimidin-4-yl]carbonyl}amino)acetic acid,
   ({[5-hydroxy-6-methyl-2-({1-[4-(pyridin-3-yl)phenyl]piperidin-4-yl}methyl)pyrimidin-4-yl]carbonyl}amino)acetic acid,
   ({[5-hydroxy-6-methyl-2-({1-[4-(thiophen-3-yl)phenyl]piperidin-4-yl}methyl)pyrimidin-4-yl]carbonyl}amino)acetic acid,
   ({[5-hydroxy-6-methyl-2-({1-[4-(1,3-thiazol-2-yl)phenyl]piperidin-4-yl}methyl)pyrimidin-4-yl]carbonyl}amino)acetic acid,
   ({[2-({1-[4-(1-benzothiophen-3-yl)phenyl]piperidin-4-yl}methyl)-5-hydroxy-6-methylpyrimidin-4-yl]carbonyl}amino)acetic acid,
   ({[5-hydroxy-2-({1-[4-(isoquinolin-5-yl)phenyl]piperidin-4-yl}methyl)-6-methylpyrimidin-4-yl]carbonyl}amino)acetic acid,
   ({[2-({1-[4-(1,4-benzodioxan-5-yl)phenyl]piperidin-4-yl}methyl)-5-hydroxy-6-methylpyrimidin-4-yl]carbonyl}amino)acetic acid,
   [({5-hydroxy-6-methyl-2-[(1-{4-[(pyridin-3-yl)methyl]phenyl}piperidin-4-yl)methyl]pyrimidin-4-yl}carbonyl)amino]acetic acid,
   [({5-hydroxy-6-methyl-2-[(1-{4-[(6-methylpyridin-3-yl)methyl]phenyl}piperidin-4-yl)methyl]pyrimidin-4-yl}carbonyl)amino]acetic acid,
   [({5-hydroxy-6-methyl-2-[(1-{4-[(4-methylpyridin-3-yl)methyl]phenyl}piperidin-4-yl)methyl]pyrimidin-4-yl}carbonyl)amino]acetic acid,
   [({5-hydroxy-6-methyl-2-[(1-{4-[(6-methoxypyridin-3-yl)methyl]phenyl piperidin-4-yl)methyl]pyrimidin-4-yl}carbonyl)amino]acetic acid,
   [({5-hydroxy-6-methyl-2-[(1-{[3-methyl-4-(pyridin-3-yl)methyl]phenyl}piperidin-4-yl)methyl]pyrimidin-4-yl}carbonyl)amino] acetic acid,
   [({2-[(1-{[3-fluoro-4-(pyridin-3 -yl)methyl]phenyl}piperidin-4-yl)methyl]-5-hydroxy-6-methylpyrimidin-4-yl}carbonyl)amino]acetic acid,
   [({5-hydroxy-6-methyl-2-[(1-{4-[(quinolin-3-yl)methyl]phenyl}piperidin-4-yl)methyl]pyrimidin-4-yl}carbonyl)amino]acetic acid,
   ({[2-(1-[4-(cyclopropylmethyl)phenyl]piperidin-4-yl}methyl)-5-hydroxy-6-methylpyrimidin-4-yl]carbonyl}amino)acetic acid,
   ({[5-hydroxy-6-methyl-2-({1-[4-(1-methyl-1-phenylethyl)phenyl]piperidin-4-yl}methyl)-pyrimidin-4-yl]carbonyl}amino)acetic acid,
   {[(2-{[1-(biphenyl-4-yl)-3,3-dimethylpiperidin-4-yl]methyl}-5-hydroxy-6-methylpyrimidin-4-yl)carbonyl]amino}acetic acid,
   {[(2-{[trans-1-(4-tert-butytphenyl)-2-methylpiperidin-4-yl]methyl}-5-hydroxy-6-methylpyrimidin-4-yl)carbonyl]amino}acetic acid,
   {[(2-{[1-(4-tert-butylphenyl)-1,2,3,6-tetrahydropyridin-4-yl]methyl}-5-hydroxy-6-methylpyrimidin-4-yl)carbonyl]amino}acetic acid,
   {[(2-{[6-(4-tert-butylphenyl)pyridin-3-yl]methyl}-5-hydroxy-6-methylpyrimidin-4-yl)carbonyl]amino}acetic acid,
   {[(2-[6-(biphenyl-4-yl)pyridin-3-yl]methyl}-5-hydroxy-6-methylpyrimidin-4-yl)carbonyl]amino}acetic acid,
   {[(2-{[2-(4-tert-butylphenyl)pyrimidin-5-yl]methyl}-5-hydroxy-6-methylpyrimidin-4-yl)carbonyl]amino}acetic acid,
   {[(2-[5-(4-tert-butylphenyl)pyridin-2-yl]methyl}-5-hydroxy-6-methylpyrimidin-4-yl)carbonyl]amino}acetic acid,
   ({[2-{[1-(4-tert-butylphenyl)piperidin-4-yl]methyl}-5-hydroxy-6-(methylsulfanyl)pyrimidin-4-yl]carbonyl}amino)acetic acid,
   ({[2-{[6-(4-tert-butylphenyl)pyridin-3-yl]methyl}-5-hydroxy-6-(methylsulfanyl)pyrimidin-4-yl]carbonyl}amino)acetic acid, and
   {[(2-{[trans-5-(biphenyl-4-yl)-1,3-dioxan-2-yl]methyl} -5-hydroxy-6-methylpyrimidin-4-yl)carbonyl]amino}acetic acid,
(30) a medicament comprising as an active ingredient a compound or a pharmacologically acceptable salt thereof according to any one of (1) to (29) above,
(31) the medicament according to (30) above, for prophylaxis and/or treatment of anemia,
(32) the medicament according to (31) above, wherein the anemia is nephrogenic anemia, anemia of prematurity, anemia incidental to chronic diseases, anemia incidental to cancer chemotherapy or cancerous anemia,
(33) the medicament according to (30) above, for producing erythropoietin,
(34) a use of a compound or a pharmacologically acceptable salt thereof according to any one of (1) to (29) above for manufacturing a medicament,
(35) the use according to (34) above, wherein the medicament is a medicament for prophylaxis and/or treatment of anemia,
(36) the use according to (35) above, wherein the anemia is nephrogenic anemia, anemia of prematurity, anemia incidental to chronic diseases, anemia incidental to cancer chemotherapy or cancerous anemia,
(37) a method for producing erythropoietin, comprising administering an effective amount of a compound or a pharmacologically acceptable salt thereof according to any one of (1) to (29) above to a mammal or bird,
(38) the method according to (37) above for prophylaxis and/or treatment of anemia,
(39) the method according to (38) above, wherein the anemia is nephrogenic anemia, anemia of prematurity, anemia incidental to chronic diseases, anemia incidental to cancer chemotherapy or cancerous anemia, and,
(40) the method according to (37) above, wherein the mammal is a human, horse, cow or pig, and the bird is a chicken.

The compounds of the present invention represented by the aforementioned formula (1) are characterized in terms of their chemical structure in having a 5-hydroxypyrimidine-4-carboxamide backbone and being substituted with a heterocycle which may be substituted, through a methylene chain and the like at the 2-position of the pyrimidine ring, and are characterized pharmacologically in having an activity of promoting EPO production.

The following provides an explanation of substituents in the compound (1) of the present invention.

The "halogen atom" in the definitions of substituent group α, substituent group β, substituent group γ, R¹¹ and R¹², refers to a fluorine atom, a chlorine atom, a bromine atom or an iodine atom.

The "C₁-C₃ alkyl group" in the definition of R² refers to a straight or branched chain alkyl group having 1 to 3 carbon atoms. Examples include a methyl group, an ethyl group, an n-propyl group and an isopropyl group.

The "C₁-C₆ alkyl group" in the definitions of substituent group α, substituent group β, substituent group γ, R¹¹ and R¹², refers to a straight or branched chain alkyl group having 1 to 6 carbon atoms. Examples include the aforementioned "C₁-C₃ alkyl group", an n-butyl group, an isobutyl group, a sec-butyl group, a tert-butyl group, a pentyl group, an isopentyl group, a 2-methylbutyl group, a neopentyl group, a 1-ethylpropyl group, a hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group and a 2-ethylbutyl group.

The "halogenated C₁-C₆ alkyl group" in the definitions of substituent group α, substituent group β, substituent group γ, R¹¹ and R¹², refers to a group in which 1 or more hydrogen atoms of the aforementioned "C₁-C₆ alkyl group" are substituted with the aforementioned "halogen atom". Examples include a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 2,2,2-trifluoroethyl group, a 2,2,2-trichloroethyl group, a 2-bromoethyl group, a 2-chloroethyl group, a 2-fluoroethyl group, a 2-iodoethyl group, a 3-chloropropyl group, a 4-fluorobutyl group, a 6-iodohexyl group and a 2,2-dibromoethyl group.

The "C₁-C₆ alkoxy group" in the definitions of substituent group β, substituent group γ, R¹¹ and R¹², refers to a group in which the aforementioned "C₁-C₆ alkyl group" is bonded to an oxygen atom. Examples include a methoxy group, an ethoxy group, an n-propoxy group, an isopropoxy group, an n-butoxy group, an isobutoxy group, a sec-butoxy group, a tert-butoxy group, an n-pentyloxy group, an isopentyloxy group, a 2-methylbutoxy group, a neopentyloxy group, an n-hexyloxy group, a 4-methylpentyloxy group, a 3-methylpentyloxy group, a 2-methylpentyloxy group, a 3,3-dimethylbutoxy group, a 2,2-dimethylbutoxy group, a 1,1-dimethylbutoxy group, a 1,2-dimethylbutoxy group, a 1,3-dimethylbutoxy group and a 2,3-dimethylbutoxy group.

The "halogenated C₁-C₆ alkoxy group" in the definitions of substituent group β, substituent group γ, R¹¹ and R¹², refers to a group in which the "C₁-C₆ alkoxy group" is substituted with 1 or more halogen atoms, examples of which include a fluoromethoxy group, a chloromethoxy group, a difluoromethoxy group, a dichloromethoxy group, a dibromomethoxy group, a trifluoromethoxy group, a trichloromethoxy group, a 2,2,2-trichloroethoxy group, a 2,2,2-trifluoroethoxy group, a 2-bromoethoxy group, a 2-chloroethoxy group, a 2-fluoroethoxy group and a 2,2-dibromoethoxy group.

The "C₁-C₆ alkylsulfanyl group" in the definitions of substituent group β and substituent group γ refers to a group in which the aforementioned "C₁-C₆ alkyl group" is bonded to a sulfur atom. Examples include a methylsulfanyl group, an ethylsulfanyl group, a propylsulfanyl group, an isopropylsulfanyl group, a butylsulfanyl group, an isobutylsulfanyl group, a sec-butylsulfanyl group, a tert-butylsulfanyl group, a pentylsulfanyl group, an isopentylsulfanyl group, a 2-methylbutylsulfanyl group, a neopentylsulfanyl group, a 1-ethylpropylsulfanyl group, a hexylsulfanyl group, a 4-methylpentylsulfanyl group, a 3-methylpentylsulfanyl group, a 2-methylpentylsulfanyl group, a 1-methylpentylsulfanyl group, a 3,3-dimethylbutylsufanyl group, a 2,2-dimethylbutylsulfanyl group, a 1,1-dimethylbutylsulfanyl group, a 1,2-dimethylbutylsulfanyl group, a 1,3-dimethylbutylsulfanyl group, a 2,3-dimethylbutylsulfanyl group and a 2-ethylbutylsulfanyl group.

The "C₁-C₆ alkylamino group" in the definitions of substituent group β and substituent group γ refers to a group in which one of the aforementioned "C₁-C₆ alkyl group" is bonded to an amino group. Examples include a methylamino group, an ethylamino group, a propylamino group, an isopropylamino group, a butylamino group, an isobutylamino group, a sec-butylamino group, a tert-butylamino group, a pentylamino group, an isopentylamino group, a 2-methylbutylamino group, a neopentylamino group, a 1-ethylpropylamino group, a hexylamino group, a 4-methylpentylamino group, a 3-methylpentylamino group, a 2-methylpentylamino group, a 1-methylpentylamino group, a 3,3-dimethylbutylamino group, a 2,2-dimethylbutylamino group, a 1,1-dimethylbutylamino group, a 1,2-dimethylbutylamino group, a 1,3-dimethylbutylamino group, a 2,3-dimethylbutylamino group and a 2-ethylbutylamino group.

The "(C₁-C₆ alkyl)(C₁-C₆ alkyl)amino group" in the definitions of substituent group β and substituent group γ refers to a group in which an amino group is substituted with two of the same or different aforementioned C₁-C₆ alkyl groups. Examples include a dimethylamino group, a methylethylamino group, a diethylamino group, a din-propylamino group, a diisopropylamino group, an N-(n-propyl)-N-ethylamino group, a di-n-butylamino group, a diisobutylamino group, a di-s-butylamino group, a di-tert-butylamino group, a di-n-pentylamino group, a diisopentylamino group, a di-2-methylbutylamino group, a dineopentylamino group, a di-1-ethylpropylamino group, a di-n-hexylamino group, a di-4-methylpentylamino group, a di-3-methylpentylamino group, a di-2-methylpentylamino group, a di-1-methylpentylamino group, a di-3,3-dimethylbutylamino group, a di-2,2-dimethylbutylamino group, a di-1,1-dimethylbutylamino group, a di-1,2-dimethylbutylamino group, a di-1,3-dimethylbutylamino group, a di-2,3-dimethylbutylamino group and a di-2-ethylbutylamino group.

The "C₂-C₇ alkanoyl group" in the definitions of substituent group β and substituent group γ refers to an alkanoyl group having 2 to 7 carbon atoms. Examples include an acetyl group, a propionyl group, a butyryl group, an isobutyryl group, a pentanoyl group, a pivaloyl group, a valeryl group, an isovaleryl group, a hexanoyl group and a heptanoyl group.

The "C₃-C₇ cycloalkyl group" in the definition of Q³ refers to a cycloalkyl group having 3 to 7 carbon atoms. Examples include a cyclopropyl group, a cyclobutyl group, a cyclopentyl group, a cyclohexyl group and a cycloheptyl group.

The "C₃-C₇ cycloalkenyl group" in the definition of Q³ refers to a cycloalkenyl group having 3 to 7 carbon atoms. Examples include a cyclopropenyl group, a cyclobutenyl group, a cyclopentenyl group, a cyclohexenyl group and a cycloheptenyl group.

The "monocyclic or bicyclic hydrocarbon ring group" in the definition of Q² refers to a saturated or unsaturated, monocyclic or bicyclic hydrocarbon group having 3 to 10 carbon atoms. Examples include an aromatic hydrocarbon ring group such as a phenyl group; the aforementioned C₃-C₇ cycloalkyl group; the aforementioned C₃-C₇ cycloalkenyl group, and a bicyclic hydrocarbon group such as a naphthalenyl group, a tetrahydronaphthalenyl group, a decahydronaphthalenyl group, an indanyl group, an indenyl group or a norboranyl group.

The "monocyclic or bicyclic heterocyclic group" in the definitions of Q¹, Q² and Q³ refers to a saturated or unsaturated, 4- to 10-membered monocyclic or bicyclic heterocyclic group containing 1 or 2 atoms selected from the group consisting of a nitrogen atom, a sulfur atom and an oxygen atom. Examples include a monocyclic non-aromatic heterocyclic group such as a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a dihydropyrrolyl group, a dihydropyridyl group, a tetrahydropyridyl group, a piperazinyl group, a morpholinyl group, a dihydrooxazolyl group or a dihydrothiazolyl group; a monocyclic aromatic heterocyclic group such as a pyrrolyl group, a pyridyl group, a thienyl group, a furyl group, a pyrimidinyl group, a pyridazinyl group, a pyrazinyl group, a pyrazolyl group, an imidazolyl group, a thiazolyl group, an isothiazolyl group, an oxazolyl group or an isoxazolyl group; and a bicyclic heterocyclic group such as a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a quinoxalyl group, a benzothiazolyl group, a tetrahydroquinolyl group, an isoquinolyl group, a tetrahydroisoquinolyl group, a benzoxazolyl group, a benzodioxanyl group, an indolidinyl group, a thienopyridyl group, a dihydrothienopyridyl group, a furopyridyl group or a, dihydrofuropyridyl group. In addition, a heterocyclic group having bridging bonds such as a tropinyl group indicated below are included in the aforementioned "bicyclic heterocyclic group".

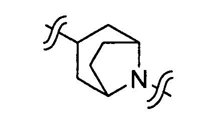

In compound (1) of the present invention, R¹ represents a group -X-Q¹, X-Q¹-Y-Q² or -X-Q¹-Y-Q²-Z-Q³.

Q¹ and Q² can be a divalent substituent in the case that R¹ represents a group X-Q¹-Y-Q² or -X-Q¹-Y Q²-Z-Q³ and these are indicated using the same notation as a monovalent substituent in the present specification.

For example, in the case that R¹ represents a group X-Q¹-Y-Q² and Q¹ is a group having a heterocycloalkyl structure, Q¹ is a "heterocycloalkylene (or heterocycloalkanediyl)", that is a divalent substituent having bonds with X and Y, and it is indicated as a "heterocycloalkyl group". This applies similarly in the case that Q¹ represents other cyclic structures as well. In addition, Q² is a "heterocycloalkylene (or heterocycloalkanediyl)", that is a divalent substituent having bonds with Y and Z, in the case that R¹ represents a group -X-Q¹-Y-Q²-Z-Q³ and Q² is a group having a heterocycloalkyl structure, and it is indicated as a "heterocycloalkyl group". This applies similarly in the case that Q² represents other cyclic structures as well. Furthermore, Q¹, Q² and Q³ may have further substituents.

Next, an explanation is provided of substitution positions of the group -Y-Q² or the group -Y-Q²-Z-Q³ in Q¹ in the case that R¹ represents a group X-Q¹-Y-Q² or -X-Q¹-Y-Q²-Z-Q³.

In the case that Q¹ is a 5-membered ring and the location of the atom that bonds with X is the 1-position, then the substitution position of the group -Y-Q² or the group -Y-Q²-Z-Q³ is preferably the 3- or 4-position.

In the case that Q¹ is a 6-membered ring and the location of the atom that bonds with X is the 1-position, then the substitution position of the group Y-Q² or the group Y-Q²-Z-Q³ is preferably the 4-position.

In the case that Q¹ is a 7-membered ring and the location of the atom that bonds with X is the 1-position, then the substitution position of the group -Y-Q² or the group -Y-Q²-Z-Q³ is preferably the 4- or 5-position.

For example, in the case that Q¹ is a piperidyl group, the substitution position of the group -Y-Q² or the group Y-Q²-Z-Q³ is preferably a substitution position as indicated below.

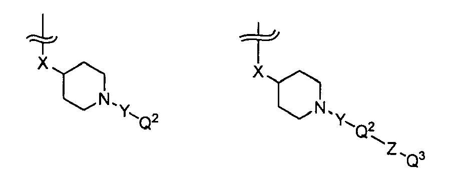

In addition, in the case that Q¹ is a pyridyl group, for example, the substitution position of the group -Y-Q² or the group Y-Q²-Z-Q³ is preferably a substitution position as indicated below.

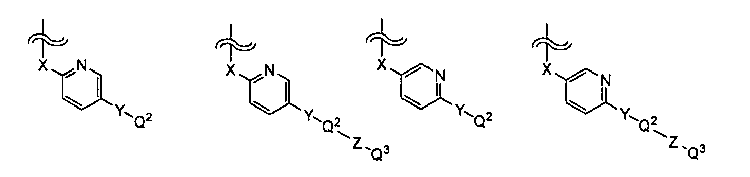

In the present invention, X is preferably a single bond, -CH₂-, -(CH₂)₂- or - (CH₂)₃-, more preferably a single bond or -CH₂-, and even more preferably a single bond. Furthermore, the bond on the right side of each group represents a bond with which the following Q¹ is bonded.

In the present invention, Q¹ is preferably a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a piperazinyl group, a dihydrooxazolyl group, a dihydrothiazolyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a tropinyl group, a chromanyl group, an isochromanyl group, a quinolyl group, a tetrahydroquinolyl group, an isoquinolyl group or a tetrahydroisoquinolyl group, each of which may have 1 or 2 substituents selected from substituent group α, more preferably a dioxanyl group, a dioxepanyl group, a pyrrolidinyl group, a piperidyl group, an azepanyl group, a pyrrolyl group, a pyridyl group, a dihydropyridyl group, a tetrahydropyridyl group, a pyrimidinyl group, a thiazolyl group, a tropinyl group or a tetrahydroisoquinolyl group, each of which may have 1 or 2 substituents selected from substituent group α, and even more preferably a piperidyl group or a pyridyl group, each of which may have 1. or 2 substituents selected from substituent group α.

In the present invention, substituent group α is preferably a group consisting of a methyl group and an oxo group, and more preferably a group consisting of a methyl group.

In the present invention, Y is preferably a single bond, -CH₂-, -(CH₂)₂- or - (CH₂)₃-, and more preferably a single bond. Furthermore, the bond on the left side of each group represents a bond with which the aforementioned Q¹ is bonded.

In the present invention, Q² is preferably a cyclopentyl group, cyclohexyl group, cycloheptyl group, cyclopentenyl group, cyclohexenyl group, cycloheptenyl group, phenyl group, naphthalenyl group, tetrahydronaphthalenyl group, decahydronaphthalenyl group, indanyl group, indenyl group, norboranyl group, tetrahydrofuranyl group, tetrahydropyranyl group, dioxolanyl group, dioxanyl group, dioxepanyl group, azetidinyl group, pyrrolidinyl group, piperidyl group, azepanyl group, piperazinyl group, dihydrooxazolyl group, dihydrothiazolyl group, morpholinyl group, pyrrolyl group, dihydropyrrolyl group, pyridyl group, dihydropyridyl group, tetrahydropyridyl group, thienyl group, furyl group, pyrimidinyl group, pyridazinyl group, pyrazinyl group, pyrazolyl group, thiazolyl group, isothiazolyl group, oxazolyl group, isoxazolyl group, chromanyl group, isochromanyl group, benzofuranyl group, dihydrobenzofuranyl group, benzothiophenyl group, dihydrobenzothiophenyl group, indolyl group, isoindolyl group, quinolyl group, tetrahydroquinolyl group, isoquinolyl group, tetrahydroisoquinolyl group, benzoxazolyl group, quinoxalyl group, benzothiazolyl group, benzodioxanyl group, indolidinyl group, thienopyridiyl group, dihydrothienopyridyl group, furopyridyl group or dihydrofuropyridyl group, each of which may have 1 or 2 substituents selected from substituent group β, more preferably a phenyl group, naphthalenyl group, pyridyl group, thienyl group, pyrimidinyl group, pyridazinyl group, pyrazinyl group, thiazolyl group, oxazolyl group, dihydrobenzofuranyl group, benzothiophenyl group, indolyl group, quinolyl group, isoquinolyl group, benzoxazolyl group, quinoxalyl group or benzothiazolyl group, each of which may have 1 or 2 substituents selected from substituent group β, and even more preferably a phenyl group, pyridyl group, thienyl group, pyrimidinyl group, pyrazinyl group or thiazolyl group, each of which may have 1 or 2 substituents selected from substituent group β.

In the present invention, substituent group β is preferably a group consisting of a halogen atom, a hydroxyl group, a cyano group, a nitro group, a formyl group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a (C₁-C₆ alkyl)(C₁-C₆ alkyl)amino group and a C₂-C₇ alkanoyl group, more preferably a group consisting of a fluorine atom, a chlorine atom, a hydroxyl group, a cyano group, a nitro group, a methyl group, an n-propyl group, an isopropyl group, an n-butyl group, a tert-butyl group, a trifluoromethyl group, a methylsulfanyl group and a trifluoromethoxy group, and even more preferably a group consisting of a fluorine atom, a methyl group, a tert-butyl group and a trifluoromethyl group.

In the present invention, Z is preferably a single bond, -CR¹¹R¹²-, -(CH₂)₂₋, - CH₂O- -OCH₂-, -O-, -CO- or -NCH₃-, and R¹¹ and R¹² preferably each independently represents a hydrogen atom, a methyl group or a hydroxyl group. More preferably, Z is a single bond or -CR¹¹R¹²-, and R¹¹ and R¹² each independently represents a hydrogen atom, a methyl group or a hydroxyl group. Furthermore, the bond on the left side of each group represents a bond with which the aforementioned Q² is bonded.

In the present invention, Q³ is preferably a cyclopropyl group, a cyclobutyl group, a cyclopentyl group, a cyclohexyl group, a cycloheptyl group, a cyclopentenyl group, a cyclohexenyl group, a cycloheptenyl group, a phenyl group, a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a dihydrooxazolyl group, a dihydrothiazolyl group, a morpholinyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, a isoxazolyl group, a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a tetrahydroquinolyl group, an isoquinolyl group, a tetrahydroisoquinolyl group, an imidazopyridyl group, a benzoxazolyl group, a quinoxalyl group, a benzothiazolyl group, a benzodioxanyl group, an indolidinyl group, a thienopyridiyl group, a dihydrothienopyridyl group, a furopyridyl group or a dihydrofuropyridyl group, each of which may have 1 or 2 substituents selected from substituent group γ, more preferably a cyclopropyl group, a cyclohexyl group, a cyclohexenyl group, a phenyl group, a dioxanyl group, a piperidyl group, a pyrrolyl group, a pyridyl group, a thienyl group, a pyrazolyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, a quinolyl group, a tetrahydroquinolyl group, a isoquinolyl group, an imidazopyridyl group or a benzodioxanyl group, each of which may have 1 or 2 substituents selected from substituent group γ, and even more preferably a cyclopropyl group, a phenyl group, a pyridyl group, a thienyl group or a thiazolyl group, each of which may have 1 or 2 substituents selected from substituent group γ.

In the present invention, substituent group γ is preferably a group consisting of a halogen atom, a hydroxyl group, a nitro group, an amino group, a cyano group, a C₁-C₆ alkyl group, a halogenated C₁-C₆ alkyl group and a C₁-C₆ alkoxy group, more preferably a group consisting of a fluorine atom, a chlorine atom, a hydroxyl group, an amino group, a cyano group, a methyl group, a methoxy group, a tert-butyl group and a trifluoromethyl group, and even more preferably a group consisting of a fluorine atom, a methyl group and a methoxy group.

In the present invention, R² is preferably a methyl group, a methylsulfanyl group or a difluoromethyl group, and more preferably a methyl group or a methylsulfanyl group.

In the present invention, R³ is preferably a hydrogen atom.

In the present invention, in the case that R¹ represents a group X-Q¹-Y-Q², preferable combinations of X, Q¹, Y, Q², substituent group α and substituent group β are those in which X is a single bond, -CH₂-, -(CH₂)₂- or -(CH₂)₃-, Q¹ is a piperidyl group or a pyridyl group, each of which may have 1 or 2 substituents selected from substituent group α, substituent group α is a group consisting of a methyl group, Y is a single bond, Q² is a phenyl group, a naphthalenyl group, a pyridyl group, a thienyl group, a thiazolyl group, a benzothiophenyl group, a quinolyl group, an isoquinolyl group, a benzoxazolyl group, a dihydrobenzofuranyl group, a benzothiazolyl group or a quinoxalyl group, each of which may have 1 or 2 substituents selected from substituent group β, and substituent group β is a group consisting of a fluorine atom, a methyl group, a tert-butyl group and a trifluoromethyl group. In these combinations, X is more preferably a single bond, and Q² is more preferably a phenyl group, a pyridyl group, a thienyl group or a thiazolyl group, each of which may have 1 or 2 substituents selected from substituent group β.

In addition, in the present invention, in the case that R¹ represents a group -X-Q¹-Y-Q²-Z-Q³, preferable combinations of X, Q¹, Y, Q², Z, Q³, substituent group α, substituent group β and substituent group γ are those in which X is a single bond, Q¹ is a piperidyl group or a pyridyl group, each of which may have 1 or 2 substituents selected from substituent group α, substituent group α is a group consisting of a methyl group, Y is a single bond, Q² is a phenyl group, a pyridyl group, a thienyl group, a pyrimidinyl group, a pyridazinyl group, a pyrazinyl group, a thiazolyl group, an oxazolyl group or an indolyl group, each of which may have 1 or 2 substituents selected from substituent group β, substituent group β is a group consisting of a fluorine atom and a methyl group, Z is a single bond, -CR¹¹R¹²-, -(CH₂)₂-, -CH₂O-, -OCH₂-, -O-, -CO- or -NCH₃-, R¹¹ and R¹² each independently represents a hydrogen atom, a methyl group or a hydroxyl group, Q³ is a cyclopropyl group, a cyclohexyl group, a cyclohexenyl group, a phenyl group, a dioxanyl group, a piperidyl group, a pyrrolyl group, a pyrazolyl group, a pyridyl group, a thienyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, an imidazopyridyl group, a quinolyl group, an isoquinolyl group, a tetrahydroquinolyl group or a benzodioxanyl group, each of which may have 1 or 2 substituents selected from substituent group γ, and substituent group γ is a group consisting of a fluorine atom, a methyl group and a methoxy group. In these combinations, Q² is more preferably a phenyl group, a pyridyl group, a thienyl group, a pyrimidinyl group, a pyrazinyl group or a thiazolyl group, each of which may have 1 or 2 substituents selected from substituent group β, Z is more preferably a single bond or -CR¹¹R¹²-, R¹¹ and R¹² more preferably each independently represents a hydrogen atom, a methyl group or a hydroxyl group, and Q³ is more preferably a cyclopropyl group, a phenyl group, a pyridyl group, a thienyl group or a thiazolyl group, each of which may have 1 or 2 substituents selected from substituent group γ.

Compound (1) of the present invention may have geometrical isomers or tautomers depending on the types of substituents. In addition, compound (1) of the present invention may have an asymmetric carbon atom. These separated isomers and mixtures thereof are included in the present invention. In addition, labeled forms, namely compounds in which one or more atoms of compounds of the present invention have been substituted with a radioactive isotope or non-radioactive isotope, are also included in the present invention.

The compounds of the present invention can be in the form of pharmacologically acceptable salts as desired in the case of having a basic group such as an amino group. Examples of such salts include salts of hydrohalogenic acids such as hydrofluorides, hydrochlorides, hydrobromides or hydroiodides; salts of inorganic acids such as nitrates, perchlorates, sulfates or phosphates; salts of lower alkanesulfonic acids such as methanesulfonates, trifluoromethanesulfonates or ethanesulfonates; salts of arylsulfonic acids such as benzenesulfonates or p-toluenesulfonates; salts of organic acids such as acetates, malates, fumarates, succinates, citrates, tartrates, oxalates or maleates; and salts of amino acids such as ornithinates, glutamates or aspartates, and salts of hydrohalogenic acids and salts of organic acids are preferable.

In addition, the compounds of the present invention can generally form a base addition salt in the case of having an acidic group such as a carboxy group. Examples of pharmacologically acceptable salts include inorganic salts including salts of alkali metals such as sodium salts, potassium salts or lithium salts; salts of alkaline earth metals such as calcium salts or magnesium salts; and ammonium salts; and organic amine salts such as dibenzylamine salts, morpholine salts, phenylglycine alkyl ester salts, ethylenediamine salts, N-methylglucamine salts, diethylamine salts, triethylamine salts, cyclohexylamine salts, dicyclohexylamine salts, N,N'-dibenzylethylenediamine salts, diethanolamine salts, N-benzyl-N-(2-phenylethoxy)amine salts, piperazine salts, tetramethylammonium salts or tris(hydroxymethyl)aminomethane salts.

The compounds of the present invention can be present as a free form or solvate. There are no particular limitations on the solvate provided it is pharmacologically acceptable and preferable specific examples include hydrates and ethanolates and the like. In addition, in the case a nitrogen atom is present in a compound represented by general formula (1), it may be in the form of a N-oxide, and these solvates and N-oxide forms are also included within the scope of the present invention.

The compounds of the present invention can be present in the form of various isomers including geometrical isomers such as a cis form or trans form, tautomers, or optical isomers such as a d form or 1 form according to the types of substituents and combinations thereof, unless specifically limited otherwise, compounds of the present invention include all isomers, stereoisomers and mixtures of isomers and stereoisomers in any ratios thereof.

Representative examples of compounds represented by general formula (1) of the present invention include the compounds indicated below, but the present invention is not limited to these compounds.

Examples include:
{[(2-{[1-(4-tert-butylphenyl)piperidin-4-yl]methyl}-5-hydroxy-6-methylpyrimidin-4-yl)carbonyl]amino}acetic acid,
[({5-hydroxy-6-methyl-2-[(1-phenylpiperidin-4-yl)methyl]pyrimidin-4-yl}carbonyl)amino]acetic acid,
{[(2-{[1-(4-fluorophenyl)piperidin-4-yl]methyl}-5-hydroxy-6-methylpyrimidin-4-yl)carbonyl]amino}acetic acid,
({[5-hydroxy-6-methyl-2-({1-[4-(trifluoromethyl)phenyl]piperidin-4-yl}methyl)pyrimidin-4-yl]carbonyl}amino)acetic acid,
{[(2-{[1-(biphenyl-4-yl)piperidin-4-yl]methyl}-5-hydroxy-6-methylpyrimidin-4-yl)carbonyl]amino}acetic acid,
{[(2-{[1-(2'-fluorobiphenyl-4-yl)piperidin-4-yl]methyl}-5-hydroxy-6-methylpyrimidin-4-yl)carbonyl]amino}acetic acid,
{[(2-{[1-(3'-fluorobiphenyl-4-yl)piperidin-4-yl]methyl}-5-hydroxy-6-methylpyrimidin-4-yl)carbonyl]amino}acetic acid,
{[(2-{[1-(4'-fluorobiphenyl-4-yl)piperidin-4-yl]methyl}-5-hydroxy-6-methylpyrimidin-4-yl)carbonyl]amino}acetic acid,
{[(2-{[1-(4'-cyanobiphenyl-4-yl)piperidin-4-yl]methyl}-5-hydroxy-6-methylpyrimidin-4-yl)carbonyl]amino}acetic acid,
{[(5-hydroxy-2-{[1-(4'-methoxybiphenyl-4-yl)piperidin-4-yl]methyl}-6-methylpyrimidin-4-yl)carbonyl]amino}acetic acid,
{[(2-{[1-(2',4'-difluorobiphenyl-4-yl)piperidin-4-yl]methyl}-5-hydroxy-6-methylpyrimidin-4-yl)carbonyl]amino}acetic acid,
{[(2-{[1-(4'-fluoro-2-methylbiphenyl-4-yl)piperidin-4-yl]methyl}-5-hydroxy-6-methylpyrimidin-4-yl)carbonyl]amino}acetic acid,
{[(2-{[1-(4-benzylphenyl)piperidin-4-yl]methyl}-5-hydroxy-6-methylpyrimidin-4-yl)carbonyl]amino}acetic acid,
({[2-({1[4-(4-fluorobenzyl)phenyl]piperidin-4-yl}methyl)-5-hydroxy-6-methylpyrimidin-4-yl]carbonyl}amino)acetic acid,
({[5-hydroxy-6-methyl-2-({1-[4-(3-methylbenzyl)phenyl]piperidin-4-yl}methyl)pyrimidin-4-yl]carbonyl}amino)acetic acid,
({[5-hydroxy-6-methyl-2-({1-[4-(4-methylbenzyl)phenyl]piperidin-4-yl}methyl)pyrimidin-4-yl]carbonyl}amino)acetic acid,
({[5-hydroxy-2-({1-[4-(4-methoxybenzyl)phenyl]piperidin-4-yl}Methyl)-6-methylpyrimidin-4-yl]carbonyl}amino)acetic acid,
{[(2-{[1-(4-benzyl-3-fluorophenyl)piperidin-4-yl]methyl}-5-hydroxy-6-methylpyrimidin-4-yl)carbonyl]amino}acetic acid,
({[5-hydroxy-6-methyl-2-({1-[5-(4-methylphenyl)pyrimidin-2-yl]piperidin-4-yl}methyl)pyrimidin-4-yl]carbonyl}amino)acetic acid,
({[5-hydroxy-6-methyl-2-({1-[5-(4-methylphenyl)pyrazin-2-yl]piperidin-4-yl}methyl)pyrimidin-4-yl]carbonyl}amino)acetic acid,
{[(5-hydroxy-6-methyl-2-{[1-(5-phenylpyridin-2-yl)piperidin-4-yl]methyl}pyrimidin-4-yl)carbonyl]amino}acetic acid,
({[5-hydroxy-6-methyl-2-({1-[6-(4-methylphenyl)pyridin-3-yl]piperidin-4-yl}methyl)pyrimidin-4-yl]carbonyl}amino)acetic acid,
({[5-hydroxy-6-methyl-2-(1-[5-(4-methylphenyl)thiophen-2-yl]piperidin-4-yl}methyl)pyrimidin-4-yl]carbonyl}amino)acetic acid,
({[5-hydroxy-6-methyl-2-({1-[5-(4-methylphenyl)-1,3-thiazol-2-yl]piperidin-4-yl}methyl)pyrimidin-4-yl]carbonyl}amino)acetic acid,
({[5-hydroxy-6-methyl-2-({1-[4-(pyridin-2-yl)phenyl]piperidin-4-yl}methyl)pyrimidin-4-yl]carbonyl}amino)acetic acid,
({[5-hydroxy-6-methyl-2-({1-[4-(pyridin-3-yl)phenyl]piperidin-4-yl}methyl)pyrimidin-4-yl]carbonyl}amino)acetic acid,
({[5-hydroxy-6-methyl-2-({1-[4-(thiophen-3-yl)phenyl]piperidin-4-yl}methyl)pyrimidin-4-yl]carbonyl}amino)acetic acid,
({[5-hydroxy-6-methyl-2-({1-[4-(1,3-thiazol-2-yl)phenyl]piperidin-4-yl}methyl)pyrimidin-4-yl]carbonyl}amino)acetic acid,
({[2-({1-[4-(1-benzothiophen-3-yl)phenyl]piperidin-4-yl}methyl)-5-hydroxy-6-methylpyrimidin-4-yl]carbonyl}amino)acetic acid,
({[5-hydroxy-2-({1-[4-(isoquinolin-5-yl)phenyl]piperidin-4-yl}methyl)-6-methylpyrimidin-4-yl]carbonyl}amino)acetic acid,
({[2-({1-[4-(1,4-benzodioxan-5-yl)phenyl]piperidin-4-yl}methyl)-5-hydroxy-6-methylpyrimidin-4-yl]carbonyl}amino)acetic acid,
[({5-hydroxy-6-methyl-2-[(1-{4-[(pyridin-3-yl)methyl]phenyl}piperidin-4-yl)methyl]pyrimidin-4-yl}carbonyl)amino] acetic acid,
[({5-hydroxy-6-methyl-2-[(1-{4-[(6-methylpyridin-3-yl)methyl]phenyl}piperidin-4-yl)methyl]pyrimidin-4-yl}carbonyl)amino]acetic acid,
[({5-hydroxy-6-methyl-2-[(1-{4-[(4-methylpyridin-3-yl)methyl]phenyl}piperidin-4-yl)methyl]pyrimidin-4-yl}carbonyl)amino]acetic acid,
[({5-hydroxy-6-methyl-2-[(1-{4-[(6-methoxypyridin-3-yl)methyl]phenyl}piperidin-4-yl)methyl]pyrimidin-4-yl}carbonyl)amino]acetic acid,
[({5-hydroxy-6-methyl-2-[(1-{[3-methyl-4-(pyridin-3-yl)methyl]phenyl}piperidin-4-yl)methyl]pyrimidin-4-yl}carbonyl)amino] acetic acid,
[({2-[(1-{[3-fluoro-4-(pyridin-3-yl)methyl]phenyl}piperidin-4-yl)methyl]-5-hydroxy-6-methylpyrimidin-4-yl}carbonyl)amino]acetic acid,
[({5-hydroxy-6-methyl-2-[(1-{4-[(quinolin-3-yl)methyl]phenyl}piperidin-4-yl)methyl]pyrimidin-4-yl}carbonyl)amino]acetic acid,
({[2-({1-[4-(cyclopropylmethyl)phenyl]piperidin-4-yl}methyl)-5-hydroxy-6-methylpyrimidin-4-yl]carbonyl}amino)acetic acid,
({[5-hydroxy-6-methyl-2-({1-[4-(1-methyl-1-phenylethyl)phenyl]piperidin-4-yl}methyl)-pyrimidin-4-yl]carbonyl}amino)acetic acid,
{[(2-{[1-(biphenyl-4-yl)-3,3-dimethylpiperidin-4-yl]methyl}-5-hydroxy-6-methylpyrimidin-4-yl)carbonyl]amino}acetic acid,
{[(2-{[trans-1-(4-tert-butylphenyl)-2-methylpiperidin-4-yl]methyl}-5-hydroxy-6-methylpyrimidin-4-yl)carbonyl]amino} acetic acid,
{[(2-{[1-(4-tert-butylphenyl)-1,2,3,6-tetrahydropyridin-4-yl]methyl}-5-hydroxy-6-methylpyrimidin-4-yl)carbonyl]amino}acetic acid,
{[(2-{[6-(4-tert-butylphenyl)pyridin-3-yl]methyl}-5-hydroxy-6-methylpyrimidin-4-yl)carbonyl]amino}acetic acid,
{[(2-[6-(biphenyl-4-yl)pyridin-3-yl]methyl}-5-hydroxy-6-methylpyrimidin-4-yl)carbonyl]amino}acetic acid,
{[(2-{[2-(4-tert-butylphenyl)pyrimidin-5-yl]methyl}-5-hydroxy-6-methylpyrimidin-4-yl)carbonyl]amino}acetic acid,
{[(2-([5-(4-tert-butylphenyl)pyridin-2-yl]methyl}-5-hydroxy-6-methylpyrimidin-4-yl)carbonyl]amino}acetic acid,
({[2-{[1-(4-tert-butylphenyl)piperidin-4-yl]methyl}-5-hydroxyl-6-(methylsulfanyl)pyrimidin-4-yl]carbonyl}amino)acetic acid,
({[2-{[6-(4-tert-butylphenyl)pyridin-3-yl]methyl}-5-hydroxy-6-(methylsulfanyl)pyrimidin-4-yl]carbonyl}amino)acetic acid, and
{[(2-{[trans-5-(biphenyl-4-yl)-1,3-dioxan-2-yl]methyl}-5-hydroxy-6-methylpyrimidin-4-yl)carbonyl]amino}acetic acid.

The compounds of the present invention can be produced by applying various known synthesis methods utilizing characteristics based on their backbone or types of substituents. At that time, depending on the type of functional group, it may be effective in terms of production technology to protect the functional group with a suitable protecting group at the stage of a starting material or intermediate, or substitute the functional group with a group that can be easily converted to that functional group. Examples of such functional groups include an amino group, a hydroxyl group and a carboxyl group and the like, and examples of their protecting groups include those described in, for example, Protective Groups in Organic Synthesis, 3rd ed., Greene, T.W. and Wuts, P.G.M. eds., John Wiley & Sons; New York, 1999, and these protecting groups can be suitably selected and used corresponding to the reaction conditions thereof. According to such methods, a desired compound can be obtained by introducing the protecting group and carrying out the reaction followed by removing the protecting group as necessary or converting the protecting group to a desired group. The obtained compounds of the present invention can be identified, and their composition or purity can be analyzed, by standard analytical technologies such as elemental analysis, NMR, mass spectroscopy or IR analysis.

Starting materials and reagents used to produce compounds of the present invention can be purchased from commercial suppliers, or can be synthesized according to methods described in the literature.

### Effects of the invention

A compound of the present invention or a pharmacologically acceptable salt thereof, demonstrates superior EPO production activity in an assay system using Hep3B cells. Namely, EPO can be produced by administering a compound of the present invention or pharmacologically acceptable salt thereof, to a mammal (such as a human, cow, horse, or pig) or bird (such as a chicken). Thus, a compound of the present invention or a pharmacologically acceptable salt thereof, can be used for the prophylaxis and/or treatment of diseases caused by decreased EPO, for auto-transfusion in patients scheduled to undergo surgery, or the like. Examples of diseases caused by decreased EPO include anemia, particularly nephrogenic anemia (dialysis stage, conservation stage), anemia of prematurity, anemia incidental to chronic diseases, anemia incidental to cancer chemotherapy, and cancerous anemia. A compound of the present invention or a pharmacologically acceptable salt thereof can also be used as a medicament for prophylaxis and /or treatment of other diseases and pathological states presenting decreased EPO such as ischemic brain diseases.

### Mode for carrying out the invention

The following indicates representative examples of methods for producing compounds of the present invention. Furthermore, the production method of the present invention is not limited to the following examples.

### (Step 1)

Step 1 is a step for producing compound (1) from compound (2) to be subsequently described.

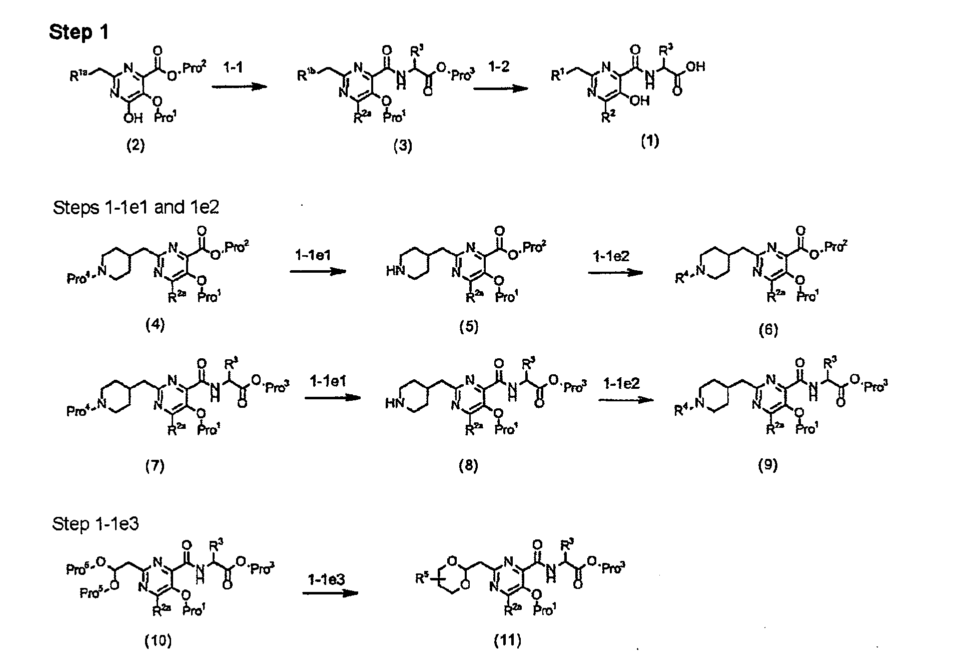

In the above formulae, Through R³ have the same meanings as previously defined, R⁴ and R⁵ represent a substituted or unsubstituted aryl group or heteroaryl group, R^{1a} and R^{1b} represent the aforementioned R¹ substituent or a precursor thereof, R^{2a} represents the aforementioned R² substituent or a precursor thereof, and Pro¹ through Pro⁵ represent a protecting group of each functional group selected according to a known method (see, for example, Protective Groups in Organic Synthesis, 3rd ed., Greene, T.W. and Wuts, P.G.M. eds., John Wiley & Sons, New York, 1999). There are no particular limitations on Pro¹ through Pro⁵ provided they are stable during the reaction and do not inhibit the reaction, and Pro¹ preferably represents a benzyl group, Pro² preferably represents a tert-butyl group, Pro³ preferably represents a methyl group or an ethyl group, Pro⁴ preferably represents a tert-butoxycarbonyl group, and Pro⁵ preferably represents a methyl group or an ethyl group.

The following provides a detailed description of each step.

### (Step 1-1)

Step 1-1 is a step for producing a compound represented by general formula (3) from a compound represented by general formula (2) to be subsequently described. Examples of essential reactions include:
step 1-1a: deprotecting reaction of protecting group Pro2;
step 1-1b: condensation reaction with an amino acid or amino acid salt represented by the general formula H₂NCH(R³)CO₂Pro³;
step 1-1c: reaction for introducing a leaving group (-OX¹) onto the hydroxyl group at the 6-position; and
step 1-1d: reaction for converting leaving group (-OX¹) to substituent R^{2a}.
   The following step can be included as necessary:
step 1-1e: reaction for converting R^{1a} to R^{1b}

Furthermore, there are no particular limitations on X¹ provided it is a substituent that forms a leaving group together with the oxygen to which it is bonded, and a trifluoromethanesulfonyl group is preferable.

Steps 1-1a through 1-1e may be carried out in any order, and the order in which they are carried out can be easily and suitably selected by one of ordinary skill in the art.

### (Step 1-1a)

This step is a step for deprotecting the protecting group Pro². A known method which is described in, for example, Protective Groups, in Organic Synthesis, 3rd ed., Greene, T.W. and Wuts, P.G.M. eds., John Wiley & Sons, New York, 1999, is suitably selected corresponding to the Pro² used, and this step is carried out according thereto. Here, a tert-butyl group is selected as a preferable example of Pro² and a method in which Pro² is converted to a hydrogen atom using a base in an inert solvent is described, and the method used is not limited thereto.

There are no particular limitations on the solvent used provided it does not inhibit the reaction and dissolves the starting substance to a certain extent, and preferable examples include aromatic hydrocarbons such as benzene, toluene or xylene; halogenated hydrocarbons such as methylene chloride or chloroform; ethers such as diethyl ether, tetrahydrofuran, 1,4-dioxane or 1,2-dimethoxyethane; alcohols such as methanol, ethanol or tert-butanol; esters such as ethyl acetate or propyl acetate; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the base used provided it is used as a base in ordinary reactions, and preferable examples include organic bases such as triethylamine; alkali metal carbonates such as sodium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide or potassium hydroxide; alkaline earth metal hydroxides such as cesium hydroxide; and alkali metal phosphates such as tripotassium phosphate.

Varying according to the starting material compounds, reagents and the like, the reaction temperature is usually -10 to 150°C and preferably 10 to 90°C.

Varying according to the starting material compounds, reagents and the like, the reaction time is usually 1 minute to 24 hours and preferably 10 minutes to 6 hours.

Following completion of the reaction, the desired compound can be obtained as a solid by distilling off the organic solvent, adding water and then adding an acid. On the other hand, in the case that a solid is unable to be obtained by adding an acid, the desired compound can be obtained by extracting the organic compound with an organic solvent such as ethyl acetate, followed by concentrating the organic layer after having dried with a commonly used procedure, or concentrating under reduced pressure after adding an acid.

### (Step 1-1b)

This step is a step for condensing an amino acid or amino acid salt represented by the general formula H₂NCH(R³)CO₂Pro³, and is carried out using a condensation agent in an inert solvent and in the presence or absence of a base.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol, ethanol or tert-butanol; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the base used provided it is used as a base in ordinary reactions, and preferable examples include organic bases such as triethylamine, diisopropylethylamine, 4-methylmorpholine, lutidine or pyridine; alkali metal carbonates such as sodium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide; alkaline earth metal hydroxides such as cesium hydroxide; and alkali metal phosphates such as tripotassium phosphate.

There are no particular limitations on the condensation agent used provided it is used as a condensation agent that forms an amide bond (in a method such as that described in Kusumoto, S., Experimental Science Course IV, Chemical Society of Japan, Maruzen Publishing, 1990, Izumiya, N., Peptide Synthesis Basics and Experimentation, Maruzen Publishing, 1985, or the like), and preferable examples include O-benzotriazole-N,N,N',N'-tetramethyluronium hexafluorophosphate (HBTU), 2-(1H-benzotriazol-1-yl)-1,1,3,3-tetramethyluronium tetrafluoroborate (TBTU), 1-(3-dimethylaminopropyl)-3-ethylcarbodiimide hydrochloride (EDCI), 4-(2-{[(cyclohexylimino)methylene]amino}ethyl-4-methylmorphol-4-inium para-toluenesulfonate (CMC), dicyclohexylcarbodiimide (DCC), 1,1'-carbonylbis(1H-imidazole) (CDI), (1H-benzotriazol-1-yloxy)(tripyrrolidin-1-yl)phosphonium hexafluorophosphate (PyBOP), bromo(tripyrrolidin-1-yl)phosphonium hexafluorophosphate (PyBrOP), 4-(4,6-dimethoxy-1,3,5-triazin-2-yl)-4-methylmorpholinium chloride (DMT-MM) and 2-chloro-4,6-dimethoxy-1,3,5-triazine (DMT). An additive such as 1-hydroxybenzotriazole (HOBT) or N,N-dimethylaminopyridine may also be added.

Varying according to the starting material compounds, reagents and the like, the reaction temperature is usually -10 to 150°C and preferably 0 to 100°C.

Although varying according to the starting material compounds, reagents and the like, the reaction time is usually 5 minutes to 48 hours and preferably 10 minutes to 24 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-1c)

This step is a step for introducing a leaving group (-OX¹) onto the hydroxyl group at the 6-position, and is carried out by reacting with an acid chloride or acid anhydride in an inert solvent and in the presence or absence of a base.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; nitriles such as acetonitrile; amides such as formamide or N,N-dimethylformamide; sulfoxides such as dimethyl sulfoxide; and a mixture of a plurality of organic solvents mixed in an arbitrary ratio.

There are no particular limitations on the acid chloride or acid anhydride used provided it is an acid chloride or acid anhydride that has X¹ such that an -OX¹ group is a known leaving group, and preferable examples include substituted or unsubstituted alkylsulfonic anhydrides or arylsulfonic anhydrides such as trifluoromethanesulfonic anhydride, substituted or unsubstituted alkylsulfonyl chlorides or arylsulfonyl chlorides such as methanesulfonyl chloride or p-toluenesulfonyl chloride, and substituted or unsubstituted alkyl phosphoric acid chlorides or aryl phosphoric acid chlorides.

There are no particular limitations on the base used provided it is used as a base in ordinary reactions, and preferable examples include organic bases such as triethylamine, diisopropylethylamine, 4-methylmorpholine, lutidine or pyridine; alkali metal carbonates such as sodium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide; alkaline earth metal hydroxides such as cesium hydroxide; and alkali metal phosphates such as tripotassium phosphate.

Varying according to the starting material compounds, reagents and the like, the reaction temperature is usually -100 to 150°C and preferably -80 to 40°C.

Varying according to the starting material compounds, reagents and the like, the reaction time is usually 5 minutes to 24 hours and preferably 10 minutes to 6 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-1d)

This step is a step for converting a leaving group (-OX¹) to substituent R^{2a}. In the case that R^{2a} is an alkyl group or an alkenyl group, this step is carried out by reacting with an alkyl boron compound or an alkenyl boron compound in an inert solvent, in the presence or absence of a base, in the presence or absence of an additive, and in the presence of a metal catalyst (1-1d1). In the case that R^{2a} is a methylsulfanyl group, this step is carried out by reacting with methanethiol or a metal salt of methanethiol in an inert solvent and in the presence or absence of base (1-1d2). In the case of a difluoromethyl group or fluoromethyl group, this step is carried out by allowing a base to act in an inert solvent on a compound which is obtained in the aforementioned step 1-1d1 and in which a methyl group is introduced for R^{2a}, followed by reacting with an electrophilic fluorinating reagent (1-1d3).

### (Step 1-1d1)

This step is a step for converting a leaving group (-OX¹) to an alkyl group or an alkenyl group as R^{2a}, and is carried out by reacting with an alkyl boron compound or alkenyl boron compound in an inert solvent, in the presence or absence of a base, in the presence or absence of an additive, and in the presence of a metal catalyst. A known method described in, for example, Zou, G., Reddy, Y.K. and Falck, J.R., Tetrahedron Lett., 2001, 42, 7213; Molander, GA. and Yun, C.-S., Tetrahedron Lett., 2002, 58, 1465; Tsuji, J., Palladium Reagents and Catalysts, John Wiley & Sons, England, 2004; or, Metal-Catalyzed Cross-Coupling Reactions, de Meijere, A. and Diederich, F., eds., Wiley-VCH, Weinheim, 2004, is suitably selected for the reaction conditions, and the reaction is carried out in accordance therewith. The following indicates preferable conditions, and the reaction conditions are not limited thereto.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol, ethanol or tert-butanol; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the base used provided it is used as a base in ordinary reactions, and preferable examples include organic bases such as triethylamine, diisopropylethylamine, 4-methylmorpholine, lutidine or pyridine; alkali metal carbonates such as potassium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide; alkaline earth metal hydroxides such as cesium hydroxide; alkali metal phosphates such as tripotassium phosphate; and metal alkoxides such as sodium tert-butoxide or potassium tert-butoxide.

There are no particular limitations on the additives used provided they are used in known methods, and preferable examples include metal oxides such as silver oxide or alumina; phosphines such as triphenylphosphine, tri-tert-butylphosphine, tricyclohexylphosphine, tri(o-tolyl)phosphine, diphenylphosphinoferrocene, 2-dicyclohexylphosphino-2',6'-dimethoxy-1,1'-biphenyl (S-PHOS), 2-dicyclohexylphosphino-2',4',6'-triisopropyl-1,1'-biphenyl (X-PHOS) or 2,2'-bis(diphenylphosphino)-1,1'-binaphthyl (BINAP); phosphine oxides such as triphenylphosphine oxide; metal salts such as lithium chloride, potassium fluoride or cesium fluoride; and ammonium salts such as tetrabutylammonium bromide. These may be used in combination in an arbitrary ratio.

There are no particular limitations on the metal catalyst used provided it is used in known methods, and preferable examples include palladium catalysts such as tetrakis(triphenylphosphine) palladium, bis(tri-tert-butylphosphine) palladium, palladium diacetate, palladium dichloride-diphenylphosphinoferrocene complex, palladium dichloride-benzonitrile complex, palladium dichloride-acetonitrile complex, bis(dibenzylideneacetone) palladium, tris(dibenzylideneacetone) dipalladium, bis[1,2-bis(diphenylphosphino)ethane] palladium, 3-chloropyridine[1,3-bis(2,6-diisopropylphenyl)imidazo-2-ylidene] palladium or palladium-activated carbon.

There are no particular limitations on the alkyl boron compound and alkenyl boron compound used provided it is used as a known reactant, and preferable examples include methylboronic acid, methylboronic acid ester, trifluoro(methyl)boranuide metal salt, ethylboronic acid, ethylboronic acid ester or ethyltrifluoroboranuide metal salt in the case that R^{2a} is an alkyl group, and vinylboronic acid, 4,4,5,5-tetramethyl-2-vinyl-1,3,2-dioxaborolane, vinylboronic acid ester, vinyltrifluoroboranuide metal salt, allylboronic acid, allylboronic acid ester or allyl(trifluoro)boranuide metal salt in the case that R^{2a} is an alkenyl group.

There are no particular limitations on the ester moiety of the alkylboronic acid ester, the metal of the trifluoro(alkyl) boranuide metal salt, the ester moiety of the alkenylboronic acid ester or the metal of the trifluoro(alkenyl)boranuide, provided they are known to be known compounds or are synthesized according to methods similar to known methods.

Varying according to the starting material compounds, reagents and the like, the reaction temperature is usually -10 to 200°C and preferably 0 to 150°C.

Although varying according to the starting material compounds, reagents and the like, the reaction time is usually 5 minutes to 48 hours and preferably 10 minutes to 12 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

Furthermore, in the case that R^{2a} represents an alkenyl group, it can be converted to the corresponding alkyl group as R² by carrying out the hydrogenolysis reaction of step 1-2a1 to be described later.

### (Step 1-1d2)

This step is a step for converting a leaving group (-OX¹) to a methylsulfanyl group as R^{2a}, and is carried out by reacting methanethiol or a metal salt of methanethiol in an inert solvent and in the presence or absence of a base.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; nitriles such as acetonitrile; amides such as formamide or N,N-dimethylformamide; alcohols such as methanol or ethanol; sulfoxides such as dimethyl sulfoxide; a mixture of a plurality of organic solvents mixed in an arbitrary ratio; and a mixture thereof with water mixed in an arbitrary ratio.

There are no particular limitations on the metal used in the metal salt of methanethiol used, and preferable examples include alkali metals such as sodium and alkaline earth metals such as magnesium.

There are no particular limitations on the base used provided it is used as a base in ordinary reactions, and preferable examples include organic bases such as pyridine; alkali metal carbonates such as sodium carbonate or potassium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide; alkaline earth metal hydroxides such as cesium hydroxide; alkali metal phosphates such as tripotassium phosphate; and metal alkoxides such as sodium tert-butoxide or potassium tert-butoxide.

Varying according to the starting material compounds, reagents and the like, the reaction temperature is usually -10 to 150°C and preferably 0 to 100°C.

Varying according to the starting material compounds, reagents and the like, the reaction time is usually 5 minutes to 48 hours and preferably 10 minutes to 12 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified- as necessary using an ordinary method such as recrystallization, reprecipitation or silica gel column chromatography.

### (Step 1-1d3)

This step is a step for converting R^{2a} of the compound which is obtained in the aforementioned step 1-1d1 and in which a methyl group is introduced as R^{2a}, to a difluoromethyl group or a fluoromethyl group, and is carried out by allowing a base to act in an inert solvent followed by reacting with an electrophilic fluorinating reagent. The desired compound can be produced by suitably adjusting the number of equivalents of the base and electrophilic fluorinating agent used.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; ethers such as diethyl ether, tetrahydrofuran, 1,4-dioxane or 1,2-dimethoxyethane; nitriles such as acetonitrile; amides such as formamide or N,N-dimethylformamide; sulfoxides such as dimethyl sulfoxide; and a mixture of a plurality of organic solvents mixed in an arbitrary ratio.

There are no particular limitations on the base used provided it is used as a base in ordinary reactions, and preferable examples include organic metal amides such as lithium diisopropylamide or sodium hexamethyldisilazide, organometal compounds such as tert-butyl lithium, and metal hydrides such as potassium hydride.

There are no particular limitations on the electrophilic fluorinating agent used provided it is used as a known reagent (see, for example, Baudoux, J. and Cahard, D., Electrophilic Fluorination with N-F Reagent, In Organic Reaction, Overman, L.E., ed., John Wiley & Sons, Inc., New Jersey, 2007, Vol. 69, Chapter 2, or Ishikawa, N., Synthesis and Functions of Fluorine Compounds, CMC Inc., Tokyo, 1987), and N-fluorobenzene sulfonimide (NFSI) is used preferably.

Varying according to the starting material compounds, reagents and the like, the reaction temperature is usually -100 to 100°C and preferably -80 to 50°C.

Varying according to the starting material compounds, reagents and the like, the reaction time is usually 5 minutes to 48 hours and preferably 10 minutes to 12 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-1e)

This step is a step for converting R^{1a} to R^{1b}, and since the synthesis method varies according to the type of heterocycle at the Q¹ position, the detailed description is divided into the following cases:
(1) a case of R^{1a} containing a heterocycle Q¹ and having a protecting group Pro⁴ on a nitrogen atom within the ring of Q¹, and
(2) a case of R^{1a} having an acetal structure..

Examples of essential reactions of (1) of Step (1-1e) include the following:
step 1-1e1: deprotecting reaction of protecting group Pro⁴; and,
step 1-1e2: reaction for introducing substituent R⁴.
The case of having a 1-(tert-butoxycarbonyl) piperidin-4-yl group as R^{1a} is indicated here, and the present invention is not limited thereto.

### (Step 1-1e1)

This step is a step for producing a compound represented by general formula (5) or (8). A known method which is described in, for example, Protective Groups in Organic Synthesis, 3rd ed., Greene, T.W. and Wuts, P.G.M. eds., John Wiley & Sons, New York, 1999, is suitably selected corresponding to the Pro⁴ used as an amine protecting group, and this step is carried out according thereto. Here, reaction conditions are described for the case of using a tert-butoxycarbonyl group as a preferable example of Pro⁴, and the reaction conditions are not limited thereto. The reaction is carried out by adding a suitable reagent to a compound represented by general formula (4) or (7) in an inert solvent.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; ethers such as diethyl ether, tetrahydrofuran, 1,4-dioxane or 1,2-dimethoxyethane; esters such as ethyl acetate or propyl acetate; nitriles such as acetonitrile; amides such as formamide or N,N-dimethylformamide; sulfoxides such as dimethyl sulfoxide; and a mixture of a plurality of organic solvents mixed in an arbitrary ratio.

There are no particular limitations on the reagent used provided it is used as a reagent that deprotects tert-butoxycarbonyl groups in ordinary reactions, and preferable examples include inorganic acids such as hydrochloric acid or sulfuric acid; organic acids such as acetic acid or trifluoroacetic acid; Lewis acids such as trimethylsilyl iodide or boron trifluoride; acid chlorides such as acetyl chloride; and alkali metal hydroxides such as sodium hydroxide.

Varying according to the starting material compounds, reagents and the like, the reaction temperature is usually -10 to 100°C and preferably 10 to 50°C.

Varying according to the starting material compounds, reagents and the like, the reaction time is usually 5 minutes to 24 hours and preferably 10 minutes to 6 hours.

Following completion of the reaction, the desired compound can be obtained as a solid by distilling off the organic solvent, adding water and then adding an acid. On the other hand, in the case that a solid is unable to be obtained by adding an acid, the desired compound can be obtained by extracting the organic compound with an organic solvent such as ethyl acetate, followed by concentrating the organic layer after having dried with a commonly used procedure, or concentrating under reduced pressure after adding an acid.

### (Step 1-1e2)

This step is a step for producing a compound represented by general formula (6) or (9), and is carried out by reacting a substituted or unsubstituted aryl halide, heteroaryl halide, aryl pseudohalide or heteroaryl pseudohalide with a compound represented by general formula (5) or (8) in an inert solvent, in the presence or absence of a base, in the presence or absence of an additive, and in the presence of a metal catalyst. A known method described in, for example, Tsuji, J., Palladium Reagents and Catalysts, John Wiley & Sons, England, 2004; or, Jiang, L., Buchwald, S.L., Palladium-Catalyzed Aromatic Carbon-Nitrogen Bond Formation, in Metal-Catalyzed Cross-Coupling Reactions, de Meijere, A. and Diederich, F., eds., Wiley-VCH, Weinheim, 2004, Chapter 13, is suitably selected for the reaction conditions, and the reaction is carried out in accordance therewith. The following indicates preferable conditions, and the reaction conditions are not limited thereto.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol, ethanol or tert-butanol; nitriles such as acetonitrile, amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the base used provided it is used as a base in ordinary reactions, and preferable examples include organic bases such as triethylamine, diisopropylethylamine, 4-methylmorpholine, lutidine or pyridine; alkali metal carbonates such as potassium carbonate; alkaline earth metal carbonates such as cesium carbonate; alkali metal hydrogencarbonates such as potassium hydrogencarbonate; alkaline earth metal hydrogencarbonates such as calcium hydrogencarbonate; alkali metal hydroxides such as sodium hydroxide; alkaline earth metal hydroxides such as cesium hydroxide; alkali metal phosphates such as tripotassium phosphate; metal alkoxides such as sodium tert-butoxide or potassium tert-butoxide; organometallic amides such as lithium diisopropylamide or sodium hexamethyldisilazide; organometallic compounds such as tert-butyl lithium; and metal hydrides such as potassium hydride.

There are no particular limitations on the additives used provided they are used in known methods, and preferable examples include metal oxides such as silver oxide or alumina; phosphines such as triphenylphosphine, tri-tert-butylphosphine, tricyclohexylphosphine, tri(o-tolyl)phosphine, diphenylphosphinoferrocene, 2-dicyclohexylphosphino-2',6'-dimethoxy-1,1'-biphenyl (S-PHOS), 2-dicyclohexylphosphino-2',4',6'-triisopropyl-1,1'-biphenyl (X-PHOS) or 2,2'-bis(diphenylphosphino)-1,1'-binaphthyl (BINAP); phosphine oxides such as triphenylphosphine oxide; metal salts such as lithium chloride, potassium fluoride or cesium fluoride; and ammonium salts such as tetrabutylammonium bromide. These may be used in combination in an arbitrary ratio.

There are no particular limitations on the metal catalyst used provided it is used in known methods, and preferable examples include palladium catalysts such as tetrakis(triphenylphosphine) palladium, bis(tri-tert-butylphosphine) palladium, palladium diacetate, palladium dichloride-diphenylphosphinoferrocene complex, palladium dichloride-benzonitrile complex, palladium dichloride-acetonitrile complex, bis(dibenzylideneacetone) palladium, tris(dibenzylideneacetone) dipalladium, bis[1,2-bis(diphenylphosphino)ethane] palladium, 3-chloropyridine[1,3-bis(2,6-diisopropylphenyl)imidazo-2-ylidene] palladium or palladium-activated carbon.

A pseudohalide refers to a substituent that is known to undergo oxidative addition to a low-valent transition metal catalyst in the same manner as a halogen atom in a coupling reaction using a transition metal catalyst. There are no particular limitations on the pseudohalide provided it is a substituent for which this oxidative addition reaction is known to occur, and examples include sulfonyloxy groups such as a trifluoromethylsulfonyloxy group, a methanesulfonyloxy group or a p-toluenesulfonyloxy group; acyloxy groups such as an acetyloxy group; diazonium groups and phosphonyloxy groups.

There are no particular limitations on the substituted or unsubstituted aryl halide, heteroaryl halide, aryl pseudohalide or heteroaryl pseudohalide used provided it is known to be a known compound or is synthesized according to methods similar to known methods.

Varying according to the starting material compounds, reagents and the like, the reaction temperature is usually -10 to 200°C and preferably 0 to 150°C.

Varying according to the starting material compounds, reagents and the like, the reaction time is usually 5 minutes to 48 hours and preferably 10 minutes to 12 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

Examples of essential reactions of (2) of Step (1-1e) include the following:
step 1-1e3: transacetalization reaction with a substituted alkanediol.
   The synthesis of a compound having a 1,3-dioxane structure is described here, and the present invention is not limited thereto. Furthermore, a compound having a 1,3-dioxolane structure is obtained by using a substituted ethane-1,2-diol instead of a substituted propane-1,3-diol, and a compound having a 1,3-dioxepane structure is obtained by using a substituted butane-1,4-diol instead of a substituted propane-1,3-diol.

### (Step 1-1e3)

This step is a step for producing general formula (11), and is carried out by allowing a substituted propane-1,3-diol to react with a compound represented by general formula (10) in an inert solvent and in the presence of a catalyst. A known method described in, for example, Protective Groups in Organic Synthesis, Greene, T.W. and Wuts, P.GM. eds., John Wiley & Sons, New York, 1999, Chapter 2, is suitably selected for the reaction conditions, and the reaction is carried out in accordance therewith. Preferable conditions are described below, and the method is not limited thereto.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; ethers such as diethyl ether, tetrahydrofuran, 1,4-dioxane or 1,2-dimethoxyethane; nitriles such as acetonitrile; amides such as formamide or N,N-dimethylformamide; sulfoxides such as dimethyl sulfoxide; alcohols such as methanol, ethanol or tert-butanol; and a mixture of a plurality of organic solvents mixed in an arbitrary ratio.

Examples of the catalyst used include inorganic acids such as hydrochloric acid or sulfuric acid, organic acids such as acetic acid, trifluoroacetic acid, p-toluenesulfonic acid or methanesulfonic acid, and Lewis acids such as trimethylsilyl trifluoromethanesulfonate.

There are no particular limitations on the substituted propane-1,3-diol used, and it can be synthesized by combining known methods.

Varying according to the starting material compounds, reagents and the like, the reaction temperature is usually -10 to 150°C and preferably 0 to 120°C.

Varying according to the starting material compounds, reagents and the like, the reaction time is usually 5 minutes to 48 hours and preferably 10 minutes to 12 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-2)

Step 1-2 is a step for producing a compound represented by general formula (1) from a compound represented by general formula (3).

Examples of essential reactions include:
step 1-2a: deprotection reaction of protecting group Pyro¹; and
step 1-2b: deprotection reaction of protecting group Pro³,
   and the following reactions can be added as necessary:
step 1-2c: reaction for converting R^{1b} to R¹; and
step 1-2d: reaction for converting R^{2a} to R².
   Steps 1-2a through 1-2d may be carried out in any order, and the order in which they are carried out can easily be suitably selected by one of ordinary skill in the art.

### (Step 1-2a)

This step is a step for deprotecting the protecting group Pro¹. A known method which is described in, for example, Protective Groups in Organic Synthesis, 3rd ed., Greene, T.W. and Wuts, P.G.M. eds., John Wiley & Sons, New York, 1999, is suitably selected corresponding to the Pro¹ used, and this step is carried out according thereto. Here, a benzyl group is selected as a preferable example of Pro¹, and a method in which Pro¹ is converted to a hydrogen atom using a catalyst under a hydrogen atmosphere, in an inert solvent and in the presence or absence of an additive (1-2a1), a method in which Pro¹ is converted to a hydrogen atom using a catalyst in the presence of an organic compound that can serve as a hydrogen source instead of hydrogen atoms, under nitrogen or argon atmosphere, in an inert solvent and in the presence or absence of an additive (1-2a2), and a method in which Pro¹ is converted to a hydrogen atom using a suitable acid in an inert solvent (1-2a3) are described, and the method is not limited thereto.

### (Step 1-2a1)

This step is a step for converting Pro² to a hydrogen atom using a catalyst under hydrogen atmosphere, in an inert solvent and in the presence or absence of an additive.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol, ethanol or tert-butanol; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the additive used provided it is an additive such as hydrochloric acid which is used in known methods.

There are no particular limitations on the metal catalyst used provided it is used in known methods, and preferable examples include palladium-activated carbon, tri(triphenylphosphine) rhodium chloride or palladium hydroxide.

Varying according to the starting material compounds, reagents and the like, the reaction temperature is usually -100 to 150°C and preferably 0 to 100°C.

Varying according to the starting material compounds, reagents and the like, the reaction time is usually 5 minutes to 24 hours and preferably 10 minutes to 6 hours.

Following completion of the reaction, insoluble materials are filtered off and the filtrate is distilled under reduced pressure. The resulting residue can be purified by using an ordinary method such as recrystallization, distillation or silica gel column chromatography.

Furthermore, in the case that R^{2a} represents an alkenyl group, it can be converted to the corresponding alkyl group as previously described.

### (Step 1-2a2)

This step is a step for converting Pro¹ to a hydrogen atom using a catalyst in the presence of an organic compound which can serve as a hydrogen source instead of hydrogen atoms, under a nitrogen or argon atmosphere, in an inert solvent and in the presence or absence of an additive.

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol or ethanol; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

In the case of carrying out the reaction not under a hydrogen atmosphere, there are no particular limitations on the organic compound used as a hydrogen source provided it is an organic compound such as formic acid which is used in known methods.

There are no particular limitations on the additive used provided it is an additive such as hydrochloric acid which is used in known methods.

There are no particular limitations on the metal catalyst used provided it is used in known methods, and preferable examples include palladium-activated carbon, tri(triphenylphosphine) rhodium chloride or palladium hydroxide.

Varying according to the starting material compounds, reagents and the like, the reaction temperature is usually -100 to 150°C and preferably -78 to 100°C.

Varying according to the starting material compounds, reagents and the like, the reaction time is usually 5 minutes to 24 hours and preferably 10 minutes to 6 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-2a3)

There are no particular limitations on the solvent used provided it does not inhibit the reaction and dissolves the starting material to a certain extent, and preferable examples include aromatic hydrocarbons such as benzene, toluene or xylene; halogenated hydrocarbons such as methylene chloride or chloroform; esters such as ethyl acetate or propyl acetate; ethers such as diethyl ether, tetrahydrofuran, 1,4-dioxane or 1,2-dimethoxyethane; alcohols such as methanol or ethanol; nitriles such as acetonitrile; amides such as formamide or N,N-dimethylformamide; sulfoxides such as dimethyl sulfoxide; a mixture of a plurality of organic solvents mixed in an arbitrary ratio; and a mixture thereof with water mixed in an arbitrary ratio.

There are no particular limitations on the acid used provided it is used as an acid in ordinary reactions, and examples include inorganic acids such as hydrochloric acid or sulfuric acid; Lewis acids such as boron trifluoride, boron tribromide or trimethylsilyl iodide; and organic acids such as trifluoroacetic acid.

Varying according to the starting material compounds, reagents and the like, the reaction temperature is usually -100 to 150°C and preferably -78 to 100°C.

Varying according to the starting material compounds, reagents and the like, the reaction time is usually 5 minutes to 24 hours and preferably 10 minutes to 6 hours.

Following completion of the reaction, the desired compound of this reaction can be obtained by, for example, concentrating the reaction mixture, adding water and an immiscible organic solvent such as ethyl acetate and washing with water, followed by separating the organic layer containing the desired compound, drying with anhydrous magnesium sulfate and the like, and distilling off the solvent.

The obtained compound can be further purified as necessary using an ordinary method such as recrystallization, reprecipitation or silica gel column chromatography.

### (Step 1-2b)

This step is a step for deprotecting the protecting group Pro³, and is carried out according to methods similar to those in step 1-1a.

### (Step 1-2c)

This step is a step for converting R^{1b} to R¹, and is carried out according to methods similar to those in step 1-1e.

### (Step 1-2d)

This step is a step for converting R^{2a} to R², and in the case that R^{2a} is an alkenyl group, it can be converted to the corresponding alkyl group according to methods similar to those in the aforementioned step 1-2a1.

### (Step 2)

Step 2 is a step for producing the aforementioned compound (2).

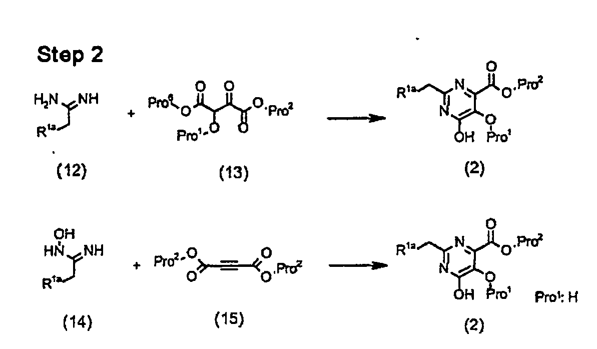

In the above formulae, R^{1a} represents the aforementioned R¹ substituent or a precursor thereof, Pro¹ and Pro² represent a protecting group of a functional group as previously described, and Pro⁶ represents a protecting group of each functional group selected according to a known method (see, for example, Protective Groups in Organic Synthesis, 3rd ed., Greene, T.W. and Wuts, P.G.M. eds., John Wiley & Sons, New York, 1999), and there are no particular limitations thereon provided it remains stable during the reaction and does not inhibit the reaction, and Pro⁶ preferably represents a methyl group.

A compound represented by general formula (2) can be synthesized using a known method (such as (i) a method in which a substituted ethaneimidamide (12) and a 2-alkyloxy-3-oxosuccinic acid diester (13), prepared according to methods similar to known methods, are condensed in the presence of base (Dreher, S.D., Ikemoto, N., Gresham, V., Liu, J., Dormer, P.G; Balsells, J., Mathre, D., Novak, T.J. and Armstrong III, J.D., Tetrahedron Lett., 2004, 45, 6023), or (ii) a method in which an N-hydroxy-substituted ethaneimidamide (14) and an acetylene dicarboxylic acid diester (15) are condensed (Culbertson, T.P., J. Heterocycl. Chem., 1979, 16, 1423-1424)), or can be synthesized using a modified version of a known method.

Reaction products obtained according to each of the aforementioned production methods are isolated and purified as free compounds, salts thereof or various types of solvates such as hydrates. Salts can be produced by subjecting the products to ordinary salt formation treatment. Isolation and purification are carried out by applying ordinary chemical procedures such as extraction, concentration, distillation, crystallization, filtration, recrystallization or various types of chromatography.

Each type of isomer can be isolated according to ordinary methods by utilizing differences in physicochemical properties therebetween. For example, optical isomers can be separated by common optical resolution methods such as fractional crystallization or chromatography. In addition, optical isomers can also be produced from suitable optically active raw material compounds.

A formulation containing a compound of the present invention as an active ingredient thereof is prepared using carriers, excipients and other additives ordinarily used in formulating procedures. Administration may be in the form of oral administration by tablets, pills, capsules, granules, grains or liquids and the like, or parenteral administration by injections such as intravenous injection or intramuscular injection, suppositories, transcutaneous agents or inhalants and the like. Dosage is suitably determined depending on an individual case in consideration of such factors as symptoms and age or gender of the administered subject, and the dosage is usually about 0.001 to 100 mg/kg for a human adult per day in the case of oral administration, and this is administered in a single administration or in 1 to 6 portions. In addition, in the case of intravenous administration, according to symptoms, the dosage is usually within the range of 0.0001 to 10 mg/kg per administration for a human adult, and this is administered one or multiple times per day.

Examples of solid compositions for oral administration according to the present invention include tablets, powders, granules and the like. In such solid compositions, one or more active substances may be mixed with at least one inert excipient such as lactose, mannitol or glucose. The composition may also contain an inert additive including a lubricant such as magnesium stearate, a disintegrant such as sodium carboxymethyl starch, or a dissolution aid, according to ordinary methods. Tablets or pills may be coated with a sugar coating, or a gastric or enteric coating as necessary.

Examples of liquid compositions for oral administration include pharmaceutically acceptable emulsions, liquids, suspensions, syrups, elixirs and the like, and contain commonly used inert solvents such as purified water or ethanol. These compositions may also contain auxiliary agents such as solubilizing agents, wetting agents or suspending agents, as well as sweeteners, corrigents, fragrances or preservatives, in addition to inert solvents.

Examples of injections for parenteral administration include sterile aqueous or non-aqueous liquids, suspensions and emulsions. Examples of aqueous solvents include distilled water for injection and physiological saline. Examples of non-aqueous solvents include propylene glycol, polyethylene glycol, vegetable oils such as olive oil, alcohols such as ethanol and Polysorbate 80 (Japanese Pharmacopoeia name) and the like. Such compositions may further contain isotonic agents, preservatives, wetting agents, emulsifiers, dispersants, stabilizers or solubilizing agents. These are sterilized for example by filtration by passing through a bacteria-retaining filter, incorporation of a bactericide, or irradiation.. In addition, these can also be used by producing a sterile solid composition and dissolving or suspending in sterile water or a sterile solvent for injection prior to use.

### Examples

The following provides a more detailed explanation of the present invention through Examples and Test Examples thereof, and the scope of the present invention is not limited thereto.

### Example 1

### {[(2-{[1-(4-tert-Butylphenyl)piperidin-4-yl]methyl}-5-hydroxy-6-methylpyrimidin-4-yl)carbonyl]amino} acetic acid

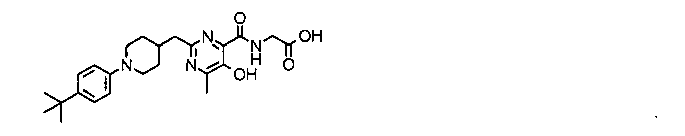

### (1) tert-Butyl 4-(cyanomethylene)piperidine-1-carboxylate

Diethyl cyanomethylphosphonate (16 mL, 100 mmol) was dissolved in tetrahydrofuran (360 mL), and under a nitrogen atmosphere at -70°C a solution of lithium hexamethyldisilazide in tetrahydrofuran (1 M, 100 mL, 100 mmol) and a solution of tert-butyl 4-oxopiperidine-1-carboxylate (18 g, 91 mmol) in tetrahydrofuran (36 mL) were added, followed by stirring at the same temperature for 40 minutes. Saturated aqueous ammonium chloride solution was added to the reaction solution, followed by extraction with ethyl acetate, and the extract was washed with saturated aqueous ammonium chloride solution. After the solvent was distilled off under reduced pressure, the resulting residue was purified by silica gel column chromatography (Moritex Corporation, elution solvent: hexane/ethyl acetate), and a fraction corresponding to the Rf value=0.41 (hexane/ethyl acetate=3/1) by thin layer chromatography was concentrated under reduced pressure to afford the title compound (22 g) as a white solid (quantitative yield).
1H-NMR (500 MHz, CDCl3) δ: 5.19 (1H, s), 3.56-3.46 (4H, m), 2.56 (2H, t, J=5 Hz), 2.33 (2H, t, J=5 Hz), 1.48 (9H, s).

### (2) tert-Butyl4-(cyanomethyl)piperidine-1-carboxylate

tert-Butyl 4-(cyanomethylene)piperidine-1-carboxylate (20 g, 91 mmol) was dissolved in ethyl acetate (400 mL), and 10% palladium-activated carbon (3.0 g) was added, followed by stirring at room temperature for 3.5 hours under a hydrogen atmosphere. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.38 (hexane/ethyl acetate=3/1) by thin layer chromatography was concentrated under reduced pressure to afford the title compound (23 g) as a white solid (quantitative yield).
1H-NMR (500 MHz, CDCl3) δ: 4.24-4.07 (2H, m), 2.78-2.64 (2H, m), 2.32 (2H, d, J=6 Hz), 1.89-1.75 (3H, m), 1.46 (9H, s), 1.32-1.21 (2H, m).

### (3) tert-Butyl 4-(2-amino-2-iminoethyl)piperidine-1-carboxylate acetic acid salt

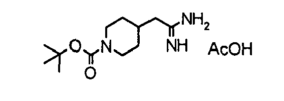

tert-Butyl 4-(cyanomethyl)piperidine-1-carboxylate (91 mmol) was dissolved in ethanol (200 mL), and aqueous hydroxylamine solution (50%, 17 mL, 16.7 mmol) was added, followed by heating to reflux for 3.5 hours. The reaction solution was cooled, and subsequently concentrated under reduced pressure to afford tert-butyl 4-[2-amino-2-(hydroxyimino)ethyl]piperidine-1-carboxylate as a colorless amorphous solid. This was dissolved in 1,4-dioxane (100 mL), and at room temperature acetic anhydride (17 mL, 181 mmol) and triethylamine (14.7 mL, 182 mmol) were added, followed by stirring at the same temperature for 2 hours. After the reaction solution was diluted with ethyl acetate, it was washed sequentially with water, dilute hydrochloric acid and water, and the organic layer was dried over sodium sulfate. After the solvent was distilled off under reduced pressure, the resulting solid was washed with hexane to afford tert-butyl 4-[2-(acetoxyimino)-2-aminoethyl]piperidine-1-carboxylate as a white solid. This was dissolved in ethanol (200 mL) and methylene chloride (40 mL), and 10% palladium-activated carbon (3.6 g) was added, followed by stirring at room temperature for 5 hours under a hydrogen atmosphere. After the reaction solution was filtered with celite, the filtrate was concentrated under reduced pressure to afford the title compound (24 g, 79 mmol) as a white solid (yield 85%).
1H-NMR (500 MHz, DMSO-d6) δ: 3.99-3.86 (2H, m), 2.81-2.58 (2H, m), 2.23 (2H, d, J=8 Hz), 1.85 (1H, m), 1.64 (3H, s), 1.64-1.56 (2H, m), 1.40 (9H, s), 0.99-1.08 (2H, m).

### (4) tert-Butyl 5-(benzyloxy)-2-{[1-(tert-butoxycarbonyl)piperidin-4-yl]methyl}-6-hydroxypyrimidine-4-carboxylate

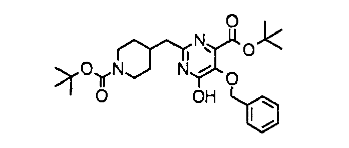

Diisopropylamine (30 mL, 210 mmol) was dissolved in tetrahydrofuran (100 mL), and a solution of n-butyllithium in hexane (2.77 M, 77 mL, 210 mmol) was added dropwise at 3°C, followed by stirring at -78°C for 30 minutes, to prepare a solution of lithium diisopropylamide (LDA) in tetrahydrofuran. tert-Butyl methyl oxalate (34 g, 210 mmol) and methyl (benzyloxy)acetate (35 g, 190 mmol) were dissolved in tetrahydrofuran (250 mL), and the prepared solution of LDA in tetrahydrofuran was added dropwise under a nitrogen atmosphere at -78°C, followed by stirring at the same temperature for 3 hours. The temperature of the reaction solution was gradually raised to -40°C, and subsequently hydrochloric acid (2 M, 210 mL) was added to the reaction solution, followed by extraction with ethyl acetate. The extract was washed with water and saturated aqueous sodium chloride solution, and dried over anhydrous magnesium sulfate, and subsequently the solvent was distilled off under reduced pressure to afford 4-tert-butyl 1-methyl 2-(benzyloxy)-3-oxosuccinate (62 g) as a yellow oil. A part of this oil (36 g, 120 mmol) and tert-butyl 4-(2-amino-2-iminoethyl)piperidine-1-carboxylate acetic acid salt (24 g, 79 mmol) were dissolved in methanol (240 mL), and at 3°C a solution of sodium methoxide in methanol (28%, 48 mL, 240 mmol) was added, followed by stirring at room temperature for 14.5 hours. After hydrochloric acid (1 M, 130 mL) was added to the reaction solution, a precipitated solid was collected by filtration to afford the title compound (26 g, 52 mmol) as a white solid (yield 66%).
1H-NMR (500 MHz, CDCl3) δ: 7.47-7.44 (2H, m), 7.40-7.31 (3H, m), 5.23 (2H, s), 4.21-3.91 (2H, m), 2.74-2.58 (2H, m), 2.62 (2H, d, J=7 Hz), 2.06 (1H, m), 1.69-1.60 (2H, m), 1.53 (9H, s), 1.43 (9H, s), 1.28-1.16 (2H, m).

### (5) tert-Butyl 5-(benzyloxy)-2-{[1-(tert-butoxycarbonyl)piperidin-4-yl]methyl}-6-{[(trifluoromethyl)sulfonyl]oxy}pyrimidine-4-carboxylate

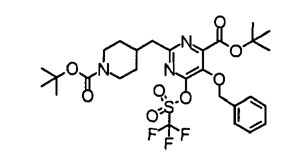

tert-Butyl 5-(benzyloxy)-2-{[1-(tert-butoxycarbonyl)piperidin-4-yl]methyl}-6-hydroxypyrimidine-4-carboxylate (5.0 g, 10 mmol) was dissolved in methylene chloride (100 mL), and trifluoromethanesulfonic anhydride (2.1 mL, 12 mmol) and triethylamine (2.1 mL, 15 mmol) were added at -78°C, followed by stirring at the same temperature for 30 minutes. After the reaction solution was concentrated under reduced pressure, the resulting residue was purified by silica gel column chromatography (Moritex Corporation, elution solvent: hexane/ethyl acetate), and a fraction corresponding to the Rf value=0.63 (hexane/ethyl acetate=2/1) by thin layer chromatography was concentrated under reduced pressure to afford the title compound (6.0 g, 9.5 mmol) as a yellow oil (yield 95%).
1H-NMR (500 MHz, CDCl3) δ: 7.44-7.36 (5H, m), 5.14 (2H, s), 4.21-3.95 (2H, m), 2.88 (2H, d, J=7 Hz), 2.80-2.62 (2H, m), 2.07 (1H, m), 1.67-1.60 (2H, m), 1.57 (9H, s), 1.46 (9H, s), 1.27-1.17 (2H, m).

### (6) tert-Butyl 5-(benzyloxy)-2-{[1-(tert-butoxycarbonyl)piperidin-4-yl]methyl}-6-methylpyrimidine-4-carboxylate

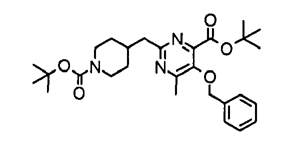

tert-Butyl 5-(benzyloxy)-2-{[1-(tert-butoxycarbonyl)piperidin-4-yl]methyl}-6-{[(trifluoromethyl)sulfonyl]oxy}pyrimidine-4-carboxylate (6.0 g, 9.5 mmol) was dissolved in tetrahydrofuran (90 mL), and at room temperature methylboronic acid (1.8 g, 30 mmol), silver oxide (6.7 g, 29 mmol), potassium carbonate (4.0 g, 29 mmol) and [1,1'-bis(diphenylphosphino)ferrocene]palladium-dichloride dichloromethane adduct (0.78 g, 0.96 mmol) were added, followed by heating to reflux for 3 hours under a nitrogen atmosphere. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.34 (hexane/ethyl acetate=2/1) by thin layer chromatography was concentrated under reduced pressure to afford the title compound (4.4 g, 8.8 mmol) as a yellow oil (yield 93%).
1H-NMR (500 MHz, CDCl3) δ: 7.44-7.34 (5H, m), 5.00 (2H, s), 4.17-3.98 (2H, m), 2.85 (2H, d, J=7 Hz), 2.77-2.64 (2H, m), 2.44 (3H, s), 2.09 (1H, m), 1.66-1.59 (2H, m), 1.59 (9H, s), 1.45 (9H, s), 1.30-1.19 (2H, m).

### (7) tert-Butyl 5-(benzyloxy)-6-methyl-2-[(piperidin-4-yl)methyl]pyrimidine-4-carboxylate hydrochloride

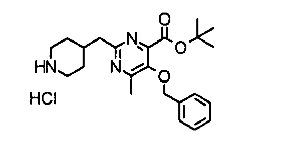

tert-Butyl 5-(benzyloxy)-2- {[1-(tert-butoxycarbonyl)piperidin-4-yl]methyl}-6-methylpyrimidine-4-carboxylate (4.4 g, 8.8 mmol) was dissolved in ethyl acetate (44 mL), and a solution of hydrogen chloride in ethyl acetate (4 M, 68 mL, 270 mmol) was added, followed by stirring at room temperature for 3 hours. Hexane (100 mL) and ethyl acetate (100 mL) were added to the reaction solution, whereby a solid was precipitated. The resulting solid was collected by filtration, and dried under reduced pressure to afford the title compound (3.7 g, 8.5 mmol) as a white solid (yield 95%).
1H-NMR (500 MHz, DMSO-d6) δ: 7.47-7.34 (5H, m), 4.99 (2H, s), 3.26-3.18 (2H, m), 2.90-2.78 (2H, m), 2.78 (2H, d, J=7 Hz), 2.46 (3H, s), 2.12 (1H, m), 1.78-1.70 (2H, m), 1.51 (9H, s), 1.51-1.39 (2H, m).

### (8) tert-Butyl 5-(benzyloxy)-2-{[1-(4-tert-butylphenyl)piperidin-4-yl]methyl}-6-methylpyrimidine-4-carboxylate

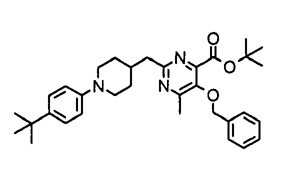

tert-Butyl 5-(benzyloxy)-6-methyl-2-[(piperidin-4-yl)methyl]pyrimidine-4-carboxylate hydrochloride (1.0 g, 2.3 mmol), 1-bromo-4-tert-butylbenzene (1.0 g, 4.6 mmol), sodium tert-butoxide (0.66 g, 6.9 mmol), 2-dicyclohexylphosphino-2',6'-dimethoxy-1,1'-biphenyl (S-PHOS) (0.19 g, 0.46 mmol) and tris(dibenzylideneacetone)dipalladium (0.21 g, 0.23 mmol) were suspended in toluene (50 mL), followed by heating to reflux for 8 hours under a nitrogen atmosphere. The reaction solution was cooled to room temperature, and subsequently filtered with celite. After the filtrate was concentrated under reduced pressure, the resulting residue was purified by silica gel column chromatography (Moritex Corporation, elution solvent: hexane/ethyl acetate), and a fraction corresponding to the Rf value=0.55 (hexane/ethyl acetate=2/1) by thin layer chromatography was concentrated under reduced pressure to afford the title compound (0.85 g, 1.6 mmol) as a pale yellow solid (yield 70%). 1H-NMR (500 MHz, CDCl3) δ: 7.43-7.36 (5H, m), 7.27-7.25 (2H, m), 6.88 (2H, d, J=9 Hz), 5.01 (2H, s), 3.63 (2H, d, J=12 Hz), 2.90 (2H, d, J=7 Hz), 2.67 (2H, t, J=11 Hz), 2.46 (3H, s), 2.10-2.01 (1H, brs), 1.77 (2H, d, J=12 Hz), 1.59 (9H, s), 1.51 (2H, d, J=10 Hz), 1.28 (9H, s).

### (9) Ethyl ({[5-(benzyloxy)-2-{[1-(4-tert-butylphenyl)piperidin-4-yl]methyl}-6-methylpyrimidin-4-yl]carbonyl}amino)acetate

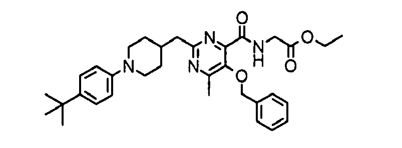

tert-Butyl 5-(benzyloxy)-2-{[1-(4-tert-butylphenyl)piperidin-4-yl]methyl}-6-methylpyrimidine-4-carboxylate (0.85 g, 1.6 mmol) was dissolved in a mixture of tetrahydrofuran (20 mL) and methanol (20 mL), and aqueous sodium hydroxide solution (8 M, 2.1 mL, 17 mmol) was added, followed by stirring at 60°C for 1 hour. The reaction solution was cooled to room temperature, concentrated under reduced pressure, and subsequently hydrochloric acid (1 M, 100 mL, 100 mmol) was added to the resulting residue, followed by extraction with ethyl acetate. The extract was washed with brine, and the organic layer was concentrated under reduced pressure to afford 5-(benzyloxy)-2-{[1-(4-tert-butylphenyl)piperidin-4-yl]methyl}-6-methylpyrimidine-4-carboxylic acid as a pale yellow solid. This was dissolved in a mixture of tetrahydrofuran (20 mL) and ethanol (20 mL), and glycine ethyl ester hydrochloride (0.27 g, 1.9 mmol) and N-methylmorpholine (0.21 mL, 1.9 mmol) were added, followed by stirring at room temperature for 5 minutes. To the reaction solution was added 4-(4,6-dimethoxy-1,3,5-triazin-2-yl)-4-methylmorpholinium chloride (0.49 g, 1.9 mmol), followed by stirring at room temperature for 12 hours. After the reaction solution was concentrated under reduced pressure, the resulting residue was purified by silica gel column chromatography (Moritex Corporation, elution solvent: hexane/ethyl acetate), and a fraction corresponding to the Rf value=0.60 (hexane/ethyl acetate=1/1) by thin layer chromatography was concentrated under reduced pressure to afford the title compound (0.81 g, 1.5 mmol) as a pale yellow oil (yield 90%).
1H-NMR (500 MHz, CDCl3) δ: 8.36 (1H, t, J=5 Hz), 7.49 (2H, d, J=6 Hz), 7.40-7.33 (3H, m), 7.28 (2H, d, J=9 Hz), 6.89 (2H, d, J=9 Hz), 5.12 (2H, s), 4.27 (2H, q, J=7 Hz), 4.25 (2H, d, J=5 Hz), 3.64 (2H, d, J=12 Hz), 2.89 (2H, d, J=7 Hz), 2.69 (2H, t, J=12 Hz), 2.47 (3H, s), 2.11-2.04 (1H, m), 1.77 (2H, d, J=12 Hz), 1.53 (2H, dq, J=12 Hz, 3 Hz), 1.32 (3H, t, J=7 Hz), 1.29 (9H, s).

### (10) Ethyl {[(2-{[1-(4-tert-butylphenyl)piperidin-4-yl]methyl}-5-hydroxy-6-methylpyrimidin-4-yl)carbonyl]amino} acetate

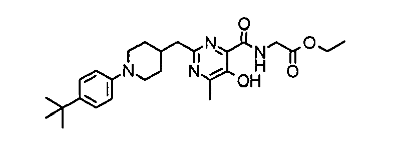

Ethyl ({[5-(benzyloxy)-2-{[1-(4-tert-butylphenyl)piperidin-4-yl]methyl} -6-methylpyrimidin-4-yl]carbonyl}amino)acetate (0.81 g, 1.5 mmol) was dissolved in ethyl acetate (20 mL), and 5% palladium-activated carbon (100 mg) was added, followed by stirring at room temperature for 2.5 hours under a hydrogen atmosphere. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.55 (hexane/ethyl acetate=2/1) by thin layer chromatography was concentrated under reduced pressure to afford the title compound (0.53 g, 1.1 mmol) as a pale yellow solid (yield 78%).
1H-NMR (500 MHz, CDCl3) δ: 11.35 (1H, s), 8.49 (1H, t, J=6 Hz), 7.26 (2H, d, J=9 Hz), 6.88 (2H, d, J=9 Hz), 4.28 (2H, q, J=7 Hz), 4.22 (2H, d, J=6 Hz), 3.64-3.61 (2H, m), 2.83-2.81 (2H, m), 2.70-2.64 (2H, m), 2.53 (3H, s), 2.05-1.94 (1H, m), 1.76-1.73 (2H, m), 1.54-1.48 (2H, m), 1.33 (3H, t, J=7 Hz), 1.28 (9H, s).

### (11) {[(2-{[1-(4-tert-Butylphenyl)piperidin-4-yl]methyl}-5-hydroxy-6-methylpyrimidin-4-yl)carbonyl]amino}acetic acid

Ethyl {[(2-{[1-(4-tert-butylphenyl)piperidin-4-yl]methyl}-5-hydroxy-6-methylpyrimidin-4-yl)carbonyl]amino}acetate (0.53 g, 1.1 mmol) was dissolved in a mixture of methanol (5 mL) and tetrahydrofuran (5 mL), and aqueous sodium hydroxide solution (1 M, 2.0 mL) was added, followed by stirring at room temperature for 1 hour. The reaction solution was concentrated under reduced pressure, and hydrochloric acid (1 M, 2.0 mL) was added to the resulting residue, whereby a solid was precipitated. The resulting solid was collected by filtration, and dried under reduced pressure to afford the title compound (0.33 g, 0.75 mmol) as a white solid (yield 68%).
mp 174-176 °C;
1H-NMR (500 MHz, DMSO-d6) δ: 12.86 (1H, s), 11.90 (1H, s), 9.38 (1H, t, J=6 Hz), 7.19 (2H, d, J=9 Hz), 6.84 (2H, d, J=9 Hz), 3.92 (2H, d, J=6 Hz), 3.59 (2H, d, J=12 Hz), 2.77 (2H, d, J=7 Hz), 2.59 (2H, t, J=12 Hz), 2.43 (3H, s), 2.06-2.01 (1H, m), 1.66 (2H, d, J=12 Hz), 1.32 (2H, dq, J=12 Hz, 3 Hz), 1.23 (9H, s).

### Example 2

### [({5-Hydroxy-6-methyl-2-[(1-phenylpiperidin-4-yl)methyl]pyrimidin-4-yl}carbonyl)amino]acetic acid

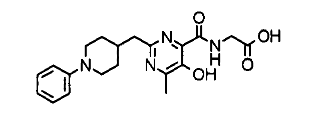

### (1) 1-Phenylpiperidin-4-one

1,4-Dioxa-8-azaspiro[4,5]-decane (5.0 g, 35 mmol), bromobenzene (7.4 mL, 70 mmol), sodium tert-butoxide (6.7 g, 70 mmol), 2-dicyclohexylphosphino-2',6'-dimethoxy-1,1'-biphenyl (S-PHOS) (1.4 g, 3.5 mmol) and tris(dibenzylideneacetone)dipalladium-chloroform adduct (1.8 g, 1.8 mmol) were dissolved in toluene (100 mL), followed by heating to reflux for 3 hours under a nitrogen atmosphere. Water was poured into the reaction solution, and organic matter was extracted with ethyl acetate. After the organic layer was concentrated under reduced pressure, the resulting residue was purified by silica gel column chromatography (Moritex Corporation, elution solvent: hexane/ethyl acetate), and a fraction corresponding to the Rf value=0.48 (hexane/ethyl acetate=5/1) by thin layer chromatography was concentrated under reduced pressure to afford (8-phenyl-1,4-dioxa-8-azaspiro[4,5]decane (7.7 g, 35 mmol) as a colorless oil (quantitative yield).

This was dissolved in ethanol (60 mL), and hydrochloric acid (2 M, 15 mL) was added, followed by heating to reflux for 5 hours. Aqueous sodium hydrogencarbonate solution was poured into the reaction solution, and organic matter was extracted with ethyl acetate. After the organic layer was concentrated under reduced pressure, the resulting residue was purified by silica gel column chromatography (Moritex Corporation, elution solvent: hexane/ethyl acetate), and a fraction corresponding to the Rf value=0.40 (hexane/ethyl acetate=5/1) by thin layer chromatography was concentrated under reduced pressure to afford the title compound (6.1 g, 35 mmol) as a colorless oil (quantitative yield).
MS m/z: 176 (M+H)⁺.

### (2) (1-Phenylpiperidin-4-yl)acetonitrile

In accordance with Examples 1-(1) and 1-(2), but using 1-phenylpiperidin-4-one (5.0 g, 29 mmol) instead of tert-butyl 4-oxopiperidine-1-carboxylate, the title compound (2.9 g, 14 mmol) was afforded as a yellow solid (yield 24%).
1H-NMR (400 MHz, CDCl3) δ: 7.26 (2H, t, J=8 Hz), 6.94 (2H, d, J=8 Hz), 6.86 (1H, t, J=8 Hz), 3.71 (2H, d, J=10 Hz), 2.74 (2H, dt, J=12 Hz, 2 Hz), 2.35 (2H, d, J=7 Hz), 1.94 (2H, d, J=13 Hz), 1.88-1.82 (1H, m), 1.55-1.46 (2H, m).

### (3) tert-Butyl 5-(benzyloxy)-6-hydroxy-2-[(1-phenylpiperidin-4-yl)methyl]pyrimidine-4-carboxylate

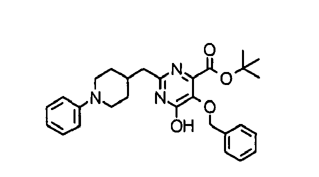

In accordance with Examples 1-(3) and 1-(4), but using (1-phenylpiperidin-4-yl)acetonitrile (2.9 g, 14 mmol) instead of tert-butyl 4-(cyanomethyl)piperidine-1-carboxylate, the title compound (4.2 g, 8.8 mmol) was afforded as a white solid (yield 76%).
1H-NMR (400 MHz, CDCl3) δ: 7.85 (2H, d, J=5 Hz), 7.51-7.47 (2H, m), 7.45 (2H, d, J=5 Hz), 7.34-7.29 (2H, m), 7.27 (2H, d, J=3 Hz), 5.26 (2H, s), 3.70 (2H, d, J=10 Hz), 3.33 (2H, t, J=12 Hz), 2.84 (2H, d, J=5 Hz), 2.65 (2H, d, J=11 Hz), 2.39 (1H, m), 1.98 (2H, d, J=12 Hz), 1.50 (9H, s).

### (4) tert-Butyl 5-(benzyloxy)-6-methyl-2-[(1-phenylpiperidin-4-yl)methyl]pyrimidine-4-carboxylate

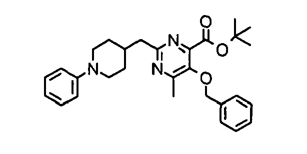

In accordance with Examples 1-(5) and 1-(6), but using tert-butyl 5-(benzyloxy)-6-hydroxy-2-[(1-phenylpiperidin-4-yl)methyl]pyrimidine-4-carboxylate (3.0 g, 6.3 mmol) instead of tert-butyl 5-(benzyloxy)-2-{[1-(tert-butoxycarbonyl)piperidin-4-yl]methyl}-6-hydroxypyrimidine-4-carboxylate, the title compound (1.5 g, 3.2 mmol) was afforded as a yellow amorphous solid (yield 50%).
1H-NMR (400 MHz, CDCl3) δ: 7.44-7.36 (5H, m), 7.24 (2H, dt, J=8 Hz, 7 Hz), 6.94 (2H, d, J=8 Hz), 6.82 (1H, t, J=7 Hz), 5.01 (2H, s), 3.67 (2H, d, J=13 Hz), 2.90 (2H, d, J=7 Hz), 2.71 (2H, dt, J=12 Hz, 2 Hz), 2.46 (3H, s), 2.10-2.07 (1H, m), 1.78 (2H, d, J=13 Hz), 1.59 (9H, s), 1.52 (2H, dq, 13 Hz, 3 Hz).

### (5) Ethyl [({5-(benzyloxy)-6-methyl-2-[(1-phenylpiperidin-4-yl)methyl]pyrimidin-4-yl}carbonyl)amino]acetate

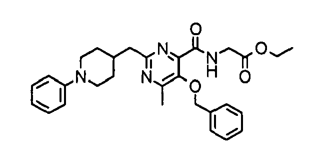

In accordance with Example 1-(9), but using tert-butyl 5-(benzyloxy)-6-methyl-2-[(1-phenylpiperidin-4-yl)methyl]pyrimidine-4-carboxylate (1.5 g, 3.2 mmol) instead of tert-butyl 5-(benzyloxy)-2-{[1-(4-tert-butylphenyl)piperidin-4-yl]methyl}-6-methylpyrimidine-4-carboxylate, the title compound (0.76 g, 1.5 mmol) was afforded as a yellow amorphous solid (yield 48%).
1H-NMR (400 MHz, CDCl3) δ: 8.36 (1H, t, J=6 Hz), 7.49 (2H, d, J=8 Hz), 7.41-7.34 (3H, m), 7.27-7.23 (2H, m), 6.95 (2H, d, J=8 Hz), 6.83 (1H, t, J=7 Hz), 5.12 (2H, s), 4.27 (2H, q, J=7 Hz), 4.25 (2H, d, J=6 Hz), 3.68 (2H, d, J=10 Hz), 2.90 (2H, d, J=7 Hz), 2.73 (2H, dt, J=13 Hz, 3 Hz), 2.46 (3H, s), 2.12-2.17 (1H, m), 1.79 (2H, d, J=13 Hz), 1.58-1.47 (2H, m), 1.32 (3H, t, J=7 Hz).

### (6) Ethyl [({5-hydroxy-6-methyl-2-[(1-phenylpiperidin-4-yl)methyl]pyrimidin-4-yl}carbonyl)amino] acetate

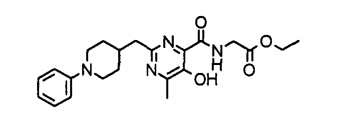

In accordance with Example 1-(10), but using ethyl [({5-(benzyloxy)-6-methyl-2-[(1-phenylpiperidin-4-yl)methyl]pyrimidin-4-yl}carbonyl)amino]acetate (760 mg, 1.5 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (460 mg, 1.1 mmol) was afforded as a solid (yield 74%).
1H-NMR (400 MHz, CDCl3) δ: 11.36 (1H, s), 8.49 (1H, t, J=6 Hz), 7.23 (2H, t, J=7 Hz), 6.94 (2H, d, J=8 Hz), 6.82 (1H, t, J=7 Hz), 4.28 (2H, q, J=7 Hz), 4.22 (2H, d, J=6 Hz), 3.67 (2H, d, J=13 Hz), 2.83 (2H, d, J=7 Hz), 2.71 (2H, dt, J=13 Hz, 3 Hz), 2.54 (3H, s), 2.07-2.01 (1H, m), 1.77 (2H, d, J=11 Hz), 1.55-1.45 (2H, m), 1.33 (3H, t, J=7 Hz).

### (7) [({5-Hydroxy-6-methyl-2-[(1-phenylpiperidin-4-yl)methyl]pyrimidin-4-yl}carbonyl)amino]acetic acid

In accordance with Example 1-(11), but using ethyl [({5-hydroxy-6-methyl-2-[(1-phenylpiperidin-4-yl)methyl]pyrimidin-4-yl}carbonyl)amino]acetate (460 mg, 1.1 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (350 mg, 0.92 mmol) was afforded as a white solid (yield 61%).
mp 141°C;
MS m/z: 385 (M+H)⁺;
Anal Calcd for C20H24N4O4: C, 62.49; H, 6.29; N, 14.57. Found: C, 62.32; H, 6.35; N, 14.36;
1H-NMR (400 MHz, CDCl3) δ: 11.58 (1H, brs), 10.53 (1H, brs), 8.60 (1H, s), 7.37-7.27 (2H, m), 7.21 (2H, d, J=8 Hz), 7.07 (1H, t, J=7 Hz), 4.21 (2H, d, J=5 Hz), 3.68 (2H, d, J=12 Hz), 2.87 (4H, d, J=8 Hz), 2.52 (3H, s), 2.09 (1H, m), 1.87-1.28 (4H, m).

### Example 3

### {[(2-{[1-(2-Fluorophenyl)piperidin-4-yl]methyl}-5-hydroxy-6-methylpyrimidin-4-yl)carbonyl]amino}acetic acid

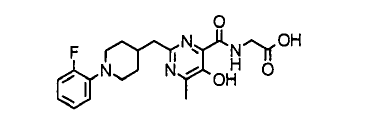

In accordance with Examples 1-(8) to 1-(11), but using 1-fluoro-2-iodobenzene (0.51 g, 2.3 mmol) instead of 1-bromo-4-tert-butylbenzene, the title compound (0.054 g, 0.26 mmol) was afforded as a white solid (yield 12%).
MS m/z: 403 (M+H)⁺;
1H-NMR (500 MHz, DMSO-d6) δ: 12.91 (1H, brs), 11.93 (1H, brs), 9.37 (1H, t, J=8 Hz), 7.14-7.05 (2H, m), 7.02 (1H, t, J=8 Hz), 6.95-6.89 (1H, m), 3.98 (2H, d, J=8 Hz), 3.35-3.30 (2H, m), 2.80 (2H, d, J=8 Hz), 2.64 (2H, t, J=10 Hz), 2.44 (3H, s), 2.19-2.02 (1H, m), 1.69 (2H, d, J=11 Hz), 1.42 (2H, dq, J=11 Hz, 3 Hz).

### Example 4

### {[(2-{[1-(3-Fluorophenyl)piperidin-4-yl]methyl}-5-hydroxy-6-methylpyrimidin-4-yl)carbonyl]amino}acetic acid

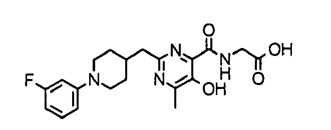

In accordance with Examples 1-(8) to 1-(11), but using 1-bromo-3-fluorobenzene (0.40 g, 2.3 mmol) instead of 1-bromo-4-tert-butylbenzene, the title compound (0.15 g, 0.37 mmol) was afforded as a pale yellowish white solid (yield 32%).
MS m/z: 403 (M+H)⁺;
1H-NMR (500 MHz, DMSO-d6) δ: 12.88 (1H, brs), 11.90 (1H, s), 9.39 (1H, t, J=6 Hz), 7.18 (1H, q, J=8 Hz), 6.76-6.66 (2H, m), 6.48 (1H, dt, J=8 Hz, 2 Hz), 4.01 (2H, d, J=6 Hz), 3.71 (2H, d, J=12 Hz), 2.77 (2H, d, J=7 Hz), 2.69 (2H, dt, J=12 Hz, 3 Hz), 2.44 (3H, s), 2.14-2.04 (1H, m), 1.65 (2H, d, J=12 Hz), 1.32 (2H, dq, J=12 Hz, 3 Hz).

### Example 5

### {[(2-{[1-(4-Fluorophenyl)piperidin-4-yl]methyl}-5-hydroxy-6-methylpyrimidin-4-yl)carbonyl]amino}acetic acid

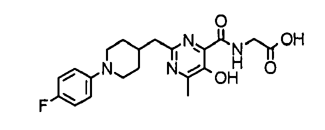

### (1) 1-(4-Fluorophenyl)piperidin-4-one

In accordance with Example 2-(1), but using 1-bromo-4-fluorobenzene (6.6 mL, 60 mmol) instead of bromobenzene, the title compound (3.7 g, 19 mmol) was afforded as an oil (yield 65%).
1H-NMR (500 MHz, CDCl3) δ: 7.02-6.98 (2H, m), 6.96-6.93 (2H, m), 3.51 (4H, t, J=6 Hz), 2.58 (4H, t, J=6 Hz).

### (2) 2-[1-(4-Fluorophenyl)piperidin-4-yl]ethanimidamide acetic acid salt

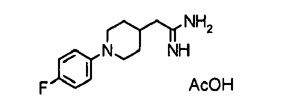

In accordance with Examples 1-(1) to 1-(3), but using 1-(4-fluorophenyl)piperidin-4-one (9.8 g, 51 mmol) instead of tert-butyl 4-oxopiperidine-1-carboxylate, the title compound (10 g, 35 mmol) was afforded as a white solid (yield 71%).
1H-NMR (500 MHz, CD3OD) δ: 7.01-6.95 (4H, m), 3.59 (2H, d, J=8 Hz), 2.67 (2H, t, J=8 Hz), 2.42 (2H, d, J=4 Hz), 1.90 (3H, s), 1.86 (2H, d, J=8 Hz), 1.85-1.83 (1H, m), 1.50-1.44 (2H, m).

### (3) Ethyl ({[5-(benzyloxy)-2-{[1-(4-fluorophenyl)piperidin-4-yl]methyl}-6-{[(trifluoromethyl)sulfonyl]oxy}pyrimidin-4-yl]carbonyl}amino)acetate

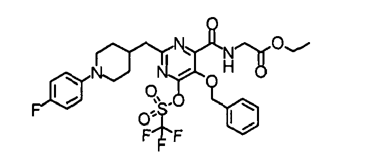

In accordance with Examples 1-(4), 1-(9) and 1-(5), but using 2-[1-(4-fluorophenyl)piperidin-4-yl]ethanimidamide acetic acid salt (10 g, 35 mmol) instead of tert-butyl 4-(2-amino-2-iminoethyl)piperidine-1-carboxylate acetic acid salt, the title compound (2.3 g, 3.5 mmol) was afforded (yield 49%).
1H-NMR (500 MHz, CDCl3) δ: 8.14 (1H, t, J=4 Hz), 7.51-7.49 (2H, m), 7.39-7.35 (3H, m), 6.96-6.93 (2H, m), 6.90-6.87 (2H, m), 5.25 (2H, s), 4.28 (2H, q, J=8 Hz), 4.23 (2H, d, J=4 Hz), 3.53 (2H, d, J=12 Hz), 2.92 (2H, d, J=4 Hz), 2.67 (2H, t, J=8 Hz), 2.07-2.02 (1H, m), 1.77 (2H, d, J=8 Hz), 1.51-1.44 (2H, m), 1.33 (3H, t, J=8 Hz).

### (4) Ethyl ({[5-(benzyloxy)-2-{[1-(4-fluorophenyl)piperidin-4-yl]methyl}-6-methylpyrimidin-4-yl]carbonyl}amino)acetate

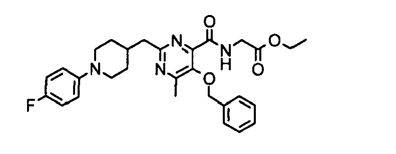

In accordance with Example 1-(6), but using ethyl ({[5-(benzyloxy)-2-{[1-(4-fluorophenyl)piperidin-4-yl]methyl}-6-{[(trifluoromethyl)sulfonyl]oxy}pyrimidin-4-yl]carbonyl}amino)acetate (0.98 g, 1.5 mmol) instead of tert-butyl 5-(benzyloxy)-2-{[1-(tert-butoxycarbonyl)piperidin-4-yl]methyl} -6-{[(trifluoromethyl)sulfonyl]oxy}pyrimidine-4-carboxylate, the title compound (0.54 g, 1.0 mmol) was afforded (yield 69%).
1H-NMR (500 MHz, CDCl3) δ: 8.34 (1H, t, J=4 Hz), 7.48 (2H, d, J=8 Hz), 7.40-7.33 (3H, m), 6.96-6.87 (4H, m), 5.12 (2H, s), 4.26 (2H, q, J=8 Hz), 4.24 (2H, d, J=4 Hz), 3.54 (2H, d, J=8 Hz), 2.89 (2H, d, J=4 Hz), 2.68 (2H, dt, J=12 Hz, 4 Hz), 2.46 (3H, s), 2.12-2.05 (1H, m), 1.78 (2H, d, J=8 Hz), 1.60-1.49 (2H, m), 1.32 (3H, t, J=8 Hz).

### (5) {[(2-{[1-(4-Fluorophenyl)piperidin-4-yl]methyl}-5-hydroxy-6-methylpyrimidin-4-yl)carbonyl]amino} acetic acid

In accordance with Examples 1-(10) and 1-(11), but using ethyl ({[5-(benzyloxy)-2- {[1-(4-fluorophenyl)piperidin-4-yl]methyl -6-methylpyrimidin-4-yl]carbonyl}amino)acetate (0.54 g, 1.0 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.085 g, 0.21 mmol) was afforded as a white solid (yield 21%).
mp 104-105 °C;
1H-NMR (400 MHz, DMSO-d6) δ: 9.38 (1H, m), 7.03-7.00 (2H, m), 6.94-6.91 (2H, m), 3.99 (2H, m), 3.56 (2H, d, J=12 Hz), 2.77 (2H, d, J=4 Hz), 2.60 (2H, dt, J=8 Hz, 4 Hz), 2.44 (3H, s), 2.08-1.98 (1H, m), 1.68 (2H, d, J=12 Hz), 1.41-1.31 (2H, m).

### Example 6

### {[(2-[1-(2-Chlorophenyl)piperidin-4-yl]methyl -5 -hydroxy-6-methylpyrimidin-4-yl)carbonyl]amino}acetic acid

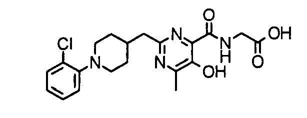

### (1) Ethyl ({[5-(benzyloxy)-2-{[1-(2-chlorophenyl)piperidin-4-yl]methyl}-6-methylpyrimidin-4-yl]carbonyl}amino)acetate

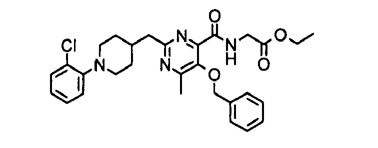

In accordance with Examples 1-(8) and 1-(9), but using 1-bromo-2-chlorobenzene (0.44 g, 2.3 mmol) instead of 1-bromo-4-tert-butylbenzene, the title compound (0.27 g, 0.50 mmol) was afforded as a yellow oil (yield 43%).
1H-NMR (500 MHz, CDCl3) δ: 8.37 (1H, t, J=6 Hz), 7.49 (2H, d, J=7 Hz), 7.41-7.32 (4H, m), 7.20 (1H, t, J=8 Hz), 7.04 (1H, d, J=8 Hz), 6.94 (1H, t, J=8 Hz), 5.12 (2H, s), 4.26 (2H, q, J=7 Hz), 4.25 (2H, d, J=6 Hz), 3.39 (2H, d, J=12 Hz), 2.92 (2H, d, J=7 Hz), 2.66 (2H, dt, J=12 Hz, 3 Hz), 2.46 (3H, s), 2.14-2.04 (1H, m), 1.76 (2H, d, J=12 Hz), 1.62 (2H, dq, J=12 Hz, 3 Hz), 1.32 (3H, t, J=7 Hz).

### (2) Ethyl {[(2-{[1-(2-chlorophenyl)piperidin-4-yl]methyl-5-hydroxy-6-methylpyrimidm-4-yl)carbonyl]amino} acetate

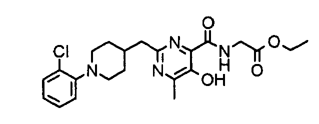

Ethyl ({[5-(benzyloxy)-2-{[1-(2-chlorophenyl)piperidin-4-yl]methyl}-6-methylpyrimidin-4-yl]carbonyl}amino)acetate (0.27 g, 0.50 mmol) was dissolved in methylene chloride (3 mL), and trifluoroacetic acid (3 mL) was added, followed by stirring at room temperature for 4 hours. After the reaction solution was concentrated under reduced pressure, the resulting residue was purified by silica gel column chromatography (Moritex Corporation, elution solvent: hexane/ethyl acetate), and a fraction corresponding to the Rf value=0.55 (hexane/ethyl acetate=2/1) by thin layer chromatography was concentrated under reduced pressure to afford the title compound (0.19 g, 0.41 mmol) as a yellow solid (yield 84%).
1H-NMR (500 MHz, CDC13) δ: 11.35 (1H, s), 8.50 (1H, t, J=5 Hz), 7.35 (1H, d, J=8 Hz), 7.20 (1H, t, J=8 Hz), 7.03 (1H, d, J=8 Hz), 6.94 (1H, t, J=8 Hz), 4.29 (2H, q, J=7 Hz), 4.23 (2H, d, J=5 Hz), 3.38 (2H, d, J=12 Hz), 2.86 (2H, d, J=7Hz), 2.64 (2H, dt, J=12 Hz, 3 Hz), 2.54 (3H, s), 2.09-1.99 (1H, m), 1.75 (2H, d, J=12 Hz), 1.62 (2H, dq, J=12 Hz, 3 Hz), 1.33 (3H, t, J=7 Hz).

### (3) {[(2-{[1-(2-Chlorophenyl)piperidin-4-yl]methyl}-5-hydroxy-6-methylpyrimidin-4-yl)carbonyl]amino} acetic acid

In accordance with Example 1-(11), but using ethyl {[(2-{[1-(2-chlorophenyl)piperidin-4-yl]methyl}-5-hydroxy-6-methylpyrimidin-4-yl)carbonyl]amino} acetate (0.19 g, 0.41 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.15 g, 0.36 mmol) was afforded as a yellow solid (yield 88%).
MS m/z: 419 (M+H)⁺;
1H-NMR (500 MHz, DMSO-d6) δ: 12.87 (1H, brs), 11.90 (1H, s), 9.40 (1H, t, J=6 Hz), 7.38 (1H, d, J=8 Hz), 7.27 (1H, t, J=8 Hz), 7.13 (1H, d, J=8 Hz), 7.00 (1H, t, J=8 Hz), 4.01 (2H, d, J=6 Hz), 3.25 (2H, d, J=12 Hz), 2.81 (2H, d, J=7 Hz), 2.62 (2H, t, J=12 Hz), 2.44 (3H, s), 2.10-2.00 (1H, m), 1.69 (2H, d, J=12 Hz), 1.44 (2H, dq, J=12 Hz, 3 Hz).

### Example 7

### {[(2-{[1-(3-Chlorophenyl)piperidin-4-yl]methyl}-5-hydroxy-6-methylpyrimidin-4-yl)carbonyl]amino}acetic acid

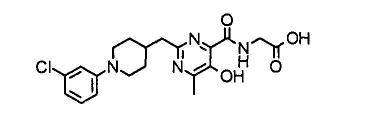

In accordance with Examples 1-(8), 1-(9), 6-(2) and 1-(11), but using 1-bromo-3-chlorobenzene (0.44 g, 2.3 mmol) instead of 1-bromo-4-tert-butylbenzene, the title compound (0.11 g, 0.26 mmol) was afforded as a yellow solid (yield 23%).
MS m/z: 419 (M+H)⁺;
1H-NMR (500 MHz, DMSO-d6) δ: 12.89 (1H, brs), 11.92 (1H, brs), 9.38 (1H, t, J=6 Hz), 7.17 (1H, t, J=8 Hz), 6.92-6.85 (2H, m), 6.73 (1H, d, J=8 Hz), 4.00 (2H, d, J=6 Hz), 3.71 (2H, d, J=12 Hz), 2.77 (2H, d, J=7 Hz), 2.69 (2H, dt, J=12 Hz, 3 Hz), 2.44 (3H, s), 2.14-2.04 (1H, m), 1.66 (2H, d, J=12 Hz), 1.32 (2H, dq, J=12 Hz, 3 Hz).

### Example 8

### {[(2-{[1-(4-Chlorophenyl)piperidin-4-yl]methyl -5-hydroxy-6-methylpyrimidin-4-yl)carbonyl]amino}acetic acid

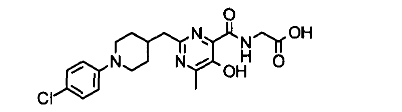

### (1) Ethyl ({[5-(benzyloxy)-2-{[1-(4-chlorophenyl)piperidin-4-yl]methyl}-6-methylpyrimidin-4-yl]carbonyl}amino)acetate

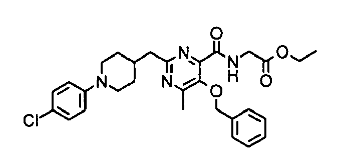

In accordance with Examples 1-(8) and 1-(9), but using 1-bromo-4-chlorobenzene (0.28 g, 1.5 mmol) instead of 1-bromo-4-tert-butylbenzene, the title compound (0.30 g, 0.56 mmol) was afforded as a yellow oil (yield 75%).
1H-NMR (500 MHz, CDCl3) δ: 8.34 (1H, t, J=5 Hz), 7.49 (2H, d, J=8 Hz), 7.41-7.34 (3H, m), 7.18 (2H, d, J=9 Hz), 6.85 (2H, d, J=9 Hz), 5.12 (2H, s), 4.27 (2H, q, J=7 Hz), 4.25 (2H, d, J=5 Hz), 3.62 (2H, d, J=12 Hz), 2.89 (2H, d, J=7 Hz), 2.71 (2H, dt, J=12 Hz, 3 Hz), 2.46 (3H, s), 2.14-2.04 (1H, m), 1.76 (2H, d, J=12 Hz), 1.50 (2H, dq, J=12 Hz, 3 Hz), 1.32 (3H, t, J=7 Hz).

### (2) Ethyl {[(2-{[1-(4-chlorophenyl)piperidin-4-yl]methyl}-5-hydroxy-6-methylpyrimidin-4-yl)carbonyl]amino}acetate

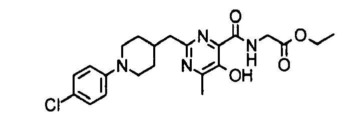

Ethyl ({[5-(benzyloxy)-2-{[1-(4-chlorophenyl)piperidin-4-yl]methyl}-6-methylpyrimidin-4-yl]carbonyl}amino)acetate (0.30 g, 0.56 mmol) was dissolved in ethanol (30 mL), and concentrated hydrochloric acid (0.99 mL) was added at room temperature, followed by stirring at 90°C for 4.5 hours. After the reaction solution was neutralized with aqueous sodium hydroxide solution (1 M, 10 mL), the solvent was distilled off under reduced pressure, and subsequently the resulting residue was extracted with methylene chloride. The extract was concentrated under reduced pressure to afford the title compound (0.24 g, 0.54 mmol) as an orange amorphous solid (yield 96%).
1H-NMR (500 MHz, CDCl3) δ: 11.37 (1H, s), 8.48 (1H, t, J=6 Hz), 7.19 (1H, d, J=9 Hz), 6.99 (2H, d, J=9 Hz), 4.29 (2H, q, J=7 Hz), 4.22 (2H, d, J=6 Hz), 3.61 (2H, d, J=12 Hz), 2.84 (2H, d, J=7 Hz), 2.75 (2H, t, J=12 Hz), 2.54 (3H, s), 2.09-1.99 (1H, m), 1.77 (2H, d, J=12 Hz), 1.62 (2H, dq, J=12 Hz, 3 Hz), 1.33 (3H, t, J=7 Hz).

### (3) {[(2-{[1-(4-Chlorophenyl)piperidin-4-yl]methyl}-5-hydroxy-6-methylpyrimidin-4-yl)carbonyl]amino}acetic acid

In accordance with Example 1-(11), but using ethyl {[(2-{[1-(4-chlorophenyl)piperidin-4-yl]methyl}-5-hydroxy-6-methylpyrimidin-4-yl)carbonyl]amino}acetate (0.24 g, 0.54 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.09 g, 0.20 mmol) was afforded as a pale yellowish white solid (yield 37%).
MS m/z: 419 (M+H)⁺;
1H-NMR (500 MHz, DMSO-d6) δ: 12.87 (1H, brs), 11.90 (1H, s), 9.38 (1H, t, J=6 Hz), 7.19 (2H, d, J=9 Hz), 6.92 (2H, d, J=9 Hz), 4.01 (2H, d, J=6 Hz), 3.65 (2H, d, J=12 Hz), 2.77 (2H, d, J=7 Hz), 2.66 (2H, dt, J=12 Hz, 3 Hz), 2.44 (3H, s), 2.13-2.02 (1H, m), 1.66 (2H, d, J=12 Hz), 1.33 (2H, dq, J=12 Hz, 3 Hz).

### Example 9

### {[(5-Hydroxy-6-methyl-2-{[1-(2-methylphenyl)piperidin-4-yl]methyl}pyrimidin-4-yl)carbonyl]amino}acetic acid

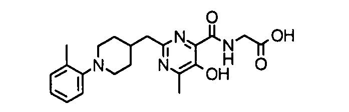

In accordance with Examples 1-(8) to 1-(11), but using 1-bromo-2-methylbenzene (0.40 g, 2.3 mmol) instead of 1-bromo-4-tert-butylbenzene, the title compound (0.18 g, 0.44 mmol) was afforded as a pale yellowish white solid (yield 38%).
MS m/z: 399 (M+H)⁺;
1H-NMR (400 MHz, DMSO-d6) δ: 9.38 (1H, t, J=6 Hz), 7.14-7.10 (2H, m), 6.99 (1H, d, J=8 Hz), 6.92 (1H, t, J=8 Hz), 3.97 (2H, d, J=6 Hz), 3.02 (2H, d, J=12 Hz), 2.80 (2H, d, J=7 Hz), 2.57 (2H, t, J=12 Hz), 2.44 (3H, s), 2.22 (3H, s), 2.09-1.97 (1H, m), 1.67 (2H, d, J=12 Hz), 1.46-1.40 (2H, m).

### Example 10

### {[(5-Hydroxy-6-methyl-2-{[1-(3-methylphenyl)piperidin-4-yl]methyl}pyrimidin-4-yl)carbonyl]amino} acetic acid

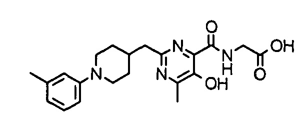

In accordance with Examples 1-(8) to 1-(11), but using 1-promo-3-methylbenzene (0.40 g, 2.3 mmol) instead of 1-bromo-4-tert-butylbenzene, the title compound (0.20 g, 0.50 mmol) was afforded as a white solid (yield 43%).
MS m/z: 399 (M+H)⁺;
1H-NMR (400 MHz, DMSO-d6) δ: 9.03 (1H, t, J=7 Hz), 7.05 (1H, t, J=8 Hz), 6.73 (1H, s), 6.70 (1H, d, J=8 Hz), 6.55 (1H, d, J=8 Hz), 3.64 (2H, d, J=7 Hz), 3.60 (2H, brs), 2.75 (2H, d, J=7 Hz), 2.61 (2H, t, J=10 Hz), 2.42 (3H, s), 2.23 (3H, s), 2.13-1.93 (1H, m), 1.68 (2H, d, J=11 Hz), 1.43-1.24 (2H, m).

### Example 11

### {[(5-Hydroxy-6-methyl-2-{[1-(4-methylphenyl)piperidin-4-yl]methyl}pyrimidin-4-yl)carbonyl]amino}acetic acid

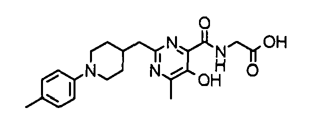

In accordance with Examples 1-(8) to 1-(11), but using 1-bromo-4-methylbenzene (0.23 g, 1.4 mmol) instead of 1-bromo-4-tert-butylbenzene, the title compound (0.08 g, 0.20 mmol) was afforded as a yellow solid (yield 29%).
MS m/z: 399 (M+H)⁺;
1H-NMR (500 MHz, CDC13) δ: 11.74 (1H, s), 8.62 (1H, brs), 7.20 (2H, d, J=8 Hz), 7.15 (2H, d, J=8 Hz), 4.19 (2H, brs), 3.61 (2H, d, J=11 Hz), 2.94-2.85 (4H, m), 2.52 (3H, s), 2.31 (3H, s), 2.06-2.00 (1H, m), 1.93-1.85 (4H, m).

### Example 12

### {[(5-Hydroxy-6-methyl-2-{[1-(4-propylphenyl)piperidin-4-yl]methyl}pyrimidin-4-yl)carbonyl]amino} acetic acid and {[(2-{[1-(4-cyclopropylphenyl)piperidin-4-yl]methyl}-5-hydroxy-6-methylpyrimidin-4-yl)carbonyl]amino} ascetic acid

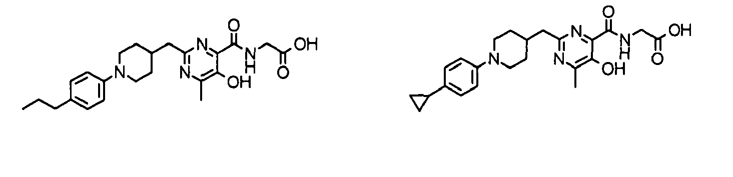

### (1) Ethyl ({[5-(benzyloxy)-2-{[1-(4-cyclopropylphenyl)piperidin-4-yl]methyl}-6-methylpyrimidin-4-yl]carbonyl}amino)acetate

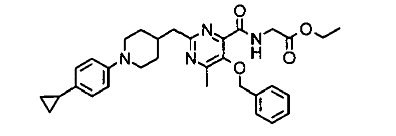

In accordance with Examples 1-(8) and 1-(9), but using 1-bromo-4-cyclopropylbenzene (1.0 g, 5.1 mmol) instead of 1-bromo-4-tert-butylbenzene, the title compound (1.3 g, 2.4 mmol) was afforded as a yellow oil (yield 58%).
1H-NMR (500 MHz, CDCl3) δ: 8.36 (1H, t, J=5 Hz), 7.49 (2H, d, J=6 Hz), 7.42-7.32 (3H, m), 6.98 (2H, d, J=9 Hz), 6.86 (2H, d, J=9 Hz), 5.12 (2H, s), 4.27 (2H, q, J=7 Hz), 4.25 (2H, d, J=5 Hz), 3.60 (2H, d, J=12 Hz), 2.89 (2H, d, J=7 Hz), 2.67 (2H, dt, J=12 Hz, 3 Hz), 2.46 (3H, s), 2.10-2.01 (1H, m), 1.86-1.79 (1H, m), 1.76 (2H, d, J=12 Hz), 1.53 (2H, dq, J=12 Hz, 3 Hz), 1.32 (3H, t, J=7 Hz), 0.90-0.85 (2H, m), 0.63-0.59 (2H, m).

### (2) {[(5-Hydroxy-6-methyl-2-{[1-(4-propylphenyl)piperidin-4-yl]methyl}pyrimidin-4-yl)carbonyl] amino}acetic acid and {[(2-{[1-(4-cyclopropylphenyl)piperidin-4-yl]methyl}-5-hydroxy-6-methylpyrimidin-4-yl)carbonyl]amino}acetic acid

Ethyl ({[5-(benzyloxy)-2-{[1-(4-cyclopropylphenyl)piperidin-4-yl]methyl}-6-methylpyrimidin-4-yl]carbonyl}amino)acetate (1.3 g, 2.4 mmol) was dissolved in ethyl acetate (40 mL), and 10% palladium-activated carbon (0.20 g) was added, followed by stirring at room temperature for 2 hours under a hydrogen atmosphere. The reaction solution was filtered with celite, and the filtrate was concentrated under reduced pressure. The resulting pale yellowish white solid was collected by filtration using (hexane/ethyl acetate=4/1) to afford a mixture (0.87 g, 1.9 mmol) of ethyl {[(5-hydroxy-6-methyl-2- {[1-(4-propylphenyl)piperidin-4-yl]methyl}pyrimidin-4-yl)carbonyl]amino}acetate and ethyl {[(2-{[1-(4-cyclopropylphenyl)piperidin-4-yl]methyl}-5-hydroxy-6-methylpyrimidin-4-yl)carbonyl]amino}acetate as a white solid (yield 80%).

This mixture (0.76 g, 1.7 mmol) was dissolved in ethanol (25 mL), and aqueous sodium hydroxide solution (1 M, 20 mL) was added, followed by stirring at room temperature for 30 minutes. The reaction solution was concentrated under reduced pressure, and hydrochloric acid (2 M, 10 mL) was added to the resulting residue, whereby a solid was precipitated. This was purified by preparative HPLC (YMC-Pack ODS-A; YMC, elution solvent: acetonitrile/water=45/55) to afford {[(5-hydroxy-6-methyl-2-{[1-(4-propylphenyl)piperidin-4-yl]methyl}pyrimidin-4-yl)carbonyl]amino}acetic acid (0.017 g, 0.04 mmol, yield 2%) as a pale yellowish white solid, and {[(2-{[1-(4-cyclopropylphenyl)piperidin-4-yl]methyl}-5-hydroxy-6-methylpyrimidin-4-yl)carbonyl]amino}acetic acid (0.12 g, 0.27 mmol, yield 16%) as a pale yellowish white solid.
Low polarity compound: {[(5-hydroxy-6-methyl-2-{[1-(4-propylphenyl)piperidin-4-yl]methyl}pyrimidin-4-yl)carbonyl]amino}acetic acid
MS m/z: 427 (M+H)⁺;
1H-NMR (500 MHz, DMSO-d6) δ: 12.89 (1H, brs), 11.91 (1H, s), 9.41 (1H, t, J=5 Hz), 7.00 (2H, dd, J=8 Hz, 2 Hz), 6.83 (2H, dd, J=8 Hz, 2 Hz), 4.00 (2H, d, J=5 Hz), 3.58 (2H, d, J=12 Hz), 2.77 (2H, d, J=6 Hz), 2.58 (2H, t, J=12 Hz), 2.44 (3H, s), 2.42 (2H, t, J=7 Hz), 2.08-1.98 (1H, m), 1.66 (2H, d, J=12 Hz), 1.56-1.47 (2H, m), 1.35 (2H, q, J=12 Hz), 0.86 (3H, dt, J=7 Hz, 2 Hz).
High polarity compound: {[(2-{[1-(4-cyclopropylphenyl)piperidin-4-yl]methyl}-5-hydroxy-6-methylpyrimidin-4-yl)carbonyl]amino}acetic acid
MS m/z: 425 (M+H)⁺;
1H-NMR (500 MHz, DMSO-d6) δ: 12.92 (1H, brs), 11.93 (1H, s), 9.40 (1H, t, J=5 Hz), 6.91 (2H, dd, J=9 Hz, 1 Hz), 6.81 (2H, dd, J=9 Hz, 1 Hz), 3.99 (2H, d, J=5 Hz), 3.57 (2H, d, J=12 Hz), 2.77 (2H, d, J=7 Hz), 2.57 (2H, t, J=12 Hz), 2.44 (3H, s), 2.08-1.98 (1H, m), 1.83-1.76 (1H, m), 1.65 (2H, d, J=12 Hz), 1.35 (2H, dq, J=12 Hz, 3 Hz), 0.86-0.81 (2H, m), 0.55-0.51 (2H, m).

### Example 13

### {[(5-Hydroxy-2-{[1-(4-isopropylphenyl)piperidin-4-yl]methyl}-6-methylpyrimidin-4-yl)carbonyl]amino}acetic acid

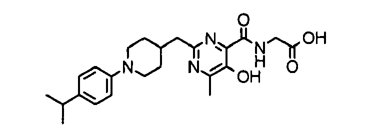

In accordance with Examples 1-(8), 1-(9), 6-(2) and 1-(11), but using 1-iodo-4-isopropylbenzene (3.4 g, 13.3 mmol) instead of 1-bromo-4-tert-butylbenzene, the title compound (1.6 g, 3.8 mmol) was afforded as a pale yellowish white solid (yield 54%).
MS m/z: 427 (M+H)⁺;
1H-NMR (500 MHz, DMSO-d6) δ: 12.87 (1H, brs), 11.90 (1H, s), 9.38 (1H, t, J=6 Hz), 7.05 (2H, d, J=8 Hz), 6.84 (2H, d, J=8 Hz), 4.01 (2H, d, J=6 Hz), 3.58 (2H, d, J=12 Hz), 2.80-2.73 (1H, m), 2.77 (2H, d, J=7 Hz), 2.59 (2H, t, J=12 Hz), 2.44 (3H, s), 2.08-1.98 (1H, m), 1.66 (2H, d, J=12 Hz), 1.35 (2H, dq, J=12 Hz, 3 Hz), 1.15 (6H, d, J=7 Hz).

### Example 14

### {[(2-{[1-(4-n-Butylphenyl)piperidin-4-yl]methyl}-5-hydroxy-6-methylpyrimidin-4-yl)carbonyl]amino} acetic acid

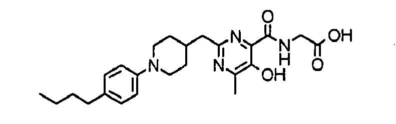

In accordance with Examples 1-(8) to 1-(11), but using 1-bromo-4-n-butylbenzene (0.50 g, 2.3 mmol) instead of 1-bromo-4-tert-butylbenzene, the title compound (0.13 g, 0.30 mmol) was afforded as a pale yellowish white solid (yield 26%).
MS m/z: 441 (M+H)⁺;
1H-NMR (500 MHz, DMSO-d6) δ:12.89 (1H, brs), 11.91 (1H, s), 9.41 (1H, t, J=6 Hz), 7.00 (2H, d, J=8 Hz), 6.82 (2H, d, J=8 Hz), 4.00 (2H, d, J=6 Hz), 3.58 (2H, d, J=12 Hz), 2.77 (2H, d, J=7 Hz), 2.58 (2H, t, J=12 Hz), 2.45 (2H, t, J=7 Hz), 2.44 (3H, s), 2.09-1.98 (1H, m), 1.66 (2H, d, J=12 Hz), 1.52-1.44 (2H, m), 1.35 (2H, dq, J=12 Hz, 3 Hz), 1.33-1.23 (2H, m), 0.87 (3H, t, J=7 Hz).

### Example 15

### {[(2-{[1-(3-tert-Butylphenyl)piperidin-4-yl]methyl}-5-hydroxy-6-methylpyrimidin-4-yl)carbonyl]amino}acetic acid

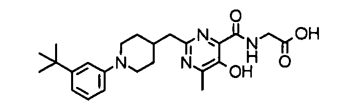

In accordance with Examples 1-(8) to 1-(11), but using 1-bromo-3-tert-butylbenzene (0.49 g, 2.3 mmol) instead of 1-bromo-4-tert-butylbenzene, the title compound (0.19 g, 0.43 mmol) was afforded as a pale yellowish white solid (yield 38%).
MS m/z: 441 (M+H)⁺;
1H-NMR (500 MHz, DMSO-d6) δ: 12.87 (1H, brs), 11.90 (1H, s), 9.39 (1H, t, J=6 Hz), 7.10 (1H, t, J=8 Hz), 6.91 (1H, s), 6.79 (1H, d, J=8 Hz), 6.72 (1H, dd, J=8 Hz, 2 Hz), 4.01 (2H, d, J=6 Hz), 3.62 (2H, d, J=12 Hz), 2.78 (2H, d, J=7 Hz), 2.63 (2H, t, J=12 Hz), 2.44 (3H, s), 2.10-1.99 (1H, m), 1.68 (2H, d, J=12 Hz), 1.37 (2H, dq, J=12 Hz, 3 Hz), 1.25 (9H, s).

### Example 16

### {[(2-{[1-(4-n-Hexylphenyl)piperidin-4-yl]methyl}-5-hydroxy-6-methylpyrimidin-4-yl)carbonyl]amino} acetic acid

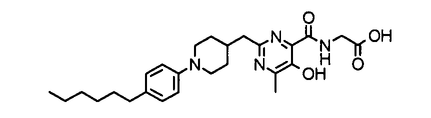

In accordance with Examples 1-(8) to 1-(11), but using 1-bromo-4-n-hexylbenzene (0.42 g, 1.8 mmol) instead of 1-bromo-4-tert-butylbenzene, the title compound (0.17 g, 0.36 mmol) was afforded as a pale yellowish white solid (yield 32%).
MS m/z: 469 (M+H)⁺;
1H-NMR (500 MHz, DMSO-d6) δ: 12.89 (1H, brs), 11.91 (1H, s), 9.40 (1H, t, J=6 Hz), 7.00 (2H, d, J=8 Hz), 6.82 (2H, d, J=8 Hz), 4.01 (2H, d, J=6 Hz), 3.58 (2H, d, J=12 Hz), 2.77 (2H, d, J=7 Hz), 2.58 (2H, t, J=12 Hz), 2.45 (2H, t, J=7 Hz), 2.44 (3H, s), 2.08-1.98 (1H, m), 1.66 (2H, d, J=12 Hz), 1.53-1.46 (2H, m), 1.35 (2H, dq, J=12 Hz, 3 Hz), 1.25 (6H, brs), 0.85 (3H, t, J=7 Hz).

### Example 17

### {[(2-{[1-(4-Cyclohexylphenyl)piperidin-4-yl]methyl -5-hydroxy-6-methylpyrimidin-4-yl)carbonyl]amino} acetic acid

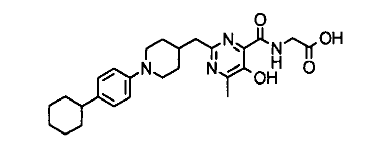

In accordance with Examples 1-(8) to 1-(11), but using 1-bromo-4-cyclohexylbenzene (0.55 g, 2.3 mmol) instead of 1-bromo-4-tert-butylbenzene, the title compound (0.19 g, 0.41 mmol) was afforded as a pale yellow solid (yield 35%).
MS m/z: 467 (M+H)⁺;
1H-NMR (500 MHz, DMSO-d6) δ: 12.90 (1H, brs), 11.91 (1H, s), 9.41 (1H, t, J=6 Hz), 7.03 (2H, d, J=9 Hz), 6.83 (2H, d, J=9 Hz), 4.00 (2H, d, J=6 Hz), 3.58 (2H, d, J=12 Hz), 2.77 (2H, d, J=7 Hz), 2.59 (2H, t, J=12 Hz), 2.44 (3H, s), 2.43-2.32 (1H, m), 2.10-1.97 (1H, m), 1.81-1.62 (6H, m), 1.42-1.14 (8H, m).

### Example 18

### ({[2-({1-[4-(1-Cyclohexen-1-yl)phenyl]piperidin-4-yl}methyl)-5-hydroxy-6-methylpyrimidin-4-yl]carbonyl}amino)acetic acid

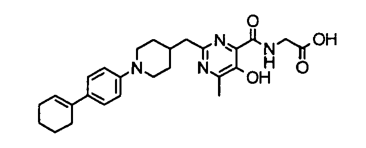

In accordance with Examples 1-(8), 1-(9), 6-(2) and 1-(11), but using 1-bromo-4-(1-cyclohexen-1-yl)benzene (0.33 g, 1.4 mmol) instead of 1-bromo-4-tert-butylbenzene, the title compound (0.22 g, 0.47 mmol) was afforded as a pink solid (yield 41%).
MS m/z: 465 (M+H)⁺;
1H-NMR (500 MHz, DMSO-d6) δ: 12.91 (1H, brs), 11.92 (1H, s), 9.42 (1H, t, J=6 Hz), 7.24 (2H, d, J=7 Hz), 6.86 (2H, d, J=7 Hz), 6.03-5.98 (1H, m), 4.01 (2H, d, J=6 Hz), 3.65 (2H, d, J=12 Hz), 2.77 (2H, d, J=7 Hz), 2.69-2.58 (2H, m), 2.44 (3H, s), 2.33-2.27 (2H, m), 2.17-2.11 (2H, m), 2.11-2.02 (1H, m), 1.73-1.62 (4H, m), 1.62-1.54 (2H, m), 1.41-1.29 (2H, m).

### Example 19

### ({[5-Hydroxy-6-methyl-2-({1-[2-(trifluoromethyl)phenyl]piperidin-4-yl}methyl)pyrimidin-4-yl]carbonyl}amino)acetic acid

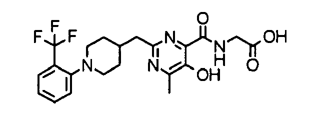

In accordance with Examples 1-(8) to 1-(11), but using 1-indo-2-(trifluoromethyl)benzene (0.50 g, 1.8 mmol) instead of 1-bromo-4-tert-butylbenzene, the title compound (0.070 g, 0.15 mmol) was afforded as a pale yellowish white solid (yield 17%).
MS m/z: 453 (M+H)⁺;
1H-NMR (500 MHz, DMSO-d6) δ: 9.27 (1H, t, J=5 Hz), 7.64 (1H, d, J=8 Hz), 7.63 (1H, t, J=8 Hz), 7.52 (1H, d, J=8 Hz), 7.30 (1H, t, J=8 Hz), 3.88 (2H, d, J=5 Hz), 2.93 (2H, d, J=11 Hz), 2.79 (2H, d, J=7 Hz), 2.73 (2H, t, J=11 Hz), 2.44 (3H, s), 2.08-1.96 (1H, m), 1.66 (2H, d, J=12 Hz), 1.43-1.35 (2H, m).

### Example 20

### ({[5-Hydroxy-6-methyl-2-({1-[4-(trifluoromethyl)phenyl]piperidin-4-yl}methyl)pyrimidin-4-yl]carbonyl}amino)acetic acid

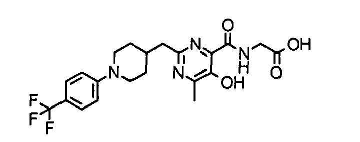

In accordance with Examples 1-(8) to 1-(11), but using 1-bromo-4-(trifluoromethyl)benzene (1.1 g, 4.9 mmol) instead of 1-bromo-4-tert-butylbenzene, the title compound (0.46 g, 1.0 mmol) was afforded as a pale yellowish white solid (yield 40%).
MS m/z: 453 (M+H)⁺;
1H-NMR (500 MHz, DMSO-d6) δ: 12.89 (1H, brs), 11.92 (1H, brs), 9.39 (1H, t, J=6 Hz), 7.47 (2H, d, J=9 Hz), 7.04 (2H, d, J=9 Hz), 3.99 (2H, d, J=6 Hz), 3.85 (2H, d, J=14 Hz), 2.82 (2H, t, J=13 Hz), 2.76 (2H, d, J=7 Hz), 2.44 (3H, s), 2.20-2.09 (1H, m), 1.67 (2H, d, J=14 Hz), 1.37-1.22 (2H, m).

### Example 21

### {[(2-{[1-(4-Cyanophenyl)piperidin-4-yl]methyl -5-hydroxy-6-methylpyrimidin-4-yl)carbonyl]amino}acetic acid

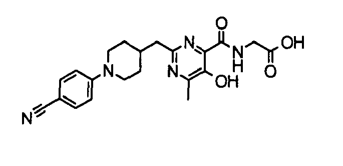

In accordance with Examples 1-(8) to 1-(11), but using 1-bromo-4-cyanobenzene (0.14 g, 0.74 mmol) instead of 1-bromo-4-tert-butylbenzene, the title compound (0.07 g, 0.17 mmol) was afforded as a pale yellow solid (yield 46%).
MS m/z: 410 (M+H)⁺;
1H-NMR (500 MHz, DMSO-d6) δ: 12.90 (1H, brs), 11.92 (1H, brs), 9.37 (1H, t, J=6 Hz), 7.54 (2H, d, J=9 Hz), 6.99 (2H, d, J=9 Hz), 4.00 (2H, d, J=6 Hz), 3.91 (2H, d, J=12 Hz), 2.85 (2H, dt, J=12 Hz, 3 Hz), 2.76 (2H, d, J=7 Hz), 2.43 (3H, s), 2.23-2.12 (1H, m), 1.66 (2H, d, J=12 Hz), 1.27 (2H, dq, J=12 Hz, 3 Hz).

### Example 22

### {[(2-{[1-(4-Formylphenyl)piperidin-4-yl]methyl}-5-hydroxy-6-methylpyrimidin-4-yl)carbonyl]amino} acetic acid

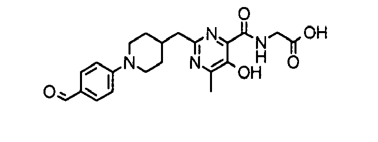

### (1) Ethyl ({[5-(benzyloxy)-2-{[1-(4-formylphenyl)piperidm-4-yl]methyl}-6-methylpyrimidin-4-yl]carbonyl}amino)acetate

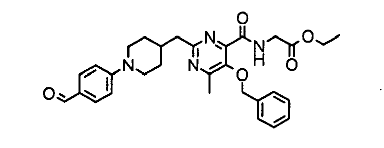

In accordance with Example 1-(8), but using 2-(4-bromophenyl)-1,3-dioxolane (2.4 g, 11 mmol) instead of 1-bromo-4-tert-butylbenzene, tert-butyl 5-(benzyloxy)-2-({1-[4-(1,3-dioxolan-2-yl)phenyl]piperidin-4-yl}methyl)-6-methylpyrimidine-4-carboxylate (1.9 g, 3.5 mmol) was afforded as a yellow solid (yield 50%).

In accordance with Example 1-(9), but using tert-butyl 5-(benzyloxy)-2-({1-[4-(1,3-dioxolan-2-yl)phenyl]piperidin-4-yl}methyl)-6-methylpyrimidine-4-carboxylate (0.35 g, 0.64 mmol) instead of tert-butyl 5-(benzyloxy)-2-{[1-(4-tert-butylphenyl)piperidin-4-yl]methyl}-6-methylpyrimidine-4-carboxylate, the title compound (0.20 g, 0.34 mmol) was afforded as a red oil (yield 29%).
1H-NMR (500 MHz, CDCl3) δ: 9.79 (1H, s), 8.35 (1H, t, J=5 Hz), 7.76 (2H, d, J=9 Hz), 7.55-7.45 (2H, m), 7.43-7.33 (3H, m), 6.93 (2H, d, J=9 Hz), 5.15 (2H, s), 4.29 (2H, q, J=8 Hz), 4.26 (2H, d, J=5 Hz), 3.97 (2H, d, J=13 Hz); 2.98 (2H, t, J=13 Hz), 2.90 (2H, d, J=7 Hz), 2.49 (3H, s), 2.35-2.15 (1H, m), 1.82 (2H, d, 12 Hz), 1.49 (2H, dq, J=11 Hz, 3 Hz), 1.35 (3H, t, J=8 Hz).

### (2) Ethyl ({[5-(benzyloxy)-2-({1-[4-(1,3-dioxan-2-yl)phenyl]piperidin-4-yl}methyl)-6-methylpyrimidin-4-yl] carbonyl}amino)acetate

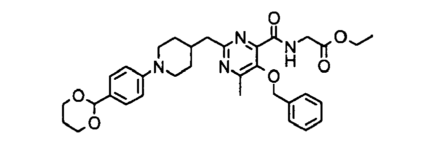

Ethyl ({[5-(benzyloxy)-2-{[1-(4-formylphenyl)piperidin-4-yl]methyl}-6-methylpyrimidin-4-yl]carbonyl}amino)acetate (0.20 g, 0.34 mmol) and propane-1,3-diol (0.031 mL, 0.41 mmol) were dissolved in benzene (20 mL), and at room temperature p-toluenesulfonic acid monohydrate (0.0072 g, 0.037 mmol) was added, followed by heating to reflux for 19.5 hours. After the reaction solution was concentrated under reduced pressure, the resulting residue was purified by silica gel column chromatography (Moritex Corporation, elution solvent: hexane/ethyl acetate), and a fraction corresponding to the Rf value=0.30 (hexane/ethyl acetate=1/1) by thin layer chromatography was concentrated under reduced pressure to afford the title compound (0.093 g, 0.16 mmol) as a yellow oil (yield 42%).
1H-NMR (500 MHz, CDCl3) δ: 8.37 (1H, t, J=5 Hz), 7.55-7.48 (2H, m), 7.43-7.31 (3H, m), 7.36 (2H, d, J=9 Hz), 6.93 (2H, d, J=9 Hz), 5.45 (1H, s), 5.14 (2H, s), 4.29 (2H, q, J=8 Hz), 4.40-4.23 (4H, m), 4.26 (2H, d, J=5 Hz), 3.97 (2H, d, J=13 Hz), 2.89 (2H, t, J=13 Hz), 2.71 (2H, d, J=7 Hz), 2.47 (3H, s), 2.30-2.20 (1H, m), 1.76 (2H, d, 12 Hz), 1.49 (2H, dq, J=11 Hz, 3 Hz), 1.35 (3H, t, J=8 Hz), 1.34-1.23 (2H, m).

### (3) {[(2-{[1-(4-Formylphenyl)piperidin-4-yl]methyl}-5-hydroxy-6-methylpyrimidin-4-yl)carbonyl]amino}acetic acid

In accordance with Examples 1-(10) and 1-(11), but using ethyl ({[5-(benzyloxy)-2-({1-[4-(1,3-dioxan-2-yl)phenyl]piperidin-4-yl}methyl)-6-methylpyrimidin-4-yl]carbonyl}amino)acetate (0.093 g, 0.16 mmol) instead of ethyl ({[5-(benzyloxy)-2-([1-(4-tert-butylphenyl)piperidin-4-yl]methyl)-6-methylpyrimidin-4-yl]carbonyl}amino)acetate, the title compound (0.024 g, 0.060 mmol) was afforded as a red solid (yield 37%).
MS m/z: 413 (M+H)⁺;
1H-NMR (500 MHz, DMSO-d6) δ: 9.68 (1H, s), 9.36 (1H, t, J=6 Hz), 7.68 (2H, d, J=9 Hz), 7.02 (2H, d, J=9 Hz), 3.99 (2H, d, J=6 Hz), 3.97 (2H, d, J=12 Hz), 2.91 (2H, t, J=12 Hz), 2.76 (2H, d, J=7 Hz), 2.44 (3H, s), 2.25-2.13 (1H, m), 1.69 (2H, d, J=12 Hz), 1.28 (2H, dq, J=12 Hz, 3 Hz).

### Example 23

### {[(5-Hydroxy-2-{[1-(4-hydroxyphenyl)piperidin-4-yl]methyl}-6-methylpyrimidin-4-yl)carbonyl]amino} acetic acid

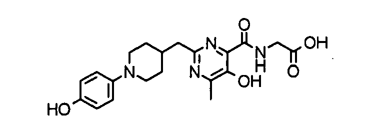

### (1) tert-Butyl 5-(benzyloxy)-2-({1-[4-(methoxymethoxy)phenyl]piperidin-4-yl}methyl)-6-methylpyrimidine-4-carboxylate

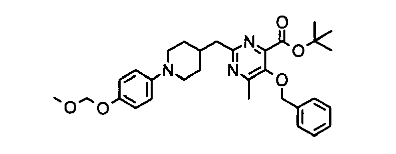

In accordance with Example 1-(8), but using 1-bromo-4-(methoxymethoxy)benzene (5.0 g; 23.0 mmol) instead of 1-bromo-4-tert-butylbenzene, the title compound (3.1 g, 5.8 mmol) was afforded as a yellow oil (yield 50%). 1H-NMR (500 MHz, CDCl3) δ: 7.45-7.35 (5H, m), 6.96 (2H, d, J=9 Hz), 6.89 (2H, d, J=9 Hz), 5.11 (2H, s), 5.01 (2H, s), 3.52 (2H, d, J=12 Hz), 3.48 (3H, s), 2.90 (2H, d, J=7 Hz), 2.63 (2H, t, J=12 Hz), 2.46 (3H, s), 2.10-2.00 (1H, m), 1.78 (2H, d, J=12 Hz), 1.59 (9H, s), 1.54 (2H, dq, J=12 Hz, 3 Hz).

### (2) tert-Butyl 5-(benzyloxy)-2- {[1-(4-hydroxyphenyl)piperidin-4-yl]methyl} -6-methylpyrimidine-4-carboxylate hydrochloride

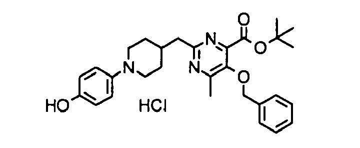

tert-Butyl 5-(benzyloxy)-2-({1-[4-(methoxymethoxy)phenyl]piperidin-4-yl}methyl)-6-methylpyrimidine-4-carboxylate (3.1 g, 5:8 mmol) was dissolved in ethyl acetate (15 mL) and tert-butanol (7.5 mL), and a solution of hydrogen chloride in dioxane (4 M, 7.3 mL) was added, followed by stirring at room temperature for 5.25 hours. After hexane (50 mL) was added to the reaction solution, the resulting solid was collected by filtration to afford the title compound (2.9 g, 5.5 mmol) as a pale yellowish white solid (yield 95%).
1H-NMR (500 MHz, DMSO-d6) δ: 12.16 (1H, brs), 7.68 (2H, d, J=9 Hz), 7.47-7.36 (5H, m), 6.99 (2H, d, J=9 Hz), 5.00 (2H, s), 3.51-3.41 (2H, m), 3.44 (2H, d, J=12 Hz), 2.84 (2H, d, J=7 Hz), 2.47 (3H, s), 2.36-2.24 (1H, m), 2.00-1.87 (2H, m), 1.84 (2H, d, J=12 Hz), 1.51 (9H, s).

### (3) {[(5-Hydroxy-2-{[1-(4-hydroxyphenyl)piperidin-4-yl]methyl}-6-methylpyrimidin-4-yl)carbonyl]amino}acetic acid

In accordance with Examples 1-(9) to 1-(11), but using tert-butyl 5-(benzyloxy)-2-{[1-(4-hydroxyphenyl)piperidin-4-yl]methyl-6-methylpyrimidine-4-carboxylate hydrochloride (0.30 g, 0.57 mmol) instead of tert-butyl 5-(benzyloxy)-2-{[1-(4-tert-butylphenyl)piperidin-4-yl]methyl}-6-methylpyrimidine-4-carboxylate, the title compound (0.15 g, 0.37 mmol) was afforded as a pale yellowish white solid (yield 66%).
MS m/z: 401 (M+H)⁺;
1H-NMR (500 MHz, DMSO-d6) δ: 12.89 (1H, brs), 11.91 (1H, s), 9.41 (1H, t, J=6 Hz), 8.79 (1H, s), 6.77 (2H, d, J=8 Hz), 6.62 (2H, d, J=8 Hz), 4.00 (2H, d, J=6 Hz), 3.38 (2H, d, J=12 Hz), 2.77 (2H, d, J=7 Hz), 2.53-2.46 (2H, m), 2.44 (3H, s), 2.04-1.93 (1H, m), 1.65 (2H, d, J=12 Hz), 1.37 (2H, dq, J=12 Hz, 3 Hz).

### Example 24

### {[(5-Hydroxy-2-{[1-(4-methoxyphenyl)piperidin-4-yl]methyl -6-methylpyrimidin-4-yl)carbonyl]amino}acetic acid

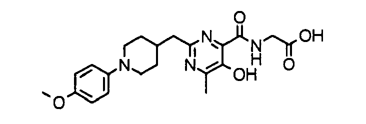

In accordance with Examples 1-(8) to 1-(11), but using 1-bromo-4-methoxybenzene (0.43 g, 2.3 mmol) instead of 1-bromo-4-tert-butylbenzene, the title compound (0.07 g, 0.17 mmol) was afforded as a pale yellow solid (yield 15%).
MS m/z: 415 (M+H)⁺;
1H-NMR (500 MHz, DMSO-d6) δ: 12.88 (1H, brs), 11.91 (1H, brs), 9.41 (1H, t, J=6 Hz), 6.88 (2H, d, J=9 Hz), 6.80 (2H, d, J=9 Hz), 4.01 (2H, d, J=6 Hz), 3.67 (3H, s), 3.47 (2H, d, J=12 Hz), 2.78 (2H, d, J=7 Hz), 2.59-2.50 (2H, m), 2.44 (3H, s), 2.07-1.96 (1H, m), 1.67 (2H, d, J=12 Hz), 1.38 (2H, dq, J=12 Hz, 3 Hz).

### Example 25

### ({[5-Hydroxy-6-methyl-2-({1-[4-(trifluoromethoxy)phenyl]piperidin-4-yl}methyl)pyrimidin-4-yl]carbonyl}amino)acetic acid

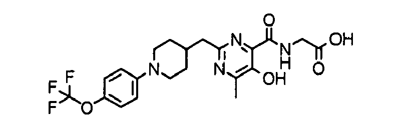

In accordance with Examples 1-(8) to 1-(11), but using 1-promo-4-(trifluoromethoxy)benzene (1.2 g, 5.0 mmol) instead of 1-bromo-4-tert-butylbenzene, the title compound (0.50 g, 1.1 mmol) was afforded as a white solid (yield 42%).
MS m/z: 469 (M+H)⁺;
1H-NMR (500 MHz, DMSO-d6) δ: 12.88 (1H, brs), 11.91 (1H, brs), 9.39 (1H, t, J=6 Hz), 7.16 (2H, d, J=9 Hz), 6.98 (2H, d, J=9 Hz), 4.00 (2H, d, J=6 Hz), 3.68 (2H, d, J=12 Hz), 2.77 (2H, d, J=7 Hz), 2.68 (2H, t, J=12 Hz), 2.44 (3H, s), 2.13-2.02 (1H, m), 1.67 (2H, d, J=13 Hz), 1.40-1.28 (2H, m).

### Example 26

### {[(5-Hydroxy-6-methyl-2-{[1-(4-phenoxyphenyl)piperidin-4-yl]methyl}pyrimidin-4-yl)carbonyl]amino}acetic acid

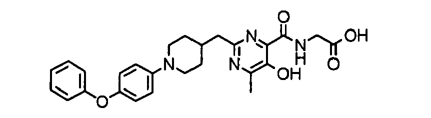

In accordance with Examples 1-(8) to 1-(11), but using 1-promo-4-phenoxybenzene (0.23 g, 0.92 mmol) instead of 1-bromo-4-tert-butylbenzene, the title compound (0.10 g, 0.21 mmol) was afforded as a pale yellowish white solid (yield 23%).
MS m/z: 477 (M+H)⁺;
1H-NMR (500 MHz, DMSO-d6) δ: 12.85 (1H, brs), 11.90 (1H, s), 9.39 (1H, t, J=6 Hz), 7.33 (2H, t, J=8 Hz), 7.05 (1H, t, J=8 Hz), 6.96-6.89 (6H, m), 4.01 (2H, d, J=6 Hz), 3.60 (2H, d, J=12 Hz), 2.78 (2H, d, J=7 Hz), 2.63 (2H, t, J=12 Hz), 2.44 (3H, s), 2.10-2.00 (1H, m), 1.69 (2H, d, J=12 Hz), 1.44-1.32 (2H, m).

### Example 27

### ({[5-Hydroxy-6-methyl-2-({1-[4-(methylsulfanyl)phenyl]piperidin-4-yl}methyl)pyrimidin-4-yl]carbonyl}amino)acetic acid

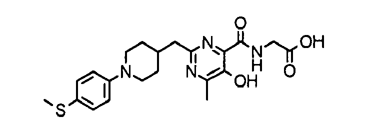

In accordance with Examples 1-(8), 1-(9), 6-(2) and 1-(11), but using 1-bromo-4-(methylsulfanyl)benzene (0.47 g, 2.3 mmol) instead of 1-bromo-4-tert-butylbenzene, the title compound (0.07 g, 0.16 mmol) was afforded as a pale yellowish white solid (yield 14%).
MS m/z: 431 (M+H)⁺;
1H-NMR (500 MHz, DMSO-d6) δ: 12.90 (1H, brs), 11.91 (1H, s), 9.41 (1H, t, J=6 Hz), 7.16 (2H, d, J=8 Hz), 6.89 (2H, d, J=8 Hz), 4.00 (2H, d, J=6 Hz), 3.64 (2H, d, J=12 Hz), 2.77 (2H, d, J=7 Hz), 2.63 (2H, t, J=12 Hz), 2.44 (3H, s), 2.38 (3H, s), 2.11-2.01 (1H, m), 1.66 (2H, d, J=12 Hz), 1.34 (2H, dq, J=12 Hz, 3 Hz).

### Example 28

### ({[2-({1-[4-(N,N-Dimethylamino)phenyl]piperidin-4-yl}methyl)-5-hydroxy-6-methylpyrimidin-4-yl]carbonyl}amino)acetic acid

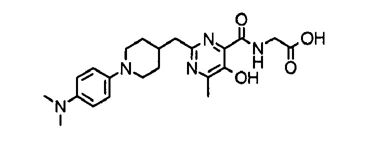

In accordance with Examples 1-(8), 1-(9), 6-(2) and 1-(11), but using 4-bromo-N,N-dimethylaniline (0.47 g, 2.4 mmol) instead of 1-bromo-4-tert-butylbenzene, the title compound (0.02 g, 0.05 mmol) was afforded as a pale yellowish white solid (yield 4%).
MS m/z: 428 (M+H)⁺;
1H-NMR (500 MHz, Pyridine-d5) δ: 12.54 (1H, brs), 10.41 (1H, t, J=6 Hz), 7.10 (2H, d, J=8 Hz), 6.85 (2H, d, J=8 Hz), 4.66 (2H, d, J=6 Hz), 3.51 (2H, d, J=12 Hz), 2.80 (2H, d, J=7 Hz), 2.77 (6H, brs), 2.60 (2H, t, J=12 Hz), 2.55 (3H, s), 2.08-1.97 (1H, m), 1.69 (2H, d, J=12 Hz), 1.52 (2H, q, J=12 Hz).

### Example 29

### [({5-Hydroxy-6-methyl-2-[(1-{4-[(N-methyl-N-phenyl)amino]phenyl}piperidin-4-yl)methyl]pyrimidin-4-yl}carbonyl)amino]acetic acid

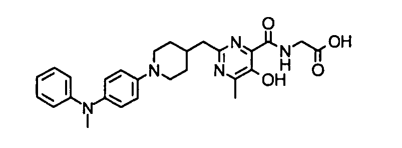

### (1) tert-Butyl 5-(benzyloxy)-6-methyl-2-{[1-(4-{[(trifluoromethyl)sulfonyl]oxy}phenyl)piperidin-4-yl]methyl}pyrimidine-4-carboxylate

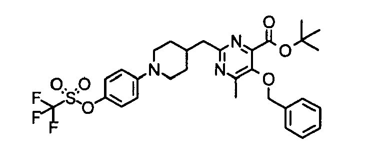

In accordance with Example 1-(5), but using tert-butyl 5-(benzyloxy)-2-{[1-(4-hydroxyphenyl)piperidin-4-yl]methyl}-6-methylpyrimidine-4-carboxylate hydrochloride (3.0 g, 5.7 mmol) obtained in Example 23-(2) instead of tert-butyl 5-(benzyloxy)-2- {[1-(tert-butoxycarbonyl)piperidin-4-yl]methyl -6-hydroxypyrimidine-4-carboxylate, the title compound (2.2 g, 3.5 mmol) was afforded (yield 62%). 1H-NMR (500 MHz, CDCl3) δ: 7.43-7.36 (5H, m), 7.11 (2H, d, J=9 Hz), 6.89 (2H, d, J=9 Hz), 5.01 (2H, s), 3.66 (2H, d, J=12 Hz), 2.90 (2H, d, J=7 Hz), 2.75 (2H, t, J=12 Hz), 2.46 (3H, s), 2.14-2.07 (1H, m), 1.78 (2H, d, J=12 Hz), 1.56 (9H, s), 1.53-1.45 (2H, m).

### (2) [({5-Hydroxy-6-methyl-2-[(1-{4-[(N-methyl-N-phenyl)amino]phenyl}piperidin-4-yl)methyl]pyrimidin-4-yl}carbonyl)amino]acetic acid

In accordance with Examples 1-(8), 1-(9), 6-(2) and 1-(11), but using N-methylaniline (0.35 g, 3.2 mmol) instead of tert-butyl 5-(benzyloxy)-6-methyl-2-(piperidin-4-ylmethyl)pyrimidine-4-carboxylate hydrochloride, and tert-butyl 5-(benzyloxy)-6-methyl-2- {[1-(4-{[(trifluoromethyl)sulfonyl]oxy}phenyl)piperidin-4-yl]methyl}pyrimidine-4-carboxylate (1.0 g, 1.6 mmol) instead of 1-bromo-4-tert-butylbenzene, the title compound (0.078 g, 0.16 mmol) was afforded (yield 10%).
MS m/z: 490 (M+H)⁺;
1H-NMR (500 MHz, DMSO-d6) δ: 9.18 (1H, brs), 7.14 (2H, t, J=8 Hz), 6.99 (2H, d, J=8 Hz), 6.93 (2H, d, J=8 Hz), 6.71-6.68 (3H, m), 3.72 (2H, brs), 3.62 (2H, d, J=11 Hz), 3.16 (3H, s), 2.78 (2H, d, J=7 Hz), 2.62 (2H, t, J=11 Hz), 2.43 (3H, s), 2.07-1.97 (1H, m), 1.69 (2H, d, J=12 Hz), 1.42-1.31 (2H, m).

### Example 30

### {[(5-Hydroxy-6-methyl-2-{[1-(4-nitrophenyl)piperidin-4-yl]methyl}pyrimidin-4-yl)carbonyl]amino}acetic acid

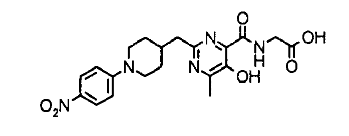

In accordance with Examples 1-(8), 1-(9), 6-(2) and 1-(11), but using 1-bromo-4-nitrobenzene (0.47 g, 2.3 mmol) instead of 1-bromo-4-tert-butylbenzene, the title compound (0.04 g, 0.10 mmol) was afforded as a yellow solid (yield 9%).
MS m/z: 430 (M+H)⁺;
1H-NMR (500 MHz, DMSO-d6) δ: 12.91 (1H, brs), 11.92 (1H, brs), 9.40 (1H, t, J=5 Hz), 8.03 (2H, dd, J=9 Hz, 1 Hz), 7.01 (2H, dd, J=9 Hz, 1 Hz), 4.05 (2H, d, J=12 Hz), 4.00 (2H, d, J=5 Hz), 2.99 (2H, t, J=12 Hz), 2.77 (2H, d, J=7 Hz), 2.44 (3H, s), 2.29-2.19 (1H, m), 1.69 (2H, d, J=12 Hz), 1.27 (2H, q, J=12 Hz).

### Example 31

### {[(2-{[1-(4-Acetylphenyl)piperidin-4-yl]methyl}-5-hydroxy-6-methylpyrimidin-4-yl)carbonyl]amino}acetic acid

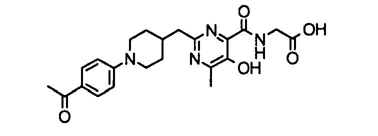

In accordance with Examples 1-(8) to 1-(11), but using 4-bromoacetophenone (0.28 g, 1.4 mmol) instead of 1-bromo-4-tert-butylbenzene, the title compound (0.072 g, 0.21 mmol) was afforded as a white solid (yield 24%).
MS m/z: 427 (M+H)⁺;
1H-NMR (500 MHz, DMSO-d6) δ: 12.92 (1H, brs), 11.92 (1H, brs), 9.40 (1H, t, J=6 Hz), 7.78 (2H, d, J=9 Hz), 6.95 (2H, d, J=9 Hz), 4.00 (2H, d, J=6 Hz), 3.93 (2H, d, J=13 Hz), 2.86 (2H, t, J=13 Hz), 2.76 (2H, d, J=7 Hz), 2.44 (6H, s), 2.23-2.12 (1H, m), 1.66 (2H, d, J=12 Hz), 1.34-1.24 (2H, m).

### Example 32

### {[(2-{[1-(4-Benzoylphenyl)piperidin-4-yl]methyl}-5-hydroxy-6-methylpyrimidin-4-yl)carbonyl]amino}acetic acid

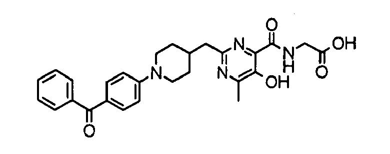

In accordance with Examples 1-(8) to 1-(11), but using 4-bromobenzophenone (0.36 g, 1.4 mmol) instead of 1-bromo-4-tert-butylbenzene, the title compound (0.14 g, 0.29 mmol) was afforded as a yellow solid (yield 42%).
MS m/z: 489 (M+H)⁺;
1H-NMR (400 MHz, DMSO-d6) δ: 12.90 (1H, brs), 11.92 (1H, s), 9.40 (1H, t, J=6 Hz), 7.65-7.59 (5H, m), 7.52 (2H, t, J=8 Hz), 7.00 (2H, d, J=8 Hz), 4.00 (2H, d, J=6 Hz), 3.97 (2H, d, J=12 Hz), 2.89 (2H, t, J=12 Hz), 2.77 (2H, d, J=7 Hz), 2.44 (3H, s), 2.26-2.10 (1H, m), 1.68 (2H, d, J=12 Hz), 1.36-1.23 (2H, m).

### Example 33

### {[(2-{[1-(2,4-Difluorophenyl)piperidin-4-yl]methyl}-5-hydroxy-6-methylpyrimidin-4-yl)carbonyl]amino} acetic acid

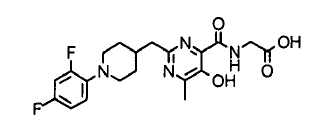

In accordance with Examples 1-(8) to 1-(11), but using 1-promo-2,4-difluorobenzene (1.68 g, 2.3 mmol) instead of 1-bromo-4-tert-butylbenzene, the title compound (0.023 g, 0.055 mmol) was afforded as a pale yellowish white solid (yield 6.5%).
MS m/z: 421 (M+H)⁺;
1H-NMR (500 MHz, DMSO-d6) δ: 11.92 (1H, brs), 9.38 (1H, t, J=6 Hz), 7.04-6.90 (3H, m), 4.00 (2H, d, J=6 Hz), 3.56 (2H, d, J=12 Hz), 2.78 (2H, d, J=7 Hz), 2.60 (2H, t, J=14 Hz), 2.44 (3H, s), 2.09-1.98 (1H, m), 1.67 (2H, d, J=14 Hz), 1.41-1.30 (2H, m).

### Example 34

### {[(2-{[1-(3,4-Difluorophenyl)piperidin-4-yl]methyl}-5-hydroxy-6-methylpyrimidin-4-yl)carbonyl]amino} acetic acid

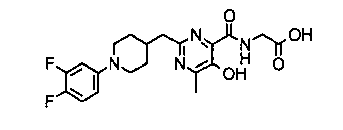

In accordance with Examples 1-(8) to 1-(11), but using 1-bromo-3,4-difluorobenzene (0.44 g, 2.3 mmol) instead of 1-bromo-4-tert-butylbenzene, the title compound (0.15 g, 0.37 mmol) was afforded as a pale yellowish white solid (yield 31%).
MS m/z: 421 (M+H)⁺;
1H-NMR (500 MHz, DMSO-d6) δ: 12.90 (1H, brs), 11.91 (1H, s), 9.40 (1H, t, J=6 Hz), 7.21 (1H, q, J=10 Hz), 6.99-6.92 (1H, m), 6.73-6.68 (1H, m), 4.01 (2H, d, J=6 Hz), 3.63 (2H, d, J=12 Hz), 2.77 (2H, d, J=7 Hz), 2.64 (2H, t, J=12 Hz), 2.44 (3H, s), 2.11-2.01 (1H, m), 1.66 (2H, d, J=12 Hz), 1.33 (2H, dq, J=12 Hz, 3 Hz).

### Example 35

### {[(2-{[1-(2,4-Dichlorophenyl)piperidin-4-yl]methyl -5-hydroxy-6-methylpyrimidin-4-yl)carbonyl]amino}acetic acid

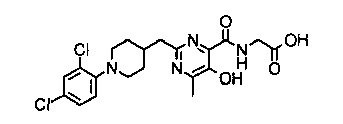

In accordance with Examples 1-(8), 1-(9), 6-(2) and 1-(11), but using 1-bromo-2,4-dichlorobenzene (1.04 g, 4.6 mmol) instead of 1-bromo-4-tert-butylbenzene, the title compound (0.12 g, 0.26 mmol) was afforded as a pale yellowish white solid (yield 12%).
MS m/z: 453 (M+H)⁺;
1H-NMR (500 MHz, DMSO-d6) δ: 12.89 (1H, brs), 11.91 (1H, s), 9.40 (1H, t, J=6 Hz), 7.53-7.51 (1H, m), 7.36-7.32 (1H, m), 7.14 (1H, d, J=9 Hz), 4.01 (2H, d, J=6 Hz), 3.23 (2H, d, J=12 Hz), 2.81 (2H, d, J=7 Hz), 2.62 (2H, t, J=12 Hz), 2.44 (3H, s), 2.11-2.00 (1H, m), 1.69 (2H, d, J=12 Hz), 1.43 (2H, dq, J=12 Hz, 3 Hz).

### Example 36

### {[(2-{[1-(3,4-Dichlorophenyl)piperidin-4-yl]methyl}-5-hydroxy-6-methylpyrimidin-4-yl)carbonyl]amino}acetic acid

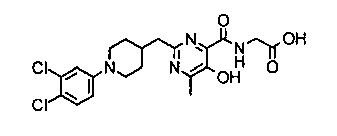

In accordance with Examples 1-(8), 1-(9), 6-(2) and 1-(11), but using 1-bromo-3,4-dichlorobenzene (0.52 g, 2.3 mmol) instead of 1-bromo-4-tert-butylbenzene, the title compound (0.15 g, 0.33 mmol) was afforded as a pale yellowish white solid (yield 29%).
MS m/z: 453 (M+H)⁺;
1H-NMR (500 MHz, DMSO-d6) δ: 12.89 (1H, brs), 11.91 (1H, s), 9.40 (1H, t, J=6 Hz), 7.36 (1H, d, J=9 Hz), 7.10 (1H, d, J=3 Hz), 6.92 (1H, dd, J=9 Hz, 3 Hz), 4.00 (2H, d, J=6 Hz), 3.72 (2H, d, J=12 Hz), 2.76 (2H, d, J=7 Hz), 2.71 (2H, t, J=12 Hz), 2.44 (3H, s), 2.15-2.05 (1H, m), 1.65 (2H, d, J=12 Hz), 1.31 (2H, dq, J=12 Hz, 3 Hz).

### Example 37

### {[(2-[1-(2,4-Dimethylphenyl)piperidin-4-yl]methyl}-5-hydroxy-6-methylpyrimidin-4-yl)carbonyl]amino acetic acid

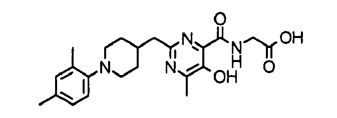

In accordance with Examples 1-(8) to 1-(11), but using 1-promo-2,4-dimethylbenzene (0.43 g, 2.3 mmol) instead of 1-bromo-4-tert-butylbenzene, the title compound (0.06 g, 0.15 mmol) was afforded as a pale yellowish white solid (yield 13%).
MS m/z: 413 (M+H)⁺;
1H-NMR (500 MHz, DMSO-d6) δ: 12.89 (1H, brs), 11.90 (1H, s), 9.40 (1H, t, J=6 Hz), 6.95 (1H, s), 6.93-6.86 (2H, m), 4.01 (2H, d, J=6 Hz), 2.96 (2H, d, J=12 Hz), 2.80 (2H, d, J=7 Hz), 2.53 (2H, t, J=12 Hz), 2.44 (3H, s), 2.19 (3H, s), 2.18 (3H, s), 2.07-1.96 (1H, m), 1.66 (2H, d, J=12 Hz), 1.41 (2H, dq, J=12 Hz, 3 Hz).

### Example 38

### {[(2-{[1-(3,4-Dimethylphenyl)piperidin-4-yl]methyl}-5-hydroxy-6-methylpyrimidin-4-yl)carbonyl]amino}acetic acid

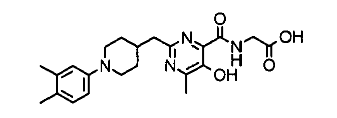

In accordance with Examples 1-(8) to 1-(11), but using 1-promo-3,4-dimethylbenzene (0.43 g, 2.3 mmol) instead of 1-bromo-4-tert-butylbenzene, the title compound (0.08 g, 0.19 mmol) was afforded as a pale yellowish white solid (yield 17%).
MS m/z: 413 (M+H)⁺;
1H-NMR (500 MHz, DMSO-d6) δ: 12.89 (1H, brs), 11.91 (1H, s), 9.40 (1H, t, J=6 Hz), 6.94 (1H, d, J=7 Hz), 6.73 (1H, s), 6.67-6.60 (1H, m), 4.01 (2H, d, J=6 Hz), 3.57 (2H, d, J=12 Hz), 2.77 (2H, d, J=7 Hz), 2.57 (2H, t, J=12 Hz), 2.44 (3H, s), 2.15 (3H, s), 2.10 (3H, s), 2.10-1.98 (1H, m), 1.65 (2H, d, J=12 Hz), 1.35 (2H, q, J=12 Hz).

### Example 39

### {[(2-{[1-(4-Fluoro-2-methylphenyl)piperidin-4-yl]methyl}-5-hydroxy-6-methylpyrimidin-4-yl)carbonyl]amino}acetic acid

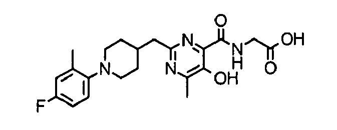

In accordance with Examples 1-(8) to 1-(11), but using 1-bromo-4-fluoro-2-methylbenzene (0.44 g, 2.3 mmol) instead of 1-bromo-4-tert-butylbenzene, the title compound (0.18 g, 0.42 mmol) was afforded as a white solid (yield 37%).
MS m/z: 417 (M+H)⁺;
1H-NMR (500 MHz, DMSO-d6) δ: 12.87 (1H, brs), 11.90 (1H, brs), 9.39 (1H, t, J=8 Hz), 7.06-6.91 (3H, m), 3.99 (2H, d, J=8 Hz), 2.96 (2H, d, J=15 Hz), 2.80 (2H, t, J=11 Hz), 2.55 (2H, t, J=10 Hz), 2.44 (3H, s), 2.23 (3H, s), 2.08-1.95 (1H, m), 1.69 (2H, d, J=15 Hz), 1.42 (2H, dq, J=11 Hz, 5 Hz).

### Example 40

### {[(2-{[1-(4-Chloro-2-methylphenyl)piperidin-4-yl]methyl}-5-hydroxy-6-methylpyrimidin-4-yl)carbonyl]amino}acetic acid

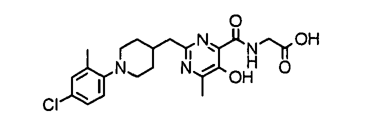

In accordance with Examples 1-(8), 1-(9), 6-(2) and 1-(11), but using 1-bromo-4-chloro-2-methylbenzene (0.28 g, 1.4 mmol) instead of 1-bromo-4-tert-butylbenzene, the title compound (0.11 g, 0.26 mmol) was afforded as a white solid (yield 38%).
MS m/z: 433 (M+H)⁺;
1H-NMR (500 MHz, DMSO-d6) δ: 11.80 (1H, brs), 9.34 (1H, t, J=8 Hz), 7.20 (1H, s), 7.15 (1H, d, J=10 Hz), 6.99 (1H, d, J=10 Hz), 3.95 (2H, d, J=8 Hz), 3.00 (2H, d, J=11 Hz), 2.80 (2H, t, J=10 Hz), 2.56 (2H, t, J=11 Hz), 2.44 (3H, s), 2.22 (3H, s), 2.08-1.98 (1H, m), 1.68 (2H, d, J=11 Hz), 1.41 (2H, dq, J=8 Hz, 5 Hz).

### Example 41

### {[(2-{[1-(4-tert-Butyl-2-chlorophenyl)piperidin-4-yl]methyl}-5-hydroxy-6-methylpyrimidin-4-yl)carbonyl]amino}acetic acid

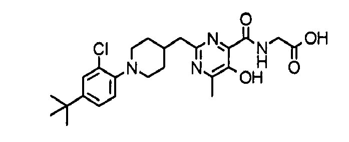

Sodium chlorite (0.08 g, 0.92 mmol) and sodium sulfamate (0.11 g, 0.92 mmol) were dissolved in tetrahydrofuran (6 mL) and water (1 mL), and at 3°C hydrochloric acid (1 M, 0.92 mL), and then {[(2-{[1-(4-tert-butylphenyl)piperidin-4-yl]methyl}-5-hydroxy-6-methylpyrimidin-4-yl)carbonyl]amino}acetic acid (0.40 g, 0.90 mmol) obtained in Example 1-(11) were added, followed by stirring at the same temperature for 2 hours. Water was added to the reaction solution, and the resulting mixture was concentrated under reduced pressure, and extracted with ethyl acetate. After the extract was concentrated under reduced pressure, the resulting residue was purified by silica gel column chromatography (Moritex Corporation, elution solvent: dichloromethane/methanol), and a fraction corresponding to the Rf value=0.25 (dichloromethane/methanol=19/1) by thin layer chromatography was concentrated under reduced pressure. The resulting residue was purified by preparative HPLC (YMC-Pack ODS-A; YMC, elution solvent: acetonitrile/water=90/10) to afford the title compound (0.18 g, 0.38 mmol) as a white solid (yield 42%).
MS m/z: 475 (M+H)⁺;
1H-NMR (500 MHz, DMSO-d6) δ: 12.90 (1H, s), 11.91 (1H, s), 9.42 (1H, t, J=6 Hz), 7.35 (1H, t, J=2 Hz), 7.28 (1H, dt, J=8 Hz, 2 Hz), 7.07 (1H, dd, J=8 Hz, 2 Hz), 4.01 (2H, d, J=6 Hz), 3.21 (2H, d, J=12 Hz), 2.81 (2H, d, J=7 Hz), 2.59 (2H, t, J=12 Hz), 2.44 (3H, s), 2.09-1.98 (1H, m), 1.68 (2H, d, J=12 Hz), 1.43 (2H, q, J=12 Hz), 1.24 (9H, s).

### Example 42

### {[(2-{[1-(4-tert-Butyl-2-methylphenyl)piperidin-4-yl]methyl}-5-hydroxy-6-methylpyrimidin-4-yl)carbonyl]amino} acetic acid

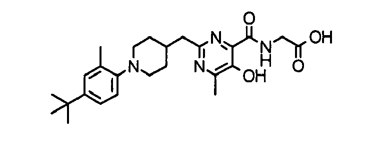

### (1) 4-tert-Butyl-2-methylphenyl trifluoromethanesulfonate

4-tert-Butyl-2-methylphenol (10 g, 61 mmol) was dissolved in methylene chloride (300 mL), and at 3°C trifluoromethanesulfonic anhydride (13 mL, 79 mmol) and pyridine (7.5 mL, 93 mmol) were added, followed by stirring at the same temperature for 30 minutes. Water was added to the reaction solution, followed by extraction with methylene chloride. After the extract was concentrated under reduced pressure, the resulting residue was purified by silica gel column chromatography (Moritex Corporation, elution solvent: hexane/ethyl acetate), and a fraction corresponding to the Rf value=0.85 (hexane/ethyl acetate=19/1) by thin layer chromatography was concentrated under reduced pressure to afford the title compound (16.7 g, 56.4 mmol) as a colorless oil (yield 92%).
1H-NMR (500 MHz, CDCl3) δ: 7.30-7.23 (2H, m), 7.14 (1H, d, J=9 Hz), 2.37 (3H, s), 1.31 (9H, s).

### (2) {[(2-{[1-(4-tert-Butyl-2-methylphenyl)piperidin-4-yl]methyl}-5-hydroxy-6-methylpyrimidin-4-yl)carbonyl]amino}acetic acid

In accordance with Examples 2-(1) to 2-(7), but using 4-tert-butyl-2-methylphenyl trifluoromethanesulfonate (14.0 g, 47.2 mmol) instead of bromobenzene, the title compound (0.69 g, 1.52 mmol) was afforded as a white solid (yield 3%).
MS m/z: 455 (M+H)⁺;
1H-NMR (500 MHz, DMSO-d6) δ: 9.00 (1H, brs), 7.15 (1H, d, J=2 Hz), 7.11 (1H, dd, J=8 Hz, 2 Hz), 6.91 (1H, d, J=8 Hz), 3.50 (2H, d, J=4 Hz), 2.98 (2H, d, J=12 Hz), 2.77 (2H, d, J=7 Hz), 2.53 (2H, t, J=12 Hz), 2.42 (3H, s), 2.22 (3H, s), 2.00-1.90 (1H, m), 1.67 (2H, d, J=12 Hz), 1.40 (2H, dq, J=12 Hz, 3 Hz), 1.23 (9H, s).

### Example 43

### {[(2-{[1-(Biphenyl-4-yl)piperidin-4-yl]methyl}-5-hydroxy-6-methylpyrimidin-4-yl)carbonyl]amino} acetic acid

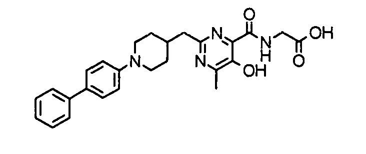

In accordance with Examples 1-(8) to 1-(11), but using 4-bromobiphenyl (0.21 g, 0.92 mmol) instead of 1-bromo-4-tert-butylbenzene, the title compound (0.11 g, 0.23 mmol) was afforded as a yellow solid (yield 50%).
MS m/z: 461 (M+H)⁺;
1H-NMR (400 MHz, DMSO-d6) δ: 12.87 (1H, brs), 11.91 (1H, s), 9.40 (1H, t, J=6 Hz), 7.59 (2H, d, J=7 Hz), 7.55-7.50 (2H, m), 7.40 (2H, t, J=7 Hz), 7.26 (1H, t, J=7 Hz), 7.06-6.97 (2H, m), 4.01 (2H, d, J=6 Hz), 3.74 (2H, d, J=12 Hz), 2.78 (2H, d, J=7 Hz), 2.78-2.67 (2H, m), 2.45 (3H, s), 2.14-2.06 (1H, m), 1.68 (2H, d, J=12 Hz), 1.43-1.31 (2H, m).

### Example 44

### {[(2-{[1-(2'-Fluorobiphenyl-4-yl)piperidin-4-yl]methyl}-5-hydroxy-6-methylpyrimidin-4-yl)carbonyl]amino}acetic acid

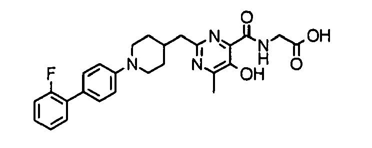

In accordance with Examples 1-(8) to 1-(11), but using 4'-bromo-2-fluorobiphenyl (1.3 g, 5.2 mmol) instead of 1-bromo-4-tert-butylbenzene, the title compound (0.81 g, 1.69 mmol) was afforded as a pale yellowish white solid (yield 41%).
MS m/z: 479 (M+H)⁺;
1H-NMR (500 MHz, DMSO-d6) δ: 11.96 (1H, brs), 9.40 (1H, t, J=5 Hz),7.50-7.45 (1H, m), 7.40 (2H, d, J=9 Hz), 7.36-7.30 (1H, m), 7.28-7.22 (2H, m), 7.02 (2H, dd, J=9 Hz, 2 Hz), 3.99 (2H, d, J=5 Hz), 3.77 (2H, d, J=12 Hz), 2.79 (2H, d, J=6 Hz), 2.72 (2H, t, J=12 Hz), 2.44 (3H, s), 2.16-2.05 (1H, m), 1.69 (2H, d, J=12 Hz), 1.37 (2H, q, J=12 Hz).

### Example 45

### {[(2-{[1-(3'-Fluorobiphenyl-4-yl)piperidin-4-yl]methyl}-5-hydroxy-6-methylpyrimidin-4-yl)carbonyl]amino}acetic acid

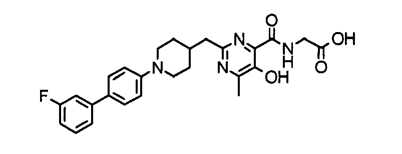

In accordance with Examples 1-(8) to 1-(11), but using 4'-bromo-3-fluorobiphenyl (2.6 g, 10.4 mmol) instead of 1-bromo-4-tert-butylbenzene, the title compound (0.64 g, 1.69 mmol) was afforded as a pale yellowish white solid (yield 16%).
MS m/z: 479 (M+H)⁺;
1H-NMR(500 MHz, DMSO-d6) δ: 12.91 (1H, brs), 11.93 (1H, s), 9.43 (1H, t, J=6 Hz), 7.56 (2H, d, J=7 Hz), 7.48-7.40 (3H, m), 7.11-7.05 (1H, m), 7.01 (2H, d, J=7 Hz), 4.02 (2H, d, J=6 Hz), 3.77 (2H, d, J=12 Hz), 2.79 (2H, d, J=6 Hz), 2.73 (2H, t, J=12 Hz), 2.45 (3H, s), 2.17-2.05 (1H, m), 1.68 (2H, d, J=12 Hz), 1.36 (2H, q, J=12 Hz).

### Example 46

### {[(2-{[1-(4'-Fluorobiphenyl-4-yl)piperidin-4-yl]methyl}-5-hydroxy-6-methylpyrimidin-4-yl)carbonyl]amino} acetic acid

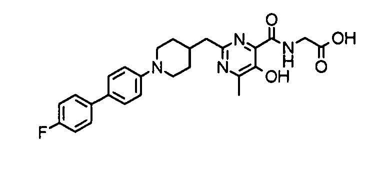

In accordance with Examples 1-(8) to 1-(11), but using 4-bromo-4'-fluorobiphenyl (0.50 g, 2.0 mmol) instead of 1-bromo-4-tert-butylbenzene, the title compound (0.18 g, 0.38 mmol) was afforded as a pale yellowish white solid (yield 33%).
MS m/z: 479 (M+H)⁺;
1H-NMR (500 MHz, DMSO-d6) δ: 9.29 (1H, brs), 7.62 (1H, d, J=8 Hz), 7.61 (1H, d, J=8 Hz), 7.48 (2H, d, J=9 Hz), 7.23 (1H, d, J=8 Hz), 7.21 (1H, d, J=8 Hz), 6.99 (2H, d, J=9 Hz), 3.89 (2H, d, J=5 Hz), 3.74 (2H, d, J=12 Hz), 2.78 (2H, d, J=7 Hz), 2.70 (2H, t, J=12 Hz), 2.44 (3H, s), 2.14-2.03 (1H, m), 1.69 (2H, d, J=12 Hz), 1.36 (2H, dq, J=12 Hz, 3 Hz).

### Example 47

### {[(5-Hydroxy-6-methyl-2-{[1-(4'-methylbiphenyl-4-yl)piperidin-4-yl]methyl}pyrimidin-4-yl)carbonyl]amino}acetic acid

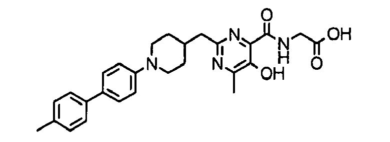

In accordance with Examples 1-(8) to 1-(11), but using 4-bromo-4'-methylbiphenyl (0.57 g, 2.3 mmol) instead of 1-bromo-4-tert-butylbenzene, the title compound (0.16 g, 0.34 mmol) was afforded as a pale yellowish white solid (yield 29%).
MS m/z: 475 (M+H)⁺;
1H-NMR (500 MHz, DMSO-d6) δ: 12.89 (1H, brs), 11.91 (1H, s), 9.41 (1H, t, J=6 Hz), 7.48 (2H, d, J=8 Hz), 7.47 (2H, d, J=8 Hz), 7.21 (2H, d, J=8 Hz), 6.98 (2H, d, J=8 Hz), 4.01 (2H, d, J=6 Hz), 3.73 (2H, d, J=12 Hz), 2.78 (2H, d, J=7 Hz), 2.69 (2H, t, J=12 Hz), 2.44 (3H, s), 2.31 (3H, s), 2.15-2.04 (1H, m), 1.68 (2H, d, J=12 Hz), 1.37 (2H, dq, J=12 Hz, 3 Hz).

### Example 48

### {[(2-{[1-(4'-tert-Butylbiphenyl-4-yl)piperidin-4-yl]methyl}-5-hydroxy-6-methylpyrimidin-4-yl)carbonyl]amino}acetic acid

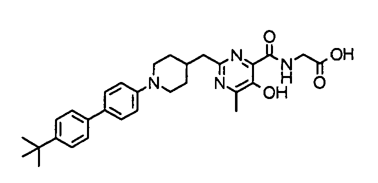

In accordance with Examples 1-(8) to 1-(11), but using 4-bromo-4'-tert-butylbiphenyl (0.67 g, 2.3 mmol) instead of 1-bromo-4-tert-butylbenzene, the title compound (0.05 g, 0.10 mmol) was afforded as a pale yellowish white solid (yield 8%).
MS m/z: 517 (M+H)⁺;
1H-NMR (500 MHz, DMSO-d6) δ: 12.91 (1H, brs), 11.92 (1H, brs), 9.40 (1H, t, J=6 Hz), 7.51 (2H, d, J=8 Hz), 7.48 (2H, d, J=8 Hz), 7.42 (2H, d, J=8 Hz), 6.99 (2H, d, J=8 Hz), 4.00 (2H, d, J=6 Hz), 3.73 (2H, d, J=12 Hz), 2.79 (2H, d, J=7 Hz), 2.70 (2H, t, J=12 Hz), 2.45 (3H, s), 2.14-2.04 (1H, m), 1.69 (2H, d, J=12 Hz), 1.37 (2H, dq, J=12 Hz, 3 Hz), 1.30 (9H, s).

### Example 49

### {[(2-{[1-(4'-Cyanobiphenyl-4-yl)piperidin-4-yl]methyl}-5-hydroxy-6-methylpyrimidin-4-yl)carbonyl]amino acetic acid

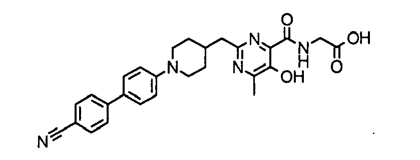

In accordance with Examples 1-(8) to 1-(11), but using 4'-bromobiphenyl-4-carbonitrile (0.69 g, 2.7 mmol) instead of 1-bromo-4-tert-butylbenzene, the title compound (0.14 g, 0.29 mmol) was afforded as a pale yellowish white solid (yield 13%).
MS m/z: 486 (M+H)⁺;
1H-NMR (500 MHz, DMSO-d6) δ: 12.88 (1H, brs), 11.91 (1H, s), 9.40 (1H, t, J=6 Hz), 7.84 (2H, d, J=8 Hz), 7.81 (2H, d, J=8 Hz), 7.63 (2H, d, J=8 Hz), 7.03 (2H, d, J=8 Hz), 4.01 (2H, d, J=6 Hz), 3.82 (2H, d, J=12 Hz), 2.81-2.72 (4H, m), 2.44 (3H, s), 2.19-2.07 (1H, m), 1.68 (2H, d, J=12 Hz), 1.35 (2H, q, J=12 Hz).

### Example 50

### {[(5-Hydroxy-2-{[1-(2'-methoxybiphenyl-4-yl)piperidin-4-yl]methyl}-6-methylpyrimidin-4-yl)carbonyl]amino} acetic acid

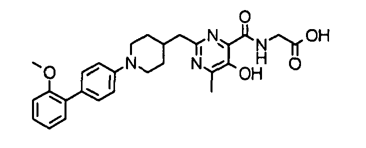

In accordance with Examples 1-(8) to 1-(11), but using 4'-bromo-2-methoxybiphenyl (0.73 g, 2.8 mmol) instead of 1-bromo-4-tert-butylbenzene, the title compound (0.35 g, 0.71 mmol) was afforded as a pale yellowish white solid (yield 31%).
MS m/z: 491 (M+H)⁺;
1H-NMR (500 MHz, DMSO-d6) δ: 12.88 (1H, s), 11.91 (1H, s), 9.41 (1H, t, J=6 Hz), 7.32 (2H, dd, J=9 Hz, 2 Hz), 7.28-7.22 (2H, m), 7.06 (1H, d, J=8 Hz), 7.00-6.95 (1H, m), 6.94 (2H, dd, J=9 Hz, 2 Hz), 4.01 (2H, d, J=6 Hz), 3.74 (3H, s), 3.72 (2H, d, J=12 Hz), 2.79 (2H, d, J=7 Hz), 2.69 (2H, t, J=12 Hz), 2.45 (3H, s), 2.14-2.04 (1H, m), 1.68 (2H, d, J=12 Hz), 1.37 (2H, q, J=12 Hz).

### Example 51

### {[(5-Hydroxy-2-{[1-(3'-methoxybiphenyl-4-yl)piperidin-4-yl]methyl}-6-methylpyrimidin-4-yl)carbonyl]amino}acetic acid

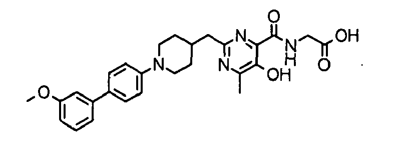

In accordance with Examples 1-(8) to 1-(11), but using 4'-bromo-3-methoxybiphenyl (0.73 g, 2.8 mmol) instead of 1-bromo-4-tert-butylbenzene, the title compound (0.42 g, 0.86 mmol) was afforded as a pale yellowish white solid (yield 37%).
MS m/z: 491 (M+H)⁺;
1H-NMR (500 MHz, DMSO-d6) δ: 12.88 (1H, s), 11.91 (1H, s), 9.43-9.38 (1H, m), 7.51 (2H, dd, J=9 Hz, 3 Hz), 7.33-7.28 (1H, m), 7.17-7.13 (1H, m), 7.11-7.08 (1H, m), 6.99 (2H, dd, J=9 Hz, 3 Hz), 6.85-6.81 (1H, m), 4.02-3.98 (2H, m), 3.80 (3H, s), 3.74 (2H, d, J=12 Hz), 2.78 (2H, d, J=7 Hz), 2.70 (2H, t, J=12 Hz), 2.44 (3H, s), 2.15-2.04 (1H, m), 1.68 (2H, d, J=12 Hz), 1.36 (2H, q, J=12 Hz).

### Example 52

### {[(5-Hydroxy-2-{[1-(4'-methoxybiphenyl-4-yl)piperidin-4-yl]methyl}-6-methylpyrimidin-4-yl)carbonyl]amino}acetic acid

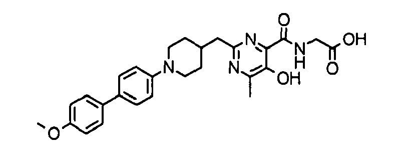

In accordance with Examples 1-(8) to 1-(11), but using 4'-bromo-4-methoxybiphenyl (0.61 g, 2.3 mmol) instead of 1-bromo-4-tert-butylbenzene, the title compound (0.05 g, 0.10 mmol) was afforded as a pale yellowish white solid (yield 9%).
MS m/z: 491 (M+H)⁺;
1H-NMR (500 MHz, DMSO-d6) δ: 9.07 (1H, brs), 7.51 (2H, d, J=8 Hz), 7.45 (2H, d, J=8 Hz), 6.97 (4H, d, J=8 Hz), 3.77 (3H, s), 3.71 (2H, d, J=12 Hz), 3.62 (2H, brs), 2.76 (2H, d, J=7 Hz), 2.67 (2H, t, J=12 Hz), 2.42 (3H, s), 2.09-1.98 (1H, m), 1.69 (2H, d, J=12 Hz), 1.37 (2H, q, J=12 Hz).

### Example 53

### {[(2-{[1-(2',4'-Difluorobiphenyl-4-yl)piperidin-4-yl]methyl}-5-hydroxy-6-methylpyrimidin-4-yl)carbonyl]amino} acetic acid

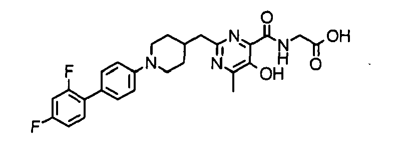

In accordance with Examples 1-(8) to 1-(11), but using 4'-bromo-2,4-difluorobiphenyl (0.75 g, 2.8 mmol) instead of 1-bromo-4-tert-butylbenzene, the title compound (0.49 g, 0.99 mmol) was afforded as a pale yellowish white solid (yield 43%).
MS m/z: 497 (M+H)⁺;
1H-NMR (500 MHz, DMSO-d6) δ: 12.8 (1h, brs), 11.91 (1H, brs), 9.40 (1H, t, J=5 Hz), 7.52 (1H, q, J=9 Hz), 7.36 (2H, d, J=8 Hz), 7.29 (1H, t, J=9 Hz), 7.13 (1H, t, J=9 Hz), 7.01 (2H, d, J=8 Hz), 4.01 (2H, d, J=5 Hz), 3.76 (2H, d, J=12 Hz), 2.78 (2H, d, J=7 Hz), 2.72 (2H, t, J=12 Hz), 2.44 (3H, s), 2.16-2.05 (1H, m), 1.68 (2H, d, J=12 Hz), 1.36 (2H, q, J=12 Hz).

### Example 54

### {[(2-{[1-(2',3'-Dimethoxybiphenyl-4-yl)piperidin-4-yl]methyl}-5-hydroxy-6-methylpyrimidin-4-yl)carbonyl]amino}acetic acid

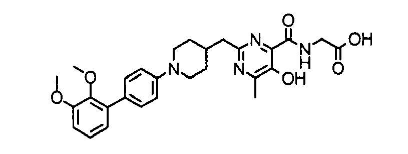

In accordance with Examples 1-(8) to 1-(11), but using 4'-bromo-2,3-dimethoxybiphenyl (0.23 g, 0.79 mmol) instead of 1-bromo-4-tert-butylbenzene, the title compound (0.14 g, 0.27 mmol) was afforded as a white solid (yield 38%).
MS m/z: 521 (M+H)⁺;
1H-NMR (500 MHz, DMSO-d6) δ: 12.89 (1H, brs), 11.91 (1H, s), 9.41 (1H, t, J=6 Hz), 7.40-7.30 (2H, m), 7.10-6.86 (5H, m), 4.01 (2H, d, J=6 Hz), 3.82 (3H, s), 3.73 (2H, d, J=11 Hz), 3.52 (3H, s), 2.80 (2H, d, J=7 Hz), 2.71 (2H, brs), 2.45 (3H, s), 2.16-2.06 (1H, m), 1.76-1.65 (2H, m), 1.46-1.32 (2H, m).

### Example 55

### {[(2-{[1-(4'-Fluoro-2-methylbiphenyl-4-yl)piperidin-4-yl]methyl}-5-hydroxy-6-methylpyrimidin-4-yl)carbonyl]amino}acetic acid

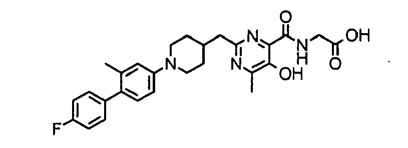

### (1) 4-Bromo-4'-fluoro-2-methylbiphenyl

4-Bromo-1-iodo-2-methylbenzene (10.5 g, 35.4 mmol) was dissolved in 1,2-dimethoxyethane (100 mL), and (4-fluorophenyl)boronic acid (7.4 g, 52.9 mmol), [1,1'-bis(diphenylphosphino)ferrocene]palladium dichloride-dichloromethane adduct (1.5 g, 1.84 mmol) and potassium phosphate hydrate (18.8 g, 88.6 mmol) were added, followed by stirring at room temperature for 16 hours under a nitrogen atmosphere. After the insoluble materials in the reaction solution were filtered off, the filtrate was concentrated under reduced pressure. The resulting residue was purified by silica gel column chromatography (Moritex Corporation, elution solvent: hexane), and a fraction corresponding to the Rf value=0.60 (hexane) by thin layer chromatography was concentrated under reduced pressure to afford the title compound (5.1 g, 19.2 mmol) as a colorless oil (yield 54%).
1H-NMR (500 MHz, CDCl3) δ: 7.42 (1H, s), 7.36 (1H, d, J=8 Hz), 7.27-7.21 (2H, m), 7.13-7.04 (3H, m), 2.22 (3H, s).

### (2) {[(2-{[1-(4'-Fluoro-2-methylbiphenyl-4-yl)piperidin-4-yl]methyl}-5-hydroxy-6-methylpyrimidin-4-yl)carbonyl]amino} acetic acid

In accordance with Examples 1-(8) to 1-(11), but using 4-bromo-4'-fluoro-2-methylbiphenyl (5.5 g, 20.7 mmol) instead of 1-bromo-4-tert-butylbenzene, the title compound (3.79 g, 7.69 mmol) was afforded as a pale yellowish white solid (yield 45%).
MS m/z: 493 (M+H)⁺;
1H-NMR (500 MHz, DMSO-d6) δ: 12.87 (1H, brs), 11.91 (1H, s), 9.41 (1H, t, J=6 Hz), 7.34-7.28 (2H, m), 7.24-7.18 (2H, m), 7.02 (1H, d, J=8 Hz), 6.84 (1H, s), 6.80 (1H, d, J=8 Hz), 4.01 (2H, d, J=6 Hz), 3.71 (2H, d, J=12 Hz), 2.78 (2H, d, J=7 Hz), 2.67 (2H, t, J=12 Hz), 2.44 (3H, s), 2.18 (3H, s), 2.14-2.03 (1H, m), 1.68 (2H, d, J=12 Hz), 1.36 (2H, q, J=12 Hz).

### Example 56

### {[(2-{[1-(4-Benzylphenyl)piperidin-4-yl]methyl}-5-hydroxy-6-methylpyrimidin-4-yl)carbonyl]amino} acetic acid

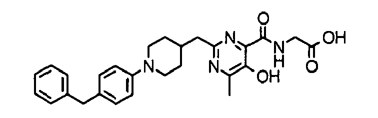

### (1) Ethyl {[(2-{[1-(4-benzylphenyl)piperidin-4-yl]methyl}-5-hydroxy-6-methylpyrimidin-4-yl)carbonyl] amino} acetate

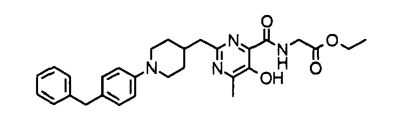

In accordance with Examples 1-(8) to 1-(10), but using 1-benzyl-4-bromobenzene (0.80 g, 3.2 mmol) instead of 1-bromo-4-tert-butylbenzene, the title compound (0.34 g, 0.68 mmol) was afforded as a pale yellow solid (yield 43%). 1H-NMR (400 MHz, CDCl3) δ: 11.35 (1H, s), 8.48 (1H, t, J=6 Hz), 7.30-7.23 (2H, m), 7.20-7.14 (3H, m), 7.06 (2H, d, J=9 Hz), 6.87 (2H, d, J=9 Hz), 4.28 (2H, q, J=7 Hz), 4.22 (2H, d, J=6 Hz), 3.90 (2H, s), 3.62 (2H, d, J=12 Hz), 2.82 (2H, d, J=7 Hz), 2.67 (2H, t, J=12 Hz), 2.53 (3H, s), 2.07-1.96 (1H, m), 1.75 (2H, d, J=12 Hz), 1.57-1.43 (2H, m), 1.32 (3H, t, J=7 Hz).

### (2) {[(2-{[1-(4-Benzylphenyl)piperidin-4-yl]methyl}-5-hydroxy-6-methylpyrimidin-4-yl)carbonyl]amino} acetic acid

In accordance with Example 1-(11), but using ethyl {[(2-{[1-(4-benzylphenyl)piperidin-4-yl]methyl}-5-hydroxy-6-methylpyrimidin-4-yl)carbonyl]amino}acetate (0.32 g, 0.63 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.23 g, 0.48 mmol) was afforded as a white solid (yield 76%).
MS m/z: 475 (M+H)⁺;
1H-NMR (400 MHz, DMSO-d6) δ: 11.86 (1H, brs), 9.35 (1H, t, J=6 Hz), 7.27-7.21 (2H, m), 7.19-7.11 (3H, m), 7.02 (2H, d, J=9 Hz), 6.81 (2H, d, J=9 Hz), 3.98 (2H, d, J=6 Hz), 3.79 (2H, s), 3.57 (2H, d, J=12 Hz), 2.74 (2H, d, J=7 Hz), 2.57 (2H, t, J=12 Hz), 2.41 (3H, s), 2.08-1.95 (1H, m), 1.63 (2H, d, J=12 Hz), 1.32 (2H, dq, J=12 Hz, 3 Hz).

### Example 57

### ({[2-({1-[4-(4-Fluorobenzyl)phenyl]piperidin-4-yl}methyl)-5-hydroxy-6-methylpyrimidin-4-yl]carbonyl}amino)acetic acid

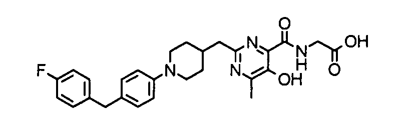

In accordance with Examples 1-(8) to 1-(11), but using 1-bromo-4-(4-fluorobenzyl)benzene (0.78 g, 2.9 mmol) instead of 1-bromo-4-tert-butylbenzene, the title compound (0.40 g, 0.81 mmol) was afforded as a pale yellowish white solid (yield 35%).
MS m/z: 493 (M+H)⁺;
1H-NMR (500 MHz, DMSO-d6) δ: 12.86 (1H, s), 11.90 (1H, s), 9.38 (1H, t, J=6 Hz), 7.21 (2H, t, J=8 Hz), 7.08 (2H, t, J=8 Hz), 7.03 (2H, d, J=8 Hz), 6.84 (2H, d, J=8 Hz), 4.00 (2H, d, J=6 Hz), 3.80 (2H, s), 3.59 (2H, d, J=12 Hz), 2.77 (2H, d, J=7 Hz), 2.59 (2H, t, J=12 Hz), 2.43 (3H, s), 2.09-1.98 (1H, m), 1.65 (2H, d, J=12 Hz), 1.34 (2H, q, J=12 Hz).

### Example 58

### ({[2-({1-[4-(4-Chlorobenzyl)phenyl]piperidin-4-yl}methyl)-5-hydroxy-6-methylpyrimidin-4-yl]carbonyl}amino)acetic acid

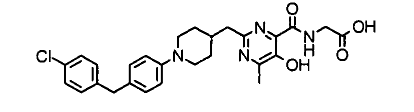

In accordance with Examples 1-(8), 1-(9), 6-(2) and 1-(11), but using 1-bromo-4-(4-chlorobenzyl)benzene (0.78 g, 2.8 mmol) instead of 1-bromo-4-tert-butylbenzene, the title compound (0.59 g, 1.60 mmol) was afforded as a pale yellowish white solid (yield 50%).
MS m/z: 509 (M+H)⁺;
1H-NMR (500 MHz, DMSO-d6) δ: 12.88 (1H, s), 11.91 (1H, s), 9.42-9.37 (1H, m), 7.33 (2H, dd, J=8 Hz, 3 Hz), 7.22 (2H, dd, J=8 Hz, 3 Hz), 7.04 (2H, dd, J=8 Hz, 3 Hz), 6.85 (2H, dd, J=8 Hz, 3 Hz), 4.02-3.98 (2H, m), 3.81 (2H, s), 3.60 (2H, d, J=12 Hz), 2.77 (2H, d, J=7 Hz), 2.59 (2H, t, J=12 Hz), 2.44 (3H, s), 2.09-1.99 (1H, m), 1.65 (2H, d, J=12 Hz), 1.34 (2H, q, J=12 Hz).

### Example 59

### ({[5-Hydroxy-6-methyl-2-({1-[4-(2-methylbenzyl)phenyl]piperidin-4-yl}methyl)pyrimidin-4-yl]carbonyl}amino)acetic acid

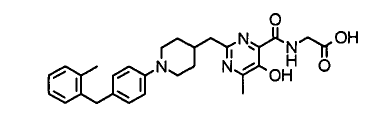

In accordance with Examples 1-(8) to 1-(11), but using 1-bromo-4-(2-methylbenzyl)benzene (0.73 g, 2.8 mmol) instead of 1-bromo-4-tert-butylbenzene, the title compound (0.51 g, 1.04 mmol) was afforded as a pale yellowish white solid (yield 45%).
MS m/z: 489 (M+H)⁺;
1H-NMR (500 MHz, DMSO-d6) δ: 12.87 (1H, s), 11.90 (1H, s), 9.39 (1H, t, J=5 Hz), 7.15-7.07 (4H, m), 6.95 (2H, d, J=8 Hz), 6.83 (2H, d, J=8 Hz), 4.00 (2H, d, J=5 Hz), 3.82 (2H, s), 3.58 (2H, d, J=12 Hz), 2.76 (2H, d, J=7 Hz), 2.58 (2H, t, J=12 Hz), 2.43 (3H, s), 2.19 (3H, s), 2.08-1.97 (1H, m), 1.65 (2H, d, J=12 Hz), 1.34 (2H, q, J=12 Hz).

### Example 60

### ({[5-Hydroxy-6-methyl-2-({1-[4-(3-methylbenzyl)phenyl]piperidin-4-yl}methyl)pyrimidin-4-yl]carbonyl}amino)acetic acid

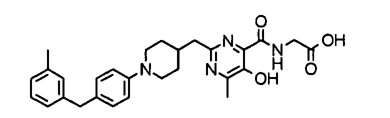

In accordance with Examples 1-(8) to 1-(11), but using 1-bromo-4-(3-methylbenzyl)benzene (0.71 g, 2.7 mmol) instead of 1-bromo-4-tert-butylbenzene, the title compound (0.46 g, 0.94 mmol) was afforded as a pale yellowish white solid (yield 41%).
MS m/z: 489 (M+H)⁺;
1H-NMR (500 MHz, DMSO-d6) δ: 12.87 (1H, s), 11.90 (1H, s), 9.39 (1H, t, J=5 Hz), 7.14 (1H, t, J=8 Hz), 7.03 (2H, d, J=8 Hz), 7.02-6.95 (3H, m), 6.83 (2H, d, J=8 Hz), 4.00 (2H, d, J=5 Hz), 3.76 (2H, s), 3.59 (2H, d, J=12 Hz), 2.77 (2H, d, J=7 Hz), 2.59 (2H, t, J=12 Hz), 2.43 (3H, s), 2.25 (3H, s), 2.08-1.98 (1H, m), 1.65 (2H, d, J=12 Hz), 1.34 (2H, q, J=12 Hz).

### Example 61

### ({[5-Hydroxy-6-methyl-2-({1-[4-(4-methylbenzyl)phenyl]piperidin-4-yl}methyl)pyrimidin-4-yl]carbonyl}amino)acetic acid

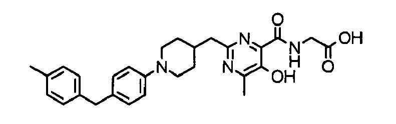

In accordance with Examples 1-(8) to 1-(11), but using 1-bromo-4-(4-methylbenzyl)benzene (0.73 g, 2.8 mmol) instead of 1-bromo-4-tert-butylbenzene, the title compound (0.46 g, 0.94 mmol) was afforded as a pale yellowish white solid (yield 41%).
MS m/z: 489 (M+H)⁺;
1H-NMR (500 MHz, DMSO-d6) δ: 12.86 (1H, s), 11.90 (1H, s), 9.38 (1H, t, J=6 Hz), 7.06 (4H, s), 7.01 (2H, d, J=8 Hz), 6.82 (2H, d, J=8 Hz), 4.00 (2H, d, J=6 Hz), 3.76 (2H, s), 3.58 (2H, d, J=12 Hz), 2.76 (2H, d, J=7 Hz), 2.58 (2H, t, J=12 Hz), 2.43 (3H, s), 2.24 (3H, s), 2.08-1.98 (1H, m), 1.65 (2H, d, J=12 Hz), 1.34 (2H, q, J=12 Hz).

### Example 62

### ({[5-Hydroxy-2-({1-[4-(4-methoxybenzyl)phenyl]piperidin-4-yl}methyl)-6-methylpyrimidin-4-yl]carbonyl}amino)acetic acid

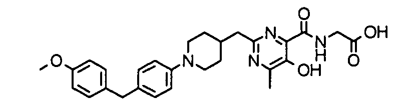

In accordance with Examples 1-(8) to 1-(11), but using 1-promo-4-(4-methoxybenzyl)benzene (1.18 g, 4.3 mmol) instead of 1-bromo-4-tert-butylbenzene, the title compound (0.56 g, 1.11 mmol) was afforded as a pale yellowish white solid (yield 32%).
MS m/z: 505 (M+H)⁺;
1H-NMR (500 MHz, DMSO-d6) δ: 12.87 (1H, s), 11.90 (1H, s), 9.39 (1H, t, J=6 Hz), 7.10 (2H, d, J=8 Hz), 7.01 (2H, d, J=8 Hz), 6.83 (4H, d, J=8 Hz), 4.00 (2H, d, J=6 Hz), 3.74 (2H, s), 3.70 (3H, s), 3.58 (2H, d, J=12 Hz), 2.77 (2H, d, J=7 Hz), 2.58 (2H, t, J=12 Hz), 2.44 (3H, s), 2.09-1.98 (1H, m), 1.65 (2H, d, J=12 Hz), 1.34 (2H, dq, J=12 Hz, 3 Hz).

### Example 63

### ({[2-({1-[4-(4-Cyanobenzyl)phenyl]piperidin-4-yl}methyl)-5-hydroxy-6-methylpyrimidin-4-yl]carbonyl}amino)acetic acid

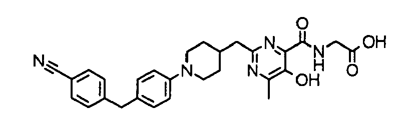

### (1) 4-(4-Bromobenzyl)benzonitrile

1-Bromo-4-(bromomethyl)benzene (2.5 g, 10.0 mmol) was dissolved in toluene (50 mL), and (4-cyanophenyl)boronic acid (2.2 g, 15.0 mmol), tetrakis(triphenylphosphine)palladium adduct (0.58 g, 0.50 mmol) and potassium phosphate hydrate (4.2 g, 19.8 mmol) were added, followed by heating to reflux for 8.5 hours under a nitrogen atmosphere. After the reaction solution was cooled to room temperature, the insoluble materials were filtered off, and the filtrate was concentrated under reduced pressure. The resulting residue was purified by silica gel column chromatography (Moritex Corporation, elution solvent: hexane/ethyl acetate), and a fraction corresponding to the Rf value=0.15 (hexane/ethyl acetate=19/1) by thin layer chromatography was concentrated under reduced pressure to afford the title compound (0.47 g, 1.73 mmol) as a white solid (yield 17%).
1H-NMR (500 MHz, CDCl3) δ: 7.58 (2H, d, J=7 Hz), 7.44 (2H, d, J=7 Hz), 7.26 (2H, d, J=7 Hz), 7.03 (2H, d, J=7 Hz), 3.98 (2H, s).

### (2) ({[2-({1-[4-(4-Cyanobenzyl)phenyl]piperidin-4-yl}methyl)-5-hydroxy-6-methylpyrimidin-4-yl]carbonyl}amino)acetic acid

In accordance with Examples 1-(8) to 1-(11), but using 4-(4-bromobenzyl)benzonitrile (0.47 g, 1.73 mmol) instead of 1-bromo-4-tert-butylbenzene, the title compound (0.05 g, 0.10 mmol) was afforded as a pale yellowish white solid (yield 7%).
MS m/z: 500 (M+H)⁺;
1H-NMR (500 MHz, DMSO-d6) δ: 12.88 (1H, s), 11.90 (1H, s), 9.39 (1H, t, J=5 Hz), 7.74 (2H, d, J=8 Hz), 7.40 (2H, d, J=8 Hz), 7.05 (2H, d, J=8 Hz), 6.85 (2H, d, J=8 Hz), 4.00 (2H, d, J=5 Hz), 3.91 (2H, s), 3.60 (2H, d, J=12 Hz), 2.76 (2H, d, J=7 Hz), 2.59 (2H, t, J=12 Hz), 2.43 (3H, s), 2.09-1.98 (1H, m), 1.65 (2H, d, J=12 Hz), 1.33 (2H, q, J=12 Hz).

### Example 64

### {[(2-{[1-(4-Benzyl-3-fluorophenyl)piperidin-4-yl]methyl}-5-hydroxy-6-methylpyrimidin-4-yl)carbonyl]amino} acetic acid

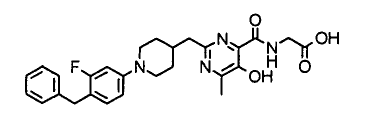

In accordance with Examples 63-(1) and 63-(2), but using 4-promo-1-(bromomethyl)-2-fluorobenzene (2.7 g, 10.1 mmol) instead of 1-bromo-4-(bromomethyl)benzene, and phenylboronic acid (1.8 g, 14.8 mmol) instead of (4-cyanophenyl)boronic acid, the title compound (0.36 g, 0.73 mmol) was afforded as a pale yellowish white solid (yield 7%).
MS m/z: 493 (M+H)⁺;
1H-NMR (500 MHz, DMSO-d6) δ: 12.82 (1H, brs), 11.91 (1H, brs), 9.39 (1H, t, J=5 Hz), 7.30-7.24 (2H, m), 7.20-7.14 (3H, m), 7.07 (1H, t, J=9 Hz), 6.71-6.65 (2H, m), 4.00 (2H, d, J=5 Hz), 3.82 (2H, s), 3.65 (2H, d, J=12 Hz), 2.76 (2H, d, J=7 Hz), 2.64 (2H, t, J=12 Hz), 2.43 (3H, s), 2.11-2.01 (1H, m), 1.64 (2H, d, J=12 Hz), 1.31 (2H, q, J=12 Hz).

### Example 65

### {[(2-{[1-(4-Benzyl-2-methylphenyl)piperidin-4-yl]methyl}-5-hydroxy-6-methylpyrimidin-4-yl)carbonyl]amino acetic acid

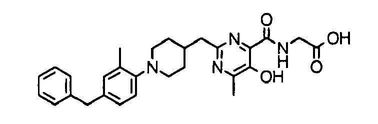

In accordance with Examples 63-(1) and 63-(2), but using 1-bromo-4-(bromomethyl)-2-methylbenzene (2.0 g, 7.6 mmol) instead of 1-bromo-4-(bromomethyl)benzene, and phenylboronic acid (1.4 g, 11.5 mmol) instead of (4-cyanophenyl)boronic acid, the title compound (0.19 g, 0.39 mmol) was afforded as a pale yellowish white solid (yield 5%).
MS m/z: 489 (M+H)⁺;
1 H-NMR (500 MHz, DMSO-d6) δ: 12.88 (1H, brs), 11.90 (1H, s), 9.40 (1H, t, J=5 Hz), 7.29-7.24 (2H, m), 7.20 (2H, d, J=7 Hz), 7.18-7.13 (1H, m), 6.99 (1H, s), 6.96 (1H, d, J=8 Hz), 6.91 (1H, d, J=8 Hz), 4.01 (2H, d, J=5 Hz), 3.81 (2H, s), 2.97 (2H, d, J=12 Hz), 2.79 (2H, d, J=7 Hz), 2.53 (2H, t, J=12 Hz), 2.43 (3H, s), 2.18 (3H, s), 2.06-1.95 (1H, m), 1.65 (2H, d, J=12 Hz), 1.40 (2H, q, J=12 Hz).

### Example 66

### {[(2-{[1-(4-Benzyl-2-bromophenyl)piperidin-4-yl]methyl}-5-hydroxy-6-methylpyrimidin-4-yl)carbonyl]amino}acetic acid

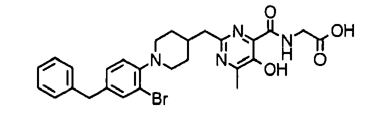

### (1) Ethyl {[(2-{[1-(4-benzyl-2-bromophenyl)piperidin-4-yl]methyl}-5-hydroxy-6-methylpyrimidin-4-yl)carbonyl] amino acetate

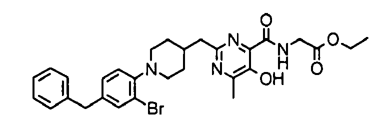

Ethyl {[(2-{[1-(4-benzylphenyl)piperidin-4-yl]methyl}-5-hydroxy-6-methylpyrimidin-4-yl)carbonyl]amino}acetate (0.36 g, 0.72 mmol) obtained in Example 56-(1) was dissolved in N,N-dimethylformamide (13 mL), and at room temperature N-bromosuccinimide (0.14 g, 0.79 mmol) was added, followed by stirring at 50°C for 2 hours. N-Bromosuccinimide (0.10 g, 0.56 mmol) was added to the reaction solution, followed by further stirring for 2 hours. Saturated aqueous sodium hydrogencarbonate solution was added to the reaction solution, followed by extraction with ethyl acetate, and the extract was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.70 (hexane/ethyl acetate=2/1) by thin layer chromatography was concentrated under reduced pressure to afford the title compound (0.13 g, 0.22 mmol) as a yellow oil (yield 31%).
1H-NMR (500 MHz, DMSO-d6) δ: 11.35 (1H, s), 8.50 (1H, t, J=6 Hz), 7.38 (1H, s), 7.30-7.16 (5H, m), 7.04 (1H, d, J=10 Hz), 6.94 (1H, d, J=8 Hz), 4.28 (2H, q, J=7 Hz), 4.22 (2H, d, J=6 Hz), 3.88 (2H, s), 3.30 (2H, d, J=11 Hz), 2.84 (2H, d, J=7 Hz), 2.60 (2H, t, J=12 Hz), 2.53 (3H, s), 2.05-1.97 (1H, m), 1.74-1.70 (2H, m), 1.60-1.53 (2H, m), 1.33 (3H, t, J=7 Hz).

### (2) {[(2-{[1-(4-Benzyl-2-bromophenyl)piperidin-4-yl]methyl}-5-hydroxy-6-methylpyrimidin-4-yl)carbonyl]amino} acetic acid

In accordance with Example 1-(11), but using ethyl {[(2-{[1-(4-benzyl-2-bromophenyl)piperidin-4-yl]methyl}-5-hydroxy-6-methylpyrimidin-4-yl)carbonyl]amino}acetate (0.13 g, 0.22 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.085 g, 0.15 mmol) was afforded as a white solid (yield 68%).
MS m/z: 553 (M+H)⁺;
1H-NMR (500 MHz, DMSO-d6) δ: 9.31 (1H, brs), 7.42 (1H, s), 7.3 0-7.17 (6H, m), 7.06 (1H, d, J=8 Hz), 3.90 (2H, brs), 3.86 (2H, s), 3.16 (2H, d, J=11 Hz), 2.78 (2H, d, J=7 Hz), 2.57 (2H, t, J=10 Hz), 2.43 (3H, s), 2.06-1.97 (1H, m), 1.66 (2H, d, J=13 Hz), 1.47-1.37 (2H, m).

### Example 67

### {[(2-{[1-(3-Benzylphenyl)piperidin-4-yl]methyl}-5-hydroxy-6-methylpyrimidin-4-yl)carbonyl]amino} acetic acid

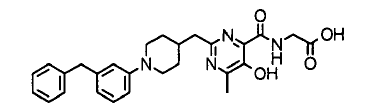

In accordance with Examples 1-(8) to 1-(11), but using 1-benzyl-3-bromobenzene (1.0 g, 4.0 mmol) instead of 1-bromo-4-tert-butylbenzene, the title compound (0.60 g, 1.26 mmol) was afforded as a pale yellowish white solid (yield 36%).
MS m/z: 475 (M+H)⁺;
1H-NMR (500 MHz, Pyridine-d5) δ: 12.49 (1H, brs), 10.41 (1H, t, J=6 Hz), 7.35-7.20 (6H, m), 7.00 (1H, s), 6.88 (1H, d, J=7 Hz), 6.80 (1H, d, J=7 Hz), 4.66 (2H, d, J=6 Hz), 3.98 (2H, s), 3.63 (2H, d, J=12 Hz), 2.75 (2H, d, J=7 Hz), 2.57 (2H, t, J=12 Hz), 2.55 (3H, s), 2.06-1.96 (1H, m), 1.63 (2H, d, J=12 Hz), 1.35 (2H, dq, J=12 Hz, 3 Hz).

### Example 68

### ({[5-Hydroxy-6-methyl-2-({1-[4-(2-phenylethyl)phenyl]piperidin-4-yl}methyl)pyrimidin-4-yl]carbonyl}amino)acetic acid

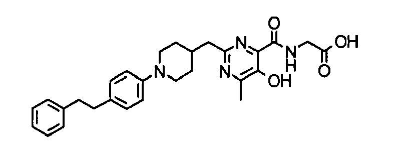

In accordance with Examples 1-(8), 1-(9), 6-(2) and 1-(11), but using 1-bromo-4-(2-phenylethyl)benzene (1.0 g, 3.9 mmol) instead of 1-bromo-4-tert-butylbenzene, the title compound (0.37 g, 0.76 mmol) was afforded as a white solid (yield 25%).
MS m/z: 489 (M+H)⁺;
1H-NMR (500 MHz, DMSO-d6) δ: 11.90 (1H, brs), 9.35 (1H, t, J=8 Hz), 7.30-7.12 (5H, m), 7.04 (2H, d, J=11 Hz), 6.83 (2H, d, J=11 Hz), 3.95 (2H, d, J=8 Hz), 3.59 (2H, d, J=15 Hz), 2.80 (2H, t, J=11 Hz), 2.85-2.72 (4H, m), 2.59 (2H, t, J=12 Hz), 2.44 (3H, s), 2.08-1.98 (1H, m), 1.66 (2H, d, J=11 Hz), 1.35 (2H, dq, J=13 Hz, 3 Hz).

### Example 69

### {[(5-Hydroxy-6-methyl-2-{[1-(1-naphthyl)piperidin-4-yl]methyl}pyrimidin-4-yl)carbonyl]amino}acetic acid

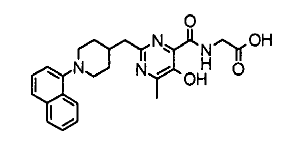

In accordance with Examples 1-(8) to 1-(11), but using 1-bromonaphthalene (0.28 mL, 2.0 mmol) instead of 1-bromo-4-tert-butylbenzene, the title compound (0.20 g, 0.47 mmol) was afforded as a white solid (yield 47%).
MS m/z: 435 (M+H)⁺;
1H-NMR (500 MHz, CDCl3) δ: 11.30 (1H, s), 8.52 (1H, t, J=5 Hz), 8.18 (1H, d, J=8 Hz), 7.80 (1H, d, J=8 Hz), 7.52 (1H, d, J=8 Hz), 7.47-7.43 (2H, m), 7.37 (1H, d, J=8 Hz), 7.06 (1H, d, J=8 Hz), 4.30 (2H, d, J=5 Hz), 3.41 (2H, d, J=11 Hz), 2.92 (2H, d, J=7 Hz), 2.76 (2H, t, J=11 Hz), 2.55 (3H, s), 2.15-2.05 (1H, m), 1.84-1.70 (4H, m).

### Example 70

### {[(5-Hydroxy-6-methyl-2-{[1-(2-naphthyl)piperidin-4-yl]methyl}pyrimidin-4-yl)carbonyl]amino} acetic acid hydrochloride

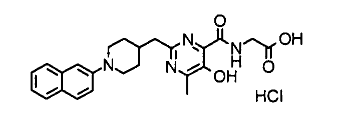

In accordance with Examples 1-(8) to 1-(11), but using 2-bromonaphthalene (0.41 g, 2.0 mmol) instead of 1-bromo-4-tert-butylbenzene, the title compound (0.33 g, 0.67 mmol) was afforded as a white solid (yield 67%).
MS m/z: 435 (M+H)⁺;
1H-NMR (500 MHz, CDCl3/DMSO-d6=5/1) δ: 11.64 (1H, s), 8.74 (1H, brs), 8.50 (1H, brs), 7.99 (1H, d, J=9 Hz), 7.94-7.89 (3H, m), 7.61-7.59 (2H, m), 4.19 (2H, d, J=5 Hz), 3.75 (2H, d, J=12 Hz), 3.59-3.51 (2H, m), 3.00 (2H, d, J=5 Hz), 2.64-2.53 (1H, m), 2.48-2.39 (2H, m), 2.53 (3H, s), 2.05-1.97 (2H, m).

### Example 71

### {[(5-Hydroxy-2-{[(1-(isoquinolin-5-yl)piperidin-4-yl]methyl}-6-methylpyrimidin-4-yl)carbonyl]amino}acetic acid

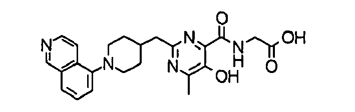

In accordance with Examples 1-(8) to 1-(11), but using 5-bromoisoquinoline (0.42 g, 2.0 mmol) instead of 1-bromo-4-tert-butylbenzene, the title compound (69 mg, 0.16 mmol) was afforded as a pale yellow solid (yield 16%).
MS m/z: 436 (M+H)⁺;
1H-NMR (400 MHz, DMSO-d6) δ: 12.89 (1H, brs), 11.91 (1H, brs), 9.42 (1H, t, J=6 Hz), 9.25 (1H, s), 8.50 (1H, d, J=6 Hz), 7.86 (1H, d, J=6 Hz), 7.74 (1H, d, J=8 Hz), 7.57 (1H, t, J=8 Hz), 7.32 (1H, d, J=8 Hz), 4.02 (2H, d, J=6 Hz), 3.35-3.29 (2H, m), 2.88 (2H, d, J=7 Hz), 2.75 (2H, t, J=12 Hz), 2.46 (3H, s), 2.19-2.09 (1H, m), 1.78 (2H, d, J=12 Hz), 1.69-1.57 (2H, m).

### Example 72

### {[(5-Hydroxy-6-methyl-2-{[1-(quinolin-6-yl)piperidin-4-yl]methyl}pyrimidin-4-yl)carbonyl]amino}acetic acid

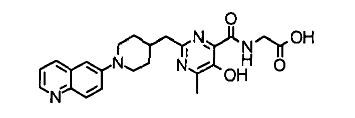

In accordance with Examples 1-(8) to 1-(11), but using 6-bromoquinoline (0.27 mL, 2.0 mmol) instead of 1-bromo-4-tert-butylbenzene, the title compound (44 mg, 0.10 mmol) was afforded as a pale yellow solid (yield 10%).
MS m/z: 436 (M+H)⁺;
1H-NMR (500 MHz, DMSO-d6) δ: 12.89 (1H, brs), 11.92 (1H, s), 9.41 (1H, t, J=6 Hz), 8.61 (1H, d, J=4 Hz), 8.11 (1H, d, J=8 Hz), 7.81 (1H, d, J=9 Hz), 7.60 (1H, dd, J=9 Hz, 3 Hz), 7.37 (1H, dd, J=8 Hz, 4 Hz), 7.16 (1H, d, J=3 Hz), 4.01 (2H, d, J=6 Hz), 3.86 (2H, d, J=12 Hz), 2.82-2.77 (4H, m), 2.45 (3H, s), 2.18-2.11 (1H, m), 1.73 (2H, d, J=12 Hz), 1.47-1.37 (2H, m).

### Example 73

### {[(2-{[1-(1-Benzothiophen-5-yl)piperidin-4-yl]methyl}-5-hydroxy-6-methylpyrimidin-4-yl)carbonyl]amino}acetic acid

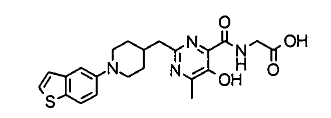

### (1) tert-Butyl2-{[1-(1-benzothiophen-5-yl)piperidin-4-yl]methyl}-5-(benzyloxy)-6-methylpyrimidine-4-carboxylate

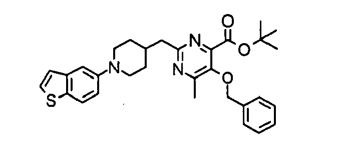

In accordance with Example 1-(8), but using 5-bromo-1-benzothiophene (0.49 g, 2.3 mmol) instead of 1-bromo-4-tert-butylbenzene, the title compound (0.29 g, 0.66 mmol) was afforded as a yellow oil (yield 57%).
1H-NMR (500 MHz, CDCl3) δ: 7.71 (1H, d, J=9 Hz), 7.45-7.36 (6H, m), 7.31 (1H, d, J=2 Hz), 7.22 (1H, d, J=5 Hz), 7.11 (1H, dd, J=9 Hz, 2 Hz), 5.01 (2H, s), 3.67 (2H, d, J=12 Hz), 2.93 (2H, d, J=7 Hz), 2.74 (2H, t, J=12 Hz), 2.47 (3H, s), 2.15-2.06 (1H, m), 1.82 (2H, d, J=12 Hz), 1.62-1.54 (2H, m), 1.60 (9H, s).

### (2) Ethyl {[(2-{[1-(1-benzothiophen-5-yl)piperidin-4-yl]methyl}-5-(benzyloxy)-6-methylpyrimidin-4-yl)carbonyl]amino}acetate

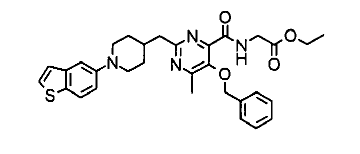

tert-Butyl 2- {[1-(1-benzothiophen-5-yl)piperidin-4-yl]methyl} -5-(benzyloxy)-6-methylpyrimidine-4-carboxylate (0.29 g, 0.66 mmol) was dissolved in a mixture of tetrahydrofuran (2.0 mL) and methanol (2.0 mL), and aqueous potassium hydroxide solution (2 M, 2.0 mL, 4 mmol) was added, followed by stirring at 60°C for 4 hours. After the reaction solution was cooled to room temperature, hydrochloric acid (1 M, 4.0 mL, 4.0 mmol) was added, followed by extraction with ethyl acetate. After the organic layer was dried over anhydrous sodium sulfate, the solvent was distilled off under reduced pressure to afford 2-{[1-(1-benzothiophen-5-yl)piperidin-4-yl]methyl}-5-(benzyloxy)-6-methylpyrimidine-4-carboxylic acid as a yellow oil.

This was dissolved in N,N-dimethylformamide (5 mL), and 1,1'-carbonylbis(1-H-imidazole) (0.16 g, 0.99 mmol) was added, followed by stirring at room temperature for 20 minutes under a nitrogen atmosphere. Glycine ethyl ester hydrochloride (0.14 g, 0.99 mmol) and diisopropylethylamine (0.35 mL, 2.0 mmol) were added to the reaction solution, followed by stirring at room temperature for 2 days. Water was added to the reaction solution, followed by extraction with ethyl acetate, and subsequently the organic layer was washed with saturated aqueous sodium chloride solution and dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.20 (hexane/ethyl acetate=2/1) by thin layer chromatography was concentrated under reduced pressure to afford the title compound (0.32 g, 0.58 mmol) as a yellow oil (yield 88%).
1H-NMR (500 MHz, CDCl3) δ: 8.37 (1H, t, J=5 Hz), 7.72 (1H, d, J=9 Hz), 7.50 (2H, d, J=7 Hz), 7.42-7.34 (4H, m), 7.31 (1H, d, J=2 Hz), 7.23 (1H, d, J=5 Hz), 7.11 (1H, dd, J=9 Hz, 2 Hz), 5.13 (2H, s), 4.29-4.24 (4H, m), 3.68 (2H, d, J=12 Hz), 2.92 (2H, d, J=7 Hz), 2.76 (2H, t, J=12 Hz), 2.47 (3H, s), 2.15-2.05 (1H, m), 1.81 (2H, d, J=12 Hz), 1.59 (2H, dq, J=12 Hz, 3 Hz), 1.32 (3H, t, J=7 Hz).

### (3) {[(2-{[1-(1-Benzothiophen-5-yl)piperidin-4-yl]methyl}-5-hydroxy-6-methylpyrimidin-4-yl)carbonyl]amino}acetic acid

In accordance with Examples 6-(2) and 1-(11), but using ethyl {[(2-{[1-(1-benzothiophen-5-yl)piperidin-4-yl]methyl}-5-(benzyloxy)-6-methylpyrimidin-4-yl)carbonyl]amino}acetate (0.32 g, 0.58 mmol) instead of ethyl ({[5-(benzyloxy)-2-{[1-(2-chlorophenyl)piperidin-4-yl]methyl}-6-methylpyrimidin-4-yl]carbonyl}amino)acetate, the title compound (0.14 g, 0.31 mmol) was afforded as a yellow oil (yield 53%).
MS m/z: 441 (M+H)⁺;
1H-NMR (500 MHz, CDCl3) δ: 11.70 (1H, brs), 8.63 (1H, t, J=5 Hz), 7.82 (1H, d, J=9 Hz), 7.67 (1H, s), 7.48 (1H, d, J=6 Hz), 7.33 (1H, d, J=9 Hz), 7.30 (1H, d, J=6 Hz), 4.23 (2H, d, J=5 Hz), 3.69 (2H, d, J=12 Hz), 2.97-2.87 (4H, m), 2.53 (3H, s), 2.10-2.02 (1H, m), 1.92-1.84 (4H, m).

### Example 74

### {[(5-Hydroxy-6-methyl-2-{[1-(2-methyl-1,3-benzothiazol-5-yl)piperidin-4-yl]methyl}pyrimidin-4-yl)carbonyl]amino}acetic acid

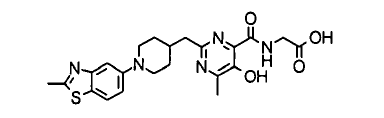

In accordance with Examples 1-(8), 73-(2), 6-(2) and 1-(11), but using 5-chloro-2-methyl-1,3-benzothiazole (422 mg, 2.3 mmol) instead of 1-bromo-4-tert-butylbenzene, the title compound (37 mg, 0.081 mmol) was afforded as a white solid (yield 7%).
MS m/z: 456 (M+H)⁺;
1H-NMR (500 MHz, CDCl3) δ: 11.47 (1H, brs), 8.63 (1H, t, J=5 Hz), 7.64 (1H, d, J=9 Hz), 7.61 (1H, s), 7.17 (1H, d, J=9 Hz), 4.27 (2H, d, J=5 Hz), 3.69 (2H, d, J=12 Hz), 2.89-2.82 (4H, m), 2.83 (3H, s), 2.53 (3H, s), 2.14-2.06 (1H, m), 1.82 (2H, d, J=12 Hz), 1.64 (2H, q, J=12 Hz).

### Example 75

### {[(2-{[1-(1,3-Benzothiazol-2-yl)piperidin-4-yl]methyl}-5-hydroxy-6-methylpyrimidin-4-yl)carbonyl]amino} acetic acid

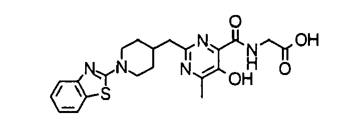

### (1) tert-Butyl 2-{[1-(1,3-benzothiazol-2-yl)piperidin-4-yl]methyl}-5-(benzyloxy)-6-methylpyrimidine-4-carboxylate

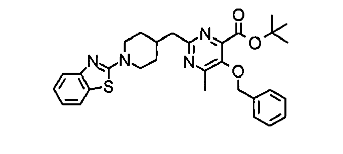

tert-Butyl 5-(benzyloxy)-6-methyl-2-[(piperidin-4-yl)methyl]pyrimidine-4-carboxylate hydrochloride (0.43 g, 1.0 mmol) obtained in Example 1-(7), 2-chloro-1,3-benzothiazole (0.34 g, 2.0 mmol) and diisopropylethylamine (0.38 mL, 2.2 mmol) were suspended in n-butanol (5 mL), followed by stirring at 100°C for 4.5 hours under a nitrogen atmosphere. After the reaction solution was concentrated under reduced pressure, the resulting residue was purified by silica gel column chromatography (Moritex Corporation, elution solvent: hexane/ethyl acetate), and a fraction corresponding to the Rf value=0.21 (hexane/ethyl acetate=2/1) by thin layer chromatography was concentrated under reduced pressure to afford the title compound (0.20 g, 0.38 mmol) as a colorless oil (yield 38%).
1H-NMR (400 MHz, CDCl3) δ: 7.58 (1H, d, J=8 Hz), 7.53 (1H, d, J=8 Hz), 7.43-7.36 (5H, m), 7.28 (1H, t, J=8 Hz), 7.05 (1H, t, J=8 Hz), 5.01 (2H, s), 4.13 (2H, d, J=13 Hz), 3.14 (2H, t, J=13 Hz), 2.90 (2H, d, J=7 Hz), 2.46 (3H, s), 2.29-2.20 (1H, m), 1.81 (2H, d, J=13 Hz), 1.59 (9H, s), 1.54-1.43 (2H, m).

### (2) {[(2-{[1-(1,3-Benzothiazol-2-yl)piperidin-4-yl]methyl}-5-hydroxy-6-methylpyrimidin-4-yl)carbonyl]amino}acetic acid

In accordance with Examples 73-(2), 6-(2) and 1-(11), but using tert-butyl 2-{[1-(1,3-benzothiazol-2-yl)piperidin-4-yl]methyl}-5-(benzyloxy)-6-methylpyrimidine-4-carboxylate (200 mg, 0.38 mmol) instead of tert-butyl 2-{[1-(benzothiophen-5-yl)piperidin-4-yl]methyl}-5-(benzyloxy)-6-methylpyrimidine-4-carboxylate, the title compound (90 mg, 0.20 mmol) was afforded as a white solid (yield 54%).
MS m/z: 442 (M+H)⁺;
1H-NMR (500 MHz, DMSO-d6) δ: 9.17 (1H, brs), 7.74 (1H, d, J=8 Hz), 7.43 (1H, d, J=8 Hz), 7.25 (1H, t, J=8 Hz), 7.04 (1H, t, J=8 Hz), 4.01 (2H, d, J=13 Hz), 3.73 (2H, brs), 3.15 (2H, t, J=13 Hz), 2.78 (2H, d, J=7 Hz), 2.43 (3H, s), 2.25-2.16 (1H, m), 1.74 (2H, d, J=13 Hz), 1.33 (2H, q, J=13 Hz).

### Example 76

### {[(2-{[1-(1,3-benzoxazol-2-yl)piperidin-4-yl]methyl}-5-hydroxy-6-methylpyrimidin-4-yl)carbonyl]amino}acetic acid

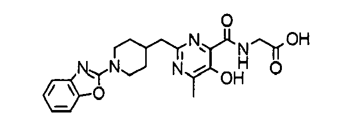

In accordance with Examples 75-(1), 73-(2), 1-(10) and 1-(11), but using 2-chloro-1,3-benzoxazole (0.31 g, 2.0 mmol) instead of 2-chloro-1,3-benzothiazole, the title compound (0.10 g, 0.24 mmol) was afforded as a white solid (yield 24%).
MS m/z: 426 (M+H)⁺;
1H-NMR (500 MHz, CDCl3) δ: 11.43 (1H, brs), 8.57 (1H, t, J=5 Hz), 7.36 (1H, d, J=8 Hz), 7.24 (1H, d, J=8 Hz), 7.17 (1H, t, J=8 Hz), 7.03 (1H, t, J=8 Hz), 4.29-4.24 (4H, m), 3.15 (2H, t, J=13 Hz), 2.83 (2H, d, J=7 Hz), 2.53 (3H, s), 2.26-2.18 (1H, m), 1.81 (2H, d, J=13 Hz), 1.44 (2H, dq, J=13 Hz, 4 Hz).

### Example 77

### {[(5-Hydroxy-6-methyl-2-{[1-(quinoxalin-2-yl)piperidin-4-yl]methyl}pyrimidin-4-yl)carbonyl]amino} acetic acid

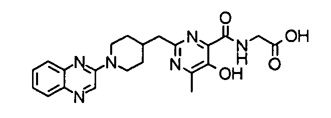

In accordance with Examples 75-(1), 73-(2), 1-(10) and 1-(11), but using 2-chloroquinoxaline (0.49 g, 3.0 mmol) instead of 2-chloro-1,3-benzothiazole, the title compound (0.11 g, 0.25 mmol) was afforded as a yellow solid (yield 13%).
MS m/z: 437 (M+H)⁺;
1H-NMR (500 MHz, CDCl3) δ: 11.43 (1H, brs), 8.63 (1H, s), 8.54 (1H, t, J=5 Hz), 7.85 (1H, d, J=8 Hz), 7.68 (1H, d, J=8 Hz), 7.58 (1H, t, J=8 Hz), 7.38 (1H, t, J=8 Hz), 4.55 (2H, d, J=13 Hz), 4.29 (2H, d, J=5 Hz), 3.05 (2H, t, J=13 Hz), 2.84 (2H, d, J=7 Hz), 2.54 (3H, s), 2.29-2.21 (1H, m), 1.84 (2H, d, J=13 Hz), 1.44 (2H, dq, J=13 Hz, 3 Hz).

### Example 78

### {[(5-Hydroxy-6-methyl-2-{[1-(quinolin-2-yl)piperidin-4-yl]methyl}pyrimidin-4-yl)carbonyl]amino} acetic acid

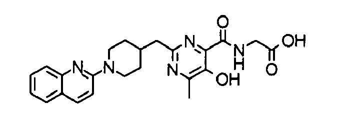

In accordance with Examples 75-(1), 73-(2), 1-(10) and 1-(11), but using 2-chloroquinoline (0.49 g, 3.0 mmol) instead of 2-chloro-1,3-benzothiazole, the title compound (0.039 g, 0.090 mmol) was afforded as a pale yellow solid (yield 5%).
MS m/z: 436 (M+H)⁺;
1 H-NMR (500 MHz, CDCl3) δ: 8.76 (1H, t, J=5 Hz), 7.99 (1H, d, J=8 Hz), 7.91 (1H, d, J=9 Hz), 7.60-7.56 (2H, m), 7.29 (1H, t, J=7 Hz), 6.96 (1H, t, d=9 Hz), 4.46 (2H, d, J=14 Hz), 4.11 (2H, d, J=5 Hz), 3.20 (2H, t, J=14 Hz), 2.78 (2H, d, J=7 Hz), 2.49 (3H, s), 2.31-2.23 (1H, m), 1.92 (2H, d, J=12 Hz), 1.48-1.40 (2H, m).

### Example 79

### {[(2-{[1-(3,3-Dimethyl-2,3-dihydro-1-benzofuran-6-yl)piperidin-4-yl]methyl}-5-hydroxy-6-methylpyrimidin-4-yl)carbonyl]amino}acetic acid

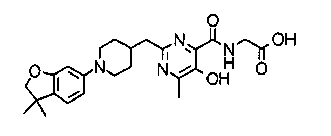

### (1) 3,3-Dimethyl-2,3-dihydro-1-benzofuran-6-yl trifluoromethanesulfonate

3,3-Dimethyl-2,3-dihydro-1-benzofuran-6-ol (1.1 g, 6.7 mmol) was dissolved in methylene chloride (20 mL), and at -78°C trifluoromethanesulfonic anhydride (1.4 mL, 8.0 mmol) and triethylamine (1.1 mL, 8.0 mmol) were added, followed by stirring at the same temperature for 30 minutes. Water was added to the reaction solution, followed by extraction with ethyl acetate, and subsequently the organic layer was dried over anhydrous sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.40 (hexane/ethyl acetate=10/1) by thin layer chromatography was concentrated under reduced pressure to afford the title compound (1.7 g, 5.6 mmol) as a colorless oil (yield 84%).
1H-NMR (500 MHz, CDCl3) δ: 7.10 (1H, d, J=8 Hz), 6.77 (1H, dd, J=8 Hz; 2 Hz), 6.68 (1H, d, J=2 Hz), 4.31 (2H, s), 1.35 (6H, s).

### (2) {[(2-{[1-(3,3-Dimethyl-2,3-dihydro-1-benzofuran-6-yl)piperidin-4-yl]methyl}-5-hydroxy-6-methylpyrimidin-4-yl)carbonyl]amino} acetic acid

In accordance with Examples 1-(8), 73-(2), 1-(10) and 1-(11), but using 3,3-dimethyl-2,3-dihydro-1-benzofuran-6-yl trifluoromethanesulfonate (0.41 g, 1.4 mmol) instead of 1-bromo-4-tert-butylbenzene, the title compound (0.17 g, 0.37 mmol) was afforded as a white solid (yield 32%).
MS m/z: 455 (M+H)⁺;
1H-NMR (500 MHz, DMSO-d6) δ: 12.87 (1H, brs), 11.90 (1H, s), 9.40 (1H, t, J=5 Hz), 6.96 (2H, d, J=8 Hz), 6.41 (2H, d, J=8 Hz), 6.33 (1H, s), 4.13 (2H, s), 4.00 (2H, d, J=5 Hz), 3.56 (2H, d, J=12 Hz), 2.76 (2H, d, J=7 Hz), 2.59 (2H, d, J=12 Hz), 2.44 (3H, s), 2.06-1.98 (1H, m), 1.64 (2H, d, J=12 Hz), 1.33 (2H, q, J=12 Hz), 1.23 (6H, s).

### Example 80

### {[(5-Hydroxy-6-methyl-2-{[1-(1-phenylindolin-5-yl)piperidin-4-yl]methyl}pyrimidin-4-yl)carbonyl]amino} acetic acid

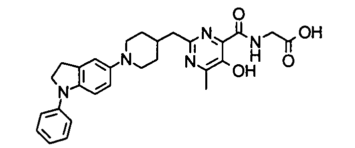

### (1) tert-Butyl 5-(benzyloxy)-2-1[1-(indolin-5-yl)piperidin-4-yl]methyl}-6-methylpyrimidine-4-carboxylate

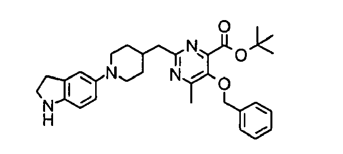

In accordance with Example 1-(8), but using tert-butyl 5-bromoindoline-1-carboxylate (1.4 g, 4.6 mmol) instead of 1-bromo-4-tert-butylbenzene, tert-butyl 5-(benzyloxy)-2-({1-[1-(tert-butoxycarbonyl)indolin-5-yl]piperidin-4-yl}methyl)-6-methylpyrimidine-4-carboxylate (1.2 g, 1.9 mmol) was afforded as a yellow oil (yield 61%).

This was dissolved in ethyl acetate (5 mL), and a solution of hydrogen chloride in ethyl acetate (4 M, 5 mL, 20 mmol) was added, followed by stirring at room temperature for 30 minutes. Saturated aqueous sodium hydrogencarbonate solution was added to the reaction solution, followed by extraction with ethyl acetate, and the organic layer was dried over anhydrous sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.14 (hexane/ethyl acetate=1/1) by thin layer chromatography was concentrated under reduced pressure to afford the title compound (0.65 g, 1.3 mmol) as a brown oil (yield 67%).
1H-NMR (500 MHz, CDCl3) δ: 7.43-7.35 (5H, m), 6.86 (1H, s), 6.68 (1H, d, J=8 Hz), 6.59 (1H, d, J=8 Hz), 5.01 (2H, s), 3.52 (2H, t, J=8 Hz), 3.43 (2H, d, J=12 Hz), 2.99 (2H, t, J=8 Hz), 2.90 (2H, d, J=7 Hz), 2.60 (2H, t, J=12 Hz), 2.46 (3H, s), 2.06-2.00 (1H, m), 1.77 (2H, d, J=12 Hz), 1.59 (9H, s), 1.59-1.51 (2H, m).

### (2) {[(5-Hydroxy-6-methyl-2-{[1-(1-phenylindolin-5-yl)piperidin-4-yl]methyl}pyrimidin-4-yl)carbonyl]amino}acetic acid

In accordance with Examples 1-(8), 73-(2), 1-(10) and 1-(11), but using tert-butyl 5-(benzyloxy)-2-{[1-(indolin-5-yl)piperidin-4-yl]methyl}-6-methylpyrimidine-4-carboxylate (0.32 g, 0.63 mmol) instead of tert-butyl 5-(benzyloxy)-6-methyl-2-[(piperidin-4-yl)methyl]pyrimidine-4-carboxylate hydrochloride, and bromobenzene (0.20 g, 1.3 mmol) instead of 1-bromo-4-tert-butylbenzene, the title compound (0.18 g, 0.36 mmol) was afforded as a pale green solid (yield 57%).
MS m/z: 502 (M+H)⁺;
1H-NMR (500 MHz, DMSO-d6) δ: 8.98 (1H, brs), 7.30 (2H, t, J=8 Hz), 7.15 (2H, d, J=8 Hz), 7.01 (1H, d, J=8 Hz), 6.89 (1H, s), 6.85 (1H, t, J=8 Hz), 6.64 (1H, d, J=8 Hz), 3.85 (2H, t, J=8 Hz), 3.50 (2H, s), 3.44 (2H, d, J=12 Hz), 3.03 (2H, t, J=8 Hz), 2.76 (2H, d, J=7 Hz), 2.56-2.50 (2H, m), 2.42 (3H, s), 1.99-1.90 (1H, m), 1.68 (2H, d, J=11 Hz), 1.41-1.34 (2H, m).

### Example 81

### {[(5-Hydroxy-6-methyl-2-{[1-(5-phenylpyrimidin-2-yl)piperidin-4-yl]methyl}pyrimidin-4-yl)carbonyl]amino}acetic acid

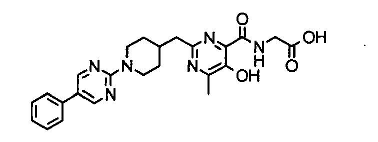

### (1) tert-Butyl 5-(benzyloxy)-2-{[1-(5-bromopyrimidin-2-yl)piperidin-4-yl]methyl}-6-methylpyrimidine-4-carboxylate

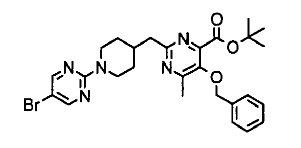

In accordance with Example 75-(1), but using 5-bromo-2-chloropyrimidine (1.3 g, 6.5 mmol) instead of 2-chloro-1,3-benzothiazole, the title compound (1.9 g, 3.3 mmol) was afforded as a yellow oil (yield 67%).
1H-NMR (500 MHz, CDCl3) δ: 8.26 (2H, s), 7.45-7.33 (5H, m), 5.01 (2H, s), 4.65 (2H, d, J=10 Hz), 2.89 (2H, t, J=8 Hz), 2.86 (2H, t, J=8 Hz), 2.46 (3H, s), 2.30-2.15 (1H, m), 1.74 (2H, d, J=10 Hz), 1.59 (9H, s), 1.31 (2H, dq, J=10 Hz, 5 Hz).

### (2) tert-Butyl 5-(benzyloxy)-6-methyl-2-{[1-(5-phenylpyrimidin-2-yl)piperidin-4-yl]methylpyrimidine-4-carboxylate

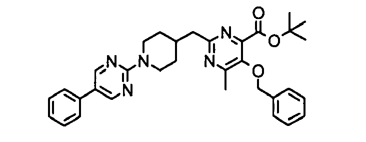

tert-Butyl 5-(benzyloxy)-2-{[1-(5-bromopyrimidin-2-yl)piperidin-4-yl]methyl}-6-methylpyrimidine-4-carboxylate (0.28 g, 0.50 mmol) was dissolved in 1,2-dimethoxyethane (3.0 mL), and phenylboronic acid (0.12 g, 1.0 mmol), [1,1'-bis(diphenylphosphino)ferrocene]palladium dichloride-dichloromethane adduct (0.041 g, 0.05 mmol) and potassium phosphate hydrate (0.23 g, 1.0 mmol) were added, followed by heating to reflux for 4 hours under a nitrogen atmosphere. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.30 (hexane/ethyl acetate=4/1) by thin layer chromatography was concentrated under reduced pressure to afford the title compound (0.23 g, 0.42 mmol) as a colorless oil (yield 83%). 1H-NMR (500 MHz, CDCl3) δ: 8.54 (2H, s), 7.50-7.24 (10H, m), 5.02 (2H, s), 4.79 (2H, d, J=13 Hz), 2.95 (2H, t, J=11 Hz), 2.89 (2H, t, J=8 Hz), 2.46 (3H, s), 2.30-2.15 (1H, m), 1.78 (2H, d, J=13 Hz), 1.59 (9H, s), 1.37 (2H, dq, J=11 Hz, 5 Hz).

### (3) {[(5-Hydroxy-6-methyl-2-{[1-(5-phenylpyrimidin-2-yl)piperidin-4-yl]methyl} pyrimidin-4-yl)carbonyl]amino} acetic acid

In accordance with Examples 1-(9) to 1-(11), but using tert-butyl 5-(benzyloxy)-6-methyl-2- {[1-(5-phenylpyrimidin-2-yl)piperidin-4-yl]methyl}pyrimidine-4-carboxylate (0.23 g, 0.42 mmol) instead of tert-butyl 5-(benzyloxy)-2- {[1-(4-tert-butylphenyl)piperidin-4-yl]methyl} -6-methylpyrimidine-4-carboxylate, the title compound (0.084 g, 0.18 mmol) was afforded as a white solid (yield 43%).
MS m/z: 463 (M+H)⁺;
1H-NMR (500 MHz, DMSO-d6) δ: 9.37 (1H, t, J=5 Hz), 8.69 (2H, s), 7.64 (2H, d, J=8 Hz), 7.44 (2H, d, J=8 Hz), 7.33 (1H, t, J=8 Hz), 4.69 (2H, d, J=12 Hz), 3.98 (2H, d, J=6 Hz), 2.94 (2H, t, J=6 Hz), 2.77 (2H, d, J=7 Hz), 2.44 (3H, s), 2.30-2.19 (1H, m), 1.69 (2H, d, J=12 Hz), 1.21 (2H, dq, J=10 Hz, 5 Hz).

### Example 82

### ({[5-Hydroxy-6-methyl-2-({1-[5-(4-methylphenyl)pyrimidin-2-yl]piperidin-4-yl}methyl)pyrimidin-4-yl]carbonyl}amino)acetic acid

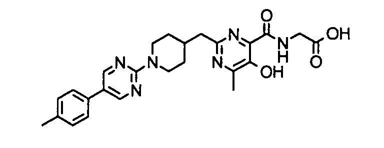

### (1) Ethyl ({[5-(benzyloxy)-2-{[1-(5-bromopyrimidin-2-yl)piperidin-4-yl]methyl}-6-methylpyrimidin-4-yl]carbonyl} amino)acetate

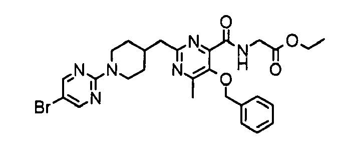

In accordance with Example 1-(9), but using tert-butyl 5-(benzyloxy)-2-{[1-(5-bromopyrimidin-2-yl)piperidin-4-yl]methyl}-6-methylpyrimidine-4-carboxylate (0.55 g, 1.0 mmol) obtained in Example 81-(1) instead of tert-butyl 5-(benzyloxy)-2-{[1-(4-tert-butylphenyl)piperidin-4-yl]methyl}-6-methylpyrimidine-4-carboxylate, the title compound (0.38 g, 0.66 mmol) was afforded as a yellow oil (yield 66%).
1H-NMR (500 MHz, CDCl3) δ: 8.35 (1H, t, J=7 Hz), 8.26 (2H, s), 7.51-7.45 (2H, m), 7.42-7.33 (3H, m), 5.12 (2H, s), 4.67 (2H, d, J=14 Hz), 4.25 (2H, q, J=7 Hz), 4.24 (2H, d, J=7 Hz), 2.89 (2H, t, J=13 Hz), 2.86 (2H, t, J=6 Hz), 2.46 (3H, s), 2.30-2.15 (1H, m), 1.73 (2H, d, J=14 Hz), 1.31 (3H, t, J=8 Hz), 1.27 (2H, dq, J=7 Hz, 3 Hz).

### (2) Ethyl ({[5-(benzyloxy)-6-methyl-2-({1-[5-(4-methylphenyl)pyrimidin-2-yl]piperidin-4-yl}methyl)pyrimidin-4-yl]carbonyl}amino)acetate

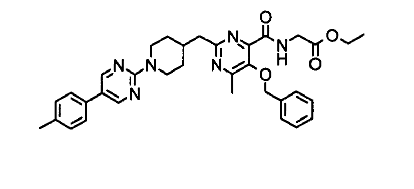

Ethyl ({[5-(benzyloxy)-2-{[1-(5-bromopyrimidin-2-yl)piperidin-4-yl]methyl}-6-methylpyrimidin-4-yl]carbonyl}amino)acetate (0.19 g, 0.32 mmol) was dissolved in 1,2-dimethoxyethane (3.0 mL), and (4-methylphenyl)boronic acid (0.088 g, 0.65 mmol), [1,1'-bis(diphenylphosphino)ferrocene]palladium dichloride-dichloromethane adduct (0.027 g, 0.032 mmol) and potassium phosphate hydrate (150 mg, 0.65 mmol) were added, followed by heating to reflux for 3 hours under a nitrogen atmosphere. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.40 (hexane/ethyl acetate=2/1) by thin layer chromatography was concentrated under reduced pressure to afford the title compound (0.14 g, 0.24 mmol) as a colorless oil (yield 73%).
1H-NMR (500 MHz, CDCl3) δ: 8.52 (2H, s), 8.36 (1H, t, J=6 Hz), 7.51-7.45 (2H, m), 7.42-7.33 (7H, m), 5.13 (2H, s), 4.79 (2H, d, J=13 Hz), 4.26 (2H, q, J=7 Hz), 4.24 (2H, d, J=6 Hz), 2.95 (2H, t, J=13 Hz), 2.89 (2H, t, J=8 Hz), 2.46 (3H, s), 2.39 (3H, s), 2.32-2.22 (1H, m), 1.76 (2H, d, J=13 Hz), 1.36 (2H, dq, J=13 Hz, 3 Hz), 1.32 (3H, t, J=7 Hz).

### (3) ({[5-Hydroxy-6-methyl-2-({1-[5-(4-methylphenyl)pyrimidin-2-yl]piperidin-4-yl}methyl)pyrimidin-4-yl]carbonyl}amino)acetic acid

In accordance with Examples 1-(10) and 1-(11), but using ethyl ({[5-(benzyloxy)-6-methyl-2-({1-[5-(4-methylphenyl)pyrimidin-2-yl]piperidin-4-yl}methyl)pyrimidin-4-yl]carbonyl}amino)acetate (0.14 g, 0.24 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.094 g, 0.20 mmol) was afforded as a white solid (yield 84%).
MS m/z: 477 (M+H)⁺;
1H-NMR (500 MHz, DMSO-d6) δ: 9.12 (1H, t, J=13 Hz), 8.65 (2H, s), 7.52 (2H, d, J=8 Hz), 7.25 (2H, d, J=8 Hz), 4.68 (2H, d, J=13 Hz), 3.70 (2H, d, J=5 Hz), 2.92 (2H, t, J=12 Hz), 2.75 (2H, d, J=7 Hz), 2.42 (3H, s), 2.33 (3H, s), 2.28-2.14 (1H, m), 1.68 (2H, d, J=11 Hz), 1.19 (2H, dq, J=12 Hz, 3 Hz).

### Example 83

### ({[5-Hydroxy-6-methyl-2-({1-[2-(4-methylphenyl)pyrimidin-5-yl]piperidin-4-yl}methyl)pyrimidin-4-yl]carbonyl}amino)acetic acid

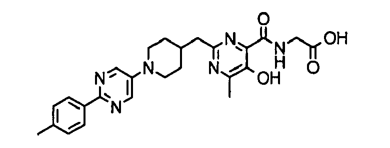

### (1) 5-Bromo-2-(4-methylphenyl)pyrimidine

2-Iodo-5-bromopyrimidine (2.0 g, 7.0 mmol) was dissolved in 1,2-dimethoxyethane (70 mL), and (4-methylphenyl)boronic acid (0.95 g, 7.0 mmol), tetrakis(triphenylphosphine)palladium adduct (0.81 g, 0.70 mmol) and aqueous sodium carbonate solution (2M, 10 mL) were added, followed by heating to reflux for 17 hours under a nitrogen atmosphere. The reaction solution was cooled to room temperature, followed by extraction with ethyl acetate. After the organic layer was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=10/1) by thin layer chromatography was concentrated under reduced pressure to afford the title compound (0.25 g, 1.0 mmol) as a white solid (yield 14%).
1H-NMR (500 MHz, CDCl3) δ: 8.81 (2H, s), 8.29 (2H, d, J=8 Hz), 7.29 (2H, d, J=8 Hz), 2.42 (3H, s).

### (2) ({[5-Hydroxy-6-methyl-2-({1-[2-(4-methylphenyl)pyrimidin-5-yl]piperidin-4-yl}methyl)pyrimidin-4-yl]carbonyl}amino)acetic acid

In accordance with Examples 1-(8) to 1-(11), but using 5-bromo-2-(4-methylphenyl)pyrimidine (0.25 g, 1.0 mmol) instead of 1-bromo-4-tert-butylbenzene, the title compound (0.14 g, 0.29 mmol) was afforded as a yellow solid (yield 29%).
MS m/z: 477 (M+H)⁺;
1H-NMR (500 MHz, DMSO-d6) δ: 9.00 (1H, t, J=9 Hz), 8.55 (2H, s), 8.15 (2H, d, J=6 Hz), 7.26 (2H, d, J=6 Hz), 3.87 (2H, d, J=9 Hz), 3.53 (2H, d, J=8 Hz), 2.79 (2H, t, J=12 Hz), 2.75 (2H, t, J=6 Hz), 2.42 (3H, s), 2.35 (3H, s), 2.13-2.03 (1H, m), 1.73 (2H, d, J=12 Hz), 1.37 (2H, dq, J=11 Hz, 3 Hz).

### Example 84

### {[(5-Hydroxy-6-methyl-2-{[1-(6-phenylpyridazin-3-yl)piperidin-4-yl]methyl}pyrimidin-4-yl)carbonyl]amino}acetic acid

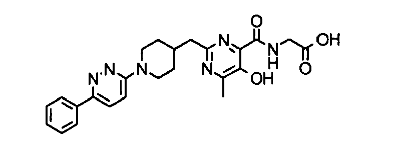

In accordance with Examples 81-(1) to 81-(3), but using 3,6-dichloropyridazine (0.45 g, 3.0 mmol) instead of 5-bromo-2-chloropyrimidine, the title compound (0.11 g, 0.23 mmol) was afforded as a white solid (yield 12%).
MS m/z: 463 (M+H)⁺;
1H-NMR (500 MHz, DMSO-d6) δ: 9.37 (1H, t, J=13 Hz), 8.01 (2H, d, J=8 Hz), 7.91 (1H, d, J=9 Hz), 7.48 (2H, t, J=7 Hz), 7.41 (1H, t, J=7 Hz), 7.34 (1H, d, J=9 Hz), 4.43 (2H, d, J=13 Hz), 3.95 (2H, d, J=7 Hz), 2.95 (2H, t, J=11 Hz), 2.77 (2H, d, J=7 Hz), 2.44 (3H, s), 2.13-2.03 (1H, m), 1.71 (2H, d, J=10 Hz), 1.29 (2H, dq, J=13 Hz, 3 Hz).

### Example 85

### {[(5-Hydroxy-6-methyl-2-{[1-(5-phenylpyrazin-2-yl)piperidin-4-yl]methyl}pyrimidin-4-yl)carbonyl]amino} acetic acid

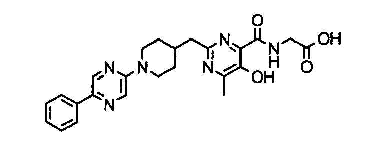

In accordance with Examples 81-(1), 81-(2), 1-(9), 6-(2) and 1-(11), but using 2,5-dichloropyrazine (1.5 g, 10.0 mmol) instead of 5-bromo-2-chloropyrimidine, the title compound (0.22 g, 0.47 mmol) was afforded as a white solid (yield 5.9%).
MS m/z: 463 (M+H)⁺;
1H-NMR (500 MHz, DMSO-d6) δ: 11.94 (1H, brs), 9.41 (1H, t, J=6 Hz), 8.67 (1H, s), 8.40 (1H, s), 7.95 (2H, d, J=8 Hz), 7.44 (2H, t, J=8 Hz), 7.34 (1H, t, J=8 Hz), 4.38 (2H, d, J=12 Hz), 3.99 (2H, d, J=6 Hz), 2.93 (2H, t, J=12 Hz), 2.77 (2H, d, J=7 Hz), 2.45 (3H, s), 2.29-2.19 (1H, m), 1.70 (2H, d, J=8 Hz), 1.26 (2H, dq, J=12 Hz, 3 Hz).

### Example 86

### ({[5-Hydroxy-6-methyl-2-({1-[5-(4-methylphenyl)pyrazin-2-yl]piperidin-4-yl}methyl)pyrimidin-4-yl]carbonyl}amino)acetic acid

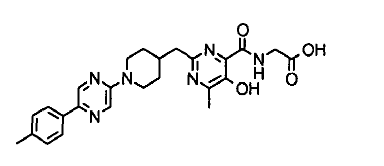

In accordance with Examples 81-(1) to 81-(3), but using 2,5-dichloropyrazine (0.89 g, 6.0 mmol) instead of 5-bromo-2-chloropyrimidine, and (4-methylphenyl)boronic acid instead of phenylboronic acid, the title compound (0.026 g, 0.054 mmol) was afforded as a yellow solid (yield 1.3%).
MS m/z: 477 (M+H)⁺;
1H-NMR (500 MHz, DMSO-d6) δ: 9.34 (1H, t, J=6 Hz), 8.63 (1H, s), 8.36 (1H, s), 7.84 (2H, d, J=8 Hz), 7.24 (2H, d, J=8 Hz), 4.35 (2H, d, J=13 Hz), 3.92 (2H, d, J=6 Hz), 2.92 (2H, t, J=5 Hz), 2.77 (2H, d, J=7 Hz), 2.44 (3H, s), 2.33 (3H, s), 2.13-2.03 (1H, m), 1.71 (2H, d, J=13 Hz), 1.27 (2H, dq, J=11 Hz, 3 Hz).

### Example 87

### ({[5-Hydroxy-6-methyl-2-({1-[5-(trifluoromethyl)pyridin-2-yl]piperidin-4-yl}methyl)pyrimidin-4-yl]carbonyl}amino)acetic acid

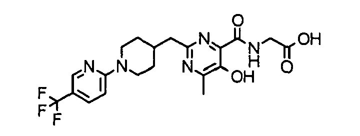

In accordance with Examples 1-(8) to 1-(11), but using 2-chloro-5-(trifluoromethyl)pyridine (0.42 g, 2.3 mmol) instead of 1-bromo-4-tert-butylbenzene, the title compound (0.082 g, 0.18 mmol) was afforded as a white solid (yield 16%).
MS m/z: 454 (M+H)⁺;
1H-NMR (500 MHz, DMSO-d6) δ: 9.26 (1H, t, J=8 Hz), 8.37 (1H, s), 7.74 (1H, d, J=6 Hz), 6.93 (1H, d, J=8 Hz), 4.39 (2H, d, J=8 Hz), 3.86 (2H, d, J=11 Hz), 2.91 (2H, t, J=8 Hz), 2.75 (2H, d, J=10 Hz), 2.43 (3H, s), 2.24-2.16 (1H, m), 1.67 (2H, d, J=11 Hz), 1.23 (2H, dq, J=11 Hz, 5 Hz).

### Example 88

### {[(2-{[1-(5-Benzylpyridin-2-yl)piperidin-4-yl]methyl}-5-hydroxy-6-methylpyrimidin-4-yl)carbonyl]amino}acetic acid

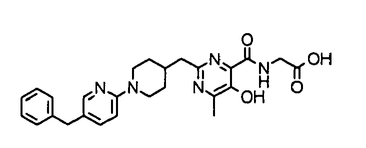

### (1) tert-Butyl 5-(benzyloxy)-2-{[1-(5-bromopyridin-2-yl)piperidin-4-yl]methyl}-6-methylpyrimidine-4-carboxylate

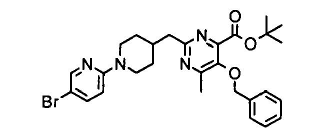

tert-Butyl 5-(benzyloxy)-6-methyl-2-[(piperidin-4-yl)methyl]pyrimidine-4-carboxylate hydrochloride (2.2 g, 5.0 mmol) obtained in Example 1-(7), 5-bromo-2-chloropyridine (1.4 g, 7.50 mmol) and potassium carbonate (2.1 g, 15 mmol) were suspended in N,N-dimethylformamide (50 mL), followed by stirring at 100°C for 17.5 hours. After the reaction solution was concentrated under reduced pressure, ethyl acetate was added, and the organic layer was washed with water. After the organic layer was concentrated under reduced pressure, the resulting residue was purified by silica gel column chromatography (Moritex Corporation, elution solvent: hexane/ethyl acetate), and a fraction corresponding to the Rf value=0.40 (hexane/ethyl acetate=4/1) by thin layer chromatography was concentrated under reduced pressure to afford the title compound (0.23 g, 0.43 mmol) as a colorless oil (yield 8.5%).
1H-NMR (500 MHz, CDCl3) δ: 8.16 (1H, s), 7.49 (1H, d, J=9 Hz), 7.45-7.33 (5H, m), 6.55 (1H, d, J=9 Hz), 5.01 (2H, s), 4.21 (2H, d, J=13 Hz), 2.87 (2H, t, J=7 Hz), 2.83 (2H, t, J=11 Hz), 2.45 (3H, s), 2.24-2.14 (1H, m), 1.75 (2H, d, J=12 Hz), 1.59 (9H, s), 1.37 (2H, dq, J=11 Hz, 3 Hz).

### (2) {[(5-Hydroxy-6-methyl-2-{[1-(5-benzylpyridin-2-yl)piperidin-4-yl]methyl}pyrimidin-4-yl)carbonyl]amino}acetic acid

In accordance with Examples 81-(2), 1-(9), 6-(2) and 1-(11), but using tert-butyl 5-(benzyloxy)-2-{[1-(5-bromopyridin-2-yl)piperidin-4-yl]methyl}-6-methylpyrimidine-4-carboxylate (0.37 g, 0.67 mmol) instead of tert-butyl 5-(benzyloxy)-2-{[1-(5-bromopyrimidin-2-yl)piperidin-4-yl]methyl}-6-methylpyrimidine-4-carboxylate, and 2-benzyl-4,4,5,5-tetramethyl-1,3,2-dioxaborolane instead of phenylboronic acid, the title compound (0.085 g, 0.18 mmol) was afforded as a white solid (yield 27%).
MS m/z: 476 (M+H)⁺;
1H-NMR (500 MHz, DMSO-d6) δ: 9.13 (1H, t, J=12 Hz), 8.00 (1H, s), 7.32 (1H, d, J=7 Hz), 7.30-7.14 (5H, m), 6.74 (1H, d, J=9 Hz), 4.19 (2H, d, J=12 Hz), 3.78 (2H, s), 3.73-3.63 (2H, m), 2.77-2.68 (4H, m), 2.42 (3H, s), 2.13-2.03 (1H, m), 1.61 (2H, d, J=12 Hz), 1.21 (2H, dq, J=7 Hz, 3 Hz).

### Example 89

### {[(5-Hydroxy-6-methyl-2-{[1-(5-phenylpyridin-2-yl)piperidin-4-yl]methyl}pyrimidin-4-yl)carbonyl]amino} acetic acid

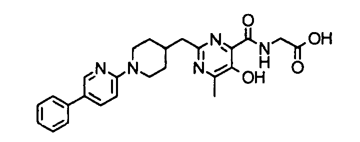

In accordance with Examples 81-(2), 1-(9), 6-(2) and 1-(11), but using tert-butyl 5-(benzyloxy)-2-{[1-(5-bromopyridin-2-yl)piperidin-4-yl]methyl}-6-methylpyrimidine-4-carboxylate (1.5 g, 2.7 mmol) instead of tert-butyl 5-(benzyloxy)-2-{[1-(5-bromopyrimidin-2-yl)piperidin-4-yl]methyl}-6-methylpyrimidine-4-carboxylate, the title compound (0.78 g, 1.7 mmol) was afforded as a white solid (yield 63%).
MS m/z: 462 (M+H)⁺;
1H-NMR (500 MHz, DMSO-d6) δ: 11.97 (1H, brs), 9.40 (1H, t, J=6 Hz), 8.43 (1H, s), 7.82 (1H, d, J=9 Hz), 7.61 (2H, d, J=8 Hz), 7.42 (2H, d, J=8 Hz), 7.29 (1H, d, J=8 Hz), 6.90 (1H, d, J=9 Hz), 4.33 (2H, d, J=13 Hz), 3.98 (2H, d, J=6 Hz), 2.84 (2H, d, J=12 Hz), 2.76 (2H, d, J=6 Hz), 2.44 (3H, s), 2.26-2.16 (1H, m), 1.66 (2H, d, J=11 Hz), 1.24 (2H, dq, J=11 Hz, 4 Hz).

### Example 90

### ({[5-Hydroxy-6-methyl-2-({1-[5-(4-methylphenyl)pyridin-2-yl]piperidin-4-yl}methyl)pyrimidin-4-yl]carbonyl}amino)acetic acid

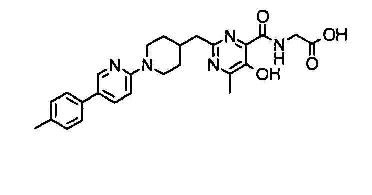

In accordance with Examples 81-(2), 1-(9), 6-(2) and 1-(11), but using tert-butyl 5-(benzyloxy)-2-{[1-(5-bromopyridin-2-yl)piperidin-4-yl]methyl}-6-methylpyrimidine-4-carboxylate (0.23 g, 0.42 mmol) instead of tert-butyl 5-(benzyloxy)-2-{[1-(5-bromopyrimidin-2-yl)piperidin-4-yl]methyl}-6-methylpyrimidine-4-carboxylate, and (4-methylphenyl)boronic acid instead of phenylboronic acid, the title compound (0.093 g, 0.19 mmol) was afforded as a white solid (yield 46%).
MS m/z: 476 (M+H)⁺;
1H-NMR (500 MHz, DMSO-d6) δ: 9.33 (1H, t, J=13 Hz), 8.40 (1H, s), 7.79 (1H, d, J=6 Hz), 7.49 (2H, d, J=8 Hz), 7.22 (2H, d, J=8 Hz), 6.88 (1H, d, J=9 Hz), 4.32 (2H, d, J=13 Hz), 3.96-3.91 (2H, m), 2.84 (2H, d, J=13 Hz), 2.76 (2H, d, J=7 Hz), 2.44 (3H, s), 2.32 (3H, s), 2.30-2.18 (1H, m), 1.67 (2H, d, J=13 Hz), 1.24 (2H, dq, J=8 Hz, 3 Hz).

### Example 91

### ({[5-Hydroxy-6-methyl-2-({1-[6-(4-methylphenyl)pyridin-3-yl]piperidin-4-yl}methyl)pyrimidin-4-yl]carbonyl}amino)acetic acid

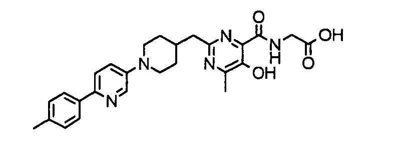

In accordance with Examples 1-(8) to 1-(11), but using 5-bromo-2-(4-methylphenyl)pyridine (0.62 g, 2.5 mmol) instead of 1-bromo-4-tert-butylbenzene, the title compound (0.28 g, 0.58 mmol) was afforded as a yellow solid (yield 29%).
MS m/z: 476 (M+H)⁺;
1H-NMR (500 MHz, DMSO-d6) δ: 9.21 (1H, t, J=6 Hz), 8.35 (1H, s), 7.87 (2H, d, J=8 Hz), 7.73 (1H, d, J=8 Hz), 7.36 (1H, d, J=8 Hz), 7.23 (2H, d, J=8 Hz), 3.81 (2H, d, J=6 Hz), 3.79 (2H, d, J=8 Hz), 2.77 (2H, t, J=7 Hz), 2.75 (2H, d, J=9 Hz), 2.43 (3H, s), 2.33 (3H, s), 2.18-2.08 (1H, m), 1.70 (2H, d, J=13 Hz), 1.37 (2H, dq, J=9 Hz, 3 Hz).

### Example 92

### {[(2-{[1-(5-tert-Butylthiophen-2-yl)piperidin-4-yl]methyl}-5-hydroxy-6-methylpiperidin-4-yl)carbonyl]amino}acetic acid

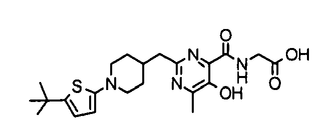

In accordance with Examples 1-(8), 1-(9), 6-(2) and 1-(11), but using 2-bromo-5-tert-butylthiophene (0.32 g, 1.5 mmol) instead of 1-bromo-4-tert-butylbenzene, the title compound (0.020 g, 0.045 mmol) was afforded as a white solid (yield 3.7%).
MS m/z: 447 (M+H)⁺;
1H-NMR (500 MHz, DMSO-d6) δ: 11.91 (1H, brs), 9.41 (1H, t, J=6 Hz), 6.80-6.71 (1H, m), 6.08-5.97 (1H, m), 4.00 (2H, d, J=6 Hz), 3.48 (2H, d, J=11 Hz), 3.31 (2H, t, J=10 Hz), 2.77 (2H, d, J=7 Hz), 2.44 (3H, s), 2.06-1.96 (1H, m), 1.65 (2H, d, J=13 Hz), 1.35 (2H, dq, J=12 Hz, 3 Hz), 1.28 (9H, s).

### Example 93

### {[(5-Hydroxy-6-methyl-2-{[1-(5-phenylthiophen-2-yl)piperidin-4-yl]methyl} piperidin-4-yl)carbonyl]amino}acetic acid

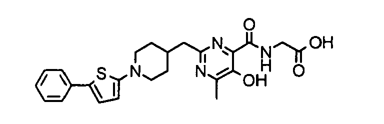

In accordance with Examples 1-(8), 1-(9), 6-(2) and 1-(11), but using 2-bromo-5-phenylthiophene (0.72 g, 3.0 mmol) instead of 1-bromo-4-tert-butylbenzene, the title compound (0.025 g, 0.053 mmol) was afforded as a white solid (yield 5.6%).
MS m/z: 467 (M+H)⁺;
1H-NMR (500 MHz, DMSO-d6) δ: 11.91 (1H, brs), 9.41 (1H, t, J=6 Hz), 7.48-7.44 (2H, m), 7.35- 7.28 (2H, m), 7.17 (1H, d, J=4 Hz), 7.17-7.11 (1H, m), 6.11 (1H, d, J=4 Hz), 4.00 (2H, d, J=6 Hz), 3.52 (2H, t, J=12 Hz), 3.38-3.31 (2H, m), 2.79 (2H, d, J=7 Hz), 2.44 (3H, s), 2.14-2.04 (1H, m), 1.69 (2H, d, J=10 Hz), 1.41 (2H, dq, J=10 Hz, 5 Hz).

### Example 94

### ({[5-Hydroxy-6-methyl-2-({1-[5-(4-methylphenyl)thiophen-2-yl]piperidin-4-yl}methyl)pyrimidin-4-yl]carbonyl}amino)acetic acid

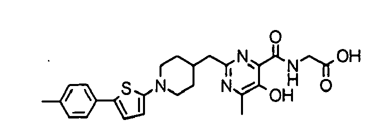

In accordance with Examples 1-(8), 1-(9), 6-(2) and 1-(11), but using 2-bromo-5-(4-methylphenyl)thiophene (1.1 g, 4.4 mmol) instead of 1-bromo-4-tert-butylbenzene, the title compound (0.17 g, 0.35 mmol) was afforded as a yellow solid (yield 8.6%).
MS m/z: 481 (M+H)⁺;
1H-NMR (500 MHz, DMSO-d6) δ: 9.23 (1H, t, J=8 Hz), 7.35 (2H, d, J=7 Hz), 7.13 (2H, d, J=7 Hz), 7.10 (1H, d, J=3 Hz), 6.08 (1H, d, J=3 Hz), 3.82 (2H, d, J=8 Hz), 3.51 (2H, d, J=11 Hz), 2.77 (2H, t, J=7 Hz), 2.50 (2H, d, J=10 Hz), 2.43 (3H, s), 2.27 (3H, s), 2.10-1.99 (1H, m), 1.69 (2H, d, J=11 Hz), 1.40 (2H, dq, J=13 Hz, 3 Hz).

### Example 95

### {[(2-{[1-(5-tert-Butyl-1,3-thiazol-2-yl)piperidin-4-yl]methyl}-5-hydroxy-6-methylpiperidin-4-yl)carbonyl]amino} acetic acid

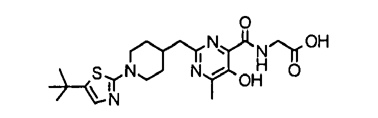

In accordance with Examples 1-(8), 1-(9), 6-(2) and 1-(11), but using 2-bromo-5-tert-butyl-1,3-thiazole (1.1 g, 5.0 mmol) instead of 1-bromo-4-tert-butylbenzene, the title compound (0.11 g, 0.25 mmol) was afforded as a white solid (yield 5.6%).
MS m/z: 448 (M+H)⁺;
1H-NMR (500 MHz, DMSO-d6) δ: 9.34 (1H, t, J=6 Hz), 6.78 (1H, s), 3.92 (2H, d, J=6 Hz), 3.79 (2H, d, J=13 Hz), 2.92 (2H, t, J=12 Hz), 2.76 (2H, d, J=7 Hz), 2.43 (3H, s), 2.18-2.08 (1H, m), 1.67 (2H, d, J=12 Hz), 1.29 (2H, dq, J=13 Hz, 3 Hz), 1.26 (9H, s).

### Example 96

### {[(5-Hydroxy-6-methyl-2-{[1-(5-phenyl-1,3-thiazol-2-yl)piperidin-4-yl]methyl} pyrimidin-4-yl)carbonyl]amino}acetic acid

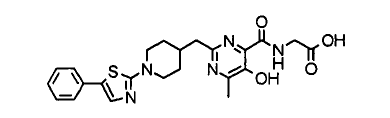

In accordance with Examples 81-(1), 81-(2), 1-(9), 6-(2) and 1-(11), but using 2,5-dibromo-1,3-thiazole (0.73 g, 3.0 mmol) instead of 5-bromo-2-chloropyrimidine, the title compound (0.42 g, 0.89 mmol) was afforded as a white solid (yield 11%).
MS m/z: 468 (M+H)⁺;
1H-NMR (500 MHz, DMSO-d6) δ: 9.28 (1H, t, J=11 Hz), 7.58 (1H, s), 7.46 (2H, d, J=8 Hz), 7.35 (2H, t, J=8 Hz), 7.20 (1H, t, J=8 Hz), 3.92 (2H, d, J=11 Hz), 3.86 (2H, d, J=5 Hz), 3.05 (2H, t, J=12 Hz), 2.78 (2H, d, J=8 Hz), 2.44 (3H, s), 2.23-2.13 (1H, m), 1.71 (2H, d, J=13 Hz), 1.33 (2H, dq, J=14 Hz, 3 Hz).

### Example 97

### ({[5-Hydroxy-6-methyl-2-({1-[5-(4-methylphenyl)-1,3-thiazol-2-yl]piperidin-4-yl}methyl)pyrimidin-4-yl]carbonyl}amino)acetic acid

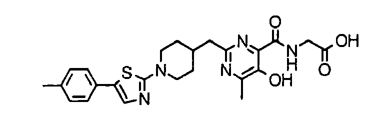

In accordance with Examples 81-(1), 81-(2), 1-(9), 6-(2) and 1-(11), but using 2,5-dibromo-1,3-thiazole (0.73 g, 3.0 mmol) instead of 5-bromo-2-chloropyrimidine, and (4-methylphenyl)boronic acid instead of phenylboronic acid, the title compound (0.058 g, 0.12 mmol) was afforded as a white solid (yield 6.0%).
MS m/z: 482 (M+H)⁺;
1H-NMR (500 MHz, DMSO-d6) δ: 9.12 (1H, t, J=12 Hz), 7.51 (1H, s), 7.34 (2H, d, J=8 Hz), 7.16 (2H, d, J=8 Hz), 3.89 (2H, d, J=12 Hz), 3.68 (2H, d, J=5 Hz), 3.03 (2H, t, J=10 Hz), 2.77 (2H, d, J=7 Hz), 2.42 (3H, s), 2.28 (3H, s), 2.19-2.09 (1H, m), 1.71 (2H, d, J=10 Hz), 1.33 (2H, dq, J=13 Hz, 3 Hz).

### Example 98

### ({[5-Hydroxy-6-methyl-2-({1-[4-(4-methylphenyl)-1,3-thiazol-2-yl]piperidin-4-yl}methyl)pyrimidin-4-yl]carbonyl}amino)acetic acid

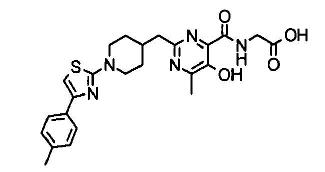

In accordance with Examples 1-(8), 1-(9), 6-(2) and 1-(11), but using 2-bromo-4-(4-methylphenyl)-1,3-thiazole (0.17 g, 0.67 mmol) instead of 1-bromo-4-tert-butylbenzene, the title compound (0.043 g, 0.089 mmol) was afforded as a yellow solid (yield 13%).
MS m/z: 482 (M+H)⁺;
1H-NMR (500 MHz, DMSO-d6) δ: 11.92 (1H, brs), 9.42 (1H, t, J=6 Hz), 7.43 (2H, d, J=8 Hz), 7.18 (2H, d, J=8 Hz), 7.16 (1H, s), 4.00 (2H, d, J=6 Hz), 3.94 (2H, d, J=11 Hz), 3.04 (2H, t, J=12 Hz), 2.79 (2H, d, J=7 Hz), 2.45 (3H, s), 2.31 (3H, s), 2.24-2.14 (1H, m), 1.72 (2H, d, J=12 Hz), 1.36 (2H, dq, J=11 Hz, 3 Hz).

### Example 99

### {[(5-Hydroxy-6-methyl-2-{[1-(5-phenyl-1,3-oxazol-2-yl)piperidin-4-yl]methyl}pyrimidin-4-yl)carbonyl]amino}acetic acid

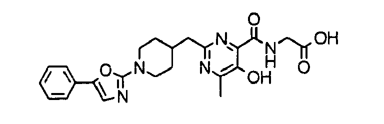

In accordance with Examples 1-(8) to 1-(11), but using 2-chloro-5-phenyl-1,3-oxazole (0.51 g, 2.8 mmol) instead of 1-bromo-4-tert-butylbenzene, the title compound (0.080 g, 0.18 mmol) was afforded as a white solid (yield 9.5%).
MS m/z: 452 (M+H)⁺;
1H-NMR (500 MHz, DMSO-d6) δ: 11.94 (1H, brs), 9.38 (1H, t, J=8 Hz), 7.54-7.51 (2H, m), 7.40-7.34 (2H, m), 7.31 (1H, s), 7.24-7.17 (1H, m), 4.02 (2H, d, J=8 Hz), 3.98 (2H, d, J=8 Hz), 2.99 (2H, t, J=11 Hz), 2.78 (2H, d, J=11 Hz), 2.44 (3H, s), 2.22-2.12 (1H, m), 1.69 (2H, d, J=11 Hz), 1.30 (2H, dq, J=11 Hz, 3 Hz).

### Example 100

### ({[5-Hydroxy-6-methyl-2-({1-[4-(pyridin-2-yl)phenyl]piperidin-4-yl}methyl)pyrimidin-4-yl]carbonyl}amino)acetic acid

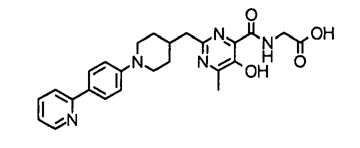

In accordance with Examples 1-(8), 1-(9), 6-(2) and 1-(11), but using 2-(4-bromophenyl)pyridine (0.54 g, 2.3 mmol) instead of 1-bromo-4-tert-butylbenzene, the title compound (0.12 g, 0.26 mmol) was afforded as a pale yellowish white solid (yield 23%).
MS m/z: 462 (M+H)⁺;
1H-NMR (500 MHz, DMSO-d6) δ: 11.92 (1H, brs), 9.40 (1H, t, J=6 Hz), 8.56 (1H, d, J=3 Hz), 7.94 (2H, d, J=7 Hz), 7.83-7.76 (2H, m), 7.22-7.20 (1H, m), 7.00 (2H, d, J=8 Hz), 4.00 (2H, d, J=6 Hz), 3.81 (2H, d, J=13 Hz), 2.79-2.73 (4H, m), 2.45 (3H, s), 2.17-2.07 (1H, m), 1.69 (2H, d, J=13 Hz), 1.43-1.32 (2H, m).

### Example 101

### ({[5-Hydroxy-6-methyl-2-({1-[4-(5-nitropyridin-2-yl)phenyl]piperidin-4-yl}methyl)pyrimidin-4-yl]carbonyl}amino)acetic acid

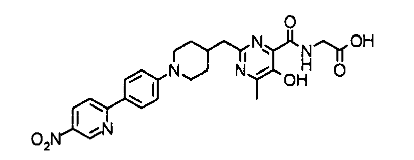

### (1) 2-(4-Bromophenyl)-5-nitropyridine

In accordance with Example 55-(1), but using 2-bromo-5-nitropyridine (0.40 g, 2.0 mmol) instead of 4-bromo-1-iodo-2-methylbenzene, and (4-bromophenyl)boronic acid (0.48 g, 2.4 mmol) instead of (4-fluorophenyl)boronic acid, the title compound (0.44 g, 1.6 mmol) was afforded as a white solid (yield 80%).
1H-NMR (500 MHz, CDCl3) δ: 9.49 (1H, brs), 8.54 (1H, t, J=9 Hz), 7.98 (2H, d, J=9 Hz), 7.90 (1H, d, J=9 Hz), 7.67 (2H, d, J=9 Hz).

### (2) Ethyl ({[5-(benzyloxy)-6-methyl-2-({1-[4-(5-nitropyridin-2-yl)phenyl]piperidin-4-yl}methyl)pyrimidin-4-yl]carbonyl}amino)acetate

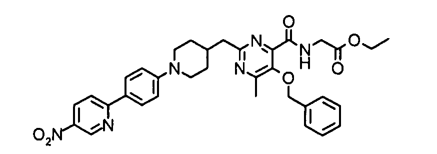

In accordance with Examples 1-(8) and 1-(9), but using 2-(4-bromophenyl)-5-nitropyridine (0.44 g, 1.6 mmol) instead of 1-bromo-4-tert-butylbenzene, the title compound (0.20 g, 0.32 mmol) was afforded as a pale yellowish white solid (yield 24%).
1H-NMR (500 MHz, CDCl3) δ: 9.41 (1H, s), 8.42 (1H, d, J=9 Hz), 8.34 (1H, brs), 8.02 (2H, d, J=9 Hz), 7.78 (1H, d, J=9 Hz), 7.49 (2H, d, J=7Hz), 7.40-7.34 (3H, m), 6.99 (2H, d, J=9 Hz), 5.12 (2H, s), 4.27 (2H, q, J=7 Hz), 4.24 (2H, d, J=6 Hz), 3.96-3.88 (2H, m), 2.93-2.89 (4H, m), 2.47 (3H, s), 2.20 (1H, brs), 1.80 (2H, d, J=7 Hz), 1.55-1.45 (2H, m), 1.32 (3H, t, J=7 Hz).

### (3) ({[5-Hydroxy-6-methyl-2-({1-[4-(5-nitropyridin-2-yl)phenyl]piperidin-4-yl}methyl)pyrimidin-4-yl]carbonyl}amino)acetic acid

In accordance with Examples 6-(2) and 1-(11), but using ethyl ({[5-(benzyloxy)-6-methyl-2-({1-[4-(5-nitropyridin-2-yl)phenyl]piperidin-4-yl}methyl)pyrimidin-4-yl]carbonyl}amino)acetate (0.040 g, 0.064 mmol) instead of ethyl ({[5-(benzyloxy)-2-{[1-(2-chlorophenyl)piperidin-4-yl]methyl}-6-methylpyrimidin-4-yl]carbonyl}amino)acetate, the title compound (0.020 g, 0.039 mmol) was afforded as a reddish brown solid (yield 62%).
MS m/z: 507 (M+H)⁺;
1H-NMR (500 MHz, DMSO-d6) δ: 9.33 (2H, brs), 8.51 (1H, d, J=9 Hz), 8.09 (3H, d, J=9 Hz), 7.05 (2H, d, J=9 Hz), 3.96-3.91 (4H, m), 2.85 (2H, t, J=12 Hz), 2.77 (2H, d, J=7 Hz), 2.44 (3H, s), 2.17 (1H, brs), 1.69 (2H, d, J=12 Hz), 1.38-1.29 (2H, m).

### Example 102

### ({[2-({1-[4-(5-Aminopyridin-2-yl)phenyl]piperidin-4-yl}methyl)-5-hydroxy-6-methylpyrimidin-4-yl]carbonyl}amino)acetic acid

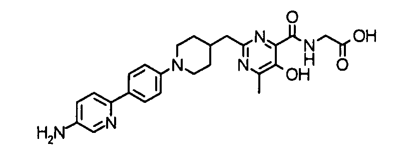

In accordance with Examples 1-(10) and 1-(11), but using ethyl ({[5-(benzyloxy)-6-methyl-2-({1-[4-(5-nitropyridin-2-yl)phenyl]piperidin-4-yl}methyl)pyrimidin-4-yl]carbonyl}amino)acetate (0.040 g, 0.064 mmol) obtained in Example 2981-(3) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.015 g, 0.031 mmol) was afforded as a yellow solid (yield 49%). MS m/z: 477 (M+H)⁺;
1H-NMR (500 MHz, DMSO-d6) δ: 9.38 (1H, brs), 7.95 (1H, s), 7.74 (2H, d, J=8 Hz), 7.49 (1H, d, J=8 Hz), 6.94 (1H, d; J=8 Hz), 6.93 (2H, d, J=8 Hz), 3.98 (2H, d, J=5 Hz), 3.72 (2H, d, J=12 Hz), 2.78 (2H, d, J=7 Hz), 2.68 (2H, t, J=12 Hz), 2.44 (3H, s), 2.09 (1H, brs), 1.68 (2H, d, J=12 Hz), 1.41-1.31 (2H, m).

### Example 103

### ({[5-Hydroxy-6-methyl-2-({1-[4-(pyridin-3-yl)phenyl]piperidin-4-yl}methyl)pyrimidin-4-yl]carbonyl}amino)acetic acid

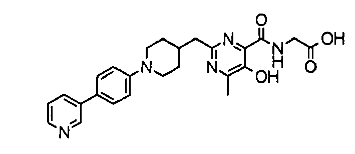

In accordance with Examples 1-(8), 1-(9), 6-(2) and 1-(11), but using 3-(4-bromophenyl)pyridine (0.58 g, 2.5 mmol) instead of 1-bromo-4-tert-butylbenzene, the title compound (0.11 g, 0.24 mmol) was afforded as a yellow solid (yield 15%).
MS m/z: 462 (M+H)⁺;
1H-NMR (500 MHz, DMSO-d6) δ: 9.38 (1H, t, J=6 Hz), 8.83 (1H, s), 8.46 (1H, d, J=4 Hz), 7.98 (1H, d, J=8 Hz), 7.58 (2H, d, J=9 Hz), 7.42 (1H, dd, J=8 Hz, 5 Hz), 7.03 (2H, d, J=9 Hz), 3.98 (2H, d, J=6 Hz), 3.78 (2H, d, J=12 Hz), 2.78 (2H, d, J=7 Hz), 2.74 (2H, t, J=10 Hz), 2.44 (3H, s), 2.16-2.07 (1H, m), 1.68 (2H, d, J=10 Hz), 1.40-1.32 (2H, m).

### Example 104

### Sodium ({[5-hydroxy-6-methyl-2-({1-[4-(pyridin-4-yl)phenyl]piperidin-4-yl}methyl)pyrimidin-4-yl]carbonyl}amino)acetate

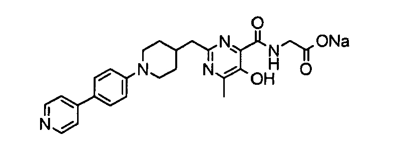

In accordance with Examples 1-(8), 1-(9), 6-(2) and 1-(11), but using 4-(4-bromophenyl)pyridine (0.47 g, 2.0 mmol) instead of 1-bromo-4-tert-butylbenzene, the title compound (0.20 g, 0.43 mmol) was afforded as a pale yellowish white solid (yield 24%).
MS m/z: 462 (M+H)⁺;
1H-NMR (500 MHz, DMSO-d6) δ: 11.71 (1H, brs), 8.52 (2H, d, J=5 Hz), 7.66 (2H, d, J=8 Hz), 7.62 (2H, d, J=8 Hz), 7.02 (2H, d, J=5 Hz), 3.80 (2H, d, J=12 Hz), 3.52 (2H, d, J=5 Hz), 2.71 (2H, t, J=12 Hz), 2.57-2.47 (2H, m), 2.21 (3H, s), 1.98-1.87 (1H, m), 1.62 (2H, d, J=12 Hz), 1.34-1.23 (2H, m).

### Example 105

### ({[5-Hydroxy-6-methyl-2-({1-[4-(pyrimidin-2-yl)phenyl]piperidin-4-yl}methyl)pyrimidin-4-yl]carbonyl}amino)acetic acid

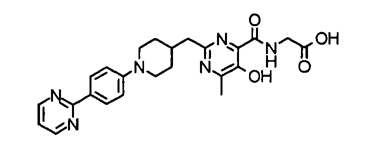

In accordance with Examples 1-(8), 1-(9), 6-(2) and 1-(11), but using 2-(4-bromophenyl)pyrimidine (0.24 g, 1.0 mmol) instead of 1-bromo-4-tert-butylbenzene, the title compound (0.053 g, 0.12 mmol) was afforded as a pale yellowish white solid (yield 12%).
MS m/z: 463 (M+H)⁺;
1H-NMR (500 MHz, DMSO-d6) δ: 12.88 (1H, brs), 11.92 (1H, s), 9.41 (1H, t, J=6 Hz), 8.78 (2H, dd, J=5 Hz, 2 Hz), 8.22 (2H, dd, J=8 Hz, 2 Hz), 7.28 (1H, dt, J=5 Hz, 2 Hz), 7.02 (2H, d, J=8 Hz), 4.00 (2H, d, J=6 Hz), 3.88 (2H, d, J=13 Hz), 2.83-2.77 (4H, m), 2.44 (3H, s), 2.20-2.10 (1H, m), 1.68 (2H, d, J=12 Hz), 1.38-1.30 (2H, m).

### Example 106

### ({[2-({1-[4-(2,5-Dimethyl-1H-pyrrol-1-yl)phenyl]piperidin-4-yl}methyl)-5-hydroxy-6-methylpyrimidin-4-yl]carbonyl}amino)acetic acid

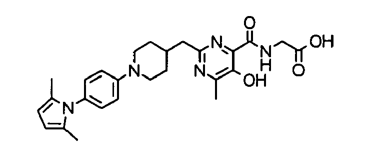

In accordance with Examples 1-(8) to 1-(11), but using 1-(4-bromophenyl)-2,5-dimethyl-1H-pyrrole (0.86 g, 3.5 mmol) instead of 1-bromo-4-tert-butylbenzene, the title compound (0.17 g, 0.36 mmol) was afforded as a pale brown solid (yield 15%).
MS m/z: 478 (M+H)⁺;
1H-NMR (500 MHz, DMSO-d6) δ: 11.92 (1H, s), 9.41 (1H, t, J=6 Hz), 7.03 (2H, d, J=8 Hz), 7.00 (2H, d, J=8 Hz), 5.73 (2H, s), 4.00 (2H, d, J=6 Hz), 3.74 (2H, d, J=12 Hz), 2.80 (2H, t, J=7 Hz), 2.71 (2H, t, J=12 Hz), 2.45 (3H, s), 2.11-2.04 (1H, m), 1.92 (6H, s), 1.70 (2H, d, J=12 Hz), 1.41-1.33 (2H, m).

### Example 107

### ({[5-Hydroxy-6-methyl-2-({1-[4-(thiophen-2-yl)phenyl]piperidin-4-yl}methyl)pyrimidin-4-yl]carbonyl}amino)acetic acid

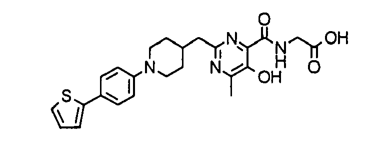

In accordance with Examples 1-(8) to 1-(11), but using 2-(4-bromophenyl)thiophene (0.94 g, 3.4 mmol) instead of 1-bromo-4-tert-butylbenzene, the title compound (0.27 g, 0.54 mmol) was afforded as a brown solid (yield 19%).
MS m/z: 467 (M+H)⁺;
1H-NMR (500 MHz, DMSO-d6) δ: 11.93 (1H, s), 9.42 (1H, t, J=6 Hz), 7.66-7.02 (7H, m), 4.01 (2H, d, J=6 Hz), 3.77-3.42 (4H, m), 2.80 (2H, d, J=7 Hz), 2.45 (3H, s), 2.23-2.11 (1H, m), 1.83-1.66 (2H, m), 1.64-1.32 (2H, m).

### Example 108

### ({[5-Hydroxy-6-methyl-2-({1-[4-(thiophen-3-yl)phenyl]piperidin-4-yl}methyl)pyrimidin-4-yl]carbonyl}amino)acetic acid

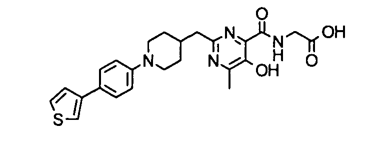

In accordance with Examples 1-(8), 1-(9), 6-(2) and 1-(11), but using 3-(4-bromophenyl)thiophene (0.88 g, 3.7 mmol) instead of 1-bromo-4-tert-butylbenzene, the title compound (0.41 g, 0.88 mmol) was afforded as a pale yellowish white solid (yield 36%).
MS m/z: 467 (M+H)⁺;
1H-NMR (500 MHz, DMSO-d6) δ: 9.35 (1H, brs), 7.64 (1H, brs), 7.57 (1H, brs), 7.54 (2H, d, J=9 Hz), 7.46 (1H, d, J=4 Hz), 6.94 (2H, d, J=9 Hz), 3.95 (2H, brs), 3.72 (2H, d, J=12 Hz), 2.78 (2H, d, J=7 Hz), 2.68 (2H, t, J=12 Hz), 2.44 (3H, s), 2.12-2.03 (1H, m), 1.68 (2H, d, J=12 Hz), 1.41-1.31 (2H, m).

### Example 109

### ({[5-Hydroxy-6-methyl-2-({1-[4-(1,3-thiazol-2-yl)phenyl]piperidin-4-yl}methyl)pyrimidin-4-yl]carbonyl}amino)acetic acid

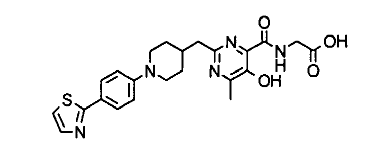

In accordance with Examples 1-(8) to 1-(11), but using 2-(4-bromophenyl)-1,3-thiazole (2.0 g, 8.5 mmol) instead of 1-bromo-4-tert-butylbenzene, the title compound (1.2 g, 2.5 mmol) was afforded as a white solid (yield 35%).
MS m/z: 468 (M+H)⁺;
1H-NMR (500 MHz, DMSO-d6) δ: 12.88 (1H, brs), 11.91 (1H, s), 9.41 (1H, t, J=6 Hz), 7.80 (1H, d, J=3 Hz), 7.76 (2H, d, J=8 Hz), 7.60 (1H, d, J=3 Hz), 7.00 (2H, d, J=8 Hz), 4.00 (2H, d, J=6 Hz), 3.83 (2H, d, J=13 Hz), 2.81-2.75 (4H, m), 2.45 (3H, s), 2.18-2.10 (1H, m), 1.68 (2H, d, J=12 Hz), 1.38-1.30 (2H, m).

### Example 110

### ({[5-Hydroxy-6-methyl-2-({1-[4-(2-methyl-1,3-thiazol-4-yl)phenyl]piperidin-4-yl}methyl)pyrimidin-4-yl]carbonyl}amino)acetic acid

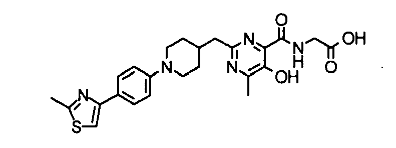

In accordance with Examples 1-(8), 1-(9), 6-(2) and 1-(11), but using 4-(4-bromophenyl)-2-methyl-1,3-thiazole (1.0 g, 3.9 mmol) instead of 1-bromo-4-tert-butylbenzene, the title compound (0.60 g, 1.2 mmol) was afforded as a pale yellow solid (yield 38%).
MS m/z: 482 (M+H)⁺;
1H-NMR (500 MHz, DMSO-d6) δ: 11.93 (1H, brs), 9.41 (1H, t, J=6 Hz), 7.75 (2H, d, J=7 Hz), 7.64 (1H, s), 6.96 (2H, d, J=7 Hz), 4.00 (2H, d, J=6 Hz), 3.74 (2H, d, J=12 Hz), 2.78 (2H, d, J=7 Hz), 2.70 (2H, t, J=12 Hz), 2.69 (3H, s), 2.45 (3H, s), 2.14-2.05 (1H, m), 1.68 (2H, d, J=12 Hz), 1.40-1.32 (2H, m).

### Example 111

### ({[5-Hydroxy-6-methyl-2-({1-[4-(1,3-oxazol-2-yl)phenyl]piperidin-4-yl}methyl)pyrimidin-4-yl]carbonyl}amino)acetic acid

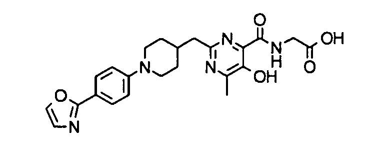

In accordance with Examples 1-(8) to 1-(11), but using 2-(4-bromophenyl)-1,3-oxazole (0.90 g, 4.0 mmol) instead of 1-bromo-4-tert-butylbenzene, the title compound (0.27 g, 0.60 mmol) was afforded as a pale brown solid (yield 15%).
MS m/z: 452 (M+H)⁺;
1H-NMR (500 MHz, DMSO-d6) δ: 12.88 (1H, brs), 11.91 (1H, s), 9.41 (1H, t, J=6 Hz), 8.08 (1H, s), 7.78 (2H, d, J=8 Hz), 7.26 (1H, s), 7.02 (2H, d, J=8 Hz), 4.00 (2H, d, J=6 Hz), 3.84 (2H, d, J=12 Hz), 2.81-2.76 (4H, m), 2.44 (3H, s), 2.19-2.10 (1H, m), 1.68 (2H, d, J=11 Hz), 1.37-1.27 (2H, m).

### Example 112

### ({[2-({1-[4-(3,5-Dimethylisoxazol-4-yl)phenyl]piperidin-4-yl}methyl)-5-hydroxy-6-methylpyrimidin-4-yl]carbonyl}amino)acetic acid

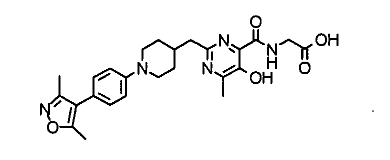

In accordance with Examples 1-(8) to 1-(11), but using 4-(4-bromophenyl)-3,5-dimethylisoxazole (0.70 g, 2.8 mmol) instead of 1-bromo-4-tert-butylbenzene, the title compound (0.50 g, 1.0 mmol) was afforded as a yellow solid (yield 45%).
MS m/z: 480 (M+H)⁺;
1H-NMR (500 MHz, DMSO-d6) δ: 12.88 (1H, brs), 11.91 (1H, s), 9.41 (1H, brs), 7.18 (2H, d, J=7 Hz), 7.00 (2H, d, J=7 Hz), 4.02 (2H, d, J=6 Hz), 3.73 (2H, d, J=11 Hz), 2.78 (2H, d, J=6 Hz), 2.70 (2H, t, J=12 Hz), 2.45 (3H, s), 2.36 (3H, s), 2.19 (3H, s), 2.13-2.04 (1H, m), 1.69 (2H, d, J=12 Hz), 1.42-1.31 (2H, m).

### Example 113

### ({[5-Hydroxy-6-methyl-2-({1-[4-(pipendin-1-yl)phenyl]piperidin-4-yl}methyl)pyrimidin-4-yl]carbonyl}amino)acetic acid

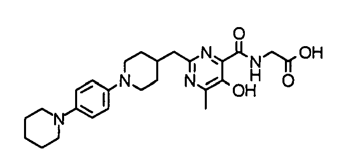

In accordance with Examples 1-(8) to 1-(11), but using 1-(4-bromophenyl)piperidine (0.78 g, 3.2 mmol) instead of 1-bromo-4-tert-butylbenzene, the title compound (0.21 g, 0.45 mmol) was afforded as a pale yellow solid (yield 30%).
MS m/z: 468 (M+H)⁺;
1H-NMR (400 MHz, DMSO-d6) δ: 9.27 (1H, t, J=6 Hz), 6.80 (4H, brs), 3.91 (2H, t, J=3 Hz), 3.46 (2H, d, J=12 Hz), 2.95 (4H, d, J=6 Hz), 2.77 (2H, d, J=7 Hz), 2.52 (2H, t, J=12 Hz), 2.43 (3H, s), 2.06-1.92 (1H, m), 1.71-1.56 (6H, m), 1.52-1.43 (2H, m), 1.42-1.30 (2H, m).

### Example 114

### ({[2-({1-[4-(1-Benzothiophen-2-yl)phenyl]piperidin-4-yl}methyl)-5-hydroxy-6-methylpyrimidin-4-yl]carbonyl}amino)acetic acid

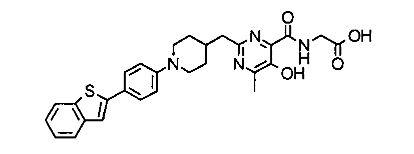

In accordance with Examples 1-(8) to 1-(11), but using 2-(4-bromophenyl)-1-benzothiophene (0.94 g, 3.3 mmol) instead of 1-bromo-4-tert-butylbenzene, the title compound (0.08 g, 0.16 mmol) was afforded as a brown solid (yield 6%).
MS m/z: 517 (M+H)⁺;
1H-NMR (500 MHz, DMSO-d6) δ: 12.89 (1H, brs), 11.92 (1H, s), 9.42 (1H, t, J=6 Hz), 7.91 (1H, d, J=8 Hz), 7.77 (1H, d, J=8 Hz), 7.64 (1H, s), 7.60 (2H, d, J=8 Hz), 7.35 (1H, t, J=8 Hz), 7.29 (1H, t, J=8 Hz), 7.01 (2H, d, J=8 Hz), 4.00 (2H, d, J=6 Hz), 3.80 (2H, d, J=12 Hz), 2.79-2.73 (4H, m), 2.45 (3H, s), 2.18-2.07 (1H, m), 1.68 (2H, d, J=11 Hz), 1.41-1.30 (2H, m).

### Example 115

### ({[2-({1-[4-(1-Benzothiophen-3-yl)phenyl]piperidin-4-yl}methyl)-5-hydroxy-6-methylpyrimidin-4-yl]carbonyl}amino)acetic acid

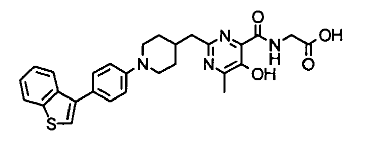

### (1) 3-(4-Bromophenyl)-1-benzothiophene

1-Bromo-4-iodobenzene (2.0 g, 7.1 mmol) was dissolved in toluene (70 mL) and water (70 mL), and at room temperature under a nitrogen atmosphere 1-benzothiophen-3-ylboronic acid (1.3 g, 7.1 mmol), tetrakis(triphenylphosphine)palladium (0.40 g, 0.35 mmol) and sodium carbonate (7.4 g, 70 mmol) were added, followed by heating to reflux for 4.5 hours. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.63 (hexane/ethyl acetate=6/1) by thin layer chromatography was concentrated under reduced pressure to afford the title compound (0.38 g, 1.3 mmol) as a colorless oil (yield 19%).
1H-NMR (500 MHz, CDCl3) δ: 7.93-7.92 (1H, m), 7.87-7.85 (1H, m), 7.62 (2H, d, J=9 Hz), 7.46 (2H, d, J=9 Hz), 7.42-7.39 (3H, m).

### (2) ({[2-({1-[4-(1-Benzothiophen-3-yl)phenyl]piperidin-4-yl}methyl)-5-hydroxy-6-methylpyrimidin-4-yl]carbonyl}amino)acetic acid

In accordance with Examples 1-(8), 1-(9), 6-(2) and 1-(11), but using 3-(4-bromophenyl)-1-benzothiophene (0.38 g, 1.3 mmol) instead of 1-bromo-4-tert-butylbenzene, the title compound (0.096 g, 0.19 mmol) was afforded as a brown solid (yield 17%).
MS m/z: 517 (M+H)⁺;
1H-NMR (500 MHz, DMSO-d6) δ: 12.89 (1H, brs), 11.93 (1H, brs), 9.41 (1H, t, J=6 Hz), 8.04 (1H, d, J=7 Hz), 7.88 (1H, d, J=7 Hz), 7.66 (1H, s), 7.45 (2H, d, J=8 Hz), 7.46-7.40 (2H, m), 7.06 (2H, d, J=8 Hz), 4.00 (2H, d, J=6 Hz), 3.78 (2H, d, J=12 Hz), 2.80 (2H, d, J=7 Hz), 2.74 (2H, t, J=12 Hz), 2.45 (3H, s), 2.16-2.07 (1H, m), 1.71 (2H, d, J=10 Hz), 1.43-1.35 (2H, m).

### Example 116

### ({[2-({1-[4-(1-Benzofuran-2-yl)phenyl]piperidin-4-yl}methyl)-5-hydroxy-6-methylpyrimidin-4-yl]carbonyl}amino)acetic acid

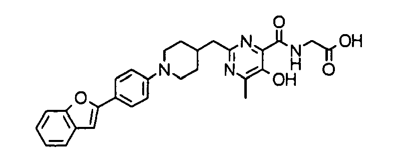

In accordance with Examples 1-(8) to 1-(11), but using 2-(4-bromophenyl)-1-benzofuran (0.94 g, 3.4 mmol) instead of 1-bromo-4-tert-butylbenzene, the title compound (0.27 g, 0.54 mmol) was afforded as a brown solid (yield 19%).
MS m/z: 501 (M+H)⁺;
1H-NMR (500 MHz, DMSO-d6) δ: 12.90 (1H, brs), 11.92 (1H, s), 9.42 (1H, t, J=6 Hz), 7.74 (2H, d, J=9 Hz), 7.57 (2H, t, J=8 Hz), 7.26-7.20 (2H, m), 7.17 (1H, s), 7.07-7.01 (2H, m), 4.00 (2H, d, J=6 Hz), 3.82 (2H, d, J=13 Hz), 2.82-2.74 (4H, m), 2.45 (3H, s), 2.17-2.09 (1H, m), 1.69 (2H, d, J=12 Hz), 1.41-1.31 (2H, m).

### Example 117

### ({[2-({1-[4-(1,3-Benzothiazol-2-yl)phenyl]piperidin-4-yl}methyl)-5-hydroxy-6-methylpyrimidin-4-yl]carbonyl}amino)acetic acid

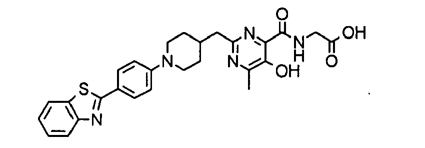

In accordance with Examples 1-(8) to 1-(11), but using 2-(4-bromophenyl)-1,3-benzothiazole (1.0 g, 3.4 mmol) instead of 1-bromo-4-tert-butylbenzene, the title compound (0.26 g, 0.51 mmol) was afforded as a yellow solid (yield 22%).
MS m/z: 518 (M+H)⁺;
1H-NMR (500 MHz, DMSO-d6) δ: 12.89 (1H, brs), 11.92 (1H, s), 9.41 (1H, t, J=6 Hz), 8.06 (1H, d, J=8 Hz), 7.94 (1H, d, J=8 Hz), 7.89 (2H, d, J=9 Hz), 7.48 (1H, t, J=8 Hz), 7.38 (1H, t, J=8 Hz), 7.06 (2H, d, J=9 Hz), 4.00 (2H, d, J=6 Hz), 3.90 (2H, d, J=13 Hz), 2.85 (2H, t, J=11 Hz), 2.78 (2H, d, J=7 Hz), 2.45 (3H, s), 2.21-2.11 (1H, m), 1.69 (2H, d, J=11 Hz), 1.39-1.30 (2H, m).

### Example 118

### ({[5-Hydroxy-2-({1-[4-(isoquinolin-5-yl)phenyl]piperidin-4-yl}methyl)-6-methylpyrimidin-4-yl]carbonyl}amino)acetic acid

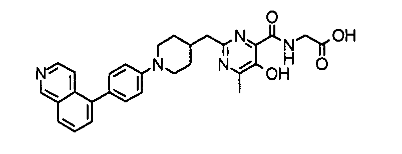

### (1) 5-[4-(Benzyloxy)phenyl]isoquinoline

In accordance with Example 115-(1), but using 5-bromoisoquinoline (2.8 g, 13 mmol) instead of 1-bromo-4-iodobenzene, and (4-benzyloxyphenyl)boronic acid (3.4 g, 15 mmol) instead of 1-benzothiophen-3-ylboronic acid, the title compound (4.2 g, 13 mmol) was afforded as a white solid (quantitative yield).
MS m/z: 312 (M+H)⁺.

### (2) 4-(Isoquinolin-5-yl)phenol

5-[4-(Benzyloxy)phenyl]isoquinoline (4.2 g, 13 mmol) was dissolved in a mixture of ethyl acetate (200 mL) and ethanol (200 mL), and 10% palladium-activated carbon (1.6 g) was added, followed by stirring at room temperature for 12 hours under a hydrogen atmosphere. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.10 (hexane/ethyl acetate=2/1) by thin layer chromatography was concentrated under reduced pressure to afford the title compound (1.5 g, 7.0 mmol) as a white solid (yield 52%).
1H-NMR (500 MHz, CDCl3) δ: 9.31 (1H, s), 8.50 (1H, d, J=6 Hz), 7.98 (1H, dd, J=7 Hz, 3 Hz), 7.77 (1H, d, J=6 Hz), 7.68-7.65 (2H, m), 7.37 (2H, d, J=8 Hz), 7.01 (2H, d, J=8 Hz).

### (3) (4-Isoquinolin-5-yl)phenyl trifluoromethanesulfonate

In accordance with Example 1-(5), but using 4-(isoquinolin-5-yl)phenol (1.5 g, 7.0 mmol) instead oftert-butyl 5-(benzyloxy)-2-{[1-(tert-butoxycarbonyl)piperidin-4-yl]methyl}-6-hydroxypyrimidine-4-carboxylate, the title compound (1.2 g, 3.4 mmol) was afforded as a yellow oil (yield 49%).
1H-NMR (500 MHz, CDCl3) δ: 9.34 (1H, s), 8.54 (1H, d, J=6 Hz), 8.04 (1H, d, J=8 Hz), 7.71-7.63 (3H, m), 7.58 (2H, d, J=8 Hz), 7.44 (2H; d, J=8 Hz).

### (4) ({[5-Hydroxy-2-({1-[4-(isoquinolin-5-yl)phenyl]piperidin-4-yl}methyl)-6-methylpyrimidin-4-yl]carbonyl}amino)acetic acid

In accordance with Examples 1-(8), 1-(9), 6-(2) and 1-(11), but using (4-isoquinolin-5-yl)phenyl trifluoromethanesulfonate (1.2 g, 3.4 mmol) instead of 1-bromo-4-tert-butylbenzene, the title compound (0.050 g, 0.19 mmol) was afforded as a brown solid (yield 17%).
MS m/z: 512 (M+H)⁺;
1H-NMR (500 MHz, DMSO-d6) δ: 12.91 (1H, brs), 11.93 (1H, brs), 9.41 (1H, t, J=6 Hz), 9.36 (1H, s), 8.48 (1H, d, J=6 Hz), 8.10 (1H, d, J=7 Hz), 7.76-7.72 (2H, m), 7.68 (1H, d, J=7 Hz), 7.36 (2H, d, J=9 Hz), 7.10 (2H, d, J=9 Hz), 4.00 (2H, d, J=6 Hz), 3.80 (2H, d, J=13 Hz), 2.80 (2H, d, J=7 Hz), 2.76 (2H, t, J=10 Hz), 2.45 (3H, s), 2.17-2.08 (1H, m), 1.72 (2H, d, J=13 Hz), 1.44-1.35 (2H, m).

### Example 119

### ({[5-Hydroxy-6-methyl-2-({1-[4-(quinolin-8-yl)phenyl]piperidin-4-yl}methyl)pyrimidin-4-yl]carbonyl}amino)acetic acid

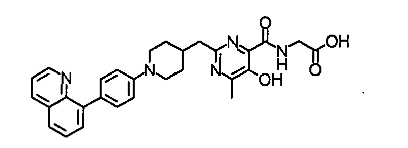

### (1) (4-Quinolin-8-yl)phenyl trifluoromethanesulfonate

In accordance with Examples 118-(1) to (3), but using 8-bromoquinoline (1.5 g, 7.2 mmol) instead of 5-bromoisoquinoline, the title compound (0.56 g, 1.6 mmol) was afforded as a yellow oil (yield 22%).
MS m/z: 354 (M+H)⁺;
1H-NMR (500 MHz, CDCl3) δ: 8.96 (1H, dd, J=4 Hz, 2 Hz), 8.24 (1H, dd, J=8 Hz, 2 Hz), 7.88 (1H, dd, J=8 Hz, 2 Hz), 7.80 (2H, d, J=9 Hz), 7.74 (1H, dd, J=7 Hz, 2 Hz), 7.63 (1H, dd, J=8 Hz, 7 Hz), 7.46 (1H, dd, J=8 Hz, 4 Hz), 7.40 (2H, d, J=9 Hz).

### (2) ({[5-Hydroxy-6-methyl-2-({1-[4-(quinolin-8-yl)phenyl]piperidin-4-yl}methyl)pyrimidin-4-yl]carbonyl}amino)acetic acid

In accordance with Examples 1-(8), 1-(9), 6-(2) and 1-(11), but using (4-quinolin-8-yl)phenyl trifluoromethanesulfonate (0.56 g, 1.6 mmol) instead of 1-bromo-4-tert-butylbenzene, the title compound (0.17 g, 0.19 mmol) was afforded as a pale yellow solid (yield 25%).
MS m/z: 512 (M+H)⁺;
1H-NMR (500 MHz, DMSO-d6) δ:11.90 (1H, brs), 9.24 (1H, t, J=6 Hz), 8.89 (1H, dd, J=4 Hz, 2 Hz), 8.40 (1H, dd, J=8 Hz, 2 Hz), 7.92 (1H, dd, J=8 Hz, 2 Hz), 7.72 (1H, dd, J=7 Hz, 2 Hz), 7.64 (1H, dd, J=8 Hz, 7 Hz), 7.56-7.53 (3H, m), 7.02 (2H, d, J=9 Hz), 4.02 (2H, d, J=6 Hz), 3.78 (2H, d, J=13 Hz), 2.80 (2H, d, J=8 Hz), 2.75 (2H, t, J=12 Hz), 2.45 (3H, s), 2.16-2.07 (1H, m), 1.70 (2H, d, J=12 Hz), 1.45-1.35 (2H, m).

### Example 120

### ({[5-Hydroxy-2-({1-[4-(imidazo[1,2-a]pyridin-3-yl)phenyl]piperidin-4-yl}methyl)-6-methylpyrimidin-4-yl]carbonyl}amino)acetic acid

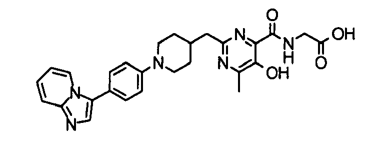

### (1) (Imidazo[1,2-a]pyridin-3-yl)phenyl trifluoromethanesulfonate

In accordance with Examples 118-(1) to (3), but using 3-bromoimidazo[1,2-a]pyridine (1.5 g, 7.6 mmol) instead of 5-bromoisoquinoline, the title compound (0.56 g, 1.6 mmol) was afforded as a yellow oil (yield 22%).
MS m/z: 343 (M+H)⁺.

### (2) ({[5-Hydroxy-2-({1-[4-(imidazo[1,2-a]pyridin-3-yl)phenyl]piperidin-4-yl}methyl)-6-methylpyrimidin-4-yl]carbonyl}amino)acetic acid

In accordance with Examples 1-(8), 1-(9), 6-(2) and 1-(11), but using (imidazo[1,2-a]pyridin-3-yl)phenyl trifluoromethanesulfonate (1.0 g, 2.9 mmol) instead of 1-bromo-4-tert-butylbenzene, the title compound (0.030 g, 0.060 mmol) was afforded as a pale yellow solid (yield 3%).
MS m/z: 501 (M+H)⁺;
1H-NMR (500 MHz, DMSO-d6) δ: 9.40 (1H, t, J=6 Hz), 8.46 (1H, d, J=7 Hz), 7.63 (1H, s), 7.62 (1H, d, J=7 Hz), 7.46 (2H, d, J=8 Hz), 7.25 (1H, t, J=7 Hz), 7.08 (2H, d, J=8 Hz), 6.93 (1H, t, J=7 Hz), 4.00 (2H, d, J=6 Hz), 3.80 (2H, d, J=13 Hz), 2.80 (2H, d, J=7 Hz), 2.75 (2H, t, J=12 Hz), 2.45 (3H, s), 2.17-2.08 (1H, m), 1.70 (2H, d, J=12 Hz), 1.43-1.33 (2H, m).

### Example 121

### ({[5-Hydroxy-2-({1-[4-(imidazo[1,2-a]pyridin-2-yl)phenyl]piperidin-4-yl}methyl)-6-methylpyrimidin-4-yl]carbonyl}amino)acetic acid

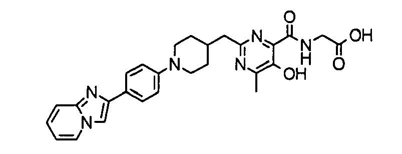

In accordance with Examples 1-(8) to 1-(11), but using 2-(4-bromophenyl)imidazo[1,2-a]pyridine (0.94 g, 3.5 mmol) instead of 1-bromo-4-tert-butylbenzene, the title compound (0.25 g, 0.50 mmol) was afforded as a yellow solid (yield 22%).
MS m/z: 501 (M+H)⁺;
1H-NMR (500 MHz, DMSO-d6) δ: 11.91 (1H, s), 9.41 (1H, t, J=6 Hz), 8.62 (1H, brs), 8.40 (1H, brs), 7.78 (2H, d, J=8 Hz), 7.68 (1H, d, J=8 Hz), 7.49 (1H, brs), 7.12 (1H, s), 7.04 (2H, d, J=8 Hz), 4.01 (2H, d, J=6 Hz), 3.81 (2H, d, J=11 Hz), 2.79-2.74 (4H, m), 2.45 (3H, s), 2.20-2.08 (1H, m), 1.69 (2H, d, J=13 Hz), 1.41-1.31 (2H, m).

### Example 122

### ({[2-({1-[4-(1,4-Benzodioxan-5-yl)phenyl]piperidin-4-yl}methyl)-5-hydroxy-6-methylpyrimidin-4-yl]carbonyl}amino)acetic acid

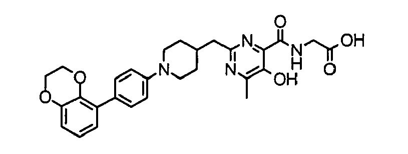

### (1) 4'-Bromo-2,3-dimethoxybiphenyl

In accordance with Example 115-(1), but using (2,3-dimethoxyphenyl)boronic acid (3.0 g, 17 mmol) instead of 1-benzothiophen-3-ylboronic acid, the title compound (1.3 g, 4.4 mmol) was afforded as a colorless oil (yield 28%).
MS m/z: 294 (M+H)⁺.

### (2) 4'-Bromobiphenyl-2,3-diol

4'-Bromo-2,3-dimethoxybiphenyl (1.0 g, 3.4 mmol) was dissolved in toluene (20 mL), and a solution of boron tribromide in methylene chloride (1.0 M, 14 mL, 14 mmol) was added, followed by stirring at room temperature for 12 hours. After the reaction solution was concentrated under reduced pressure, the resulting residue was purified by silica gel column chromatography (Moritex Corporation, elution solvent: hexane/ethyl acetate), and a fraction corresponding to the Rf value=0.60 (hexane/ethyl acetate=1/1) by thin layer chromatography was concentrated under reduced pressure to afford the title compound (1.1 g, 3.4 mmol) as a yellow oil (quantitative yield).
1H-NMR (500 MHz, CDCl3) δ: 7.60 (2H, d, J=8 Hz), 7.40 (2H, d, J=8 Hz), 6.92 (1H, dd, J=8 Hz, 2 Hz), 6.88 (1H, dd, J=8 Hz, 8 Hz), 6.82 (1H, dd, J=8 Hz, 2 Hz), 5.42 (1H, brs), 5.28 (1H, brs).

### (3) 5-(4-Bromophenyl)-1,4-benzodioxane

4'-Bromobiphenyl-2,3-diol (1.1 g, 3.4 mmol) was dissolved in N,N-dimethylformamide (15 mL), and at room temperature 1,2-dibromoethane (0.89 g, 4.8 mmol) and potassium fluoride (1.0 g, 17 mmol) were added, followed by stirring at 135°C for 4 hours. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.90 (hexane/ethyl acetate=1/1) by thin layer chromatography was concentrated under reduced pressure to afford the title compound (0.46 g, 1.6 mmol) as a yellow oil (yield 47%).
1H-NMR (500 MHz, CDCl3) δ: 7.53 (2H, d, J=8 Hz), 7.41 (2H, d, J=8 Hz), 6.95-6.85 (3H, m), 4.30-4.26 (4H, m).

### (4) ({[2-({1-[4-(1,4-Benzodioxan-5-yl)phenyl]piperidin-4-yl}methyl)-5-hydroxy-6-methylpyrimidin-4-yl]carbonyl}amino)acetic acid

In accordance with Examples 1-(8) to 1-(11), but using 5-(4-bromophenyl)-1,4-benzodioxane (0.46 g, 1.6 mmol) instead of 1-bromo-4-tert-butylbenzene, the title compound (0.20 g, 0.39 mmol) was afforded as a white solid (yield 30%).
MS m/z: 519 (M+H)⁺;
1H-NMR (500 MHz, DMSO-d6) δ: 12.89 (1H, brs), 11:91 (1H, brs), 9.40 (1H, t, J=6 Hz), 7.34 (2H, d, J=8 Hz), 6.94 (2H, d, J=8 Hz), 6.85-6.77 (3H, m), 4.23 (4H, d, J=11 Hz), 4.00 (2H, d, J=6 Hz), 3.72 (2H, d, J=12 Hz), 2.78 (2H, d, J=7 Hz), 2.69 (2H, t, J=12 Hz), 2.44 (3H, s), 2.14-2.03 (1H, m), 1.68 (2H, d, J=11 Hz), 1.42-1.32 (2H, m).

### Example 123

### [({5-Hydroxy-6-methyl-2-[(1-{4-[(pyridin-2-yl)methyl]phenyl}piperidin-4-yl)methyl]pyrimidin-4-yl}carbonyl)amino]acetic acid

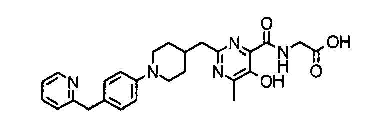

In accordance with Examples 1-(8), 1-(9), 6-(2) and 1-(11), but using 2-(4-bromobenzyl)pyridine (0.27 g, 1.1 mmol) instead of 1-bromo-4-tert-butylbenzene, the title compound (0.10 g, 0.21 mmol) was afforded as a white solid (yield 21%).
MS m/z: 476 (M+H)⁺;
1H-NMR (500 MHz, DMSO-d6) δ: 12.98 (1H, brs), 11.93 (1H, brs), 9.36 (1H, s), 8.46-8.42 (1H, m), 7.67 (1H, t, J=8 Hz), 7.21 (1H, d, J=7 Hz), 7.19 (1H, t, J=7 Hz), 7.08 (2H, d, J=9 Hz), 6.83 (2H, d, J=9 Hz), 3.96 (2H, d, J=5 Hz), 3.94 (2H, s), 3.59 (2H, d, J=13 Hz), 2.75 (2H, t, J=7 Hz), 2.59 (2H, t, J=11 Hz), 2.43 (3H, s), 2.08-1.98 (1H, m), 1.66 (2H, d, J=13 Hz), 1.34 (2H, dq, J=11 Hz, 3 Hz).

### Example 124

### [({5-Hydroxy-6-methyl-2-[(1-{4-[(pyridin-3-yl)methyl]phenyl}piperidin-4-yl)methyl]pyrimidin-4-yl} carbonyl)amino]acetic acid

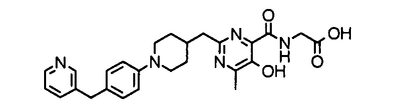

### (1) 3-(4-Bromobenzyl)pyridine

Copper bromide (2.0 g, 8.6 mmol) and tert-butyl nitrite (1.2 mL, 11 mmol) were dissolved in acetonitrile (60 mL), and at 0°C a solution of 4-[(pyridin-3-yl)methyl]aniline (1.4 g, 7.3 mmol) in acetonitrile (60 mL) was added, followed by stirring at the same temperature for 1 hour. After the temperature of the reaction solution was raised to room temperature, ethyl acetate was added to the reaction solution, and the organic layer was washed with saturated aqueous sodium hydrogencarbonate solution. After the filtrate was concentrated under reduced pressure, the resulting residue was purified by silica gel column chromatography (Moritex Corporation, elution solvent: hexane/ethyl acetate), and a fraction corresponding to the Rf value=0.70 (hexane/ethyl acetate=2/1) by thin layer chromatography was concentrated under reduced pressure to afford the title compound (0.95 g, 0.38 mmol) as a colorless oil (yield 52%).
1H-NMR (500 MHz, CDCl3) δ: 8.53-8.45 (2H, m), 7.45-7.39 (1H, m), 7.42 (2H, d, J=9 Hz), 7.23-7.19 (1H, m), 7.05 (2H, d, J=9 Hz), 3.93 (2H, s).

### (2) [({5-Hydroxy-6-methyl-2-[(1-{4-[(pyridin-3-yl)methyl]phenyl}piperidin-4-yl)methyl]pyrimidin-4-yl}carbonyl)amino]acetic acid

In accordance with Examples 1-(8), 1-(9), 6-(2) and 1-(11), but using 3-(4-bromobenzyl)pyridine (0.94 g, 3.8 mmol) instead of 1-bromo-4-tert-butylbenzene, the title compound (0.20 g, 0.41 mmol) was afforded as a yellow solid (yield 14%).
MS m/z: 476 (M+H)⁺;
1H-NMR (500 MHz, DMSO-d6) δ: 12.87 (1H, brs), 11.90 (1H, brs), 9.39 (1H, t, J=6 Hz), 8.46 (1H, s), 8.41-8.35 (1H, m), 7.61-7.55 (1H, m), 7.32-7.25 (1H, m), 6.96 (2H, d, J=9 Hz), 6.84 (2H, d, J=9 Hz), 4.00 (2H, d, J=6 Hz), 3.84 (2H, s), 3.59 (2H, d, J=11 Hz), 2.76 (2H, d, J=7 Hz), 2.59 (2H, t, J=12 Hz), 2.43 (3H, s), 2.09-1.99 (1H, m), 1.65 (2H, d, J=13 Hz), 1.33 (2H, dq, J=9 Hz, 3 Hz).

### Example 125

### [({5-Hydroxy-6-methyl-2-[(1-{4-[(6-methylpyridin-3-yl)methyl]phenyl}piperidin-4-yl)methyl]pyrimidin-4-yl}carbonyl)amino]acetic acid

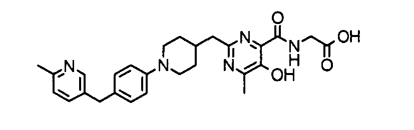

### (1) N-Methoxy-N,6-dimethylnicotinamide

6-Methylnicotinic acid (2.1 g, 15 mmol) was dissolved in a mixture of tetrahydrofuran (150 mL) and methanol (150 mL), and N,O-dimethylhydroxylamine hydrochloride (1.8 g, 18 mmol), N-methylmorpholine (2.0 mL, 18 mmol) and 4-(4,6-dimethoxy-1,3,5-triazin-2-yl)-4-methylmorpholinium chloride (5.0 g, 18 mmol) were added, followed by stirring at room temperature for 3 hours. After the reaction solution was concentrated under reduced pressure, the resulting residue was diluted with ethyl acetate, and subsequently the organic layer was washed with water. After the solvent was distilled off under reduced pressure, the resulting residue was purified by silica gel column chromatography (Moritex Corporation, elution solvent: hexane/ethyl acetate), and a fraction corresponding to the Rf value=0.10 (ethyl acetate) by thin layer chromatography was concentrated under reduced pressure to afford the title compound (2.2 g, 12 mmol) as a colorless oil (yield 82%).
1H-NMR (500 MHz, CDCl3 δ: 8.87 (1H, s), 7.95 (1H, d, J=7 Hz), 7.21 (1H, d, J=7 Hz), 3.57 (3H, s), 3.39 (3H, s), 2.61 (3H, s).

### (2) (4-Bromophenyl)(6-methylpyridin-3-yl)methanone

1,4-Dibromobenzene (5.8 g, 24 mmol) was dissolved in tetrahydrofuran (120 mL), and at -78°C a solution of n-butyllithium in hexane (2.6 M, 9.2 mL, 24 mmol) was added, followed by stirring at the same temperature for 30 minutes. To the reaction solution at -78°C was added a solution of N-methoxy-N,6-dimethylnicotinamide (2.2 g, 12 mmol) in tetrahydrofuran (20 mL), followed by stirring at the same temperature for 30 minutes. Water was added to the reaction solution, followed by extraction with ethyl acetate, and subsequently the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.40 (hexane/ethyl acetate=2/1) by thin layer chromatography was concentrated under reduced pressure to afford the title compound (2.1 g, 7.4 mmol) as a white solid (yield 61 %).
1H-NMR (500 MHz, CDCl3) δ: 8.86 (1H, s), 8.01 (1H, d, J=8 Hz), 7.71-7.63 (4H, m), 7.31 (1H, d, J=8 Hz), 2.67 (3H, s).

### (3) 5-(4-Bromobenzyl)-2-methylpyridine

(4-Bromophenyl)(6-methylpyridin-3-yl)methanone (0.88 g, 3.0 mmol), hydrazine monohydrate (1.5 mL, 30 mmol) and potassium hydroxide (0.68 g, 12 mmol) were dissolved in ethylene glycol (7.0 mL), followed by stirring at 150°C for 1 hour. After the reaction solution was cooled to room temperature, water was added, followed by extraction with ethyl acetate, and the organic layer was dried over sodium sulfate. After the solvent was distilled off under reduced pressure, the residue was purified by silica gel column chromatography (Moritex Corporation, elution solvent: hexane/ethyl acetate), and a fraction corresponding to the Rf value=0.35 (hexane/ethyl acetate=2/1) by thin layer chromatography was concentrated under reduced pressure to afford the title compound (0.62 g, 2.4 mmol) as a colorless oil (yield 79%).
1H-NMR (500 MHz, CDCl3) δ: 8.36 (1H, s), 7.41 (2H, d, J=8 Hz), 7.31 (1H, d, J=7 Hz), 7.07 (1H, d, J=7 Hz), 7.04 (2H, d, J=8 Hz), 3.89 (2H, s), 2.53 (3H, s).

### (4) [({5-Hydroxy-6-methyl-2-[(1-{4-[(6-methylpyridin-3-yl)methyl]phenyl}piperidin-4-yl)methyl]pyrimidin-4-yl}carbonyl)amino]acetic acid

In accordance with Examples 1-(8) to 1-(11), but using 5-(4-bromobenzyl)-2-methylpyridine (0.62 g, 2.4 mmol) instead of 1-bromo-4-tert-butylbenzene, the title compound (0.24 g, 0.49 mmol) was afforded as a yellow solid (yield 27%).
MS m/z: 490 (M+H)⁺;
1H-NMR (500 MHz, DMSO-d6) δ: 9.18 (1H, t, J=6 Hz), 8.31 (1H, s), 7.45 (1H, d, J=7 Hz), 7.14 (1H, d, J=7 Hz), 7.03 (2H, d, J=8 Hz), 6.84 (2H, d, J=8 Hz), 3.78 (2H, s), 3.76 (2H, d, J=6 Hz), 3.59 (2H, d, J=11 Hz), 2.74 (2H, d, J=7 Hz), 2.58 (2H, t, J=12 Hz), 2.42 (3H, s), 2.40 (3H, s), 2.09-1.99 (1H, m), 1.65 (2H, d, J=13 Hz), 1.33 (2H, dq, J=9 Hz, 3 Hz).

### Example 126

### [({5-Hydroxy-6-methyl-2-[(1-{4-[(2-methylpyridin-3-yl)methyl]phenyl}piperidin-4-yl)methyl]pyrimidin-4-yl}carbonyl)amino]acetic acid

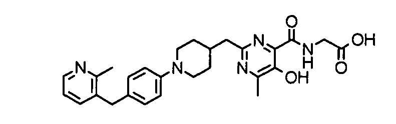

In accordance with Examples 125-(1) to 125-(4), but using 2-methylnicotinic acid (2.1 g, 15 mmol) instead of 6-methylnicotinic acid, the title compound (0.14 g, 0.29 mmol) was afforded as a yellow solid (yield 3%).
MS m/z: 490 (M+H)⁺;
1H-NMR (500 MHz, DMSO-d6) δ: 12.86 (1H, brs), 11.90 (1H, s), 9.39 (1H, t, J=6 Hz), 8.28 (1H, d, J=6 Hz), 7.45 (1H, d, J=6 Hz), 7.15 (1H, t, J=6 Hz), 6.97 (2H, d, J=8 Hz), 6.85 (2H, d, J=8 Hz), 4.00 (2H, d, J=6 Hz), 3.85 (2H, s), 3.60 (2H, d, J=12 Hz), 2.77 (2H, d, J=7 Hz), 2.59 (2H, t, J=12 Hz), 2.43 (3H, s), 2.39 (3H, s), 2.08-1.98 (1H, m), 1.66 (2H, d, J=12 Hz), 1.34 (2H, q, J=12 Hz).

### Example 127

### [({5-Hydroxy-6-methyl-2-[(1-{4-[(4-methylpyridin-3-yl)methyl]phenyl}piperidin-4-yl)methyl]pyrimidin-4-yl} carbonyl)amino] acetic acid

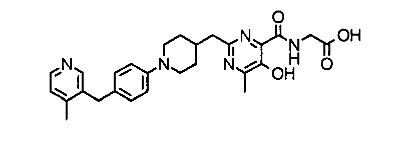

In accordance with Examples 125-(1) to 125-(4), but using 4-methylnicotinic acid hydrochloride (2.6 g, 15 mmol) instead of 6-methylnicotinic acid, the title compound (0.070 g, 0.14 mmol) was afforded as a pale yellowish white solid (yield 1%).
MS m/z: 490 (M+H)⁺;
1H-NMR (500 MHz, DMSO-d6) δ: 12.86 (1H, brs), 11.90 (1H, s), 9.39 (1H, t, J=6 Hz), 8.31 (1H, s), 8.29 (1H, d, J=5 Hz), 7.16 (1H, d, J=5 Hz), 6.97 (2H, d, J=8 Hz), 6.84 (2H, d, J=8 Hz), 4.00 (2H, d, J=6 Hz), 3.86 (2H, s), 3.59 (2H, d, J=12 Hz), 2.76 (2H, d, J=7 Hz), 2.59 (2H, t, J=12 Hz), 2.43 (3H, s), 2.19 (3H, s), 2.09-1.98 (1H, m), 1.65 (2H, d, J=12 Hz), 1.33 (2H, q, J=12 Hz).

### Example 128

### [({5-Hydroxy-6-methyl-2-[(1-{4-[(5-methylpyridin-3-yl)methyl]phenyl}piperidin-4-yl)methyl]pyrimidin-4-yl}carbonyl)amino]acetic acid

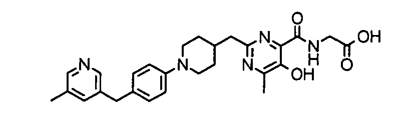

In accordance with Examples 125-(1) to 125-(4), but using 5-methylnicotinic acid (2.7 g, 20 mmol) instead of 6-methylnicotinic acid, the title compound (0.43 g, 0.88 mmol) was afforded as a pale yellowish white solid (yield 5%).
MS m/z: 490 (M+H)⁺;
1H-NMR (500 MHz, DMSO-d6) δ: 12.85 (1H, brs), 11.90 (1H, s), 9.38 (1H, t, J=6 Hz), 8.26 (1H, s), 8.22 (1H, s), 7.39 (1H, s), 7.05 (2H, d, J=8 Hz), 6.84 (2H, d, J=8 Hz), 4.00 (2H, d, J=6 Hz), 3.79 (2H, s), 3.60 (2H, d, J=12 Hz), 2.76 (2H, d, J=7 Hz), 2.59 (2H, t, J=12 Hz), 2.43 (3H, s), 2.24 (3H, s), 2.09-1.98 (1H, m); 1.65 (2H, d, J=12 Hz), 1.33 (2H, q, J=12 Hz).

### Example 129

### [({2-[(1-{4-[(5-Fluoropyridin-3-yl)methyl]phenyl}piperidin-4-yl)methyl]-5-hydroxy-6-methylpyrimidin-4-yl} carbonyl)amino]acetic acid

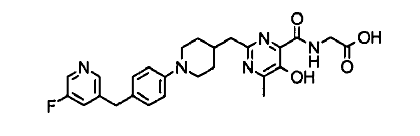

In accordance with Examples 125-(1) to 125-(4), but using 5-fluoronicotinic acid (2.5 g, 18 mmol) instead of 6-methylnicotinic acid, the title compound (0.21 g, 0.43 mmol) was afforded as a pale yellowish white solid (yield 2%).
MS m/z: 494 (M+H)⁺;
1H-NMR (500 MHz, DMSO-d6) δ: 12.89 (1H, brs), 11.92 (1H, brs), 9.38 (1H, t, J=5 Hz), 8.39 (1H, s), 8.36 (1H, s), 7.55 (1H, d, J=10 Hz), 7.09 (2H, d, J=8 Hz), 6.86 (2H, d, J=8 Hz), 3.99 (2H, d, J=5 Hz), 3.88 (2H, s), 3.61 (2H, d, J=12 Hz), 2.76 (2H, d, J=7 Hz), 2.60 (2H, t, J=12 Hz), 2.43 (3H, s), 2.09-1.98 (1H, m), 1.65 (2H, d, J=12 Hz), 1.33 (2H, q, J=12 Hz).

### Example 130

### {[(5-Hydroxy-6-methyl-2-{[1-(4-{[6-(trifluoromethyl)pyridin-3-yl]methyl}phenyl)piperidin-4-yl]methyl}pyrimidin-4-yl)carbonyl]amino}acetic acid

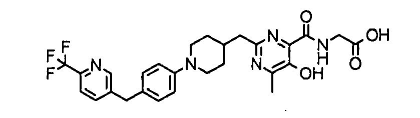

In accordance with Examples 125-(1) to 125-(4), but using 6-(trifluoromethyl)nicotinic acid (2.9 g, 15 mmol) instead of 6-methylnicotinic acid, the title compound (0.17 g, 0.32 mmol) was afforded as a yellow solid (yield 3.2%).
MS m/z: 544 (M+H)⁺;
1H-NMR (500 MHz, DMSO-d6) δ: 12.88 (1H, brs), 11.91 (1H, brs), 9.40 (1H, t, J=6 Hz), 8.68 (1H, s), 7.87 (1H, d, J=7 Hz), 7.81 (1H, d, J=7 Hz), 7.10 (2H, d, J=8 Hz), 6.87 (2H, d, J=8 Hz), 4.00 (2H, d, J=6 Hz), 3.98 (2H, s), 3.59 (2H, d, J=11 Hz), 2.75 (2H, d, J=7 Hz), 2.65-2.55 (2H, m), 2.43 (3H, s), 2.09-1.99 (1H, m), 1.65 (2H, d, J=13 Hz), 1.33 (2H, dq, J=9 Hz, 3 Hz).

### Example 131

### [({5-Hydroxy-2-[(1-{4-[(6-methoxypyridin-3-yl)methyl]phenyl}piperidin-4-yl)methyl]-6-methylpyrimidin-4-yl}carbonyl)amino] acetic acid

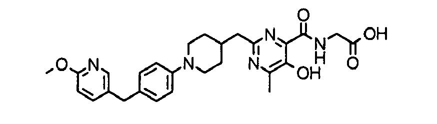

In accordance with Examples 125-(1) to 125-(4), but using 6-methoxynicotinic acid (2.3 g, 15 mmol) instead of 6-methylnicotinic acid, and pinacol instead of ethylene glycol, the title compound (0.073 g, 0.14 mmol) was afforded as a white solid (yield 2.8%).
MS m/z: 506 (M+H)⁺;
1H-NMR (500 MHz, DMSO-d6) δ: 12.89 (1H, brs), 11.95 (1H, brs), 9.36 (1H, t, J=6 Hz), 8.03 (1H, s), 7.49 (1H, d, J=8 Hz), 7.03 (2H, d, J=7 Hz), 6.84 (2H, d, J=7 Hz), 6.72 (1H, d, J=8 Hz), 3.96 (2H, d, J=6 Hz), 3.80 (3H, s), 3.75 (2H, s), 3.59 (2H, d, J=11 Hz), 2.75 (2H, d, J=7 Hz), 2.58 (2H, t, J=12 Hz), 2.43 (3H, s), 2.09-1.99 (1H, m), 1.65 (2H, d, J=13 Hz), 1.33 (2H, dq, J=9 Hz, 3 Hz).

### Example 132

### [({5-Hydroxy-6-methyl-2-[(1-{[2-methyl-4-(pyridin-3-yl)methyl]phenyl}piperidin-4-yl)methyl]pyrimidin-4-yl}carbonyl)amino]acetic acid

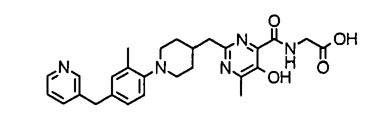

In accordance with Examples 63-(1) and 63-(2), but using 1-bromo-4-(bromomethyl)-2-methylbenzene (1.2 g, 4.4 mmol) instead of 1-bromo-4-(bromomethyl)benzene, and (pyridin-3-yl)boronic acid (0.65 g, 5.3 mmol) instead of (4-cyanophenyl)boronic acid, the title compound (0.17 g, 0.35 mmol) was afforded as a pale yellowish white solid (yield 8%).
MS m/z: 490 (M+H)⁺;
1H-NMR (500 MHz, DMSO-d6) δ: 11.91 (1H, brs), 9.38 (1H, t, J=5 Hz), 8.47 (1H, s), 8.39 (1H, d, J=6 Hz), 7.60 (1H, d, J=6 Hz), 7.29 (1H, t, J=6 Hz), 7.02 (1H, s), 6.99 (1H, d, J=8 Hz), 6.92 (1H, d, J=8 Hz), 4.00 (2H, d, J=5 Hz), 3.85 (2H, s), 2.97 (2H, d, J=12 Hz), 2.79 (2H, d, J=7 Hz), 2.53 (2H, t, J=12 Hz), 2.43 (3H, s), 2.18 (3H, s), 2.07-1.95 (1H, m), 1.65 (2H, d, J=12 Hz), 1.40 (2H, q, J=12 Hz).

### Example 133

### [({5-Hydroxy-6-methyl-2-[(1-{[3-methyl-4-(pyridin-3-yl)methyl]phenyl}piperidin-4-yl)methyl]pyrimidin-4-yl}carbonyl)amino]acetic acid

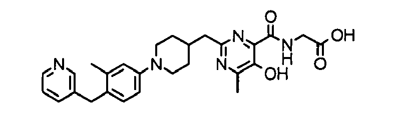

In accordance with Examples 63-(1) and 63-(2), but using 4-bromo-1-(bromomethyl)-2-methylbenzene (1.7 g, 6.3 mmol) instead of 1-bromo-4-(bromomethyl)benzene, and (pyridin-3-yl)boronic acid (0.94 g, 7.6 mmol) instead of (4-cyanophenyl)boronic acid, the title compound (0.23 g, 0.47 mmol) was afforded as a pale yellowish white solid (yield 7%).
MS m/z: 490 (M+H)⁺;
1H-NMR (500 MHz, DMSO-d6) δ: 12.84 (1H, brs), 11.91 (1H, brs), 9.39 (1H, t, J=5 Hz), 8.39 (1H, s), 8.38 (1H, d, J=6 Hz), 7.47 (1H, d, J=6 Hz), 7.28 (1H, t, J=6 Hz), 6.96 (1H, d, J=8 Hz), 6.74 (1H, s), 6.70 (1H, d, J=8 Hz), 4.00 (2H, d, J=5 Hz), 3.85 (2H, s), 3.61 (2H, d, J=12 Hz), 2.77 (2H, d, J=7 Hz), 2.60 (2H, t, J=12 Hz), 2.43 (3H, s), 2.13 (3H, s), 2.09-1.98 (1H, m), 1.65 (2H, d, J=12 Hz), 1.33 (2H, q, J=12 Hz).

### Example 134

### [({2-[(1-{[2-Fluoro-4-(pyridin-3-yl)methyl]phenyl}piperidin-4-yl)methyl]-5-hydroxy-6-methylpyrimidin-4-yl}carbonyl)amino]acetic acid

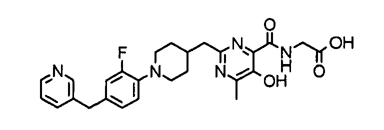

In accordance with Examples 63-(1) and 63-(2), but using 1-bromo-4-(bromomethyl)-2-fluorobenzene (1.7 g, 6.5 mmol) instead of 1-bromo-4-(bromomethyl)benzene, and (pyridin-3-yl)boronic acid (0.96 g, 7.8 mmol) instead of (4-cyanophenyl)boronic acid, the title compound (0.05 g, 0.10 mmol) was afforded as a pale yellowish white solid (yield 2%)
MS m/z: 494 (M+H)⁺;
1H-NMR (500 MHz, DMSO-d6) δ: 11.95 (1H, brs), 9.36 (1H, brs), 8.49 (1H, s), 8.42-8.38 (1H, m), 7.62 (1H, d, J=6 Hz), 7.30 (1H, t, J=6 Hz), 7.02 (1H, d, J=14 Hz), 6.98-6.91 (2H, m), 3.97 (2H, d, J=5 Hz), 3.88 (2H, s), 3.27 (2H, d, J=12 Hz), 2.78 (2H, d, J=7 Hz), 2.59 (2H, t, J=12 Hz), 2.43 (3H, s), 2.07-1.97 (1H, m), 1.67 (2H, d, J=12 Hz), 1.39 (2H, q, J=12 Hz).

### Example 135

### [({2-[(1-{[3-Fluoro-4-(pyridin-3-yl)methyl]phenyl}piperidin-4-yl)methyl]-5-hydroxy-6-methylpyrimidin-4-yl}carbonyl)amino]acetic acid

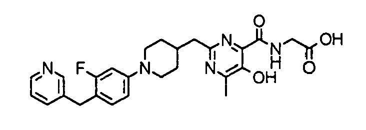

In accordance with Examples 63-(1) and 63-(2), but using 4-bromo-1-(bromomethyl)-2-fluorobenzene (1.5 g, 5.6 mmol) instead of 1-promo-4-(bromomethyl)benzene, and (pyridin-3-yl)boronic acid (1.0 g, 8.1 mmol) instead of (4-cyanophenyl)boronic acid, the title compound (0.05 g, 0.10 mmol) was afforded as a pale yellowish white solid (yield 2%).
MS m/z: 494 (M+H)⁺;
1H-NMR (500 MHz, DMSO-d6) δ: 12.87 (1H, brs), 11.91 (1H, brs), 9.39 (1H, t, J=6 Hz), 8.44 (1H, s), 8.41-8.37 (1H, m), 7.56 (1H, d, J=8 Hz), 7.32-7.27 (1H, m), 7.11 (1H, t, J=8 Hz), 6.71 (1H, s), 6.68 (1H, s), 4.00 (2H, d, J=6 Hz), 3.86 (2H, s), 3.67 (2H, d, J=12 Hz), 2.76 (2H, d, J=7 Hz), 2.65 (2H, t, J=12 Hz), 2.43 (3H, s), 2.12-2.01 (1H, m), 1.64 (2H, d, J=12 Hz), 1.31 (2H, q, J=12 Hz).

### Example 136

### [({5-Hydroxy-2-[(1-{4-[(6-hydroxypyridin-3-yl)methyl]phenyl}piperidin-4-yl)methyl]-6-methylpyrimidin-4-yl}carbonyl)amino]acetic acid

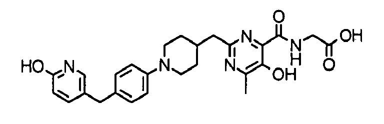

### (1) [6-(Benzyloxy)pyridin-3-yl](4-bromophenyl)methanone

In accordance with Example 125-(2), but using 2-(benzyloxy)-5-bromopyridine (2.7 g, 10 mmol) instead of 1,4-dibromobenzene, and 4-bromo-N-methoxy-N-methylbenzamide (2.2 g, 9.0 mmol) instead of N-methoxy-N,6-dimethylnicotinamide, the title compound (0.45 g, 1.2 mmol) was afforded as a white solid (yield 14%). 1H-NMR (500 MHz, CDCl3) δ: 8.60 (1H, s), 8.09 (1H, d, J=9 Hz), 7.66 (4H, s), 7.50-7.43 (2H, m), 7.42-7.34 (3H, m), 6.90 (1H, d, J=8 Hz), 5.48 (2H, s).

### (2) 2-(Benzyloxy)-5-(4-bromobenzyl)pyridine

In accordance with Example 125-(3), but using [6-(benzyloxy)pyridin-3-yl](4-bromophenyl)methanone (0.37 g, 1.0 mmol) instead of (4-bromophenyl)(6-methylpyridin-3-yl)methanone, and pinacol (4.0 mL) instead of ethylene glycol, the title compound (0.20 g, 0.57 mmol) was afforded as a colorless oil (yield 58%).
1H-NMR (500 MHz, CDCl3) δ: 8.01 (1H, s), 7.50-7.26 (7H, m), 7.04 (2H, d, J=8 Hz), 6.74 (2H, d, J=8 Hz), 5.35 (2H, s), 3.85 (2H, s).

### (3) Ethyl [({5-hydroxy-2-[(1-{4-[(6-hydroxypyridin-3-yl)methyl]phenyl}piperidin-4-yl)methyl]-6-methylpyrimidin-4-yl}carbonyl)amino]acetate

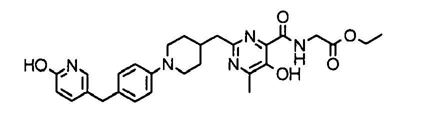

In accordance with Examples 1-(8), 1-(9) and 6-(2), but using 2-(benzyloxy)-5-(4-bromobenzyl)pyridine (1.9 g, 5.3 mmol) instead of 1-bromo-4-tert-butylbenzene, the title compound (0.24 g, 0.46 mmol) was afforded as a colorless oil (yield 11%).
1H-NMR (500 MHz, CDCl3) δ: 11.36 (1H, brs), 8.49 (1H, t, J=6 Hz), 7.30 (1H, d, J=10 Hz), 7.09-6.95 (3H, m), 6.87 (2H, d, J=8 Hz), 6.51 (1H, d, J=10 Hz), 4.27 (2H, q, J=6 Hz), 4,21 (2H, d, J=6 Hz), 3.68-3.63 (2H, m), 3.63 (2H, s), 2.82 (2H, d, J=7 Hz), 2.69 (2H, t, J=12 Hz), 2.53 (3H, s), 2.09-1.99 (1H, m), 1.74 (2H, d, J=13 Hz), 1.50 (2H, dq, J=9 Hz, 3 Hz), 1.32 (3H, t, J=6 Hz).

### (4) [({5-Hydroxy-2-[(1-{4-[(6-hydroxypyridin-3-yl)methyl]phenyl}piperidin-4-yl)methyl]-6-methylpyrimidin-4-yl}carbonyl)amino] acetic acid

In accordance with Example 1-(11), but using ethyl [({5-hydroxy-2-[(1-{4-[(6-hydroxypyridin-3-yl)methyl]phenyl}piperidin-4-yl)methyl]-6-methylpyrimidin-4-yl}carbonyl)amino]acetate (0.24 g, 0.46 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.13 g, 0.26 mmol) was afforded as a white solid (yield 56%).
MS m/z: 492 (M+H)⁺;
1H-NMR (500 MHz, DMSO-d6) δ: 11.41 (1H, brs), 9.15 (1H, t, J=2 Hz), 7.24 (1H, d, J=9 Hz), 7.13 (1H, s), 7.02 (2H, d, J=8 Hz), 6.84 (2H, d, J=8 Hz), 6.24 (1H, d, J=9 Hz), 3.74 (2H, d, J=2 Hz), 3.60 (2H, d, J=12 Hz), 3.53 (2H, s), 2.75 (2H, d, J=7 Hz), 2.59 (2H, t, J=12 Hz), 2.42 (3H, s), 2.09-1.99 (1H, m), 1.66 (2H, d, J=13 Hz), 1.34 (2H, dq, J=9 Hz, 3 Hz).

### Example 137

### [({5-Hydroxy-2-[(1-{4-[(5-hydroxypyridin-3-yl)methyl]phenyl}piperidin-4-yl)methyl]-6-methylpyrimidin-4-yl}carbonyl)amino]acetic acid

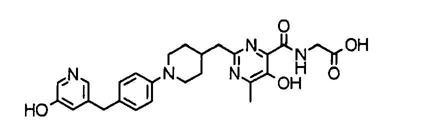

### (1) Methyl 5-(benzyloxy)nicotinate

Methyl 5-hydroxynicotinate (3.8 g, 25 mmol), benzyl bromide (5.1 g, 30 mmol) and potassium carbonate (6.9 g, 50 mmol) were dissolved in N,N-dimethylformamide (150 mL), followed by stirring at 60°C for 6.5 hours. After the reaction solution was concentrated under reduced pressure, the resulting residue was dissolved in ethyl acetate, and the organic layer was washed with water. After the solvent was distilled off under reduced pressure, the resulting residue was purified by silica gel column chromatography (Moritex Corporation, elution solvent: hexane/ethyl acetate), and a fraction corresponding to the Rf value=0.50 (hexane/ethyl acetate=2/1) by thin layer chromatography was concentrated under reduced pressure to afford the title compound (3.0 g, 12 mmol) as a yellow oil (yield 50%).
1H-NMR (500 MHz, CDCl3) δ: 8.84 (1H, s), 8.55 (1H, s), 7.85 (1H, s), 7.50-7.34 (5H, m), 5.15 (2H, s), 3.95 (3H, s).

### (2) 5-(Benzyloxy)nicotinic acid

Methyl 5-(benzyloxy)nicotinate (3.0 g, 12 mmol) was dissolved in a mixture of tetrahydrofuran (70 mL) and methanol (70 mL), and aqueous sodium hydroxide solution (1 M, 70 mL, 70 mmol) was added, followed by stirring at room temperature for 45 minutes. After the reaction solution was concentrated under reduced pressure, hydrochloric acid (1 M) was added to the residue to neutralize it, whereby a solid was precipitated. The precipitated material was filtered, and subsequently dried under reduced pressure to afford a crude product (2.9 g, 12 mmol) of the title compound (quantitative yield).
MS m/z: 230 (M+H)⁺.

### (3) 3-(Benzyloxy)-5-(4-bromobenzyl)pyridine

In accordance with Examples 125-(1) to 125(3), but using 5-(benzyloxy)nicotinic acid (2.9 g, 12 mmol) instead of 6-methylnicotinic acid, the title compound (1.5 g, 4.3 mmol) was afforded as a yellow oil (yield 41%).
1H-NMR (500 MHz, CDCl3) δ: 8.24 (1H, s), 8.11 (1H, s), 7.41 (2H, d, J=8 Hz), 7.38-7.32 (5H, m), 7.02 (2H, d, J=8 Hz), 6.97 (1H, s), 5.05 (2H, s), 3.90 (2H, s).

### (4) Ethyl {[(2-{[1-(4-{[5-(benzyloxy)pyridin-3-yl]methyl}phenyl)piperidin-4-yl]methyl}-5-hydroxy-6-methylpyrimidin-4-yl)carbonyl]amino}acetate

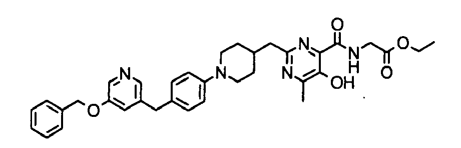

In accordance with Examples 1-(8), 1-(9) and 6-(2), but using 3-(benzyloxy)-5-(4-bromobenzyl)pyridine (1.5 g, 4.3 mmol) instead of 1-bromo-4-tert-butylbenzene, the title compound (0.95 g, 1.6 mmol) was afforded as a yellow oil (yield 48%).
1H-NMR (500 MHz, CDCl3) δ: 11.36 (1H, s), 8.49 (1H, t, J=6 Hz), 8.21 (1H, s), 8.12 (1H, s), 7.43-7.26 (5H, m), 7.04 (2H, d, J=8 Hz), 7.03 (1H, s), 6.87 (2H, d, J=8 Hz), 5.04 (2H, s), 4.28 (2H, q, J=7 Hz), 4.21 (2H, d, J=6 Hz), 3.86 (2H, s), 3.63 (2H, d, J=12 Hz), 2.82 (2H, d, J=7 Hz), 2.68 (2H, t, J=10 Hz), 2.53 (3H, s), 2.08-1.98 (1H, m), 1.76 (2H, d, J=12 Hz), 1.58-1.43 (2H, m), 1.32 (3H, t, J=7 Hz).

### (5) Ethyl [({5-hydroxy-2-[(1-{4-[(5-hydroxypyridin-3-yl)methyl]phenyl}piperidin-4-yl)methyl]-6-methylpyrimidin-4-yl}carbonyl)amino]acetate

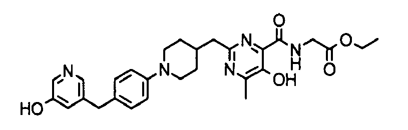

Ethyl {[(2-{[1-(4-{[5-(benzyloxy)pyridin-3-yl]methyl}phenyl)piperidin-4-yl]methyl}-5-hydroxy-6-methylpyrimidin-4-yl)carbonyl]amino}acetate (0.70 g, 1.2 mmol) was dissolved in ethyl acetate (100 mL), and 10% palladium-activated carbon (0.30 g) was added, followed by stirring at room temperature for 1 hour and 45 minutes under a hydrogen atmosphere. After the reaction solution was filtered with celite, the filtrate was concentrated under reduced pressure. The resulting residue was purified by silica gel column chromatography (Moritex Corporation, elution solvent: hexane/ethyl acetate), and a fraction corresponding to the Rf value=0.15 (ethyl acetate) by thin layer chromatography was concentrated under reduced pressure to afford the title compound (0.42 g, 0.81 mmol) as a colorless oil (yield 70%).
1H-NMR (500 MHz, CDCl3) δ: 11.36 (1H, s), 8.49 (1H, t, J=6 Hz), 8.03 (1H, s), 7.81 (1H, s), 7.06 (2H, d, J=8 Hz), 6.90 (2H, d, J=8 Hz), 6.84 (1H, s), 4.28 (2H, q, J=7 Hz), 4.21 (2H, d, J=6 Hz), 3.86 (2H, s), 3.64 (2H, d, J=13 Hz), 2.82 (2H, d, J=7 Hz), 2.70 (2H, t, J=10 Hz), 2.53 (3H, s), 2.08-1.98 (1H, m), 1.76 (2H, d, J=12 Hz), 1.58-1.43 (2H, m), 1.32 (3H, t, J=7 Hz).

### (6) [({5-Hydroxy-2-[(1-{4-[(5-hydroxypyridin-3-yl)methyl]phenyl}piperidin-4-yl)methyl]-6-methylpyrimidin-4-yl}carbonyl)amino]acetic acid

In accordance with Example 1-(11), but using ethyl [({5-hydroxy-2-[(1-{4-[(5-hydroxypyridin-3-yl)methyl]phenyl}piperidin-4-yl)methyl]-6-methylpyrimidin-4-yl}carbonyl)amino]acetate (0.42 g, 0.81 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.40 g, 0.81 mmol) was afforded as a yellow solid (yield 100%).
MS m/z: 492 (M+H)⁺;
1H-NMR (500 MHz, DMSO-d6) δ: 9.90 (1H, brs), 9.10 (1H, t, J=4 Hz), 7.93 (1H, s), 7.92 (1H, s), 7.03 (2H, d, J=8 Hz), 6.91 (1H, s), 6.84 (2H, d, J=8 Hz), 3.75 (2H, s), 3.68 (2H, d, J=4 Hz), 3.59 (2H, d, J=12 Hz), 2.74 (2H, d, J=7 Hz), 2.58 (2H, t, J=12 Hz), 2.42 (3H, s), 2.05-1.95 (1H, m), 1.65 (2H, d, J=12 Hz), 1.39-1.27 (2H, m).

### Example 138

### [({5-Hydroxy-6-methyl-2-[(1-{4-[(pyridin-4-yl)methyl]phenyl}piperidin-4-yl)methyl]pyrimidin-4-yl}carbonyl)amino]acetic acid

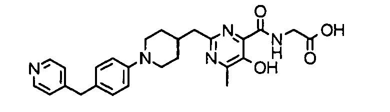

In accordance with Examples 124-(1) and 124-(2), but using 4-[(pyridin-4-yl)methyl]aniline (3.5 g, 19 mmol) instead of 4-[(pyridin-3-yl)methyl]aniline, the title compound (0.063 g, 0.13 mmol) was afforded as a yellow solid (yield 2.3%)
MS m/z: 476 (M+H)⁺;
1H-NMR (500 MHz, DMSO-d6) δ: 12.13 (1H, brs), 9.30 (1H, t, J=5 Hz), 8.42 (2H, d, J=6 Hz), 7.19 (2H, d, J=6 Hz), 7.06 (2H, d, J=9 Hz), 6.85 (2H, d, J=9 Hz), 3.90 (2H, d, J=5 Hz), 3.84 (2H, s), 3.60 (2H, d, J=11 Hz), 2.76 (2H, d, J=7 Hz), 2.59 (2H, t, J=11 Hz), 2.43 (3H, s), 2.09-1.99 (1H, m), 1.65 (2H, d, J=13 Hz), 1.33 (2H, dq, J=9 Hz, 3 Hz).

### Example 139

### [({5-Hydroxy-6-methyl-2-[(1-{4-[(pyridazin-4-yl)methyl]phenyl}piperidin-4-yl)methyl]pyrimidin-4-yl}carbonyl)amino]acetic acid

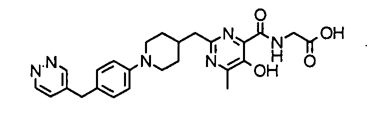

### (1) 4-(4-Bromobenzyl)pyridazine

In accordance with Examples 125-(1) to (3), but using pyridazine-4-carboxylic acid (2.7 g, 22 mmol) instead of 6-methylnicotinic acid, the title compound (0.78 g, 3.1 mmol) was afforded as a colorless solid (yield 14%).
1H-NMR (500 MHz, CDCl3) δ: 9.11-9.04 (2H, m), 7.48 (2H, d, J=7 Hz), 7.19 (1H, s), 7.05 (2H, d, J=7 Hz), 3.95 (2H, s).

### (2) [({5-Hydroxy-6-methyl-2-[(1-{4-[(pyridazin-4-yl)methyl]phenyl}piperidin-4-yl)methyl]pyrimidin-4-yl}carbonyl)amino]aceticacid

In accordance with Examples 1-(8), 1-(9), 6-(2) and 1-(11), but using 4-(4-bromobenzyl)pyridazine (0.78 g, 3.1 mmol) instead of 1-bromo-4-tert-butylbenzene, the title compound (0.084 g, 0.18 mmol) was afforded as a yellow solid (yield 7.3%).
MS m/z: 477 (M+H)⁺;
1H-NMR (500 MHz, DMSO-d6) δ: 12.87 (1H, brs), 11.90 (1H, brs), 9.39 (1H, t, J=4 Hz), 9.15-9.05 (2H, m), 7.47 (1H, s), 7.09 (2H, d, J=8 Hz), 6.87 (2H, d, J=8 Hz), 4.00 (2H, d, J=4 Hz), 3.88 (2H, s), 3.62 (2H, d, J=11 Hz), 2.76 (2H, d, J=7 Hz), 2.61 (2H, t, J=12 Hz), 2.43 (3H, s), 2.09-1.99 (1H, m), 1.65 (2H, d, J=13 Hz), 1.33 (2H, dq, J=9 Hz, 3 Hz).

### Example 140

### [({5-Hydroxy-6-methyl-2-[(1-{4-[(pyrazin-2-yl)methyl]phenyl}piperidin-4-yl)methyl]pyrimidin-4-yl}carbonyl)amino]acetic acid

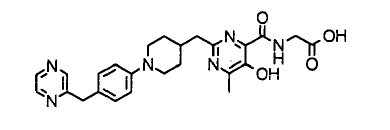

### (1) 2-(4-Bromobenzyl)pyrazine

In accordance with Examples 125-(1) to (3), but using pyrazine-2-carboxylic acid (1.9 g, 15 mmol) instead of 6-methylnicotinic acid, the title compound (1.9 g, 7.7 mmol) was afforded as a yellow oil (yield 79%).
1H-NMR (500 MHz, CDCl3) δ: 8.53-8.41 (3H, m), 7.44 (2H, d, J=8 Hz), 7.16 (2H, d, J=8 Hz), 4.12 (2H, s).

### (2) [({5-Hydroxy-6-methyl-2-[(1-{4-[(pyrazin-2-yl)methyl]phenyl}piperidin-4-yl)methyl]pyrimidin-4-yl}carbonyl)amino]acetic acid

In accordance with Examples 1-(8), 1-(9), 6-(2) and 1-(11), but using 2-(4-bromobenzyl)pyrazine (1.1 g, 4.5 mmol) instead of 1-bromo-4-tert-butylbenzene, the title compound (0.17 g, 0.35 mmol) was afforded as a yellow solid (yield 12%).
MS m/z: 477 (M+H)⁺;
1H-NMR (500 MHz, DMSO-d6) δ: 12.89 (1H, brs), 11.91 (1H, brs), 9.38 (1H, t, J=6 Hz), 8.60-8.43 (3H, m), 7.10 (2H, d, J=8 Hz), 6.84 (2H, d, J=8 Hz), 4.01 (2H, s), 3.99 (2H, d, J=6 Hz), 3.60 (2H, d, J=11 Hz), 2.74 (2H, d, J=8 Hz), 2.60 (2H, t, J=11 Hz), 2.43 (3H, s), 2.09-1.99 (1H, m), 1.65 (2H, d, J=13 Hz), 1.33 (2H, dq, J=9 Hz, 3 Hz).

### Example 141

### [({5-Hydroxy-6-methyl-2-[(1-{4-[(1H-pyrrol-1-yl)methyl]phenyl}piperidin-4-yl)methyl]pyrimidin-4-yl}carbonyl)amino]acetic acid

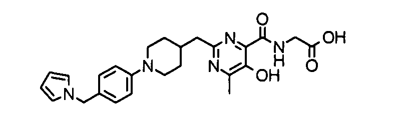

In accordance with Examples 1-(8) to 1-(11), but using 1-(4-bromobenzyl)-1H-pyrrole (2.2 g, 9.1 mmol) instead of 1-bromo-4-tert-butylbenzene, the title compound (0.84 g, 1.8 mmol) was afforded as a white solid (yield 26%).
MS m/z: 464 (M+H)⁺;
1H-NMR (500 MHz, DMSO-d6) δ: 12.94 (1H, brs), 11.93 (1H, brs), 9.38 (1H, t, J=6 Hz), 7.06 (2H, d, J=9 Hz), 6.86 (2H, d, J=9 Hz), 6.78-6.74 (1H, m), 5.99-5.94 (1H, m), 4.94 (2H, s), 3.99 (2H, d, J=6 Hz), 3.64 (2H, d, J=13 Hz), 3.35 (2H, brs), 2.76 (2H, d, J=7 Hz), 2.62 (2H, t, J=13 Hz), 2.43 (3H, s), 2.09-1.99 (1H, m), 1.65 (2H, d, J=11 Hz), 1.33 (2H, dq, J=13 Hz, 3 Hz).

### Example 142

### [({5-Hydroxy-6-methyl-2-[(1-{4-[(1H-pyrazol-1-yl)methyl]phenyl}piperidin-4-yl)methyl]pyrimidin-4-yl}carbonyl)amino]acetic acid

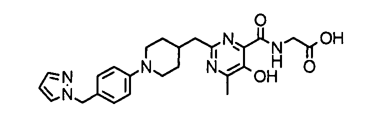

### (1) 1-(4-Bromobenzyl)-1H-pyrazole

Potassium tert-butoxide (1.4 g, 12 mmol) and 18-crown-6-ether (0.26 g, 1.0 mmol) were dissolved in diethyl ether (25 mL), and pyrazole (0.68 g, 10 mmol) was added, followed by stirring at room temperature for 15 minutes. A solution of 1-bromo-4-(bromomethyl)benzene in diethyl ether (25 mL) was added to the reaction solution, followed by stirring for 1.5 hours. Water was added to the reaction solution, followed by extraction with ethyl acetate. After the organic layer was concentrated under reduced pressure, the resulting residue was purified by silica gel column chromatography (Moritex Corporation, elution solvent: hexane/ethyl acetate), and a fraction corresponding to the Rf value=0.40 (hexane/ethyl acetate=2/1) by thin layer chromatography was concentrated under reduced pressure to afford the title compound (2.2 g, 9.5 mmol) as a colorless oil (yield 95%).
1H-NMR (500 MHz, CDCl3) δ: 7.57-7.54 (1H, m), 7.45 (2H, d, J=8 Hz), 7.42-7.36 (1H, m), 7.08 (2H, d, J=8 Hz), 6.32-6.27 (1H, m), 5.28 (2H, s).

### (2) [({5-Hydroxy-6-methyl-2-[(1-{4-[(1H-pyrazol-1-yl)methyl]phenyl}piperidin-4-yl)methyl]pyrimidin-4-yl}carbonyl)amino]acetic acid

In accordance with Examples 1-(8) to 1-(11), but using 1-(4-bromobenzyl)-1H-pyrazole (2.2 g, 9.1 mmol) instead of 1-bromo-4-tert-butylbenzene, the title compound (0.49 g, 1.1 mmol) was afforded as a white solid (yield 16%).
MS m/z: 465 (M+H)⁺;
1H-NMR (500 MHz, DMSO-d6) δ:12.89 (1H, brs), 11.91 (1H, brs), 9.38 (1H, t, J=5 Hz), 7.77-7.72 (1H, m), 7.43-7.39 (1H, m), 7.09 (2H, d, J=8 Hz), 6.87 (2H, d, J=8 Hz), 6.27-6.20 (1H, m), 5.17 (2H, s), 3.99 (2H, d, J=5 Hz), 3.64 (2H, d, J=12 Hz), 2.76 (2H, d, J=7 Hz), 2.63 (2H, t, J=11 Hz), 2.43 (3H, s), 2.09-1.99 (1H, m), 1.64 (2H, d, J=11 Hz), 1.32 (2H, dq, J=10 Hz, 3 Hz).

### Example 143

### [({5-Hydroxy-6-methyl-2-[(1-{4-[(quinolin-3-yl)methyl]phenyl}piperidin-4-yl)methyl]pyrimidin-4-yl}carbonyl)amino]acetic acid

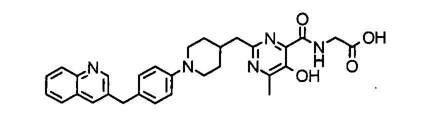

### (1) 3-(4-Bromobenzyl)quinoline

In accordance with Examples 125-(1) to (3), but using quinoline-3-carboxylic acid (2.6 g, 15 mmol) instead of 6-methylnicotinic acid, the title compound (1.5 g, 5.1 mmol) was afforded as a yellow solid (yield 34%).
1H-NMR (500 MHz, CDCl3) δ: 8.79 (1H, s), 8.09 (1H, d, J=8 Hz), 7.86 (1H, s), 7.74 (1H, d, J=8 Hz), 7.68 (1H, t, J=8 Hz), 7.53 (1H, t, J=8 Hz), 7.44 (2H, d, J=7 Hz), 7.11 (2H, d, J=7 Hz), 4.13 (2H, s).

### (2) [({5-Hydroxy-6-methyl-2-[(1-{4-[(quinolin-3-yl)methyl]phenyl}piperidin-4-yl)methyl]pyrimidin-4-yl}carbonyl)amino]acetic acid

In accordance with Examples 1-(8), 1-(9), 6-(2) and 1-(11), but using 3-(4-bromobenzyl)quinoline (1.3 g, 4.5 mmol) instead of 1-bromo-4-tert-butylbenzene, the title compound (0.73 g, 1.4 mmol) was afforded as a white solid (yield 46%).
MS m/z: 526 (M+H)⁺;
1H-NMR (500 MHz, DMSO-d6) δ: 12.95 (1H, brs), 11.94 (1H, brs), 9.33 (1H, t, J=3 Hz), 8.80 (1H, s), 8.11 (1H, s), 7.97 (1H, d, J=9 Hz), 7.91 (1H, d, J=9 Hz), 7.69 (1H, t, J=9 Hz), 7.57 (1H, t, J=9 Hz), 7.12 (2H, d, J=8 Hz), 6.87 (2H, d, J=8 Hz), 4.04 (2H, s), 3.93 (2H, d, J=3 Hz), 3.60 (2H, d, J=12 Hz), 2.74 (2H, d, J=8 Hz), 2.59 (2H, t, J=11 Hz), 2.43 (3H, s), 2.09-1.99 (1H, m), 1.65 (2H, d, J=13 Hz), 1.33 (2H, dq, J=9 Hz, 3 Hz).

### Example 144

### [({2-[(1-{4-[(1,2,3,4-Tetrahydroquinolin-1-yl)methyl]phenyl}piperidin-4-yl)methyl]-5-hydroxy-6-methylpyrimidin-4-yl}carbonyl)amino]acetic acid

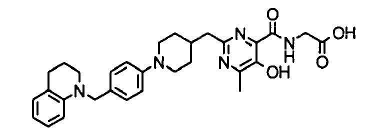

### (1) 1-(4-Bromobenzyl)-1,2,3,4-tetrahydroquinoline 4-Bromobenzaldehyde (1.9 g, 10 mmol) and 1,2,3,4-tetrahydroquinoline (1.1 g,

11 mmol) were dissolved in methylene chloride (50 mL), and sodium triacetoxyborohydride (3.0 g, 14 mmol) and acetic acid (0.80mL), 14 mmol) were added, followed by stirring at room temperature for 2 hours. Saturated aqueous sodium hydrogencarbonate solution was added to the reaction solution, followed by extraction with ethyl acetate. After the extract was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=4/1) by thin layer chromatography was concentrated under reduced pressure to afford the title compound (2.2 g, 7.4 mmol) as a colorless oil (yield 74%).
1H-NMR (500 MHz, CDCl3) δ: 7.43 (2H, d, J=6 Hz), 7.14 (2H, d, J=6 Hz), 6.98 (1H, d, J=9 Hz), 6.96 (1H, t, J=9 Hz), 6.59 (1H, t, J=9 Hz), 6.44 (1H, d, J=9 Hz), 4.41 (2H, s), 3.38-3.31 (2H, m), 2.86-2.77 (2H, m), 2.09-1.97 (2H, m).

### (2) [({2-[(1-{4-[(1,2,3,4-Tetrahydroquinolin-1-yl)methyl]phenyl}piperidin-4-yl)methyl]-5-hydroxy-6-methylpyrimidin-4-yl}carbonyl)amino]acetic acid

In accordance with Examples 1-(8) to 1-(11), but using 1-(4-bromobenzyl)-1,2,3,4-tetrahydroquinoline (2.1 g, 7.0 mmol) instead of 1-bromo-4-tert-butylbenzene, the title compound (0.62 g, 1.2 mmol) was afforded as a white solid (yield 23%).
MS m/z: 530 (M+H)⁺;
1H-NMR (500 MHz, DMSO-d6) δ: 9.29 (1H, t, J=5 Hz), 7.10-7.04 (2H, m), 6.86 (4H, d, J=8 Hz), 6.51-6.47 (1H, m), 6.46-6.40 (1H, m), 4.34 (2H, s), 3.90 (2H, d, J=5 Hz), 3.60 (2H, d, J=12 Hz), 3.31 (2H, t, J=6 Hz), 2.76 (2H, d, J=7 Hz), 2.70 (2H, t, J=6 Hz), 2.59 (2H, t, J=11 Hz), 2.43 (3H, s), 2.09-1.99 (1H, m), 1.93-1.85 (2H, m), 1.66 (2H, d, J=11 Hz), 1.33 (2H, dq, J=10 Hz, 3 Hz).

### Example 145

### [({2-[(1-{4-[(1,3-Dioxan-2-yl)methyl]phenyl}piperidin-4-yl)methyl]-5-hydroxy-6-methylpyrimidin-4-yl}carbonyl)amino]acetic acid

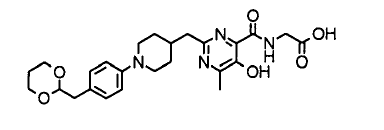

### (1) 2-(4-Bromobenzyl)-1,3-dioxane (4-Bromophenyl)acetaldehyde (3.7 g, 18 mmol) and propane-1,3-diol (1.5 g,

20 mmol) were dissolved in benzene (150 mL), and at room temperature p-toluenesulfonic acid monohydrate (0.18 g, 0.92 mmol) was added, followed by heating to reflux for 17.5 hours. After the reaction solution was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=10/1) by thin layer chromatography was concentrated under reduced pressure to afford the title compound (1.9 g, 7.5 mmol) as a yellow oil (yield 41 %).
1H-NMR (500 MHz, CDCl3) δ: 7.43 (2H, d, J=8 Hz), 7.15 (2H, d, J=8 Hz), 4.73-4.62 (1H, m), 4.19-4.08 (4H, m), 2.89-2.82 (2H, m), 2.17-2.05 (2H, m).

### (2) [({2-[(1-{4-[(1,3-Dioxan-2-yl)methyl]phenyl}piperidin-4-yl)methyl]-5-hydroxy-6-methylpyrimidin-4-yl}carbonyl)amino]acetic acid

In accordance with Examples 1-(8) to 1-(11), but using 2-(4-bromobenzyl)-1,3-dioxane (0.77 g, 3.0 mmol) instead of 1-bromo-4-tert-butylbenzene, the title compound (0.15 g, 0.32 mmol) was afforded as a white solid (yield 13%).
MS m/z: 485 (M+H)⁺;
1H-NMR (500 MHz, DMSO-d6) δ: 11.91 (1H, brs), 9.38 (1H, t, J=6 Hz), 7.02 (2H, d, 11 Hz), 6.81 (2H, d, J=11 Hz), 4.59 (1H, t, J=5 Hz), 4.00 (2H, d, J=6 Hz), 3.99-3.92 (2H, m), 3.70-3.59 (2H, m), 3.60 (2H, d, J=12 Hz), 2.77 (2H, d, J=7 Hz), 2.66 (2H, d, J=6 Hz), 2.60 (2H, t, J=11 Hz), 2.44 (3H, s), 2.09-1.99 (1H, m), 1.93-1.85 (2H, m), 1.66 (2H, d, J=12 Hz), 1.32 (2H, dq, J=13 Hz, 3 Hz).

### Example 146

### [({2-[(1-{4-[(5,5-Dimethyl-1,3-dioxan-2-yl)methyl]phenyl}piperidin-4-yl)methyl]-5-hydroxy-6-methylpyrimidin-4-yl}carbonyl)amino]acetic acid

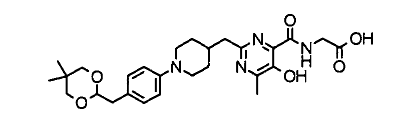

In accordance with Examples 145-(1) and 145-(2), but using 2,2-dimethylpropane-1,3-diol (1.6 g, 15.0 mmol) instead of propane-1,3-diol, the title compound (0.62 g, 1.2 mmol) was afforded as a white solid (yield 22%).
MS m/z: 513 (M+H)⁺;
1H-NMR (500 MHz, DMSO-d6) δ: 12.89 (1H, brs), 11.91 (1H, brs), 9.40 (1H, t, J=5 Hz), 7.04 (2H, d, 7 Hz), 6.81 (2H, d, J=7 Hz), 4.51 (1H, t, J=5 Hz), 4.00 (2H, d, J=5 Hz), 3.60 (2H, d, J=8 Hz), 3.50 (2H, d, 11 Hz), 3.38-3.32 (2H, m), 2.77 (2H, d, J=7 Hz), 2.71 (2H, d, J=4 Hz), 2.60 (2H, t, J=11 Hz), 2.44 (3H, s), 2.09-1.99 (1H, m), 1.66 (2H, d, J=12 Hz), 1.35 (2H, dq, J=13 Hz, 3 Hz), 1.08 (3H, s), 0.66 (3H, s).

### Example 147

### [({5-Hydroxy-6-methyl-2-[(1-{4-[(2,5,5-trimethyl-1,3-dioxan-2-yl)methyl]phenyl}piperidin-4-yl)methyl]pyrimidin-4-yl}carbonyl)amino]acetic acid

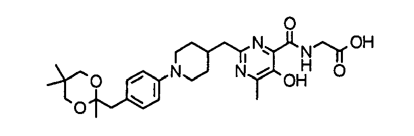

In accordance with Examples 145-(1) and 145-(2), but using (4-bromophenyl)acetone (2.6 g, 12 mmol) instead of (4-bromophenyl)acetaldehyde, and 2,2-dimethylpropane-1,3-diol (1.6 g, 13 mmol) instead of propane-1,3-diol, the title compound (0.39 g, 0.73 mmol) was afforded as a white solid (yield 18%).
MS m/z: 527 (M+H)⁺;
1H-NMR (500 MHz, DMSO-d6) δ: 12.30 (1H, brs), 9.33 (1H, t, J=6 Hz), 7.04 (2H, d, 8 Hz), 6.81 (2H, d, J=8 Hz), 3.93 (2H, d, J=6 Hz), 3.60 (2H, d, J=8 Hz), 3.48 (2H, d, 11 Hz), 3.38-3.32 (2H, m), 2.82 (3H, s), 2.77 (2H, d, J=7 Hz), 2.71 (2H, d, J=4 Hz), 2.59 (2H, t, J=11 Hz), 2.43 (3H, s), 2.09-1.99 (1H, m), 1.67 (2H, d, J=12 Hz), 1.35 (2H, dq, J=13 Hz, 3 Hz), 0.89 (3H, s), 0.84 (3H, s).

### Example 148

### {[(2-{[1-(2,3'-Bipyridin-6'-yl)piperidin-4-yl]methyl}-5-hydroxy-6-methylpyrimidin-4-yl)carbonyl]amino}acetic acid

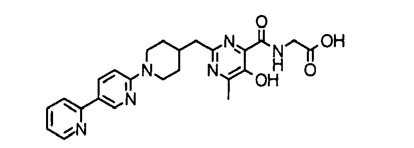

### (1) tert-Butyl 5-(benzyloxy)-2-{[1-(2,3'-bipyridin-6'-yl)pipendin-4-yl]methyl}-6-methylpyrimidine-4-carboxylate

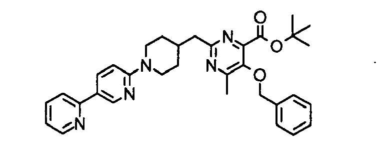

tert-Butyl 5-(benzyloxy)-2-{[1-(5-bromopyridin-2-yl)piperidin-4-yl]methyl}-6-methylpyrimidine-4-carboxylate (0.74 g, 1.3 mmol) obtained in Example 88-(1) was dissolved in tetrahydrofuran (30 mL), and at room temperature under a nitrogen atmosphere tetrakis(triphenylphosphine)palladium (0.16 g, 0.13 mmol) and a solution of 2-pyridylzinc bromide in tetrahydrofuran (0.50 M, 4.0 mL, 2.0 mmol) were added, followed by stirring at 60°C for 5 hours. After the reaction solution was concentrated under reduced pressure, the resulting residue was purified by silica gel column chromatography (Moritex Corporation, elution solvent: hexane/ethyl acetate), and a fraction corresponding to the Rf value=0.2 (hexane/ethyl acetate=1/1) by thin layer chromatography was concentrated under reduced pressure to afford the title compound (0.80 g, 1.3 mmol) as a yellow oil (quantitative yield).
MS m/z: 552 (M+H)⁺.

### (2) {[(2-{[1-(2,3'-Bipyridin-6'-yl)piperidin-4-yl]methyl}-5-hydroxy-6-methylpyrimidin-4-yl)carbonyl]amino}acetic acid

In accordance with Examples 1-(9) to 1-(11), but using tert-butyl 5-(benzyloxy)-2-{[1-(2,3'-bipyridin-6'-yl)piperidin-4-yl]methyl}-6-methylpyrimidine-4-carboxylate (0.80 g, 1.3 mmol) instead of tert-butyl 5-(benzyloxy)-2-{[1-(4-tert-butylphenyl)piperidin-4-yl]methyl}-6-methylpyrimidine-4-carboxylate, the title compound (0.12 g, 0.26 mmol) was afforded (yield 20%).
MS m/z: 463 (M+H)⁺;
1H-NMR (500 MHz, DMSO-d6) δ:12.88 (1H, brs), 11.91 (1H, s), 9.40 (1H, t, J=6 Hz), 8.81 (1H, s), 8.58 (1H, d, J=4 Hz), 8.18 (1H, d, J=9 Hz), 7.85 (1H, d, J=8 Hz), 7.80 (1H, t, J=8 Hz), 7.25-7.22 (1H, m), 6.91 (1H, d, J=9 Hz), 4.38 (2H, d, J=13 Hz), 4.00 (2H, d, J=6 Hz), 2.88 (2H, t, J=11 Hz), 2.76 (2H, d, J=7 Hz), 2.44 (3H, s), 2.26-2.17 (1H, m), 1.67 (2H, d, J=11 Hz), 1.29-1.19 (2H, m).

### Example 149

### [({5-Hydroxy-6-methyl-2-[(1-{5-[(pyridin-3-yl)methyl]pyridin-2-yl}piperidin-4-yl)methyl]pyrimidin-4-yl}carbonyl)amino]acetic acid

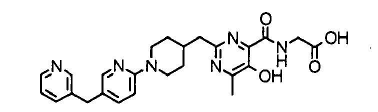

### (1) tert-Butyl 5-(benzyloxy)-6-methyl-2-[(1-{5-[(pyridin-3-yl)methyl]pyridin-2-yl}piperidin-4-yl)methyl]pyrimidine-4-carboxylate

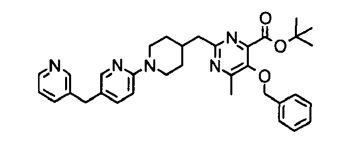

tert-Butyl 5-(benzyloxy)-2-{[1-(5-bromopyridin-2-yl)piperidin-4-yl]methyl}-6-methylpyrimidine-4-carboxylate (0.55 g, 1.0 mmol) obtained in Example 88-(1) was dissolved in 1,4-dioxane (10 mL), and 4,4,4',4',5,5,5',5'-octamethyl-2,2'-bi-1,3,2-dioxaborolane (0.31 g, 1.2 mmol), [1,1'-bis(diphenylphosphino)ferrocene]palladium dichloride-dichloromethane adduct (0.082 g, 0.10 mmol) and potassium acetate (0.30 g, 3.0 mmol) were added, followed by heating to reflux for 2.5 hours under a nitrogen atmosphere. After the reaction solution was cooled to room temperature, the insoluble materials were filtered off, and the filtrate was concentrated under reduced pressure. The resulting residue was dissolved in a mixture of toluene (8 mL), ethanol (5 mL) and water (5 mL), and at room temperature 3-(bromomethyl)pyridine hydrobromide (0.31 g, 1.2 mmol), tetrakis(triphenylphosphine)palladium adduct (0.23 g, 0.20 mmol) and sodium carbonate (0.53 g, 5.0 mmol) were added, followed by heating to reflux for 1 hour under a nitrogen atmosphere. After the reaction solution was cooled to room temperature, it was diluted with ethyl acetate, and the organic layer was washed with water. After the solvent was distilled off under reduced pressure, the resulting residue was purified by silica gel column chromatography (Moritex Corporation, elution solvent: ethyl acetate/methanol), and a fraction corresponding to the Rf value=0.30 (ethyl acetate/methanol =10/1) by thin layer chromatography was concentrated under reduced pressure to afford the title compound (0.44 g, 0.78 mmol) as a yellow oil (yield 78%).
1H-NMR (500 MHz, CDCl3) δ: 8.49 (1H, s), 8.45 (1H, s), 8.05 (1H, s), 7.73-7.62 (2H, m), 7.60-7.52 (1H, m), 7.51-7.37 (3H, m), 7.26-7.15 (2H, m), 6.61 (1H, d, J=9 Hz), 5.01 (2H, s), 4.21 (2H, d, J=13 Hz), 3.83 (2H, s), 2.87 (2H, t, J=7 Hz), 2.83 (2H, t, J=11 Hz), 2.45 (3H, s), 2.24-2.14 (1H, m), 1.75 (2H, d, J=12 Hz), 1.59 (9H, s), 1.37 (2H, dq, J=11 Hz, 3 Hz).

### (2) [({5-Hydroxy-6-methyl-2-[(1-{5-[(pyridin-3-yl)methyl]pyridin-2-yl}piperidin-4-yl)methyl]pyrimidin-4-yl}carbonyl)amino]acetic acid

In accordance with Examples 1-(9), 6-(2) and 1-(11), but using tert-butyl 5-(benzyloxy)-6-methyl-2-[(1-{5-[(pyridin-3-yl)methyl]pyridin-2-yl}piperidin-4-yl)methyl]pyrimidine-4-carboxylate (0.44 g, 0.78 mmol) instead of tert-butyl 5-(benzyloxy)-2-{[1-(4-tert-butylphenyl)piperidin-4-yl]methyl}-6-methylpyrimidine-4-carboxylate, the title compound (0.12 g, 0.25 mmol) was afforded as a white solid (yield 32%).
MS m/z: 477 (M+H)⁺;
1H-NMR (500 MHz, DMSO-d6) δ: 12.76 (1H, brs), 11.91 (1H, brs), 9.38 (1H, t, J=6 Hz), 8.49 (1H, s), 8.40 (1H, s), 8.03 (1H, s), 7.60 (1H, d, J=8 Hz), 7.36 (1H, d, J=8 Hz), 7.34-7.26 (1H, m), 6.75 (1H, d, J=9 Hz), 4.20 (2H, d, J=12 Hz), 3.99 (2H, d, J=6 Hz), 3.81 (2H, s), 2.75 (2H, d, J=6 Hz), 2.74 (2H, d, J=7 Hz), 2.43 (3H, s), 2.20-2.08 (1H, m), 1.62 (2H, d, J=13 Hz), 1.22 (2H, dq, J=8 Hz, 3 Hz).

### Example 150

### ({[2-({1-[4-(Cyclopropylmethyl)phenyl]piperidin-4-yl}methyl)-5-hydroxy-6-methylpyrimidin-4-yl]carbonyl}amino)acetic acid

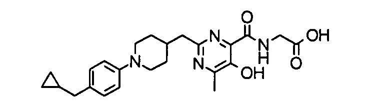

In accordance with Examples 1-(8), 73-(2), 1-(10) and 1-(11), but using 1-bromo-4-(cyclopropylmethyl)benzene (0.29 g, 1.4 mmol) instead of 1-bromo-4-tert-butylbenzene, the title compound (0.32 g, 0.74 mmol) was afforded as a white solid (yield 64%).
MS m/z: 439 (M+H)⁺;
1H-NMR (500 MHz, DMSO-d6) δ: 12.89 (1H, brs), 11.91 (1H, brs), 9.39 (1H, t, J=6 Hz), 7.06 (2H, d, J=8 Hz), 6.84 (2H, d, J=8 Hz), 4.00 (2H, d, J=6 Hz), 3.59 (2H, d, J=12 Hz), 2.77 (2H, d, J=7 Hz), 2.59 (2H, t, J=12 Hz), 2.44 (3H, s), 2.39 (2H, d, J=7 Hz), 2.08-1.99 (1H, m), 1.66 (2H, d, J=12 Hz), 1.36 (2H, q, J=12 Hz), 0.93-0.85 (1H, m), 0.43 (2H, d, J=7 Hz), 0.14 (2H, d, J=4 Hz).

### Example 151

### ({[2-({1-[4-(Cyclohexylmethyl)phenyl]piperidin-4-yl}methyl)-5-hydroxy-6-methylpyrimidin-4-yl]carbonyl}amino)acetic acid

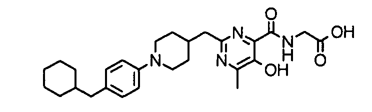

### (1) Ethyl ({[5-(benzyloxy)-2-({1-[4-(cyclohexylidenemethyl)phenyl]piperidin-4-yl}methyl)-6-methylpyrimidin-4-yl]carbonyl}amino)acetate

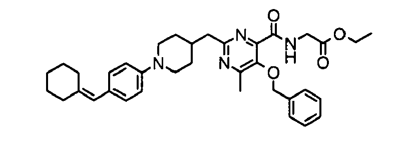

In accordance with Examples 1-(8) and 1-(9), but using 1-bromo-4-(cyclohexylidenemethyl)benzene (1.96 g, 7.8 mmol) instead of 1-bromo-4-tert-butylbenzene, the title compound (0.82 g, 1.4 mmol) was afforded as a yellow oil (yield 23%).
1H-NMR (500 MHz, CDCl3) δ: 8.35 (1H, t, J=5 Hz), 7.49 (2H, d, J=7 Hz), 7.41-7.32 (3H, m), 7.10 (2H, d, J=8 Hz), 6.88 (2H, d, J=8 Hz), 6.13 (1H, s), 5.12 (2H, s), 4.26 (2H, q, J=7 Hz), 4.24 (2H, d, J=5 Hz), 3.66 (2H, d, J=12 Hz), 2.88 (2H, d, J=7 Hz), 2.71 (2H, t, J=12 Hz), 2.46 (3H, s), 2.38 (2H, t, J=5 Hz), 2.23 (2H, m), 2.08-1.95 (1H, m), 1.76 (2H, d, J=12 Hz), 1.66-1.51 (6H, m), 1.54 (2H, dq, J=13 Hz, 3 Hz), 1.32 (3H, t, J=7 Hz).

### (2) ({[2-({1-[4-(Cyclohexylmethyl)phenyl]piperidin-4-yl}methyl)-5-hydroxy-6-methylpyrimidin-4-yl]carbonyl}amino)acetic acid

In accordance with Examples 1-(10) and 1-(11), but using ethyl ({[5-(benzyloxy)-2-({1-[4-(cyclohexylidenemethyl)phenyl]piperidin-4-yl}methyl)-6-methylpyrimidin-4-yl]carbonyl}amino)acetate (0.41 g, 0.69 mmol) instead of ethyl ({[5-(benzyloxy)-2-{[1-(4-tert-butylphenyl)piperidin-4-yl]methyl -6-methylpyrimidin-4-yl]carbonyl}amino)acetate, the title compound (0.23 g, 0.48 mmol) was afforded as a white solid (yield 70%).
MS m/z: 481 (M+H)⁺;
1H-NMR (500 MHz, CD3OD) δ: 7.40-7.33 (2H, m), 7.30-7.25 (2H, m), 4.15 (2H, s), 3.65 (2H, d, J=10 Hz), 3.46-3.36 (2H, m), 2.92 (2H, d, J=7 Hz), 2.59 (2H, t, J=10Hz), 2.52 (3H, s), 2.41-2.29 (2H, m), 2.05-1.96 (2H, m), 1.81-1.59 (4H, m), 1.57-1.47 (2H, m), 1.27-1.13 (2H, m), 1.25 (2H, dq, J=10 Hz, 5 Hz), 1.01-0.95 (2H, m).

### Example 152

### [({5-Hydroxy-2-[(1-{4-[(1-hydroxycyclohexyl)methyl]phenyl}piperidin-4-yl)methyl]-6-methylpyrimidin-4-yl}carbonyl)amino]acetic acid

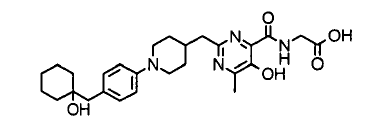

### (1) Ethyl ({[5-hydroxy-6-methyl-2-({1-[4-({[1-(2,2,2-trifluoroacetoxy)]cyclohexyl}methyl)phenyl]piperidin-4-yl}methyl)pyrimidin-4-yl]carbonyl}amino)acetate

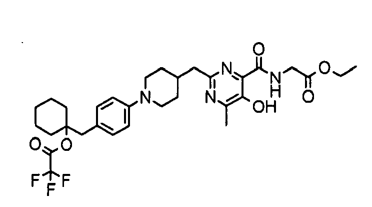

In accordance with Example 6-(2), but using ethyl ({[5-(benzyloxy)-2-({1-[4-(cyclohexylidenemethyl)phenyl]piperidin-4-yl}methyl)-6-methylpyrimidin-4-yl]carbonyl}amino)acetate (0.41 g, 0.69 mmol) obtained in Example 151-(1) instead of ethyl ({[5-(benzyloxy)-2-{[1-(2-chlorophenyl)piperidin-4-yl]methyl}-6-methylpyrimidin-4-yl]carbonyl}amino)acetate, the title compound (0.089 g, 0.18 mmol) was afforded as a colorless oil (yield 24%).
1H-NMR (500 MHz, CDCl3) δ:11.36 (1H, brs), 8.49 (1H, t, J=5 Hz), 7.05 (2H, d, J=8 Hz), 6.86 (2H, d, J=8 Hz), 4.29 (2H, q, J=8 Hz), 4.23 (2H, d, J=5 Hz), 3.61 (2H, d, J=10 Hz), 3.14 (2H, s), 2.82 (2H, d, J=7 Hz), 2.67 (2H, t, J=11 Hz), 2.53 (3H, s), 2.09-1.95 (5H, m), 1.89-1.81 (2H, m), 1.80-1.70 (2H, m), 1.62-1.48 (4H, m), 1.33 (3H, t, J=8 Hz), 1.31-1.28 (2H, m).

### (2) [({5-Hydroxy-2-[(1-{4-[(1-hydroxycyclohexyl)methyl]phenyl}piperidin-4-yl)methyl]-6-methylpyrimidin-4-yl} carbonyl)amino] acetic acid

In accordance with Example 1-(11), but using ethyl ({[5-hydroxy-6-methyl-2-({1-[4-({[1-(2,2,2-trifluoroacetoxy)]cyclohexyl}methyl)phenyl]piperidin-4-yl}methyl)pyrimidin-4-yl]carbonyl}amino)acetate (0.15 g, 0.24 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.089 g, 0.18 mmol) was afforded as a white solid (yield 76%).
MS m/z: 497 (M+H)⁺;
1H-NMR (500 MHz, DMSO-d6) δ:12.87 (1H, brs), 11.90 (1H, brs), 9.40 (1H, t, J=3 Hz), 7.71 (2H, d, J=8 Hz), 6.80 (2H, d, J=8 Hz), 4.00 (2H, d, J=3 Hz), 3.89 (1H, brs), 3.59 (2H, d, J=9 Hz), 2.77 (2H, d, J=6 Hz), 2.59 (2H, t, J=8 Hz), 2.44 (3H, s), 2.41-2.32 (2H, m), 2.05-1.96 (1H, m), 1.70-1.60 (4H, m), 1.57-1.47 (2H, m), 1.41-1.30 (4H, m), 1.29-1.19 (2H, m), 1.16-1.06 (2H, m).

### Example 153

### [({5-Hydroxy-2-[(1-{4-[(1-methoxycyclohexyl)methyl]phenyl}piperidin-4-yl)methyl]-6-methylpyrimidin-4-yl}carbonyl)amino]acetic acid

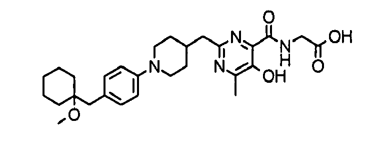

### (1) 1-(4-Bromobenzyl)cyclohexyl trifluoroacetate

1-Bromo-4-(cyclohexylidenemethyl)benzene (1.3 g, 5.0 mmol) was dissolved in methylene chloride (50 mL), and trifluoroacetic acid (50 mL) was added, followed by stirring at room temperature for 15 hours. After the reaction solution was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=10/1) by thin layer chromatography was concentrated under reduced pressure to afford the title compound (1.6 g, 4.3 mmol) as a colorless oil (yield 86%).
1H-NMR (500 MHz, CDCl3) δ: 7.42 (2H, d, J=8 Hz), 6.99 (2H, d, J=8 Hz), 3.20 (2H, s), 2.34-2.25 (2H, m), 1.68-1.55 (4H, m), 1.50-1.39 (4H, m).

### (2) 1-(4-Bromobenzyl)cyclohexanol

1-(4-Bromobenzyl)cyclohexyl trifluoroacetate (1.6 g, 4.3 mmol) was dissolved in a mixture of tetrahydrofuran (25 mL) and methanol (25 mL), and aqueous sodium hydroxide solution (1 M, 25 mL) was added, followed by stirring at room temperature for 18 hours. After the reaction solution was concentrated under reduced pressure, the resulting residue was neutralized with hydrochloric acid (1 M), followed by extraction with ethyl acetate. After the organic layer was concentrated under reduced pressure, the resulting residue was purified by silica gel column chromatography (Moritex Corporation, elution solvent: hexane/ethyl acetate), and a fraction corresponding to the Rf value=0.30 (hexane/ethyl acetate=10/1) by thin layer chromatography was concentrated under reduced pressure to afford the title compound (1.1 g, 3.9 mmol) as a white solid (yield 91 %).
1H-NMR (500 MHz, CDCl3) δ: 7.42 (2H, d, J=8 Hz), 7.09 (2H, d, J=8 Hz), 3.57 (1H, brs), 2.70 (2H, s), 1.62-1.38 (10H, m).

### (3) 1-(4-Bromobenzyl)cyclohexyl methyl ether

1-(4-Bromobenzyl)cyclohexanol (1.1 g, 3.9 mmol) was dissolved in N,N-dimethylformamide (50 mL), and at room temperature sodium hydride (0.19 g, 4.3 mmol) was added, followed by stirring at 50°C for 30 minutes. Methyl iodide (0.29 mL, 4.7 mmol) was added to the reaction solution, followed by further stirring at the same temperature for 2 hours. After the reaction solution was cooled to room temperature, water was added, followed by extraction with ethyl acetate, and the extract was washed with water. After the solvent was distilled off under reduced pressure, the resulting residue was purified by silica gel column chromatography (Moritex Corporation, elution solvent: hexane/ethyl acetate), and a fraction corresponding to the Rf value=0.70 (hexane/ethyl acetate=10/1) by thin layer chromatography was concentrated under reduced pressure to afford the title compound (0.38 g, 1.3 mmol) as a colorless oil (yield 34%).
1H-NMR (500 MHz, CDCl3) δ: 7.38 (2H, d, J=8 Hz), 7.03 (2H, d, J=8 Hz), 3.29 (3H, s), 2.68 (2H, s), 1.62-1.60 (2H, m), 1.56-1.40 (4H, m), 1.28-1.14 (4H, m).

### (4) [({5-Hydroxy-2-[(1-{4-[(1-methoxycyclohexyl)methyl]phenyl}piperidin-4-yl)methyl]-6-methylpyrimidin-4-yl}carbonyl)amino]acetic acid

In accordance with Examples 1-(8) to 1-(11), but using 1-(4-bromobenzyl)cyclohexyl methyl ether (0.38 g, 1.3 mmol) instead of 1-bromo-4-tert-butylbenzene, the title compound (0.11 g, 0.21 mmol) was afforded as a white solid (yield 21%).
MS m/z: 511 (M+H)⁺;
1H-NMR (500 MHz, DMSO-d6) δ: 9.33 (1H, t, J=6 Hz), 6.96 (2H, d, J=9 Hz), 6.81 (2H, d, J=9 Hz), 3.95 (2H, d, J=6 Hz), 3.60 (2H, d, J=12 Hz), 3.17 (3H, s), 2.77 (2H, d, J=7 Hz), 2.58 (2H, t, J=12 Hz), 2.57 (2H, s), 2.44 (3H, s), 2.09-1.99 (1H, m), 1.65 (2H, d, J=13 Hz), 1.60-1.44 (2H, m), 1.44-1.30 (4H, m), 1.33 (2H, dq, J=9 Hz, 3 Hz), 1.28-1.14 (4H, m).

### Example 154

### ({[5-Hydroxy-6-methyl-2-({1-[4-(1-methyl-1-phenylethyl)phenyl]piperidin-4-yl}methyl)pyrimidin-4-yl]carbonyl}amino)acetic acid

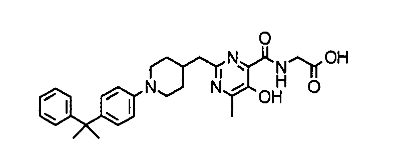

### (1) 4-(1-Methyl-1-phenylethyl)phenyl trifluoromethanesulfonate

In accordance with Example 79-(1), but using 4-(1-methyl-1-phenylethyl)phenol (2.1 g, 10 mmol) instead of 3,3-dimethyl-2,3-dihydro-1-benzofuran-6-ol, the title compound (3.4 g, 10 mmol) was afforded as a colorless oil (quantitative yield).
1H-NMR (500 MHz, CDCl3) δ: 7.35-7.10 (9H, m), 1.68 (6H, s).

### (2) ({[5-Hydroxy-6-methyl-2-({1-[4-(1-methyl-1-phenylethyl)phenyl]piperidin-4-yl}methyl)pyrimidin-4-yl]carbonyl}amino)acetic acid

In accordance with Examples 1-(8), 1-(9), 6-(2) and 1-(11), but using 4-(1-methyl-1-phenylethyl)phenyl trifluoromethanesulfonate (3.4 g, 10 mmol) instead of 1-bromo-4-tert-butylbenzene, the title compound (0.41 g, 0.82 mmol) was afforded as a white solid (yield 10%).
MS m/z: 503 (M+H)⁺;
1H-NMR (500 MHz, DMSO-d6) δ: 9.14 (1H, t, J=11 Hz), 7.29-7.10 (5H, m), 7.02 (2H, d, J=10 Hz), 6.81 (2H, d, J=10 Hz), 3.78-3.68 (2H, m), 3.59 (2H, d, J=10 Hz), 2.75 (2H, t, J=8 Hz), 2.58 (2H, t, J=10 Hz), 2.42 (3H, s), 2.08-1.98 (1H, m), 1.66 (2H, d, J=11 Hz), 1.58 (6H, s), 1.34 (2H, dq, J=13 Hz, 5 Hz).

### Example 155

### [({5-Hydroxy-2-[(1-{4-[hydroxy(phenyl)methyl]phenyl}piperidin-4-yl)methyl]-6-methylpyrimidin-4-yl}carbonyl)amino]acetic acid

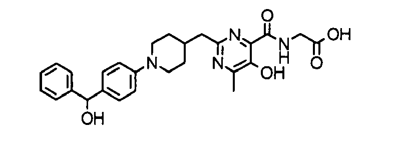

In accordance with Examples 1-(8), 1-(9), 6-(2) and 1-(11), but using (4-bromophenyl)(phenyl)methyl methyl ether (0.95 g, 3.4 mmol) instead of 1-bromo-4-tert-butylbenzene, the title compound (0.078 g, 0.16 mmol) was afforded as a white solid (yield 6.1 %).
MS m/z: 491 (M+H)⁺;
1H-NMR(500 MHz, DMSO-d6) δ: 12.86 (1H, brs), 11.90 (1H, brs), 9.38 (1H, t, J=8 Hz), 7.35-7.31 (2H, m), 7.30-7.25 (2H, m), 7.20-7.11 (1H, m), 7.15 (2H, d, J=11 Hz), 6.83 (2H, d, J=11 Hz), 5.65 (1H, s), 5.58 (1H, s), 3.99 (2H, d, J=8 Hz), 3.60 (2H, d, J=8 Hz), 2.75 (2H, t, J=5 Hz), 2.60 (2H, t, J=11 Hz), 2.43 (3H, s), 2.08-1.98 (1H, m), 1.64 (2H, d, J=11 Hz), 1.33 (2H, dq, J=11 Hz, 5 Hz).

### Example 156

### [({5-Hydroxy-6-methyl-2-[(1-{4-[(2-pyridin-2-yl)ethyl]phenyl}piperidin-4-yl)methyl]pyrimidin-4-yl}carbonyl)amino]acetic acid

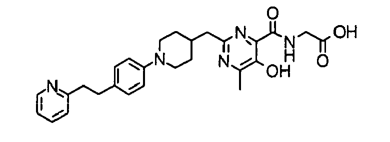

### (1) 4-[(2-Pyridin-2-yl)ethyl]phenyl trifluoromethanesulfonate

In accordance with Example 79-(1), but using 4-[(2-pyridin-2-yl)ethyl]phenol (0.82 g, 4.1 mmol) instead of 3,3-dimethyl-2,3-dihydro-1-benzofuran-6-ol, the title compound (1.2 g, 3.7 mmol) was afforded as a pale yellow solid (yield 90%). 1H-NMR (500 MHz, CDCl3) δ: 8.57 (1H, d, J=4 Hz), 7.57 (1H, t, J=8 Hz), 7.25 (2H, d, J=8 Hz), 7.17-7.12 (3H, m), 7.05 (1H, d, J=8 Hz), 3.09 (4H, brs).

### (2) ({[5-Hydroxy-6-methyl-2-({1-[4-(2-pyridin-2-ylethyl)phenyl]piperidin-4-yl}methyl)pyrimidin-4-yl]carbonyl}amino)acetic acid

In accordance with Examples 1-(8), 73-(2), 6-(2) and 1-(11), but using 4-[(2-pyridin-2-yl)ethyl]phenyl trifluoromethanesulfonate (0.46 g, 1.4 mmol) instead of 1-bromo-4-tert-butylbenzene, the title compound (0.12 g, 0.24 mmol) was afforded as a white solid (yield 21%).
MS m/z: 490 (M+H)⁺;
1H-NMR (500 MHz, DMSO-d6) δ: 12.88 (1H, brs), 11.91 (1H, brs), 9.39 (1H, t, J=5 Hz), 8.49 (1H, d, J=4 Hz), 7.66 (1H, t, J=8 Hz), 7.23-7.17 (2H, m), 7.03 (2H, d, J=8 Hz), 6.82 (2H, d, J=8 Hz), 4.01 (2H, d, J=5 Hz), 3.59 (2H, d, J=12 Hz), 2.96 (2H, t, J=8 Hz), 2.86 (2H, t, J=8 Hz), 2.77 (2H, d, J=7 Hz), 2.59 (2H, t, J=12 Hz), 2.44 (3H, s), 2.07-2.00 (1H, m), 1.66 (2H, d, J=13 Hz), 1.39-1.31 (2H, m).

### Example 157

### [({5-Hydroxy-6-methyl-2-[(1-{4-[(2-pyridin-3-yl)ethyl]phenyl}piperidin-4-yl)methyl]pyrimidin-4-yl}carbonyl)amino]acetic acid

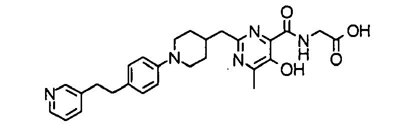

### (1) 4-[(2-Pyridin-3-yl)ethyl]phenyl trifluoromethanesulfonate

In accordance with Example 79-(1), but using 4-[(2-pyridin-3-yl)ethyl]phenol (0.52 g, 2.6 mmol) instead of 3,3-dimethyl-2,3-dihydro-1-benzofuran-6-ol, the title compound (0.22 g, 0.66 mmol) was afforded as a colorless oil (yield 25%). 1H-NMR (500 MHz, CDCl3) δ: 8.47 (1H, d, J=5 Hz), 8.42 (1H, s), 7.41 (1H, d, J=7 Hz), 7.22-7.18 (5H, m), 2.98-2.91 (4H, m).

### (2) ({[5-Hydroxy-6-methyl-2-({1-[4-(2-pyridin-3-ylethyl)phenyl]piperidin-4-yl}methyl)pyrimidin-4-yl]carbonyl}amino)acetic acid

In accordance with Examples 1-(8), 73-(2), 6-(2) and 1-(11), but using 4-[(2-pyridin-3-yl)ethyl]phenyl trifluoromethanesulfonate (220 mg, 0.66 mmol) instead of 1-bromo-4-tert-butylbenzene, the title compound (41 mg, 0.084 mmol) was afforded as a white solid (yield 15%).
MS m/z: 490 (M+H)⁺;
1H-NMR (500 MHz, DMSO-d6) δ:12.86 (1H, brs), 11.90 (1H, brs), 9.39 (1H, t, J=6 Hz), 8.40-8.36 (2H, m), 7.62 (1H, dt, J=8 Hz, 2 Hz), 7.29 (1H, dd, J=8 Hz, 5 Hz), 7.03 (2H, d, J=9 Hz), 6.82 (2H, d, J=9 Hz), 4.01 (2H, d, J=6 Hz), 3.60 (2H, d, J=12 Hz), 2.86-2.81 (2H, m), 2.80-2.75 (4H, m), 2.59 (2H, t, J=12 Hz), 2.44 (3H, s), 2.09-1,99 (1H, m), 1.66 (2H, d, J=12 Hz), 1.40-1.30 (2H, m).

### Example 158

### ({[2-({1-[4-(Benzyloxy)phenyl]piperidin-4-yl}methyl)-5-hydroxy-6-methylpyrimidin-4-yl]carbonyl}amino)acetic acid

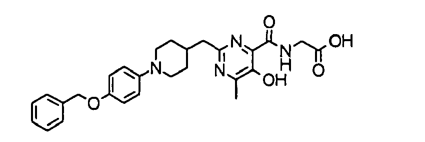

In accordance with Examples 1-(8), 73-(2), 6-(2) and 1-(11), but using 1-(benzyloxy)-4-bromobenzene (0.36 g, 1.4 mmol) instead of 1-bromo-4-tert-butylbenzene, the title compound (0.28 g, 0.58 mmol) was afforded as a white solid (yield 50%).
MS m/z: 491 (M+H)⁺;
1H-NMR (500 MHz, DMSO-d6) δ:12.86 (1H, brs), 11.90 (1H, s), 9.39 (1H, t, J=5 Hz), 7.42 (2H, d, J=7 Hz), 7.38 (2H, t, J=7 Hz), 7.31 (1H, t, J=7 Hz), 6.87 (4H, brs), 5.01 (2H, s), 4.01 (2H, d, J=5 Hz), 3.47 (2H, d, J=12 Hz), 2.78 (2H, d, J=7 Hz), 2.57-2.50 (2H, m), 2.44 (3H, s), 2.04-1.97 (1H, m), 1.66 (2H, d, J=12 Hz), 1.37 (2H, q, J=12 Hz).

### Example 159

### ({[5-Hydroxy-6-methyl-2-({1-[4-(phenoxymethyl)phenyl]piperidin-4-yl}methyl)pyrimidin-4-yl]carbonyl}amino)acetic acid

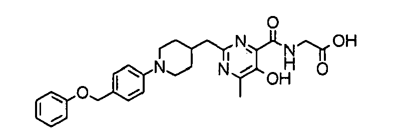

In accordance with Examples 1-(8), 73-(2), 1-(10) and 1-(11), but using 1-bromo-4-(phenoxymethyl)benzene (0.36 g, 1.4 mmol) instead of 1-bromo-4-tert-butylbenzene, the title compound (0.32 g, 0.65 mmol) was afforded as a pale red solid (yield 56%).
MS m/z: 491 (M+H)⁺;
1H-NMR (500 MHz, DMSO-d6) δ: 12.90 (1H, brs), 11.92 (1H, brs), 9.39 (1H, t, J=6 Hz), 7.29-7.24 (4H, m), 6.98 (2H, d, J=8 Hz), 6.94-6.90 (3H, m), 4.94 (2H, s), 4.00 (2H, d, J=6 Hz), 3.69 (2H, d, J=12 Hz), 2.77 (2H, d, J=7 Hz), 2.66 (2H, d, J=12 Hz), 2.44 (3H, s), 2.12-2.03 (1H, m), 1.66 (2H, d, J=12 Hz), 1.35 (2H, q, J=12 Hz).

### Example 160

### {[(2-{[1-(4-tert-Butylphenyl)pyrrolidin-3-yl]methyl}-5-hydroxy-6-methylpyrimidin-4-yl)carbonyl]amino}acetic acid

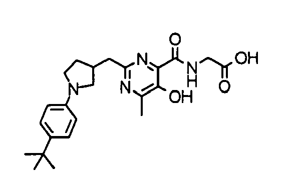

### (1) tert-Butyl 3-(2-amino-2-iminoethyl)pyrrolidine-1-carboxylate acetic acid salt

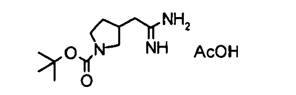

In accordance with Example 1-(3), but using tert-butyl 3-(cyanomethyl)pyrrolidine-1-carboxylate (2.1 g, 9.6 mmol) instead of tert-butyl 4-(cyanomethyl)piperidine-1-carboxylate, the title compound (2.8 g, 9.6 mmol) was afforded as a white solid (quantitative yield).
1H-NMR (500 MHz, DMSO-d6) δ: 3.49-3.33 (2H, m), 3.24-3.16 (1H, m), 2.89 (1H, dd, J=10 Hz, 8 Hz), 2.54-2.45 (1H, m), 2.42-2.30 (2H, m), 1.98-1.91 (1H, m), 1.70 (3H, s), 1.57-1.48 (1H, m), 1.40 (9H, s).

### (2) tert-Butyl 5-(benzyloxy)-2-{[1-(tert-butoxycarbonyl)pyrrolidin-3-yl]methyl}-6-hydroxypyrimidine-4-carboxylate

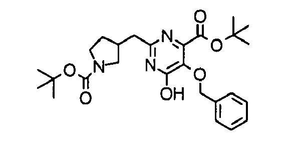

In accordance with Example 1-(4), but using tert-butyl 3-(2-amino-2-iminoethyl)pyrrolidine-1-carboxylate acetic acid salt (2.8 g, 9.6 mmol) instead of tert-butyl 4-(2-amino-2-iminoethyl)piperidine-1-carboxylate acetic acid salt, the title compound (2.7 g, 5.6 mmol) was afforded as a white solid (yield 59%).
1H-NMR (500 MHz, CDC3) δ: 12.68 (1H, brs), 7.47 (2H, d, J=7 Hz), 7.38-7.31 (3H, m), 5.23 (2H, d, J=8 Hz), 3.61-3.39 (2H, m), 3.32-3.23 (1H, m), 3.07-2.98 (1H, m), 2.79-2.70 (3H, m), 2.06-1.99 (1H, m), 1.68-1.58 (1H, m), 1.53 (9H, s), 1.44 (9H, s).

### (3) tert-Butyl 5-(benzyloxy)-6-methyl-2-[(pyrrolidin-3-yl)methyl]pyrimidine-4-carboxylate hydrochloride

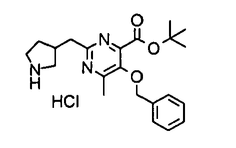

In accordance with Examples 1-(5) to 1-(7), but using tert-butyl 5-(benzyloxy)-2-{[1-(tert-butoxycarbonyl)pyrrolidin-3-yl]methyl}-6-hydroxypyrimidine-4-carboxylate (1.5 g, 3.0 mmol) instead of tert-butyl 5-(benzyloxy)-2-{[1-(tert-butoxycarbonyl)piperidin-4-yl]methyl}-6-hydroxypyrimidine-4-carboxylate, the title compound (0.90 g, 2.2 mmol) was afforded as a pale yellow solid (yield 72%).
1H-NMR (500 MHz, DMSO-d6) δ:7.45-7.37 (5H, m), 5.00 (2H, s), 3.34-3.33 (1H, m), 3.28-3.22 (1H, m), 3.16-3.08 (1H, m), 3.00 (2H, d, J=7 Hz), 2.90-2.84 (1H, m), 2.75-2.69 (1H, m), 2.46 (3H, s), 2.10-2.02 (1H, m), 1.66-1.58 (1H, m), 1.51 (9H, s).

### (4) {[(2-{[1-(4-tert-Butylphenyl)pyrrolidin-3-yl]methyl}-5-hydroxy-6-methylpyrimidin-4-yl)carbonyl]amino}acetic acid

In accordance with Examples 1-(8) to 1-(11), but using tert-butyl 5-(benzyloxy)-6-methyl-2-[(pyrrolidin-3-yl)methyl]pyrimidine-4-carboxylate hydrochloride (0.29 g, 0.70 mmol) instead of tert-butyl 5-(benzyloxy)-6-methyl-2-[(piperidin-4-yl)methyl]pyrimidine-4-carboxylate hydrochloride, the title compound (0.14 g, 0.33 mmol) was afforded as a pale yellow solid (yield 46%).
MS m/z: 427 (M+H)⁺;
1H-NMR (500 MHz, CDCl3) δ:11.35 (1H, s), 8.50 (1H, t, J=5 Hz), 7.26 (2H, d, J=9 Hz), 6.56 (2H, d, J=9 Hz), 4.24 (2H, d, J=6 Hz), 3.48 (1H, t, J=8 Hz), 3.42-3.37 (1H, m), 3.35-3.30 (1H, m), 3.08 (1H, t, J=8 Hz), 3.01 (2H, d, J=7 Hz), 2.95-2.87 (1H, m), 2.53 (3H, s), 2.18-2.12 (1H, m), 1.84-1.77 (1H, m), 1.28 (9H, s).

### Example 161

### {[(2-([1-(4-tert-Butylphenyl)azepan-4-yl]methyl)-5-hydroxy-6-methylpyrimidin-4-yl)carbonyl]amino}acetic acid

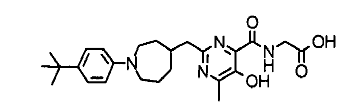

### • tert-Butyl 4-(cyanomethyl)azepane-1-carboxylate

In accordance with Examples 1-(1) and 1-(2), but using tert-butyl 4-oxoazepane-1-carboxylate (2.3 g, 11 mmol) instead of tert-butyl 4-oxopiperidine-1-carboxylate, the title compound (2.6 g, 11 mmol) was afforded as a colorless oil (yield 98%).
1H-NMR (500 MHz, CDCl3) δ: 3.65-3.55 (1H, m), 3.49-3.37 and 3.32-3.16 (total 3H, each m), 2.36-2.25 (2H, m), 1.98-1.80 (4H, m), 1.66-1.56 (1H, m), 1.54-1.42 (1H, m), 1.46 (9H, s), 1.41-1.33 (1H, m).

### • tert-Butyl 4-(2-amino-2-iminoethyl)azepane-1-carboxylate acetic acid salt

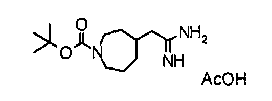

In accordance with Example 1-(3), but using tert-butyl 4-(cyanomethyl)azepane-1-carboxylate (2.6 g, 11 mmol) instead of tert-butyl 4-(cyanomethyl)piperidine-1-carboxylate, the title compound (2.5 g, 8.0 mmol) was afforded as a white solid (yield 74%).
1H-NMR (500 MHz, DMSO-d6) δ: 3.51-3.43 (1H, m), 3.36-3.24 (2H, m), 3.14-3.08 (1H, m), 2.20 (2H, dd, J=7 Hz, 2 Hz), 1.87-1.78 (2H, m), 1.75-1.69 (1H, m), 1.67-1.59 (1H, m), 1.64 (3H, s), 1.54-1.45 (1H, m), 1.40 (9H, s), 1.29-1.21 (1H, m), 1.14-1.06 (1H, m).

### (3) tert-Butyl 4-{[5-(benzyloxy)-4-(tert-butoxycarbonyl)-6-hydroxypyrimidin-2-yl]methyl}azepane-1-carboxylate

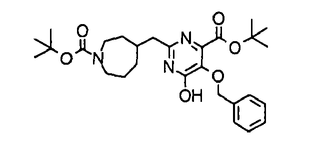

In accordance with Example 1-(4), but using tert-butyl 4-(2-amino-2-iminoethyl)azepane-1-carboxylate acetic acid salt (2.5 g, 8.0 mmol) instead of tert-butyl 4-(2-amino-2-iminoethyl)piperidine-1-carboxylate acetic acid salt, the title compound (2.5 g, 4.8 mmol) was afforded as a pale yellow solid (yield 60%).
1H-NMR (500 MHz, CDCl3) δ: 12.45 and 12.39 (total 1H, each brs), 7.47 (2H, d, J=7 Hz), 7.38-7.31 (3H, m), 5.24 (2H, s), 3.67-3.63 and 3.55-3.51 (total 1H, each m), 3.40-3.32 (2H, m), 3.15-3.05 (1H, m), 2.65-2.57 (2H, m), 2.11-2.05 (1H, m), 1.87-1.71 (3H, m), 1.56-1.23 (3H, m), 1.52 (9H, s), 1.43 (9H, s).

### (4) tert-Butyl 2-[(azepan-4-yl)methyl]-5-(benzyloxy)-6-methylpyrimidine-4-carboxylate hydrochloride

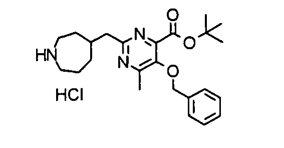

In accordance with Examples 1-(5) to 1-(7), but using tert-butyl 4-{[5-(benzyloxy)-4-(tert-butoxycarbonyl)-6-hydroxypyrimidin-2-yl]methyl}azepane-1-carboxylate (2.5 g, 4.8 mmol) instead of tert-butyl 5-(benzyloxy)-2-{[1-(tert-butoxycarbonyl)piperidin-4-yl]methyl}-6-hydroxypyrimidine-4-carboxylate, the title compound (1.8 g, 4.1 mmol) was afforded as a pale yellow solid (yield 84%).
1H-NMR (500 MHz, DMSO-d6) δ: 7.46-7.37 (5H, m), 4.99 (2H, s), 3.20-3.09 (2H, m), 3.04-2.95 (2H, m), 2.78 (2H, d, J=7 Hz), 2.45 (3H, s), 2.22-2.16 (1H, m), 1.86-1.81 (2H, m), 1.77-1.56 (3H, m), 1.51 (9H, s), 1.38-1.31 (1H, m).

### (5) {[(2-{[1-(4-tert-Butylphenyl)azepan-4-yl]methyl}-5-hydroxy-6-methylpyrimidin-4-yl)carbonyl]amino}acetic acid

In accordance with Examples 1-(8) to 1-(11), but using tert-butyl 2-[(azepan-4-yl)methyl]-5-(benzyloxy)-6-methylpyrimidine-4-carboxylate hydrochloride (0.36 g, 0.80 mmol) instead of tert-butyl 5-(benzyloxy)-6-methyl-2-[(piperidin-4-yl)methyl]pyrimidine-4-carboxylate hydrochloride, the title compound (0.10 g, 0.22 mmol) was afforded as a pale yellow solid (yield 27%).
MS m/z: 455 (M+H)⁺;
1H-NMR (500 MHz, DMSO-d6) δ: 8.99 (1H, brs), 7.14 (2H, d, J=9 Hz), 6.58 (2H, d, J=9 Hz), 3.56 (2H, brs), 3.53-3.24 (4H, m), 2.70 (2H, d, J=6 Hz), 2.38 (3H, s), 2.10-2.02 (1H, m), 1.90-1.83 (1H, m), 1.80-1.74 (1H, m), 1.66-1.57 (2H, m), 1.48-1.40 (1H, m), 1.22 (9H, s), 1.24-1.14 (1H, m).

### Example 162

### {[(2-{[1-(4-Fluorophenyl)azepan-4-yl]methyl}-5-hydroxy-6-methylpyrimidin-4-yl)carbonyl]amino}acetic acid

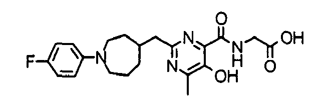

In accordance with Examples 1-(8) to 1-(11), but using tert-butyl 2-[(azepan-4-yl)methyl]-5-(benzyloxy)-6-methylpyrimidine-4-carboxylate hydrochloride (358 mg, 0.80 mmol) instead of tert-butyl 5-(benzyloxy)-6-methyl-2-[(piperidin-4-yl)methyl]pyrimidine-4-carboxylate hydrochloride, and 1-bromo-4-fluorobenzene (0.18 mL, 1.6 mmol) instead of 1-bromo-4-tert-butylbenzene, the title compound (26 mg, 0.06 mmol) was afforded as a yellow solid (yield 8%).
MS m/z: 417 (M+H)⁺;
1H-NMR (500 MHz, CDCl3) δ: 11.29 (1H, brs), 8.43 (1H, brs), 6.91 (2H, t, J=9 Hz), 6.62 (2H, dd, J=9 Hz, 4 Hz), 4.26 (2H, d, J=5 Hz), 3.52-3.45 (2H, m), 3.37-3.30 (2H, m), 2.79 (2H, d, J=7 Hz), 2.51 (3H, s), 2.18-2.10 (1H, m), 1.96-1.90(1H, m), 1.87-1.81 (1H, m), 1.76-1.68 (2H, m), 1.59-1.50 (1H, m), 1.35-1.25 (1H, m).

### Example 163

### {[(2-{[1-(4-tert-Butylphenyl)-3,3-dimethylpiperidin-4-yl]methyl}-5-hydroxy-6-methylpyrimidin-4-yl)carbonyl]amino}acetic acid

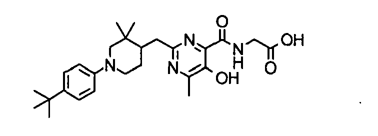

### (1) tert-Butyl 4-(2-amino-2-iminoethyl)-3,3-dimethylpiperidine-1-carboxylateacetic acid salt

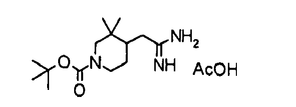

In accordance with Examples 1-(1) to 1-(3), but using tert-butyl 3,3-dimethyl-4-oxopiperidine-1-carboxylate (2.6 g, 12 mmol) instead of tert-butyl 4-oxopiperidine-1-carboxylate, the title compound (2.5 g, 7.6 mmol) was afforded as a white solid (yield 66%).
1H-NMR (500 MHz, DMSO-d6) δ: 4.01-3.90 (1H, m), 3.66-3.48 (1H, m), 2.76-2.37 (3H, m), 1.90 (1H, t, J=12 Hz), 1.69-1.62 (1H, m), 1.63 (3H, s), 1.40-1.31 (1H, m), 1.38 (9H, s), 1.29-1.20 (1H, m), 0.91 (3H, s), 0.73 (3H, s).

### (2) tert-Butyl 5-(benzyloxy)-2-{[1-(tert-butoxycarbonyl)-3,3-dimethylpiperidin-4-yl]methyl}-6-hydroxypyrimidine-4-carboxylate

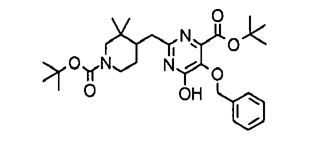

In accordance with Example 1-(4), but using tert-butyl 4-(2-amino-2-iminoethyl)-3,3-dimethylpiperidine-1-carboxylate acetic acid salt (2.5 g, 7.6 mmol) instead of tert-butyl 4-(2-amino-2-iminoethyl)piperidine-1-carboxylate acetic acid salt, the title compound (2.9 g, 5.4 mmol) was afforded as a white solid (yield 71 %).
1H-NMR (500 MHz, CDCl3) δ: 12.45 (1H, brs), 7.43 (2H, d, J=7 Hz), 7.37-7.31 (3H, m), 5.24 (2H, s), 4.19-4.12 and 4.05-3.98 (total 1H, each m), 3.80-3.73 and 3.63-3.56 (total 1H, each m), 2.89 (1H, dd, J=14 Hz, 3 Hz), 2.73-2.58 (1H, m), 2.57-2.40 (1H, m), 2.34 (1H, dd, J=14 Hz, 11 Hz), 1.89-1.80 (1H, m), 1.51 (9H, s), 1.46-1.37 (2H, m), 1.43 (9H, s), 0.95 (3H, s), 0.83 (3H, s).

### (3) tert-Butyl 5-(benzyloxy)-2-{[1-(tert-butoxycarbonyl)-3,3-dimethylpiperidin-4-yl]methyl}-6-methylpyrimidine-4-carboxylate

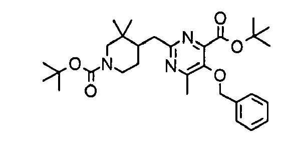

In accordance with Examples 1-(5) and 1-(6), but using tert-butyl 5-(benzyloxy)-2-{[1-(tert-butoxycarbonyl)-3,3-dimethylpiperidin-4-yl]methyl}-6-hydroxypyrimidine-4-carboxylate (2.9 g, 5.4 mmol) instead of tert-butyl 5-(benzyloxy)-2-{[1-(tert-butoxycarbonyl)piperidin-4-yl]methyl}-6-hydroxypyrimidine-4-carboxylate, the title compound (2.5 g, 4.7 mmol) was afforded as a yellow oil (yield 87%). 1H-NMR (500 MHz, CDCl3) δ: 7.43-7.34 (5H, m), 5.00 (2H, s), 4.01-3.94 and 3.83-3.77 (total 1H, each m), 3.68-3.61 (1H, m), 3.11 (1H, dd, J=14 Hz, 3 Hz), 2.71-2.48 (3H, m), 2.44 (3H, s), 1.99-1.90 (1H, m), 1.59 (9H, s), 1.45 (9H, s), 1.44-1.36 (1H, m), 1.35-1.24 (1H, m), 0.98 (3H, s), 0.90 (3H, s).

### (4) tert-Butyl 5-(benzyloxy)-2-[(3,3-dimethylpiperidin-4-yl)methyl]-6-methylpyrimidine-4-carboxylate hydrochloride

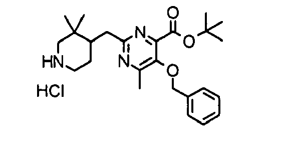

In accordance with Example 1-(7), but using tert-butyl 5-(benzyloxy)-2-{[1-(tert-butoxycarbonyl)-3,3-dimethylpiperidin-4-yl]methyl}-6-methylpyrimidine-4-carboxylate (2.9 g, 5.4 mmol) instead of tert-butyl 5-(benzyloxy)-2-{[1-(tert-butoxycarbonyl)piperidin-4-yl]methyl}-6-methylpyrimidine-4-carboxylate, the title compound (2.0 g, 4.2 mmol) was afforded as a pale yellow solid (yield 90%).
MS m/z: 426 (M+H)⁺.

### (5) {[(2-{[1-(4-tert-Butylphenyl)-3,3-dimethylpiperidin-4-yl]methyl}-5-hydroxy-6-methylpyrimidin-4-yl)carbonyl]amino}acetic acid

In accordance with Examples 1-(8), 73-(2), 1-(10) and 1-(11), but using tert-butyl 5-(benzyloxy)-2-[(3,3-dimethylpiperidin-4-yl)methyl]-6-methylpyrimidine-4-carboxylate hydrochloride (0.52 g, 1.1 mmol) instead of tert-butyl 5-(benzyloxy)-6-methyl-2-[(piperidin-4-yl)methyl]pyrimidine-4-carboxylate hydrochloride, the title compound (0.14 g, 0.30 mmol) was afforded as a pale yellow solid (yield 27%).
MS m/z: 469 (M+H)⁺;
1H-NMR (500 MHz, CDCl3) δ: 11.35 (1H, brs), 8.52 (1H, brs), 7.29-7.26 (2H, m), 6.92 (2H, d, J=8 Hz), 4.26 (2H, d, J=5 Hz), 3.57 (1H, d, J=12 Hz), 3.22 (1H, d, J=12 Hz), 3.10 (1H, d, J=14 Hz), 2.62-2.51 (3H, m), 2.53 (3H, s), 1.86-1.80 (1H, m), 1.70-1.61 (1H, m), 1.51-1.46 (1H, m), 1.28 (9H, s), 1.09 (3H, s), 1.03 (3H, s).

### Example 164

### {[(2-{[1-(Biphenyl-4-yl)-3,3-dimethylpiperidin-4-yl]methyl}-5-hydroxy-6-methylpyrimidin-4-yl)carbonyl]amino}acetic acid

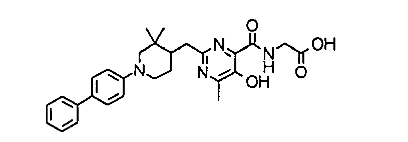

In accordance with Examples 1-(8), 73-(2), 1-(10) and 1-(11), but using tert-butyl 5-(benzyloxy)-2-[(3,3-dimethylpiperidin-4-yl)methyl]-6-methylpyrimidine-4-carboxylate hydrochloride (0.52 g, 1.1 mmol) instead of tert-butyl 5-(benzyloxy)-6-methyl-2-[(piperidin-4-yl)methyl]pyrimidine-4-carboxylate hydrochloride, and 4-bromobiphenyl (0.51 g, 2.2 mmol) instead of 1-bromo-4-tert-butylbenzene, the title compound (0.26 g, 0.52 mmol) was afforded as a pale yellow solid (yield 48%).
MS m/z: 489 (M+H)⁺;
1H-NMR (500 MHz, DMSO-d6) δ: 9.07 (1H, brs), 7.57 (2H, d, J=8 Hz), 7.49 (2H, d, J=8 Hz), 7.39 (2H, t, J=8 Hz), 7.25 (1H, t, J=8 Hz), 6.98 (2H, d, J=8 Hz), 3.64 (1H, d, J=12 Hz), 3.54 (2H, brs), 3.41 (1H, d, J=12 Hz), 3.05 (1H, d, J=14 Hz), 2.58-2.40 (3H, m), 2.42 (3H, s), 1.89-1.82 (1H, m), 1.52-1.39 (2H, m), 1.01 (3H, s), 1.00 (3H, s).

### Example 165

### { [(2-{[trans-1-(4-tert-Butylphenyl)-2-methylpiperidin-4-yl]methyl}-5-hydroxy-6-methylpyrimidin-4-yl)carbonyl]amino}acetic acid

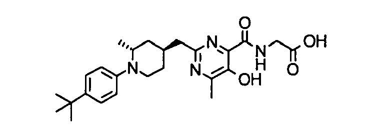

### (1) tert-Butyl 4-(2-amino-2-iminoethyl)-2-methylpiperidine-1-carboxylate acetic acid salt

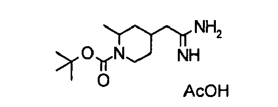

In accordance with Examples 1-(1) to 1-(3), but using tert-butyl 2-methyl-4-oxopiperidine-1-carboxylate (2.1 g, 9.6 mmol) instead of tert-butyl 4-oxopiperidine-1-carboxylate, the title compound (1.0 g, 3.2 mmol) was afforded as a grey solid (yield 33%).
1H-NMR (500 MHz, DMSO-d6) δ: 3.99-3.88 and 3.80-3.73 (total 1H, each m), 3.87-3.79 and 3.56-3.50 (total 1H, each m), 3.12-3.07 and 3.01-2.97 (total 1H, each m), 2.33-2.11 (2H, m), 1.96-1.85 (1H, m), 1.77-1.67 and 1.65-1.57 (total 2H, each m), 1.65 (3H, s), 1.40 and 1.39 (total 9H, each s), 1.20-1.11 (2H, m), 1.14 and 1.08 (total 3H, each d, J=7 Hz).

### (2) tert-Butyl 5-(benzyloxy)-2-{[1-(tert-butoxycarbonyl)-2-methylpiperidin-4-yl]methyl}-6-hydroxypyrimidine-4-carboxylate

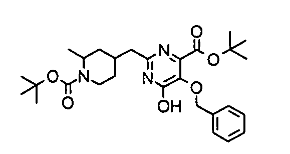

In accordance with Example 1-(4), but using tert-butyl 4-(2-amino-2-iminoethyl)-2-methylpiperidine-1-carboxylate acetic acid salt (1.0 g, 3.2 mmol) instead of tert-butyl 4-(2-amino-2-iminoethyl)piperidine-1-carboxylate acetic acid salt, the title compound (1.1 g, 2.0 mmol) was afforded as a white solid (yield 64%).
1H-NMR (500 MHz, CDCl3) δ: 12.65 (1H, brs), 7.47 (2H, d, J=7 Hz), 7.39-7.31 (3H, m), 5.23 (2H, s), 3.90-3.83 (1H, m), 3.72-3.67 (1H, ddd, J=14 Hz, 7 Hz, 3 Hz), 3.08-3.02 (1H, m), 2.71-2.54 (2H, m), 2.21-2.14 (1H, m), 1.92-1.84 (1H, m), 1.79-1.74 (1H, m), 1.53 (9H, s), 1.43 (9H, s), 1.31-1.19 (2H, m), 1.14 and 1.09 (total 3H, each d, J=7 Hz).

### (3) tert-Butyl 5-(benzyloxy)-2-{[1-(tert-butoxycarbonyl)-2-methylpiperidin-4-yl]methyl}-6-methylpyrimidine-4-carboxylate

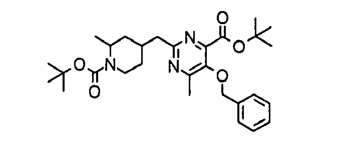

In accordance with Examples 1-(5) and 1-(6), but using tert-butyl 5-(benzyloxy)-2-{[1-(tert-butoxycarbonyl)-2-methylpiperidin-4-yl]methyl}-6-hydroxypyrimidine-4-carboxylate (1.1 g, 2.0 mmol) instead of tert-butyl 5-(benzyloxy)-2-{[1-(tert-butoxycarbonyl)piperidin-4-yl]methyl}-6-hydroxypyrimidine-4-carboxylate, the title compound (1.0 g, 2.0 mmol) was afforded as a colorless oil (yield 97%). 1H-NMR (500 MHz, CDCl3) δ: 7.43-7.34 (5H, m), 5.00 (2H, s), 3.93-3.86 (1H, m), 3.72 (1H, ddd, J=14 Hz, 7 Hz, 3 Hz), 3.10 (1H, ddd, J=16 Hz, 10 Hz, 6 Hz), 2.95-2.79 (2H, m), 2.45 (3H, s), 2.27-2.18 (1H, m), 1.87-1.81 (1H, m), 1.80-1.73 (1H, m), 1.59 (9H, s), 1.45 (9H, s), 1.33-1.22 (2H, m), 1.19 and 1.13 (total 3H, each d, J=7 Hz).

### (4) tert-Butyl 5-(benzyloxy)-2-{[trans-1-(4-tert-butylphenyl)-2-methylpiperidin-4-yl]methyl}-6-methylpyrimidine-4-carboxylate and tert-butyl 5-(benzyloxy)-2-{[cis-1-(4-tert-butylphenyl)-2-methylpiperidin-4-yl]methyl}-6-methylpyrimidine-4-carboxylate

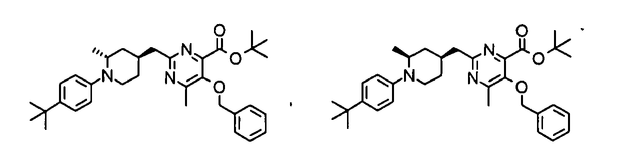

In accordance with Example 1-(7), but using tert-butyl 5-(benzyloxy)-2-{[1-(tert-butoxycarbonyl)-2-methylpiperidin-4-yl]methyl}-6-methylpyrimidine-4-carboxylate (1.0 g, 2.0 mmol) instead of tert-butyl 5-(benzyloxy)-2-{[1-(tert-butoxycarbonyl)piperidin-4-yl]methyl}-6-methylpyrimidine-4-carboxylate, tert-butyl 5-(benzyloxy)-6-methyl-2-[(2-methylpiperidin-4-yl)methyl]pyrimidine-4-carboxylate hydrochloride (0.88 g, 1.9 mmol) was afforded as a white solid (yield 96%).

In accordance with Example 1-(8), but using tert-butyl 5-(benzyloxy)-6-methyl-2-[(2-methylpiperidin-4-yl)methyl]pyrimidine-4-carboxylate hydrochloride (0.40 g, 0.9 mmol) instead of tert-butyl 5-(benzyloxy)-6-methyl-2-[(piperidin-4-yl)methyl]pyrimidine-4-carboxylate hydrochloride, the title compounds (trans-form: 0.032 g, 0.058 mmol, yield 6.4%, cis-form: 0.076 g, 0.14 mmol, yield 16%) were each afforded as an oil.
trans-form:
Rf value=0.47 (hexane/ethyl acetate=2/1);
MS m/z: 544 (M+H)⁺.
cis-form:
Rf value=0.30 (hexane/ethyl acetate=2/1);
MS m/z: 544 (M+H)⁺.

### (5) {[(2-{[trans-1-(4-tert-Butylphenyl)-2-methylpiperidin-4-yl]methyl}-5-hydroxy-6-methylpyrimidin-4-yl)carbonyl]amino}acetic acid

In accordance with Examples 73-(2), 1-(10) and 1-(11), but using tert-butyl 5-(benzyloxy)-2- {[trans-1-(4-tert-butylphenyl)-2-methylpiperidin-4-yl]methyl}-6-methylpyrimidine-4-carboxylate (32 mg, 0.058 mmol) instead of tert-butyl 2-{[1-(1-benzothiophen-5-yl)piperidin-4-yl]methyl}-5-(benzyloxy)-6-methylpyrimidine-4-carboxylate, the title compound (6.4 mg, 0.014 mmol) was afforded as a pale yellow solid (yield 24%).
MS m/z: 455 (M+H)⁺;
1H-NMR (500 MHz, CDCl3) δ: 11.75 (1H, brs), 8.64 (1H, brs), 7.38 (2H, d, J=9 Hz), 7.33-7.26 (2H, m), 4.24-4.14 (2H, m), 3.96 (1H, brs), 3:42-3.31 (2H, m), 2.97-2.89 (2H, m), 2.52 (3H, s), 2.45 (1H, brs), 2.19 (1H, brs), 2.02 (2H, brs), 1.85-1.60 (1H, m), 1.29 (9H, s), 1.02 (3H, d, J=6 Hz).

### Example 166

### {[(2-{[cis-1-(4-tert-Butylphenyl)-2-methylpiperidin-4-yl]methyl}-5-hydroxy-6-methylpyrimidin-4-yl)carbonyl]amino}acetic acid

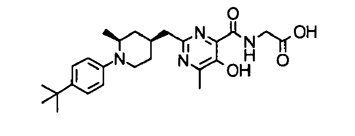

In accordance with Examples 73-(2), 1-(10) and 1-(11), but using tert-butyl 5-(benzyloxy)-2- {[cis-1-(4-tert-butylphenyl)-2-methylpiperidin-4-yl]methyl}-6-methylpyrimidine-4-carboxylate (76 mg, 0.14 mmol) instead of tert-butyl 2-{[1-(1-benzothiophen-5-yl)piperidin-4-yl]methyl}-5-(benzyloxy)-6-methylpyrimidine-4-carboxylate, the title compound (16 mg, 0.036 mmol) was afforded as a pale yellow solid (yield 26%).
MS m/z: 455 (M+H)⁺;
1H-NMR (500 MHz, CDCl3) δ: 11.92 (1H, brs), 8.79 (1H, d, J=5 Hz), 7.55 (2H, brs), 7.45 (2H, d, J=9 Hz), 4.22 (1H, dd, J=18 Hz, 5 Hz), 4.16 (1H, dd, J=18 Hz, 5 Hz), 3.67 (1H, d, J=12 Hz), 3.42 (1H, brs), 3.16 (1H, t, J=12 Hz), 2.96-2.88 (2H, m), 2.52 (3H, s), 2.29 (2H, brs), 2.22-2.14 (1H, m), 1.96 (1H, d, J=14 Hz), 1.90 (1H, d, J=9 Hz), 1.30 (9H, s), 1.17 (3H, d, J=6 Hz).

### Example 167

### {[(2-{[1-(4-tert-Butylphenyl)-1,2,3,6-tetrahydropyridin-4-yl]methyl}-5-hydroxy-6-methylpyrimidin-4-yl)carbonyl]amino}acetic acid

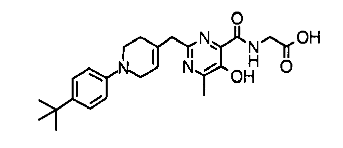

### (1) 1-(4-tert-Butylphenyl)piperidin-4-one

In accordance with Example 2-(1), but using 1-bromo-4-tert-butylbenzene instead of bromobenzene, the title compound (18 g, 78 mmol) was afforded as a solid (quantitative yield).
1H-NMR (500 MHz, CDCl3) δ: 7.32 (2H, d, J=9 Hz), 6.93 (2H, d, J=9 Hz), 3.57 (4H, t, J=6 Hz), 2.55 (4H, t, J=6 Hz), 1.30 (9H, s).

### (2) [1-(4-tert-Butylphenyl)-1,2,3,6-tetrahydropyridin-4-yl]acetonitrile

1-(4-tert-Butylphenyl)piperidin-4-one (5.0 g, 22 mmol) was dissolved in toluene (150 mL), and cyanoacetic acid (4.0 g, 48 mmol) and ammonium acetate (0.83 g, 11 mmol) were added, followed by heating to reflux for 12 hours. Cyanoacetic acid (1.0 g, 12 mmol) and ammonium acetate (0.83 g, 11 mmol) were added to the reaction solution, followed by heating to reflux for further 6 hours. After the reaction solution was cooled to room temperature, the solvent was distilled off under reduced pressure to afford the title compound (3.2 g, 13 mmol).
MS m/z: 255 (M+H)⁺.

### (3) 2-[1-(4-tert-Butylphenyl)-1,2,3,6-tetrahydropyridin-4-yl]ethanimidamide hydrochloride

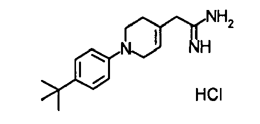

[1-(4-tert-Butylphenyl)-1,2,3,6-tetrahydropyridin-4-yl]acetonitrile (3.2 g, 13 mmol) was dissolved in ethanol, and stirred for 1 hour while bubbling hydrogen chloride at 0°C, followed by stirring at the same temperature for 3 hours. The reaction solution was concentrated under reduced pressure to afford a white solid. This was dissolved in ethanol (50 mL), and a solution of ammonia in methanol (7.0 M, 20 mL) was added, followed by stirring at room temperature overnight. The reaction solution was concentrated under reduced pressure to afford the title compound (3.7 g, 12 mmol) as a white solid (yield 92%).
MS m/z: 272 (M+H)⁺.

### (4) tert-Butyl 5-(benzyloxy)-2-{[1-(4-tert-butylphenyl)-1,2,3,6-tetrahydropyridin-4-yl]methyl}-6-hydroxypyrimidine-4-carboxylate

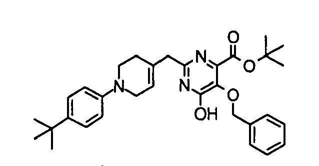

In accordance with Example 1-(4), but using [1-(4-tert-butylphenyl)-1,2,3,6-tetrahydropyridin-4-yl]ethanimidamide hydrochloride (3.7 g, 12 mmol) instead of tert-butyl 4-(2-amino-2-iminoethyl)piperidine-1-carboxylate acetic acid salt, the title compound (5.3 g, 10 mmol) was afforded as a pale brown solid (yield 79%).
MS m/z: 530 (M+H)⁺.

### (5) {[(2-{[1-(4-tert-Butylphenyl)-1,2,3,6-tetrahydropyridin-4-yl]methyl}-5-hydroxy-6-methylpyrimidin-4-yl)carbonyl]amino} acetic acid

In accordance with Examples 1-(5), 1-(6) and 1-(9) to 1-(11), but using tert-butyl 5-(benzyloxy)-2-{[1-(4-tert-butylphenyl)-1,2,3,6-tetrahydropyridin-4-yl]methyl}-6-hydroxypyrimidine-4-carboxylate (5.3 g, 10 mmol) instead of tert-butyl 5-(benzyloxy)-2-{[1-(tert-butoxycarbonyl)piperidin-4-yl]methyl}-6-hydroxypyrimidine-4-carboxylate, the title compound (0.53 g, 1.2 mmol) was afforded as a yellow solid (yield 12%).
MS m/z: 439 (M+H)⁺;
1H-NMR (500 MHz, DMSO-d6) δ: 11.97 (1H, brs), 9.37 (1H, brs), 7.20 (2H, d, J=8 Hz), 6.83 (2H, d, J=8 Hz), 5.45 (1H, s), 4.00 (2H, d, J=6 Hz), 3.56 (2H, s), 3.58-3.25 (4H, m), 2.44 (3H, s), 2.21 (2H, brs), 1.23 (9H, s).

### Example 168

### {[(2-{[4-(4-tert-Butylphenyl)-2-oxopyridin-1(2H)-yl]methyl}-5-hydroxy-6-methylpyrimidin-4-yl)carbonyl]amino}acetic acid

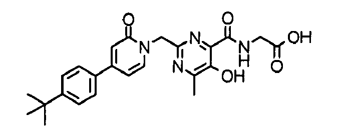

### (1) [4-(4-tert-Butylphenyl)-2-oxopyridin-1(2H)-yl]acetonitrile

2-(Benzyloxy)-4-(tert-butylphenyl)pyridine (1.6 g, 5.0 mmol) was dissolved in acetonitrile (20 mL), and bromoacetonitrile (2.1 mL, 30 mmol) was added, followed by heating to reflux for 13 hours under a nitrogen atmosphere. The reaction solution was cooled to room temperature, followed by concentration under reduced pressure. The resulting residue was purified by silica gel column chromatography (Moritex Corporation, elution solvent: hexane/ethyl acetate), and a fraction corresponding to the Rf value=0.04 (hexane/ethyl acetate=2/1) by thin layer chromatography was concentrated under reduced pressure to afford the title compound (0.64 g, 2.4 mmol) as a white solid (yield 48%).
1H-NMR (500 MHz, CDCl3) δ: 7.53 (2H, d, J=9 Hz), 7.50 (2H, d, J=9 Hz), 7.41 (1H, d, J=7 Hz), 6.84 (1H, d, J=2 Hz), 6.58 (1H, dd, J=7 Hz, 2 Hz), 4.90 (2H, s), 1.36 (9H, s).

### (2) N'-Acetoxy-2-[4-(4-tert-butylphenyl)-2-oxopyridin-1(2H)-yl]ethanimidamide

[4-(4-tert-Butylphenyl)-2-oxopyridin-1(2H)-yl]acetonitrile (1.0 g, 3.8 mmol) was dissolved in ethanol (10 mL), and aqueous hydroxylamine solution (50%, 0.28 mL, 4.5 mmol) was added, followed by heating to reflux for 1 hour. The reaction solution was cooled, and subsequently concentrated under reduced pressure to afford 2-[4-(4-tert-butylphenyl)-2-oxopyridin-1 (2H)-yl]-N'-(hydroxy)ethanimidamide as a solid. This was dissolved in 1,4-dioxane (15 mL), and acetic anhydride (0.42 mL, 4.5 mmol) and triethylamine (0.63 mL, 4.5 mmol) were added, followed by stirring at room temperature for 3 hours. After water was added to the reaction solution, the precipitated solid was collected by filtration to afford the title compound (1.3 g, 3.8 mmol) as a white solid (yield 99%).
1H-NMR (500 MHz, CDCl3) δ: 7.53 (2H, d, J=9 Hz), 7.50-7.47 (3H, m), 6.83 (1H, d, J=2 Hz), 6.57 (1H, dd, J=7 Hz, 2 Hz), 4.71 (2H, s), 2.16 (3H, s), 1.35 (9H, s).

### (3) 2-[4-(4-tert-Butylphenyl)-2-oxopyridin-1(2H)-yl]ethanimidamide acetic acid salt

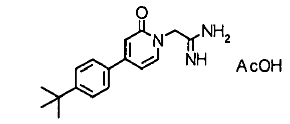

N'-Acetoxy-2-[4-(4-tert-butylphenyl)-2-oxopyridin-1 (2H)-yl]ethanimidamide (0.61 g, 1.8 mmol) was dissolved in ethanol (10 mL) and methylene chloride (4 mL), and 10% palladium-activated carbon (0.10 g) was added, followed by stirring at room temperature for 4 hours under a hydrogen atmosphere. 10% Palladium-activated carbon (0.05 g) was added to the reaction solution, followed by further stirring at room temperature for 3 hours under a hydrogen atmosphere. After the reaction solution was filtered with celite, the filtrate was concentrated under reduced pressure to afford the title compound (0.68 g) as a pale yellow solid (quantitative yield).
1H-NMR (500 MHz, DMSO-d6/CDCl3=1/10) δ: 7.72 (1H, d, J=7 Hz), 7.54 (2H, d, J=8 Hz), 7.50 (2H, d, J=8 Hz), 6.80 (1H, d, J=2 Hz), 6.60 (1H, dd, J=7 Hz, 2 Hz), 4.92 (2H, s), 1.94 (3H, s), 1.36 (9H, s).

### (4) tert-Butyl 5-(benzyloxy)-2-{[4-(4-tert-butylphenyl)-2-oxopyridin-1(2H)-yl]methyl}-6-hydroxypyrimidine-4-carboxylate

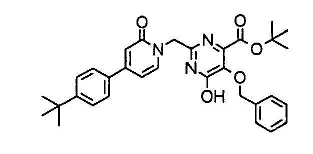

In accordance with Example 1-(4), but using 2-[4-(4-tert-butylphenyl)-2-oxopyridin-1(2H)-yl]ethanimidamide acetic acid salt (0.75 g, 2.1 mmol) instead of tert-butyl 4-(2-amino-2-iminoethyl)piperidine-1-carboxylate acetic acid salt, the title compound (0.49 g, 0.91 mmol) was afforded as a pale yellow solid (yield 43%).
1H-NMR (500 MHz, CDCl3) δ: 11.69 (1H, brs), 7.54-7.49 (5H, m), 7.44 (2H, d, J=7 Hz), 7.36-7.30 (3H, m), 6.91 (1H, d, J=2 Hz), 6.61 (1H, dd, J=7 Hz, 2 Hz), 5.32 (2H, s), 5.02 (2H, s), 1.50 (9H, s), 1.36 (9H, s).

### (5) Ethyl ({[5-(benzyloxy)-2-{[4-(4-tert-butylphenyl)-2-oxopyridin-1(2H)-yl]methyl}-6-hydroxypyrimidin-4-yl]carbonyl}amino)acetate

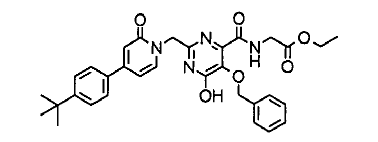

tert-Butyl 5-(benzyloxy)-2- {[4-(4-tert-butylphenyl)-2-oxopyridin-1 (2H)-yl]methyl}-6-hydroxypyrimidine-4-carboxylate (0.49 g, 0.91 mmol) was dissolved in a mixture of methanol (4.0 mL) and tetrahydrofuran (4.0 mL), and aqueous potassium hydroxide solution (2 M, 2.0 mL) was added, followed by stirring at 60°C for 1 hour. Aqueous potassium hydroxide solution (2 M, 4.0 mL) was added to the reaction solution, followed by further stirring at 60°C for 6.5 hours. After the reaction solution was cooled to room temperature, hydrochloric acid (1.0 M) was added, and the precipitated solid was collected by filtration to afford 5-(benzyloxy)-2-{[4-(4-tert-butylphenyl)-2-oxopyridin-1(2H)-yl]methyl}-6-hydroxypyrimidine-4-carboxylic acid as a white solid. This was dissolved in N,N-dimethylformamide (5.0 mL), and 1,1'-carbonylbis(1H-imidazole) (0.18 g, 1.1 mmol) was added, followed by stirring at room temperature for 20 minutes under a nitrogen atmosphere. Ethyl glycine hydrochloride (0.15 g, 1.1 mmol) and diisopropylethylamine (0.38 mL, 2.2 mmol) were added to the reaction solution, followed by stirring at room temperature for 2.5 hours. Water and hydrochloric acid (1.0 M) were added to the reaction solution, followed by extraction with ethyl acetate. After the extract was washed with saturated aqueous sodium chloride solution, the organic layer was concentrated under reduced pressure to afford the title compound (0.53 g) as a pale yellow solid (quantitative yield).
1H-NMR (500 MHz, CDCl3) δ: 8.02 (1H, brs), 7.54-7.45 (7H, m), 7.37-7.30 (3H, m), 6.92 (1H, d, J=2 Hz), 6.61 (1H, dd, J=7 Hz, 2 Hz), 5.46 (2H, s), 5.05 (2H, s), 4.24 (2H, q, J=7 Hz), 4.13 (2H, d, J=5 Hz), 1.36 (9H, s), 1.30 (3H, t, J=7 Hz).

### (6) Ethyl ({[5-(benzyloxy)-2-{[4-(4-tert-butylphenyl)-2-oxopyridin-1(2H)-yl]methyl}-6-methylpyrimidin-4-yl]carbonyl}amino)acetate

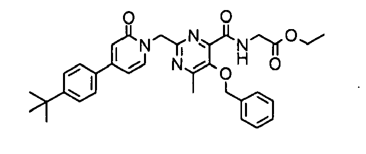

In accordance with Examples 1-(5) and (6), but using ethyl ({[5-(benzyloxy)-2-{[4-(4-tert-butylphenyl)-2-oxopyridin-1 (2H)-yl]methyl} -6-hydroxypyrimidin-4-yl]carbonyl}amino)acetate (0.53 g, 0.91 mmol) instead of tert-butyl 5-(benzyloxy)-2-{[1-(tert-butoxycarbonyl)piperidin-4-yl]methyl}-6-hydroxypyrimidine-4-carboxylate, the title compound (93 mg, 0.16 mmol) was afforded as a pale yellow solid (yield 18%).
1H-NMR (500 MHz, CDCl3) δ: 8.15 (1H, t, J=5 Hz), 7.60 (2H, d, J=8 Hz), 7.50-7.46 (5H, m), 7.40-7.34 (3H, m), 6.87 (1H, d, J=2 Hz), 6.57 (1H, dd, J=7 Hz, 2 Hz), 5.35 (2H, s), 5.09 (2H, s), 4.16 (2H, q, J=7 Hz), 4.14 (2H, d, J=5 Hz), 2.44 (3H, s), 1.36 (9H, s), 1.24 (3H, t, J=7 Hz).

### (7) Ethyl {[(2-{[4-(4-tert-butylphenyl)-2-oxopyridin-1(2H)-yl]methyl}-5-hydroxy-6-methylpyrimidin-4-yl)carbonyl]amino}acetate

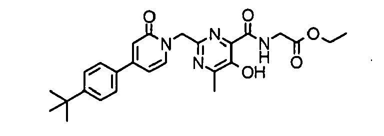

In accordance with Example 1-(10), but using ethyl ({[5-(benzyloxy)-2-{[4-(4-tert-butylphenyl)-2-oxopyridin-1 (2H)-yl]methyl} -6-methylpyrimidin-4-yl]carbonyl}amino)acetate (93 mg, 0.16 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (85 mg) was afforded as a yellow oil (quantitative yield).
1H-NMR (500 MHz, CDCl3) δ: 11.49 (1H, s), 8.25 (1H, brs), 7.59 (2H, d, J=8 Hz), 7.48 (2H, d, J=8 Hz), 7.46 (1H, d, J=7 Hz), 6.85 (1H, d, J=2 Hz), 6.54 (1H, dd, J=7 Hz, 2 Hz), 5.29 (2H, s), 4.20 (2H, q, J=7 Hz), 4.12 (2H, d, J=5 Hz), 2.52 (3H, s), 1.36 (9H, s), 1.26 (3H, t, J=7 Hz).

### (8) {[(2-{[4-(4-tert-Butylphenyl)-2-oxopyridin-1(2H)-yl]methyl}-5-hydroxy-6-methylpyrimidin-4-yl)carbonyl]amino}acetic acid

In accordance with Example 1-(11), but using ethyl {[(2-{[4-(4-tert-butylphenyl)-2-oxopyridin-1(2H)-yl]methyl}-5-hydroxy-6-methylpyrimidin-4-yl)carbonyl]amino}acetate (85 mg, 0.16 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (64 mg, 0.14 mmol) was afforded as a pale yellow solid (yield 87%).
MS m/z: 451 (M+H)⁺;
1H-NMR (500 MHz, CDCl3) δ: 11.75 (1H, s), 8.43 (1H, t, J=6 Hz), 7.59 (1H, d, J=7 Hz), 7.56 (2H, d, J=8 Hz), 7.48 (2H, d, J=8 Hz), 6.91 (1H, d, J=2 Hz), 6.67 (1H, dd, J=7 Hz, 2 Hz), 5.33 (2H, s), 4.16 (2H, d, J=6 Hz), 2.51 (3H, s), 1.35 (9H, s).

### Example 169

### {[(2-{[4-(4-tert-Butylphenyl)-2-oxopiperidin-1-yl]methyl}-5-hydroxy-6-methylpyrimidin-4-yl)carbonyl]amino}acetic acid

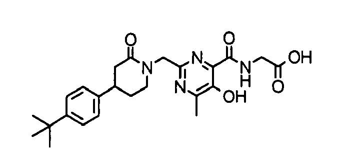

### (1) 2-[4-(4-tert-Butylphenyl)-2-oxopiperidin-1-yl]ethanimidamide acetic acid salt

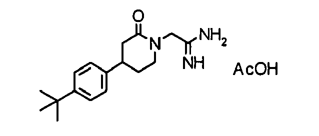

N'-Acetoxy-2-[4-(4-tert-butylphenyl)-2-oxopyridin-1(2H)-yl]ethanimidamide (0.67 g, 2.0 mmol) obtained in Example 168-(2) was dissolved in acetic acid (10 mL), and 20% palladium hydroxide-activated carbon (0.10 g) was added, followed by stirring at room temperature for 4 hours under a hydrogen atmosphere. After the reaction solution was filtered with celite, the filtrate was concentrated under reduced pressure to afford the title compound (0.75 g) as a grey solid (quantitative yield).
1H-NMR (500 MHz, DMSO-d6/CDCl3=1/10) δ: 7.37 (2H, d, J=9 Hz), 7.14 (2H, d, J=9 Hz), 4.36 (1H, d, J=16 Hz), 4.22 (1H, d, J=16 Hz), 3.50-3.44 (1H, m), 3.41-3.36 (1H, m), 3.18-3.11 (1H, m), 2.74 (1H, dd, J=18 Hz, 5 Hz), 2.61-2.55 (1H, m), 2.16-2.04 (2H, m), 1.98 (3H, s), 1.32 (9H, s).

### (2) tert-Butyl 5-(benzyloxy)-2-{[4-(4-tert-butylphenyl)-2-oxopiperidin-1-yl]methyl}-6-hydroxypyrimidine-4-carboxylate

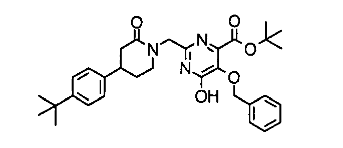

In accordance with Example 1-(4), but using 2-[4-(4-tert-butylphenyl)-2-oxopiperidin-1-yl]ethanimidamide acetic acid salt (0.75 g, 2.1 mmol) instead of tert-butyl 4-(2-amino-2-iminoethyl)piperidine-1-carboxylate acetic acid salt, the title compound (0.51 g, 0.93 mmol) was afforded as a brown solid (yield 44%).
1H-NMR (500 MHz, CDCl3) δ: 10.96 (1H, brs), 7.46 (2H, d, J=8 Hz), 7.38-7.30 (5H, m), 7.13 (2H, d, J=8 Hz), 5.32 (2H, s), 4.55 (1H, d, J=14 Hz), 4.31 (1H, d, J=14 Hz), 3.56-3.53 (2H, m), 3.13-3.07 (1H, m), 2.81 (1H, dd, J=18 Hz, 5 Hz), 2.59 (1H, dd, J=18 Hz, 11 Hz), 2.16-2.12 (1H, m), 2.02-1.95 (1H, m), 1.50 (9H, s), 1.32 (9H, s).

### (3) Ethyl ({[5-(benzyloxy)-2-{[4-(4-tert-butylphenyl)-2-oxopiperidin-1-yl]methyl}-6-hydroxypyrimidin-4-yl]carbonyl}amino)acetate

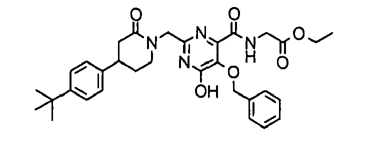

In accordance with Example 168-(5), but using tert-butyl 5-(benzyloxy)-2-{[4-(4-tert-butylphenyl)-2-oxopiperidin-1-yl]methyl}-6-hydroxypyrimidine-4-carboxylate (0.51 g, 0.93 mmol) instead of tert-butyl 5-(benzyloxy)-2-{[4-(4-tert-butylphenyl)-2-oxopyridin-1(2H)-yl]methyl}-6-hydroxypyrimidine-4-carboxylate, the title compound (0.54 g) was afforded as a pale yellow solid (quantitative yield).
1H-NMR (500 MHz, CDCl3) δ: 11.04 (1H, brs), 8.00 (1H, t, J=5 Hz), 7.52 (2H, d, J=8 Hz), 7.38-7.32 (5H, m), 7.13 (2H, d, J=8 Hz), 5.44 (2H, s), 4.57 (1H, d, J=14 Hz), 4.34 (1H, d, J=14 Hz), 4.24 (2H, q, J=7 Hz), 4.13 (2H, d, J=5 Hz), 3.58-3.49 (2H, m), 3.14-3.07 (1H, m), 2.82 (1H, dd, J=18 Hz, 5 Hz), 2.60 (1H, dd, J=18 Hz, 11 Hz), 2.18-2.12 (1H, m), 2.03-1.95 (1H, m), 1.31 (9H, s), 1.30 (3H, t, J=7 Hz).

### (4) Ethyl ({[5-(benzyloxy)-2-{[4-(4-tert-butylphenyl)-2-oxopiperidin-1-yl]methyl}-6-methylpyrimidin-4-yl]carbonyl}amino)acetate

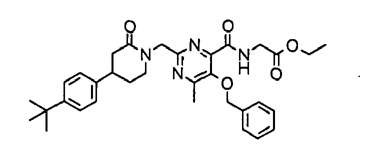

In accordance with Examples 1-(5) and 1-(6), but using ethyl ({[5-(benzyloxy)-2-{[4-(4-tert-butylphenyl)-2-oxopiperidin-1-yl]methyl}-6-hydroxypyrimidin-4-yl]carbonyl}amino)acetate (0.54 g) instead oftert-butyl 5-(benzyloxy)-2-{[1-(tert-butoxycarbonyl)piperidin-4-yl]methyl}-6-hydroxypyrimidine-4-carboxylate, the title compound (0.21 g, 0.36 mmol) was afforded as a pale yellow solid (yield 38%).
1H-NMR (500 MHz, CDCl3) δ: 8.40 (1H, t, J=5 Hz), 7.51 (2H, d, J=7 Hz), 7.41-7.34 (5H, m), 7.26-7.24 (2H, m), 5.11 (2H, s), 4.86 (1H, d, J=17 Hz), 4.81 (1H, d, J=17 Hz), 4.28 (2H, q, J=7 Hz), 4.23 (2H, d, J=5 Hz), 3.68-3.62 (1H, m), 3.51-3.47 (1H, m), 3.29-3.22 (1H, m), 2.86 (1H, dd, J=18 Hz, 5 Hz), 2.69 (1H, dd, J=18 Hz, 11 Hz), 2.46 (3H, s), 2.19-2.14 (2H, m), 1.33 (9H, s), 1.32 (3H, t, J=7 Hz).

### (5) Ethyl {[(2-{[4-(4-tert-butylphenyl)-2-oxopiperidin-1-yl]methyl}-5-hydroxy-6-methylpyrimidin-4-yl)carbonyl]amino}acetate

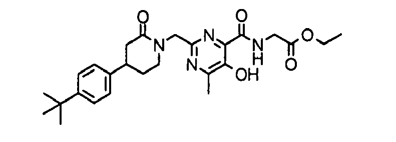

In accordance with Example 1-(10), but using ethyl ({[5-(benzyloxy)-2-{[4-(4-tert-butylphenyl)-2-oxopiperidin-1-yl]methyl}-6-methylpyrimidin-4-yl]carbonyl}amino)acetate(0.21 g, 0.36 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.17 g) was afforded as a pale yellow oil (quantitative yield).
1H-NMR (500 MHz, CDCl3) δ: 11.41 (1H, s), 8.51 (1H, t, J=5 Hz), 7.36 (2H, d, J=8 Hz), 7.24 (2H, d, J=8 Hz), 4.81 (1H, d, J=16 Hz), 4.74 (1H, d, J=16 Hz), 4.30 (2H, q, J=7 Hz), 4.20 (2H, d, J=5 Hz), 3.61 (1H, dt, J=11 Hz, 5 Hz), 3.48-3.44 (1H, m), 3.27-3.20 (1H, m), 2.85 (1H, dd, J=18 Hz, 5 Hz), 2.66 (1H, dd, J=18 Hz, 11 Hz), 2.54 (3H, s), 2.19-2.12 (2H, m), 1.33 (3H, t, J=7 Hz), 1.32 (9H, s).

### (6) {[(2-{[4-(4-tert-Butylphenyl)-2-oxopiperidin-1-yl]methyl}-5-hydroxy-6-methylpyrimidin-4-yl)carbonyl]amino}acetic acid

In accordance with Example 1-(11), but using ethyl {[(2-{[4-(4-tert-butylphenyl)-2-oxopiperidin-1-yl]methyl -5-hydroxy-6-methylpyrimidin-4-yl)carbonyl]amino}acetate (0.17 g) instead of ethyl {[(2-{[1-(4-tert-butylphenyl)piperidin-4-yl]methyl}-5-hydroxy-6-methylpyrimidin-4-yl)carbonyl]amino}acetate, the title compound (0.13 g, 0.29 mmol) was afforded as a white solid (yield 82%).
MS m/z: 455 (M+H)⁺;
1H-NMR (500 MHz, CDCl3) δ: 11.61 (1H, s), 8.49 (1H, t, J=5 Hz), 7.33 (2H, d, J=8 Hz), 7.16 (2H, d, J=8 Hz), 4.82 (1H, d, J=16 Hz), 4.71 (1H, d, J=16 Hz), 4.16 (2H, d, J=5 Hz), 3.58 (1H, dt, J=11 Hz, 4 Hz), 3.52-3.47 (1H, m), 3.20-3.13 (1H, m), 2.88 (1H, dd, J=18 Hz, 5 Hz), 2.64 (1H, dd, J=18 Hz, 11 Hz), 2.53 (3H, s), 2.17-2.12 (1H, m), 2.09-2.01 (1H, m), 1.30 (9H, s).

### Example 170

### {[(2-{[1-(4-tert-Butylphenyl)-2-oxopiperidin-4-yl]methyl}-5-hydroxy-6-methylpyrimidin-4-yl)carbonyl]amino}acetic acid

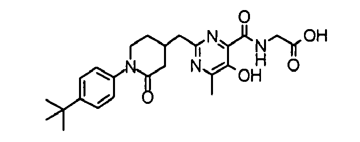

### (1) Methyl 1-(4-tert-butylphenyl)-2,4-dioxopiperidine-3-carboxylate

Ethyl 3-[(4-tert-butylphenyl)amino]-3-oxopropanoate (7.1 g, 28 mmol) was dissolved in methylene chloride (60 mL), and pyridine (3.4 mL, 43 mmol), 4-dimethylaminopyridine (0.020 g, 0.16 mmol) and a solution of ethyl 3-chloro-3-oxopropanoate (4.4 mL, 34 mmol) in methylene chloride (10 mL) were added at 0°C, followed by stirring at room temperature for 8 hours. After the reaction solution was diluted with ethyl acetate, it was washed with water, and the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.40 (hexane/ethyl acetate=3/1) by thin layer chromatography was concentrated under reduced pressure to afford ethyl N-(4-tert-butylphenyl)-N-(3-ethoxy-3-oxopropanoyl)-β-alaninate as a yellow oil.

This was dissolved in toluene (120 mL), and a solution of sodium methoxide in methanol (28%, 7.0mL, 34 mmol) was added, followed by stirring at 90°C for 15 hours. A solution of sodium methoxide in methanol (28%, 3.0 mL, 14 mmol) was added to the reaction solution, followed by stirring for further 10 hours. After the reaction solution was cooled to room temperature, hydrochloric acid (1.0 M, 100 mL) was added, followed by extraction with ethyl acetate, then with isopropyl alcohol/chloroform (1/3), and the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.60(ethyl acetate) by thin layer chromatography was concentrated under reduced pressure to afford the title compound (5.5 g, 18 mmol) as a yellow oil (yield 64%).
MS m/z: 304 (M+H)⁺.

### (2) [1-(4-tert-Butylphenyl)-2-oxopiperidin-4-yl]acetonitrile

Methyl 1-(4-tert-butylphenyl)-2,4-dioxopiperidine-3-carboxylate (5.5 g, 18 mmol) was dissolved in a mixture of acetonitrile (200 mL) and water (3 mL), followed by heating to reflux for 8 hours. After the reaction solution was cooled to room temperature, it was concentrated under reduced pressure to afford 1-(4-tert-butylphenyl)piperidine-2,4-dione as an orange solid.

This was dissolved in toluene (20 mL), and cyanoacetic acid (1.7 g, 20 mmol), ammonium acetate (0.35 g, 4.5 mmol) and acetic acid (0.54 g, 9.1 mmol) were added, followed by heating to reflux for 12 hours. After the reaction solution was cooled to room temperature, water was added, followed by extraction with ethyl acetate. The extract was washed with water and saturated aqueous sodium chloride solution, and the organic layer was dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.40 (hexane/ethyl acetate=1/2) by thin layer chromatography was concentrated under reduced pressure to afford [1-(4-tert-butylphenyl)-2-oxopiperidin-4-ylidene]acetonitrile (2.0 g, 7.5 mmol).

In accordance with Example 1-(1), but using [1-(4-tert-butylphenyl)-2-oxopiperidin-4-ylidene]acetonitrile (2.0 g, 7.5 mmol) instead of tert-butyl 4-(cyanomethylene)piperidine-1-carboxylate, the title compound (1.0 g, 3.7 mmol) was afforded as a pale yellow oil (yield 21 %).
MS m/z: 271 (M+H)⁺.

### (3) tert-Butyl 5-(benzyloxy)-2-{[1-(4-tert-butylphenyl)-2-oxopiperidin-4-yl]methyl}-6-hydroxypyrimidine-4-carboxylate

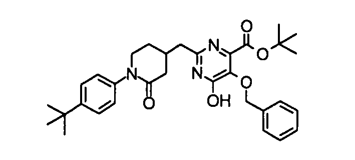

In accordance with Examples 1-(3) and 1-(4), but using [1-(4-tert-butylphenyl)-2-oxopiperidin-4-yl]acetonitrile (1.0 g, 3.7 mmol) instead of tert-butyl 4-(cyanomethyl)piperidine-1-carboxylate, the title compound (0.64 g, 1.2 mmol) was afforded as a pale brown solid (yield 32%).
1H-NMR (500 MHz, CDCl3) δ: 7.48 (2H, d, J=8 Hz), 7.38-7.33 (5H, m), 7.12 (2H, d, J=8 Hz), 5.27-5.22 (2H, m), 3.70-3.55 (2H, m), 2.80-2.60 (4H, m), 2.34-2.29 (1H, m), 1.76-1.55 (2H, m), 1.53 (9H, s), 1.30 (9H, s).

### (4) {[(2-{[1-(4-tert-Butylphenyl)-2-oxopiperidin-4-yl]methyl}-5-hydroxy-6-methylpyrimidin-4-yl)carbonyl]amino}acetic acid

In accordance with Examples 1-(5), 1-(6) and 1-(9) to 1-(11), but using tert-butyl 5-(benzyloxy)-2-{[1-(4-tert-butylphenyl)-2-oxopiperidin-4-yl]methyl}-6-hydroxypyrimidine-4-carboxylate (0.64 g, 1.2 mmol) instead of tert-butyl 5-(benzyloxy)-2-{[1-(tert-butoxycarbonyl)piperidin-4-yl]methyl}-6-hydroxypyrimidine-4-carboxylate, the title compound (0.29 g, 0.64 mmol) was afforded as a white solid (yield 53%).
MS m/z: 455 (M+H)⁺;
1H-NMR (500 MHz, DMSO-d6) δ: 11.94 (1H, brs), 9.43 (1H, t, J=6 Hz), 7.38 (2H, d, J=9 Hz), 7.18 (2H, d, J=9 Hz), 4.00 (2H, d, J=6 Hz), 3.69-3.63 (1H, m), 3.56-3.52 (1H, m), 2.88 (2H, d, J=6 Hz), 2.65-2.57 (1H, m), 2.45 (3H, s), 2.44-2.40 (1H, m), 2.22 (1H, dd, J=16 Hz, 10Hz), 1.93 (1H, d, J=13 Hz), 1.70-1.62 (1H, m), 1.28 (9H, s).

### Example 171

### {[(2-{[2-(4-tert-Butylphenyl)-1,2,3,4-tetrahydroisoquinolin-6-yl]methyl}-5-hydroxy-6-methylpyrimidin-4-yl)carbonyl]amino}acetic acid

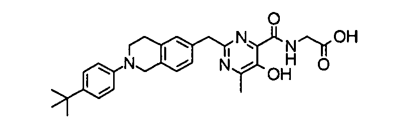

### (1) tert-Butyl 6-(cyanomethyl)-1,2,3,4-tetrahydroisoquinoline-2-carboxylate

tert-Butyl 6-(methoxycarbonyl)-1,2,3,4-tetrahydroisoquinoline-2-carboxylate (3.2 g, 11 mmol) was dissolved in tetrahydrofuran (50 mL), and at room temperature methanol (0.88 mL, 22 mmol) and lithium borohydride (0.71 g, 33 mmol) were added, followed by heating to reflux for 2 hours under a nitrogen atmosphere. After the reaction solution was cooled to room temperature, saturated aqueous ammonium chloride solution was added, followed by extraction with ethyl acetate. After the organic layer was dried over anhydrous sodium sulfate, it was concentrated under reduced pressure to afford tert-butyl 6-(hydroxymethyl)-1,2,3,4-tetrahydroisoquinoline-2-carboxylate as a colorless oil. This was dissolved in tetrahydrofuran (60 mL), and at 0°C phosphorus tribromide (0.51 mL, 5.5 mmol) was added, followed by stirring at room temperature for 3 hours under a nitrogen atmosphere. Aqueous sodium hydrogencarbonate solution was added to the reaction solution, followed by extraction with ethyl acetate, and the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.62 (hexane/ethyl acetate=2/1) by thin layer chromatography was concentrated under reduced pressure to afford tert-butyl 6-(bromomethyl)-1,2,3,4-tetrahydroisoquinoline-2-carboxylate (2.0 g, 6.2 mmol) as a colorless oil. This was dissolved in acetonitrile (30 mL), and tetrabutylammonium cyanide (1.8 g, 6.9 mmol) was added, followed by stirring for 3 days under a nitrogen atmosphere. After the reaction solution was concentrated under reduced pressure, the resulting residue was purified by silica gel column chromatography (Moritex Corporation, elution solvent: hexane/ethyl acetate), and a fraction corresponding to the Rf value=0.19 (hexane/ethyl acetate=5/1) by thin layer chromatography was concentrated under reduced pressure to afford the title compound (1.1 g, 4.1 mmol) as a colorless oil (yield 66%).
1H-NMR (500 MHz, CDCl3) δ: 7.15-7.11 (3H, m), 4.56 (2H, s), 3.71 (2H, s), 3.68-3.62 (2H, m), 2.83 (2H, t, J=6 Hz), 1.49 (9H, s).

### (2) tert-Butyl 5-(benzyloxy)-2-{[2-(tert-butoxycarbonyl)-1,2,3,4-tetrahydroisoquinolin-6-yl]methyl}-6-hydroxypyrimidine-4-carboxylate

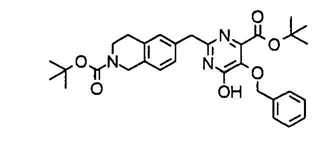

In accordance with Examples 1-(3) and 1-(4), but using tert-butyl 6-(cyanomethyl)-1,2,3,4-tetrahydroisoquinoline-2-carboxylate (1.1 g, 4.1 mmol) instead of tert-butyl 4-(cyanomethyl)piperidine-1-carboxylate, the title compound (1.5 g, 2.7 mmol) was afforded as a brown oil (yield 66%).
1H-NMR (500 MHz, CDCl3) δ: 7.45 (2H, d, J=8 Hz), 7.39-7.30 (3H, m), 7.15 (1H, d, J=8 Hz), 7.11 (1H, s), 7.07 (1H, d, J=8 Hz), 5.23 (2H, s), 4.54 (2H, s), 3.96 (2H, s), 3.65-3.59 (2H, m), 2.77 (2H, t, J=5 Hz), 1.53 (9H, s), 1.49 (9H, s).

### (3) tert-Butyl 5-(benzyloxy)-6-methyl-2-(1,2,3,4-tetrahydroisoquinolin-6-ylmethyl)pyrimidine-4-carboxylate hydrochloride

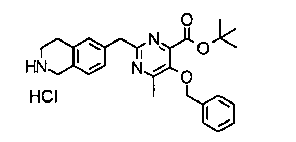

In accordance with Examples 1-(5) to 1-(7), but using tert-butyl 5-(benzyloxy)-2-{[2-(tert-butoxycarbonyl)-1,2,3,4-tetrahydroisoquinolin-6-yl]methyl}-6-hydroxypyrimidine-4-carboxylate (1.5 g, 2.7 mmol) instead of tert-butyl 5-(benzyloxy)-2-{[1-(tert-butoxycarbonyl)piperidin-4-yl]methyl -6-hydroxypyrimidine-4-carboxylate, the title compound (0.65 g, 1.3 mmol) was afforded as a white solid (yield 49%). 1H-NMR (500 MHz, DMSO-d6) δ: 7.45-7.36 (5H, m), 7.18-7.13 (3H, m), 4.98 (2H, s), 4.20 (2H, s), 4.13 (2H, s), 3.35-3.30 (2H, m), 2.97 (2H, t, J=6 Hz), 2.44 (3H, s), 1.50 (9H, s).

### (4) {[(2-{[2-(4-tert-Butylphenyl)-1,2,3,4-tetrahydroisoquinolin-6-yl]methyl}-5-hydroxy-6-methylpyrimidin-4-yl)carbonyl]amino} acetic acid

In accordance with Examples 1-(8), 73-(2), 1-(10) and 1-(11), but using tert-butyl 5-(benzyloxy)-6-methyl-2-(1,2,3,4-tetrahydroisoquinolin-6-ylmethyl)pyrimidine-4-carboxylate hydrochloride (0.32 g, 0.67 mmol) instead of tert-butyl 5-(benzyloxy)-6-methyl-2-[(piperidin-4-yl)methyl]pyrimidine-4-carboxylate hydrochloride, the title compound (0.092 g, 0.19 mmol) was afforded as a pale yellow solid (yield 28%).
MS m/z: 489 (M+H)⁺;
1H-NMR (500 MHz, DMSO-d6) δ: 9.23 (1H, brs), 7.22 (2H, d, J=9 Hz), 7.12-7.08 (3H, m), 6.91 (2H, d, J=9 Hz), 4.28 (2H, s), 4.07 (2H, s), 3.77 (2H, s), 3.45 (2H, t, J=6 Hz), 2.85 (2H, t, J=6 Hz), 2.40 (3H, s), 1.23 (9H, s).

### Example 172

### {[(2-{[8-(4-tert-Butylphenyl)-8-azabicyclo[3.2.1]oct-3-yl]methyl}-5-hydroxy-6-methylpyrimidin-4-yl)carbonyl]amino}acetic acid

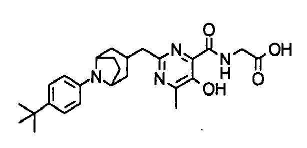

### (1) 8-(4-tert-Butylphenyl)-8-azabicyclo[3.2.1]octan-3-one

Spiro[8-azabicyclo[3.2.1]octane-3,2'-[1,3]dioxolane] (2.2 g, 13 mmol), 1-bromo-4-tert-butylbenzene (2.9 mL, 17 mmol), sodium tert-butoxide (2.5 g, 26 mmol), dicyclohexyl(2',4',6'-triisopropylbiphenyl-2-yl)phosphine (X-PHOS) (0.62 g, 1.3 mmol) and tris(dibenzylideneacetone)dipalladium (0.59 g, 0.65 mmol) were suspended in toluene (70 mL), followed by heating at 100°C for 5 hours under a nitrogen atmosphere. The reaction solution was cooled to room temperature, and subsequently filtered with celite. After the filtrate was concentrated under reduced pressure, the resulting residue was purified by silica gel column chromatography (Moritex Corporation, elution solvent: hexane/ethyl acetate), and a fraction corresponding to the Rf value=0.34 (hexane/ethyl acetate=10/1) by thin layer chromatography was concentrated under reduced pressure to afford 8-(4-tert-butylphenyl)spiro[8-azabicyclo[3.2.1]octane-3,2'-[1,3]dioxolane] as a yellow oil. This was dissolved in tetrahydrofuran (30 mL), and hydrochloric acid (5 M, 15 mL, 75 mmol) was added, followed by stirring at room temperature for 24 hours. The reaction solution was added dropwise to saturated aqueous sodium hydrogencarbonate solution, followed by extraction with ethyl acetate, and drying over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.23 (hexane/ethyl acetate=5/1) by thin layer chromatography was concentrated under reduced pressure to afford the title compound (2.2 g, 8.5 mmol) as a pale yellow solid (yield 66%).
1H-NMR (500 MHz, CDCl3) δ: 7.32 (2H, d, J=8 Hz), 6.83 (2H, d, J=8 Hz), 4.48 (2H, s), 2.71 (2H, d, J=14 Hz), 2.30 (2H, d, J=14 Hz), 2.19-2.15 (2H, m), 1.80-1.76 (2H, m), 1.30 (9H, s).

### (2) [8-(4-tert-Butylphenyl)-8-azabicyclo[3.2.1]oct-3-yl]acetonitrile

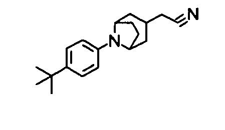

In accordance with Example 1-(1), but using 8-(4-tert-butylphenyl)-8-azabicyclo[3.2.1]octan-3-one (2.2 g, 8.5 mmol) instead of tert-butyl 4-oxopiperidine-1-carboxylate, [8-(4-tert-butylphenyl)-8-azabicyclo[3.2.1]oct-3-ylidene]acetonitrile (2.3 g, 8.3 mmol) was afforded as a white solid (yield 98%).

To magnesium powder (9.5 g, 0.39 mol), at 0°C, was added a solution of [8-(4-tert-butylphenyl)-8-azabicyclo[3.2.1]oct-3-ylidene]acetonitrile (3.6 g, 13 mmol) in methanol (200 mL) at 0°C, followed by vigorously stirring at the same temperature for 2.5 hours under a nitrogen atmosphere. Saturated aqueous ammonium chloride solution was added to the reaction solution, followed by extraction with ethyl acetate, and drying over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.28 (hexane/ethyl acetate=5/1) by thin layer chromatography was concentrated under reduced pressure to afford a stereoisomeric mixture (2.9 g, 10 mmol) of the title compound as a white solid (yield 78%).
1H-NMR (500 MHz, CDCl3) δ: 7.23 (2H, d, J=8 Hz), 6.70 and 6.68 (total 2H, each d, J=8 Hz), 4.25 and 4.23 (total 2H, each s), 2.47 and 2.11 (total 2H, each d, J=8 Hz), 2.43-2.38 and 2.14-2.08 and 1.35-1.31 (total 4H, each m), 2.35-2.25 and 1.99-1.91 (total 1H, each m), 1.77 and 1.72 (total 2H, each d, J=8 Hz), 1.62 and 1.60 (total 2H, each s), 1.28 (9H, s).

### (3) tert-Butyl 5-(benzyloxy)-2-{[8-(4-tert-butylphenyl)-8-azabicyclo[3.2.1]oct-3-yl]methyl}-6-hydroxypyrimidine-4-carboxylate

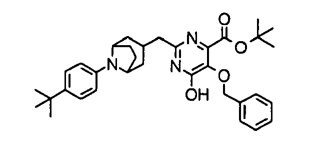

In accordance with Examples 1-(3) and 1-(4), but using [8-(4-tert-butylphenyl)-8-azabicyclo[3.2.1]oct-3-yl]acetonitrile (2.9 g, 10 mmol) instead of tert-butyl 4-(cyanomethyl)piperidine-1-carboxylate, a stereoisomeric mixture (3.1 g, 5.6 mmol) of the title compound was afforded as a pale yellow solid (yield 55%).
1H-NMR (500 MHz, CDCl3) δ: 7.42 (2H, d, J=7 Hz), 7.37-7.31 (3H, m), 7.20 (2H, d, J=8 Hz), 6.67 and 6.65 (total 2H, each d, J=8 Hz), 5.22 (2H, s), 4.16 (2H, brs), 2.89 and 2.41 (total 2H, each d, J=7 Hz), 2.55-2.47 and 2.31-2.25 and 1.68-1.61 (total 3H, each m), 2.05-1.98 (2H, m), 1.90 and 1.75 (total 2H, each d, J=7 Hz), 1.51 (9H, s), 1.44-1.38 and 1.28-1.22 (total 2H, each m), 1.26 (9H, s).

### (4) tert-Butyl 5-(benzyloxy)-2-{[8-(4-tert-butylphenyl)-8-azabicyclo[3.2.1]oct-3-yl]methyl}-6-methylpyrimidine-4-carboxylate

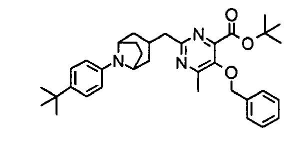

In accordance with Examples 1-(5) and 1-(6), but using tert-butyl 5-(benzyloxy)-2-{[8-(4-tert-butylphenyl)-8-azabicyclo[3.2.1]oct-3-yl]methyl}-6-hydroxypyrimidine-4-carboxylate (3.3 g, 5.8 mmol) instead of tert-butyl 5-(benzyloxy)-2-{[1-(tert-butoxycarbonyl)piperidin-4-yl]methyl}-6-hydroxypyrimidine-4-carboxylate, a stereoisomeric mixture (1.2 g, 2.2 mmol) of the title compound was afforded as an amorphous solid (yield 37%).
1H-NMR (500 MHz, CDCl3) δ: 7.43-7.35 (5H, m), 7.22 (2H, d, J=8 Hz), 6.69 (2H, d, J=8 Hz), 4.99 (2H, s), 4.18 (2H, brs), 3.15 and 2.67 (total 2H, each d, J=8 Hz), 2.59-2.51 and 2.38-2.32 (total 1H, each m), 2.43 and 2.42 (total 3H, each s), 2.28-2.22 and 1.72-1.66 (total 2H, each m), 2.13-2.09 and 2.05-2.00 (total 2H, each m), 2.02 and 1.80 (total 2H, each d, J=7 Hz), 1.59 and 1.58 (total 9H, each s), 1.36-1.31 and 1.29-1.25 (total 2H, each m), 1.29 and 1.28 (total 9H, each s).

### (5) {[(2-{[8-(4-tert-Butylphenyl)-8-azabicyclo[3.2.1]oct-3-yl]methyl}-5-hydroxy-6-methylpyrimidin-4-yl)carbonyl]amino} acetic acid

In accordance with Examples 1-(9) to 1-(11), but using tert-butyl 5-(benzyloxy)-2-{[8-(4-tert-butylphenyl)-8-azabicyclo[3.2.1]oct-3-yl]methyl}-6-methylpyrimidine-4-carboxylate (1.2 g, 2.2 mmol) instead of tert-butyl 5-(benzyloxy)-2-{[1-(4-tert-butylphenyl)piperidin-4-yl]methyl}-6-methylpyrimidine-4-carboxylate, a stereoisomeric mixture of the title compound was afforded as a pale yellow solid. This was purified by preparative HPLC (YMC-Pack ODS-A; YMC, elution solvent: acetonitrile/triethylamine (0.025%) and trifluoroacetic acid (0.05%) aqueous solution =45/55) to afford a high polarity isomer (0.022 g, 0.050 mmol, yield 2.3%) of the title compound as a pale yellow solid, and a low polarity isomer (0.11 g, 0.23 mmol, yield 10%) of the title compound as a white solid.
High polarity isomer
MS m/z: 467 (M+H)⁺;
1H-NMR (500 MHz, DMSO-d6) δ: 12.87 (1H, brs), 11.89 (1H, s), 9.40 (1H, t, J=6 Hz), 7.18 (2H, d, J=8 Hz), 6.68 (2H, d, J=8 Hz), 4.16 (2H, s), 3.98 (2H, d, J=6 Hz), 2.57-2.49 (3H, m), 2.41 (3H, s), 1.94-1.90 (2H, m), 1.74 (2H, d, J=7 Hz), 1.49 (2H, t, J=11 Hz), 1.31-1.23 (2H, m), 1.24 (9H, s).
Low polarity isomer
MS m/z: 467 (M+H)⁺;
1H-NMR (500 MHz, DMSO-d6) δ: 12.85 (1H, brs), 11.88 (1H, s), 9.36 (1H, t, J=6 Hz), 7.16 (2H, d, J=8 Hz), 6.68 (2H, d, J=8 Hz), 4.16 (2H, s), 3.98 (2H, d, J=6 Hz), 3.05 (2H, d, J=8 Hz), 2.41 (3H, s), 2.34-2.28 (1H, m), 2.13-2.08 (2H, m), 2.03-1.95 (4H, m), 1.22 (9H, s), 1.18 (2H, d, J=14 Hz).

### Example 173

### {[(2-{[6-(4-tert-Butylphenyl)pyridin-3-yl]methyl}-5-hydroxy-6-methylpyrimidin-4-yl)carbonyl]amino}acetic acid

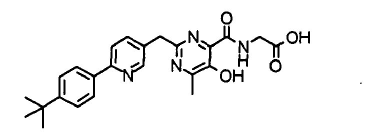

### (1) [6-(4-tert-Butylphenyl)pyridin-3-yl]acetonitrile

(6-Chloropyridin-3-yl)acetonitrile (5.0 g, 33 mmol) was dissolved in 1,2-dimethoxyethane (100 mL), and (4-tert-butylphenyl)boronic acid (8.8 g, 49 mmol), tri-potassium phosphate hydrate (18 g, 66 mmol) and [1,1'-bis(diphenylphosphino)ferrocene]palladium dichloride-dichloromethane adduct (0.81 g, 1.0 mmol) were added at room temperature under a nitrogen atmosphere, followed by heating to reflux for 7 hours. After the reaction solution was cooled to room temperature, the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.40 (hexane/ethyl acetate=3/1) by thin layer chromatography was concentrated under reduced pressure to afford the title compound (5.1 g, 20 mmol) as a pale yellowish white solid (yield 62%).
1H-NMR (500 MHz, CDCl3) δ: 8.62 (1H, s), 7.93 (2H, d, J=8 Hz), 7.76 (2H, s), 7.52 (2H, d, J=8 Hz), 3.80 (2H, s), 1.37 (9H, s).

### (2) 2-[6-(4-tert-Butylphenyl)pyridin-3-yl]ethanimidamide acetic acid salt

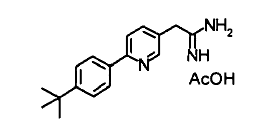

In accordance with Example 1-(3), but using [6-(4-tert-butylphenyl)pyridin-3-yl]acetonitrile (5.1 g, 20 mmol) instead of tert-butyl 4-(cyanomethyl)piperidine-1-carboxylate, the title compound (7.0 g, 20 mmol) was afforded as a pale yellow solid (yield 100%).
1H-NMR (500 MHz, DMSO-d6) δ: 8.55 (1H, s), 8.00 (2H, d, J=8 Hz), 7.95 (1H, d, J=8 Hz), 7.86 (1H, d, J=8 Hz), 7.52 (2H, d, J=8 Hz), 3.67 (2H, s), 1.65 (3H, s), 1.32 (9H, s).

### (3) tert-Butyl 5-(benzyloxy)-2-{[6-(4-tert-butylphenyl)pyridin-3-yl]methyl}-6-hydroxypyrimidine-4-carboxylate

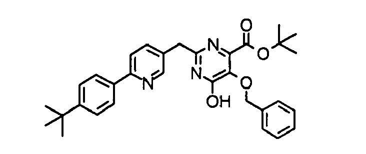

In accordance with Example 1-(4), but using 2-[6-(4-tert-butylphenyl)piperidin-3-yl]ethanimidamide acetic acid salt (16 g, 48 mmol) instead of tert-butyl 4-(2-amino-2-iminoethyl)piperidine-1-carboxylate acetic acid salt, the title compound (19 g, 36 mmol) was afforded as a pale brown solid (yield 74%).
MS m/z: 526 (M+H)⁺.

### (4) tert-Butyl 5-(benzyloxy)-2-{[6-(4-tert-butylphenyl)pyridin-3-yl]methyl}-6-{[(trifluoromethyl)sulfonyl]oxy}pyrimidine-4-carboxylate

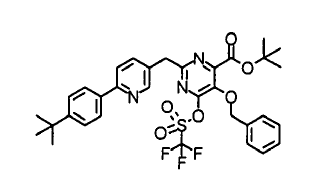

In accordance with Example 1-(5), but using tert-butyl 5-(benzyloxy)-2-{[6-(4-tert-butylphenyl)pyridin-3-yl]methyl}-6-hydroxypyrimidine-4-carboxylate (19 g, 36 mmol) instead of tert-butyl 5-(benzyloxy)-2-{[1-(tert-butoxycarbonyl)piperidin-4-yl]methyl}-6-hydroxypyrimidine-4-carboxylate, the title compound (17 g, 26 mmol) was afforded as a yellow oil (yield 72%).
1H-NMR (500 MHz, CDCl3) δ: 8.66 (1H, s), 7.91 (2H, d, J=8 Hz), 7.77 (1H, d, J=8 Hz), 7.69 (1H, d, J=8 Hz), 7.50 (2H, d, J=8 Hz), 7.43-7.37 (5H, m), 5.13 (2H, s), 4.29 (2H, s), 1.58 (9H, s), 1.36 (9H, s).

### (5) tert-Butyl 5-(benzyloxy)-2-{[6-(4-tert-butylphenyl)pyridin-3-yl]methyl}-6-methylpyrimidine-4-carboxylate

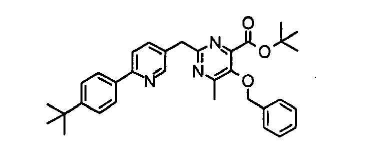

In accordance with Example 1-(6), but using tert-butyl 5-(benzyloxy)-2-{[6-(4-tert-butylphenyl)pyridin-3-yl]methyl}-6-{[(trifluoromethyl)sulfonyl]oxy}pyrimidine-4-carboxylate (16 g, 24 mmol) instead oftert-butyl 5-(benzyloxy)-2-{[1-(tert-butoxycarbonyl)piperidin-4-yl]methyl}-6-{[(trifluoromethyl)sulfonyl]oxy}pyrimidine-4-carboxylate, the title compound (9.6 g, 18 mmol) was afforded as a pale yellow solid (yield 75%).
1H-NMR (500 MHz, CDCl3) δ: 8.70 (1H, s), 7.90 (2H, d, J=8 Hz), 7.78 (1H, d, J=8 Hz), 7.65 (1H, d, J=8 Hz), 7.48 (2H, d, J=8 Hz), 7.42-7.35 (5H, m), 4.99 (2H, s), 4.28 (2H, s), 2.44 (3H, s), 1.59 (9H, s), 1.36 (9H, s).

### (6) Ethyl ({[5-(benzyloxy)-2-{[6-(4-tert-butylphenyl)pyridin-3-yl]methyl}-6-methylpyrimidin-4-yl]carbonyl}amino)acetate

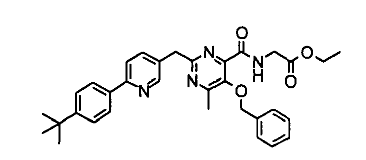

In accordance with Example 1-(9), but using tert-butyl 5-(benzyloxy)-2-{[6-(4-tert-butylphenyl)pyridin-3-yl]methyl}-6-methylpyrimidine-4-carboxylate (9.6 g, 18 mmol) instead of tert-butyl 5-(benzyloxy)-2-{[1-(4-tert-butylphenyl)piperidin-4-yl]methyl}-6-methylpyrimidine-4-carboxylate, the title compound (9.2 g, 17 mmol) was afforded as a pale yellow oil (yield 91 %).
1H-NMR (500 MHz, CDCl3) δ: 8.69 (1H, s), 8.28 (1H, t, J=5 Hz), 7.91 (2H, d, J=8 Hz), 7.76 (1H, d, J=8 Hz), 7.69 (1H, d, J=8 Hz), 7.50-7.47 (4H, m), 7.39-7.34 (3H, m), 5.10 (2H, s), 4.28 (2H, s), 4.25 (2H, q, J=7 Hz), 4.21 (2H, d, J=5 Hz), 2.46 (3H, s), 1.36 (9H, s), 1.31 (3H, t, J=7 Hz).

### (7) {[(2-{[6-(4-tert-Butylphenyl)pyridin-3-yl]methyl}-5-hydroxy-6-methylpyrimidin-4-yl)carbonyl]amino} acetic acid

In accordance with Examples 6-(2) and 1-(11), but using ethyl ({[5-(benzyloxy)-2-{[6-(4-tert-butylphenyl)pyridin-3-yl]methyl}-6-methylpyrimidin-4-yl]carbonyl}amino)acetate (9.2 g, 17 mmol) instead of ethyl ({[5-(benzyloxy)-2-{[1-(2-chlorophenyl)piperidin-4-yl]methyl}-6-methylpyrimidin-4-yl]carbonyl}amino)acetate, the title compound (7.0 g, 16 mmol) was afforded as a white solid (yield 95%).
MS m/z: 435 (M+H)⁺;
1H-NMR (500 MHz, DMSO-d6) δ: 11.98 (1H, brs), 9.51 (1H, t, J=6 Hz), 8.65 (1H, s), 7.98 (2H, d, J=7 Hz), 7.86 (1H, d, J=8 Hz), 7.82 (1H, d, J=8 Hz), 7.50 (2H, d, J=7 Hz), 4.22 (2H, s), 4.02 (2H, d, J=6 Hz), 2.43 (3H, s), 1.32 (9H, s).

### Example 174

### {[(2-{[6-(4-Fluorophenyl)pyridin-3-yl]methyl}-5-hydroxy-6-methylpyrimidin-4-yl)carbonyl]amino} acetic acid

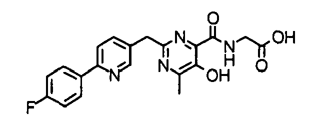

In accordance with Examples 173-(1) to 173-(7), but using (4-fluorophenyl)boronic acid (2.1 g, 15 mmol) instead of (4-tert-butylphenyl)boronic acid, the title compound (0.90 g, 2.3 mmol) was afforded as a white solid (yield 23%).
MS m/z: 397 (M+H)⁺;
1H-NMR (500 MHz, DMSO-d6) δ: 11.99 (1H, brs), 9.48 (1H, t, J=6 Hz), 8.77 (1H, s), 8.15-8.06 (4H, m), 7.41-7.37 (2H, m), 4.32 (2H, s), 4.02 (2H, d, J=6 Hz), 2.43 (3H, s).

### Example 175

### ({[5-Hydroxy-6-methyl-2-({6-[4-(trifluoromethyl)phenyl]pyridin-3-yl}methyl)pyrimidin-4-yl]carbonyl}amino)acetic acid

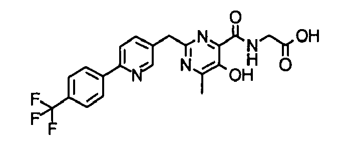

In accordance with Examples 173-(1) to 173-(7), but using [4-(trifluoromethyl)phenyl]boronic acid (3.0 g, 20 mmol) instead of (4-tert-butylphenyl)boronic acid, the title compound (2.5 g, 5.6 mmol) was afforded as a white solid (yield 28%).
MS m/z: 447 (M+H)⁺;
1H-NMR (500 MHz, DMSO-d6) δ: 9.47 (1H, t, J=6 Hz), 8.73 (1H, s), 8.28 (2H, d, J=8 Hz), 8.01 (1H, d, J=8 Hz), 7.90 (1H, d, J=8 Hz), 7.84 (2H, d, J=8 Hz), 4.25 (2H, s), 4.02 (2H, d, J=6 Hz), 2.42 (3H, s).

### Example 176

### {[(2-{[6-(Biphenyl-4-yl)pyridin-3-yl]methyl}-5-hydroxy-6-methylpyrimidin-4-yl)carbonyl]amino} acetic acid

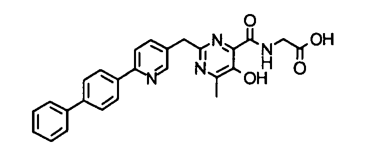

In accordance with Examples 173-(1) to 173-(7), but using (biphenyl-4-yl)boronic acid (3.0 g, 15 mmol) instead of (4-tert-butylphenyl)boronic acid, the title compound (0.24 g, 2.3 mmol) was afforded as a white solid (yield 5.5%).
MS m/z: 455 (M+H)⁺;
1H-NMR (500 MHz, DMSO-d6) δ: 9.46 (1H, brs), 8.69 (1H, s), 8.16 (2H, d, J=7 Hz), 7.96 (1H, d, J=8 Hz), 7.84 (1H, d, J=8 Hz), 7.79 (2H, d, J=7 Hz), 7.74 (2H, d, J=7 Hz), 7.50 (2H, t, J=7 Hz), 7.40 (1H, t, J=7 Hz), 4.24 (2H, s), 3.98 (2H, d, J=6 Hz), 2.43 (3H, s).

### Example 177

### {[(2-{[6-(4-Benzylphenyl)pyridin-3-yl]methyl}-5-hydroxy-6-methylpyrimidin-4-yl)carbonyl]amino} acetic acid

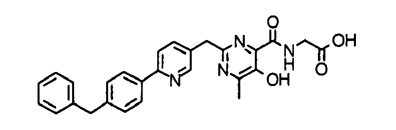

In accordance with Examples 173-(1) to 173-(7), but using (4-benzylphenyl)boronic acid pinacol ester (10 g, 35 mmol) instead of (4-tert-butylphenyl)boronic acid, the title compound (2.4 g, 5.6 mmol) was afforded as a white solid (yield 18%).
MS m/z: 469 (M+H)⁺;
1H-NMR (500 MHz, DMSO-d6) δ: 11.97 (1H, brs), 9.46 (1H, t, J=6 Hz), 8.68 (1H, s), 7.96 (2H, d, J=8 Hz), 7.92 (2H, s), 7.36 (2H, d, J=8 Hz), 7.32-7.16 (5H, m), 4.24 (2H, s), 4.02 (2H, d, J=6 Hz), 4.00 (2H, s), 2.42 (3H, s).

### Example 178

### {[(2-{[6-(4-tert-Butylphenyl)-2-methylpyridin-3-yl]methyl}-5-hydroxy-6-methylpyrimidin-4-yl)carbonyl]amino}acetic acid

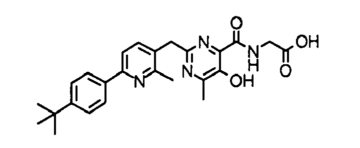

### (1) 1-(4-tert-Butylphenyl)-3-(dimethylamino)propan-1-one

4-tert-Butylacetophenone (5.1 g, 29 mmol) was dissolved in ethanol (15 mL), and dimethylamine hydrochloride (3.1 g, 28 mmol), paraformaldehyde (1.5 g, 500 mmol) and concentrated hydrochloric acid (0.090 mL) were added, followed by heating to reflux for 12 hours under a nitrogen atmosphere. After the reaction solution was cooled, it was concentrated under reduced pressure, and the resulting solid was collected by filtration using ethanol to afford the title compound (3.9 g, 17 mmol) as a white solid (yield 58%).
MS m/z: 234 (M+H)⁺.

### (2) Methyl 6-(4-tert-butylphenyl)-2-methylnicotinate

1-(4-tert-Butylphenyl)-3-(dimethylamino)propan-1-one (3.9 g, 17 mmol) was dissolved in acetic acid (50 mL), and at room temperature methyl 3-amino-2-butenoate (1.9 g, 17 mmol) was added, followed by heating to reflux for 10 hours. Methyl 3-amino-2-butenoate (1.9 g, 17 mmol) was added to the reaction solution, followed by heating to reflux for a further 10 hours. After the reaction solution was cooled, water was added, followed by extraction with ethyl acetate, and the extract was washed with saturated aqueous ammonium chloride solution. After the solvent was distilled off under reduced pressure, the resulting residue was purified by silica gel column chromatography (Moritex Corporation, elution solvent: hexane/ethyl acetate), and a fraction corresponding to the Rf value=0.60 (hexane/ethyl acetate=4/1) by thin layer chromatography was concentrated under reduced pressure to afford the title compound (1.4 g, 4.9 mmol) as a colorless oil (yield 58%).
MS m/z: 284 (M+H)⁺.

### (3) [6-(4-tert-Butylphenyl)-2-methylpyridin-3-yl]methanol

Methyl 6-(4-tert-butylphenyl)-2-methylnicotinate (1.4 g, 4.9 mmol) was dissolved in tetrahydrofuran (10 mL), and at 0°C a solution of diisobutylaluminium hydride in hexane (1.0 M, 12 mL, 12 mmol) was added dropwise, followed by stirring at the same temperature for 1 hour. Magnesium sulfate decahydrate (2.0 g) was added to the reaction solution, followed by stirring at room temperature for 30 minutes. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.15 (hexane/ethyl acetate=2/1) by thin layer chromatography was concentrated under reduced pressure to afford the title compound (1.0 g, 4.0 mmol) as a colorless oil (yield 82%).
MS m/z: 256 (M+H)⁺.

### (4) 3-(Bromomethyl)-6-(4-tert-butylphenyl)-2-methylpyridine

[6-(4-tert-Butylphenyl)-2-methylpyridin-3-yl]methanol (1.0 g, 4.0 mmol) was dissolved in a mixture of toluene (8.0 mL) and methylene chloride (4.0 mL), and at 0°C phosphorus tribromide (0.75 mL, 8.0 mmol) was added dropwise, followed by stirring at the same temperature for 1.5 hours. Saturated aqueous sodium hydrogencarbonate solution was added to the reaction solution, followed by extraction with methylene chloride. After the extract was concentrated under reduced pressure, the resulting residue was purified by silica gel column chromatography (Moritex Corporation, elution solvent: hexane/ethyl acetate), and a fraction corresponding to the Rf value=0.70 (hexane/ethyl acetate=4/1) by thin layer chromatography was concentrated under reduced pressure to afford the title compound (1.2 g, 3.7 mmol) as a pale yellow oil (yield 91 %).
1H-NMR (500 MHz, CDCl3) δ: 7.92 (2H, d, J=8 Hz), 7.64 (1H, d, J=8 Hz), 7.53 (1H, d, J=8 Hz), 7.49 (2H, d, J=8 Hz), 4.55 (2H, s), 2.70 (3H, s), 1.35 (9H, s).

### (5) [6-(4-tert-Butylphenyl)-2-methylpyridin-3-yl]acetonitrile

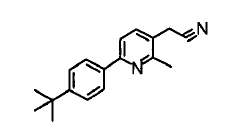

3-(Bromomethyl)-6-(4-tert-butylphenyl)-2-methylpyridine (1.2 g, 3.7 mmol) was dissolved in acetonitrile (50 mL), and at room temperature tetrabutylammonium cyanide (0.99 g, 3.7 mmol) was added, followed by stirring at the same temperature for 5 hours. After the reaction solution was concentrated under reduced pressure, the resulting residue was purified by silica gel column chromatography (Moritex Corporation, elution solvent: hexane/ethyl acetate); and a fraction corresponding to the Rf value=0.60 (hexane/ethyl acetate=3/1) by thin layer chromatography was concentrated under reduced pressure to afford the title compound (0.91 g, 3.4 mmol) as a white solid (yield 94%).
1H-NMR (500 MHz, CDCl3) δ: 7.92 (2H, d, J=8 Hz), 7.72 (1H, d, J=8 Hz), 7.58 (1H, d, J=8 Hz), 7.50 (2H, d, J=8 Hz), 3.74 (2H, s), 2.63 (3H, s), 1.36 (9H, s).

### (6) {[(2-{[6-(4-tert-Butylphenyl)-2-methylpyridin-3-yl]methyl}-5-hydroxy-6-methylpyrimidin-4-yl)carbonyl]amino} acetic acid

In accordance with Examples 173-(2) to 173-(7), but using [6-(4-tert-butylphenyl)-2-methylpyridin-3-yl]acetonitrile (0.91 g, 3.4 mmol) instead of [6-(4-tert-butylphenyl)pyridin-3-yl]acetonitrile, the title compound (0.44 g, 0.98 mmol) was afforded as a white solid (yield 29%).
MS m/z: 449 (M+H)⁺;
1H-NMR (500 MHz, DMSO-d6) δ: 12.89 (1H, brs), 11.96 (1H, s), 9.38 (1H, t, J=6 Hz), 7.96 (2H, d, J=8 Hz), 7.68 (1H, d, J=8 Hz), 7.63-7.60 (1H, m), 7.48 (2H, d, J=8 Hz), 4.22 (2H, s), 4.02 (2H, d, J=6 Hz), 2.58 (3H, s), 2.41 (3H, s), 1.31 (9H, s).

### Example 179

### {[(2-{[6-(4-tert-Butylphenyl)-5-methylpyridin-3-yl]methyl}-5-hydroxy-6-methylpyrimidin-4-yl)carbonyl]amino} acetic acid

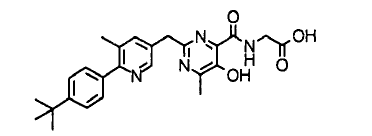

### (1) 2-(4-tert-Butylphenyl)-3-methyl-5-nitropyridine

2-Chloro-3-methyl-5-nitropyridine (2.0 g, 12 mmol) was dissolved in a mixture of 1,2-dimethoxyethane (50 mL) and water (1.0 mL), and (4-tert-butylphenyl)boronic acid (2.5 g, 14 mmol), tri-potassium phosphate hydrate (6.2 g, 23 mmol) and [1,1'-bis(diphenylphosphino)ferrocene]palladium dichloride-dichloromethane adduct (0.28 g, 0.35 mmol) were added at room temperature under a nitrogen atmosphere, followed by heating to reflux for 7 hours. After the reaction solution was cooled to room temperature, the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.60 (hexane/ethyl acetate=4/1) by thin layer chromatography was concentrated under reduced pressure to afford the title compound (3.0 g, 11 mmol) as a pale yellowish white solid (yield 96%).
1H-NMR (500 MHz, CDCl3) δ: 9.33 (1H, d, J=2 Hz), 8.38 (1H, d, J=2 Hz), 7.52 (4H, s), 2.54 (3H, s), 1.37 (9H, s).

### (2) 3-Amino-6-(4-tert-butylphenyl)-5-methylpyridine

2-(4-tert-Butylphenyl)-3-methyl-5-nitropyridine (4.6 g, 18 mmol) was dissolved in a mixture of ethyl acetate (50 mL), ethanol (200 mL) and methanol (200 mL), and 5% palladium-activated carbon (1.2 g) was added, followed by stirring at room temperature for 2 hours under a hydrogen atmosphere. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.80 (ethyl acetate) by thin layer chromatography was concentrated under reduced pressure to afford the title compound (4.5 g, 18 mmol) as a white solid (quantitative yield).
1H-NMR (500 MHz, CDCl3) δ: 8.03 (1H, d, J=3 Hz), 7.42 (4H, s), 6.91 (1H, d, J=3 Hz), 2.32 (3H, s), 1.36 (9H, s).

### (3) 5-Bromo-2-(4-tert-butylphenyl)-3-methylpyridine

3-Amino-6-(4-tert-butylphenyl)-5-methylpyridine (4.5 g, 18 mmol) was dissolved in acetonitrile (100 mL), and at 0°C copper(II) bromide (6.0 g, 27 mmol) and tert-butyl nitrite (4.8 mL, 36 mmol) were added, followed by stirring for 3 hours. After stirring further at room temperature for 5 hours, saturated aqueous ammonium chloride solution (20 mL) was added, followed by extraction with ethyl acetate. The extract was washed sequentially with water and saturated aqueous sodium chloride solution, and subsequently dried over anhydrous magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.80 (hexane/ethyl acetate=4/1) by thin layer chromatography was concentrated under reduced pressure to afford the title compound (2.0 g, 6.6 mmol) as a yellow solid (yield 37%).
1H-NMR (500 MHz, CDCl3) δ: 8.56 (1H, brs), 7.73 (1H, brs), 7.48-7.43 (4H, m), 2.38 (3H, s), 1.36 (9H, s).

### (4) [6-(4-tert-Butylphenyl)-5-methylpyridin-3-yl](phenylsulfonyl)acetonitrile

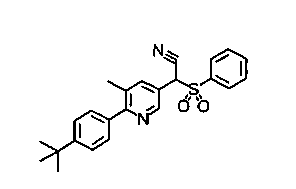

(Phenylsulfonyl)acetonitrile (4.7 g, 26 mmol) was dissolved in 1,2-dimethoxyethane (40 mL), and tetrakis(triphenylphosphine)palladium adduct (0.75 g, 0.65 mmol) and sodium hydride (62%, 1.2 g, 33 mmol) were added at room temperature, followed by stirring at the same temperature for 10 minutes. A solution of 5-bromo-2-(4-tert-butylphenyl)-3-methylpyridine (2.0 g, 6.6 mmol) in 1,2-dimethoxyethane (30 mL) was added to the reaction solution, followed by heating to reflux for 5 hours. After the reaction solution was cooled to room temperature, saturated aqueous ammonium chloride solution (20 mL) was added, followed by extraction with ethyl acetate. The extract was washed with water and saturated aqueous sodium chloride solution, and subsequently dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.50 (hexane/ethyl acetate=2/1) by thin layer chromatography was concentrated under reduced pressure to afford the title compound (2.5 g, 6.2 mmol) as a pale yellow oil (yield 94%).
MS m/z: 405 (M+H)⁺.

### (5) [6-(4-tert-Butylphenyl)-5-methylpyridin-3-yl]acetonitrile

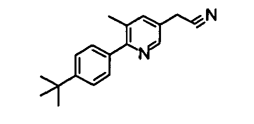

[6-(4-tert-Butylphenyl)-5-methylpyridin-3-yl](phenylsulfonyl)acetonitrile (2.5 g, 6.2 mmol) was dissolved in ethanol (15 mL), and at room temperature acetic acid (8 mL) and zinc powder (2.0 g) were added, followed by stirring for 1 hour. After the reaction solution was concentrated under reduced pressure, saturated aqueous sodium hydrogencarbonate solution was added, followed by extraction with ethyl acetate. The extract was washed with water and saturated aqueous sodium chloride solution, and dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.30 (hexane/ethyl acetate=2/1) by thin layer chromatography was concentrated under reduced pressure to afford the title compound (1.5 g, 5.7 mmol) as a pale yellow oil (yield 92%).
1H-NMR (500 MHz, CDC13) δ: 8.45 (1H, s), 7.61 (1H, s), 7.47-7.46 (4H, m), 3.78 (2H, s), 2.42 (3H, s), 1.36 (9H, s).

### (6) {[(2-{[6-(4-tert-Butylphenyl)-5-methylpyridin-3-yl]methyl}-5-hydroxy-6-methylpyrimidin-4-yl)carbonyl]amino}acetic acid

In accordance with Examples 173-(2) to 173-(7), but using [6-(4-tert-butylphenyl)-5-methylpyridin-3-yl]acetonitrile (1.5 g, 5.7 mmol) instead of [6-(4-tert-butylphenyl)pyridin-3-yl]acetonitrile, the title compound (0.50 g, 1.1 mmol) was afforded as a yellow solid (yield 20%).
MS m/z: 449 (M+H)⁺;
1H-NMR (500 MHz, DMSO-d6) δ: 12.90 (1H, brs), 11.97 (1H, s), 9.47 (1H, t, J=6 Hz), 8.48 (1H, s), 7.66 (1H, s), 7.46 (4H, s), 4.18 (2H, s), 4.02 (2H, d, J=6 Hz), 2.43 (3H, s), 2.30 (3H, s), 1.32 (9H, s).

### Example 180

### {[(2-{[6-(4-tert-Butylphenyl)-4-methylpyridin-3-yl]methyl}-5-hydroxy-6-methylpyrimidin-4-yl)carbonyl]amino}acetic acid

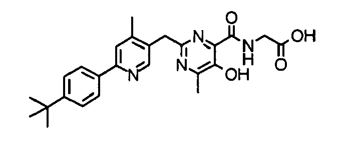

### (1) [6-(4-tert-Butylphenyl)-4-methylpyridin-3-yl]acetonitrile

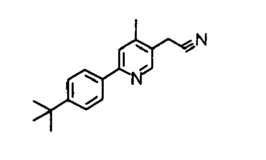

In accordance with Examples 179-(1) to 179-(5), but using 2-chloro-4-methyl-5-nitropyridine (2.0 g, 12 mmol) instead of 2-chloro-3-methyl-5-nitropyridine, the title compound (0.88 g, 3.3 mmol) was afforded as a white solid (yield 28%).
1H-NMR (500 MHz, CDCl3) δ: 8.55 (1H, s), 7.91 (2H, d, J=9 Hz), 7.58 (1H, s), 7.50 (2H, d, J=9 Hz), 3.71 (2H, s), 2.46 (s, 3H), 1.37 (9H, s).

### (2) {[(2-{[6-(4-tert-Butylphenyl)-4-methylpyridin-3-yl]methyl}-5-hydroxy-6-methylpyrimidin-4-yl)carbonyl]amino} acetic acid

In accordance with Examples 173-(2) to 173-(7), but using [6-(4-tert-butylphenyl)-4-methylpyridin-3-yl]acetonitrile (0.88 g, 3.3 mmol) instead of [6-(4-tert-butylphenyl)pyridin-3-yl]acetonitrile, the title compound (0.30 g, 0.67 mmol) was afforded as a yellow solid (yield 20%).
MS m/z: 449 (M+H)⁺;
1H-NMR (500 MHz, DMSO-d6) δ: 12.90 (1H, brs), 11.95 (1H, brs), 9.36 (1H, t, J=6 Hz), 8.45 (1H, s), 7.98 (2H, d, J=8 Hz), 7.74 (1H, s), 7.48 (2H, d, J=8 Hz), 4.22 (2H, s), 4.00 (2H, d, J=6 Hz), 2.40 (3H, s), 2.39 (3H, s), 1.31 (9H, s).

### Example 181

### {[(2-{[2-(4-tert-Butylphenyl)pyrimidin-5-yl]methyl}-5-hydroxy-6-methylpyrimidin-4-yl)carbonyl]amino}acetic acid

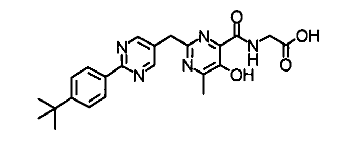

### (1) 2-(4-tert-Butylphenyl)-5-methylpyrimidine

2-Chloro-5-methylpyrimidine (1.9 g, 15 mmol) was dissolved in a mixture of toluene (30 mL), ethanol (15 mL) and water (15 mL), and (4-tert-butylphenyl)boronic acid (2.9 g, 17 mmol), sodium carbonate (4.8 g, 45 mmol) and [1,1'-bis(diphenylphosphino)ferrocene]palladium dichloride-dichloromethane adduct (1.2 g, 1.5 mmol) were added at room temperature under a nitrogen atmosphere, followed by heating to reflux for 8 hours. The reaction solution was cooled to room temperature, and the insoluble materials were filtered off. The filtrate was extracted with ethyl acetate, and the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.70 (hexane/ethyl acetate=3/1) by thin layer chromatography was concentrated under reduced pressure to afford the title compound (2.5 g, 11 mmol) as a white solid (yield 73%).
1H-NMR (500 MHz, CDCl3) δ: 8.62 (2H, s), 8.32 (2H, d, J=8 Hz), 7.50 (2H, d, J=8 Hz), 2.34 (3H, s), 1.37 (9H, s).

### (2) 5-(Bromomethyl)-2-(4-tert-butylphenyl)pyrimidine

2-(4-tert-Butylphenyl)-5-methylpyrimidine (2.5 g, 11 mmol) was dissolved in carbon tetrachloride (100 mL), and N-bromosuccinimide (2.4 g, 14 mmol) and 2,2'-azobis(isobutyronitrile) (0.30 g, 1.8 mmol) were added, followed by heating to reflux for 3 hours. N-Bromosuccinimide (0.60 g, 3.4 mmol) and 2,2'-azobis(isobutyronitrile) (0.30 g, 1.8 mmol) were added to the reaction solution, followed by heating to reflux for a further 7 hours. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), and a fraction corresponding to the Rf value=0.70 (hexane/ethyl acetate=3/1) by thin layer chromatography was concentrated under reduced pressure to afford the title compound (2.4 g, 8.0 mmol) as a white solid (yield 65%).
1H-NMR (500 MHz, CDCl3) δ: 8.81 (2H, s), 8.36 (2H, d, J=8 Hz), 7.52 (2H, d, J=8 Hz), 4.48 (2H, s), 1.37 (9H, s).

### (3) [2-(4-tert-butylphenyl)pyrimidin-5-yl]acetonitrile

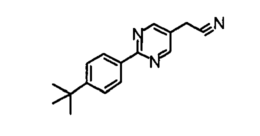

In accordance with Example 178-(5), but using 5-(bromomethyl)-2-(4-tert-butylphenyl)pyrimidine (2.4 g, 8.0 mmol) instead of 3-(bromomethyl)-6-(4-tert-butylphenyl)-2-methylpyridine, the title compound (1.5 g, 6.0 mmol) was afforded as a pale yellow solid (yield 75%).
1H-NMR (500 MHz, CDCl3) δ: 8.78 (2H, s), 8.37 (2H, d, J=8 Hz), 7.53 (2H, d, J=8 Hz), 3.79 (2H, s), 1.38 (9H, s).

### (4) {[(2-{[2-(4-tert-Butylphenyl)pyrimidin-5-yl]methyl}-5-hydroxy-6-methylpyrimidin-4-yl)carbonyl]amino}acetic acid

In accordance with Examples 173-(2) to 173-(7), but using [2-(4-tert-butylphenyl)pyrimidin-5-yl]acetonitrile (1.5 g, 6.0 mmol) instead of [6-(4-tert-butylphenyl)pyridin-3-yl]acetonitrile, the title compound (0.81 g, 1.9 mmol) was afforded as a white solid (yield 31 %).
MS m/z: 436 (M+H)⁺;
1H-NMR (500 MHz, DMSO-d6) δ: 9.46 (1H, t, J=6 Hz), 8.90 (2H, s), 8.30 (2H, d, J=7 Hz), 7.54 (2H, d, J=7 Hz), 4.24 (2H, s), 4.02 (2H, d, J=6 Hz), 2.42 (3H, s), 1.32 (9H, s).

### Example 182

### {[(2-{[2-(4-Benzylphenyl)pyrimidin-5-yl]methyl}-5-hydroxy-6-methylpyrimidin-4-yl)carbonyl]amino} acetic acid

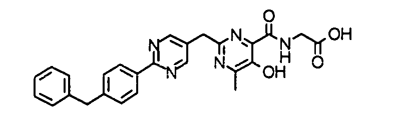

In accordance with Examples 173-(1) to 173-(7), but using (2-chloropyrimidin-5-yl)acetonitrile (0.26 g, 1.7 mmol) instead of (6-chloropyridin-3-yl)acetonitrile, and (4-benzylphenyl)boronic acid pinacol ester (0.75 g, 2.5 mmol) instead of (4-tert-butylphenyl)boronic acid, the title compound (0.069 g, 0.15 mmol) was afforded as a white solid (yield 8.6%).
MS m/z: 470 (M+H)⁺;
1H-NMR (500 MHz, DMSO-d6) δ: 9.41 (1H, brs), 8.89 (2H, s), 8.29 (2H, d, J=8 Hz), 7.37 (2H, d, J=8 Hz), 7.32-7.18 (5H, m), 4.23 (2H, s), 4.01 (2H, s), 3.96 (2H, d, J=5 Hz), 2.41 (3H, s).

### Example 183

### {[(2-{[5-(4-tert-Butylphenyl)pyridin-2-yl]methyl}-5-hydroxy-6-methylpyrimidin-4-yl)carbonyl]amino} acetic acid

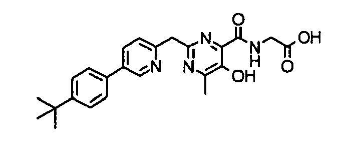

### (1) [5-(4-tert-Butylphenyl)pyridin-2-yl]acetonitrile

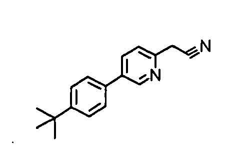

In accordance with Examples 181-(1) to (3), but using 5-bromo-2-methylpyridine (5.0 g, 29 mmol) instead of 2-chloro-5-methylpyrimidine, the title compound (3.5 g, 14 mmol) was afforded as a yellow solid (yield 48%).
1H-NMR (500 MHz, CDCl3) δ: 8.81 (1H, d, J=3 Hz), 7.92 (1H, dd, J=8 Hz, 3 Hz), 7.53 (4H, s), 7.49 (1H, d, J=8 Hz), 3.99 (2H, s), 1.37 (9H, s).

### (2) tert-Butyl 5-(benzyloxy)-2-{[5-(4-tert-butylphenyl)pyridin-2-yl]methyl}-6-hydroxypyrimidine-4-carboxylate

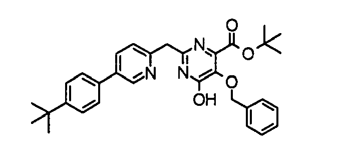

In accordance with Examples 173-(2) and 173-(3), but using [5-(4-tert-butylphenyl)pyridin-2-yl]acetonitrile (5.0 g, 20 mmol) instead of [6-(4-tert-butylphenyl)pyridin-3-yl]acetonitrile, the title compound (3.7 g, 6.9 mmol) was afforded as a brown oil (yield 35%).
MS m/z: 526 (M+H)⁺.

### (3) tert-Butyl 5-(benzyloxy)-2-{[5-(4-tert-butylphenyl)pyridin-2-yl]methyl}-6-methylpyrimidine-4-carboxylate

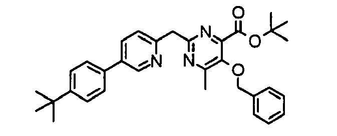

In accordance with Examples 173-(4) and 173-(5), but using tert-butyl 5-(benzyloxy)-2-{[5-(4-tert-butylphenyl)pyridin-2-yl]methyl}-6-hydroxypyrimidine-4-carboxylate (3.7 g, 6.9 mmol) instead of tert-butyl 5-(benzyloxy)-2-{[6-(4-tert-butylphenyl)pyridin-3-yl]methyl}-6-hydroxypyrimidine-4-carboxylate, the title compound (0.27 g, 0.52 mmol) was afforded as a pale yellow solid (yield 7.5%). 1H-NMR (500 MHz, CDCl3) δ: 8.78 (1H, s), 7.80 (1H, m), 7.53-7.48 (4H, m), 7.43-7.35 (6H, m), 5.00 (2H, s), 4.52 (2H, s), 2.46 (3H, s), 1.57 (9H, s), 1.36 (9H, s).

### (4) {[(2-{[5-(4-tert-Butylphenyl)pyridin-2-yl]methyl}-5-hydroxy-6-methylpyrimidin-4-yl)carbonyl]amino}acetic acid

In accordance with Examples 173-(6) and 173-(7), but using tert-butyl 5-(benzyloxy)-2-{[5-(4-tert-butylphenyl)pyridin-2-yl]methyl}-6-methylpyrimidine-4-carboxylate (0.27 g, 0.52 mmol) instead of tert-butyl 5-(benzyloxy)-2-{[6-(4-tert-butylphenyl)pyridin-3-yl]methyl}-6-methylpyrimidine-4-carboxylate, the title compound (0.13 g, 0.30 mmol) was afforded as a pale yellow solid (yield 58%).
MS m/z: 435 (M+H)⁺;
1H-NMR (500 MHz, DMSO-d6) δ; 9.42 (1H, brs), 8.74 (1H, s), 7.98 (1H, d, J=8 Hz), 7.62 (2H, d, J=8 Hz), 7.50 (2H, d, J=8 Hz), 7.42 (1H, d, J=8 Hz), 4.38 (2H, s), 3.98 (2H, d, J=6 Hz), 2.42 (3H, s), 1.31 (9H, s).

### Example 184

### {[(2-{[2-(4-tert-Butylphenyl)-1,3-thiazol-5-yl]methyl}-5-hydroxy-6-methylpyrimidin-4-yl)carbonyl]amino}acetic acid

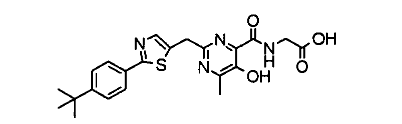

### (1) Methyl 2-(4-tert-butylphenyl)-1,3-thiazole-5-carboxylate

Methyl 2-bromo-1,3-thiazol-5-carboxylate (1.8 g, 7.9 mmol) was dissolved in 1,2-dimethoxyethane (50 mL), and (4-tert-butylphenyl)boronic acid (1.7 g, 9.5 mmol), tri-potassium phosphate hydrate (6.3 g, 24 mmol) and [1,1'-bis(diphenylphosphino)ferrocene]palladium dichloride-dichloromethane adduct (0.64 g, 0.80 mmol) were added at room temperature under a nitrogen atmosphere, followed by heating to reflux for 7 hours. The reaction solution was cooled to room temperature, and the insoluble materials were filtered off. After the filtrate was concentrated under reduced pressure, the resulting residue was purified by silica gel column chromatography (Moritex Corporation, elution solvent: hexane/ethyl acetate), a fraction corresponding to the Rf value=0.80 (hexane/ethyl acetate=2/1) by thin layer chromatography was concentrated under reduced pressure to afford the title compound (1.5 g, 5.3 mmol) as a white solid (yield 67%).
1H-NMR (500 MHz, CDCl3) δ: 8.41 (1H, s), 7.92 (2H, d, J=8 Hz), 7.49 (2H, d, J=8 Hz), 3.93 (3H, s), 1.36 (9H, s).

### (2) [2-(4-tert-Butylphenyl)-1,3-thiazol-5-yl]methanol

Lithium aluminium hydride (0.30 g, 8.0 mmol) was suspended in tetrahydrofuran (100 mL), and a solution of methyl 2-(4-tert-butylphenyl)-1,3-thiazol-5-carboxylate (1.5 g, 5.3 mmol) in tetrahydrofuran (20 mL) was added dropwise, followed by stirring at room temperature for 2 hours. Magnesium sulfate decahydrate (2.0 g) was added to the reaction solution, followed by stirring at room temperature for 30 minutes. The reaction solution was filtered with celite, and the filtrate was concentrated under reduced pressure. The resulting residue was purified by silica gel column chromatography (Moritex Corporation, elution solvent: hexane/ethyl acetate), and a fraction corresponding to the Rf value=0.20 (hexane/ethyl acetate=2/1) by thin layer chromatography was concentrated under reduced pressure to afford the title compound (1.4 g, 5.7 mmol) as a pale yellow oil (quantitative yield).
1H-NMR (500 MHz, CDCl3) δ: 7.86 (2H, d, J=8 Hz), 7.69 (1H, s), 7.46 (2H, d, J=8 Hz), 4.90 (2H, s), 1.35 (9H, s).

### (3) 5-(Bromomethyl)-2-(4-tert-butylphenyl)-1,3-thiazole

[2-(4-tert-Butylphenyl)-1,3-thiazol-5-yl]methanol (1.4 g, 5.7 mmol) was dissolved in methylene chloride (20 mL), and at 0°C carbon tetrabromide (2.0 g, 6.0 mmol) and triphenylphosphine (1.6 g, 6.0 mmol) were added, followed by stirring at room temperature for 2 hours. After the reaction solution was concentrated under reduced pressure, ethyl acetate was added, and the insoluble materials were filtered off. After the filtrate was concentrated under reduced pressure, the resulting residue was purified by silica gel column chromatography (Moritex Corporation, elution solvent: hexane/ethyl acetate), a fraction corresponding to the Rf value=0.80 (hexane/ethyl acetate=3/1) by thin layer chromatography was concentrated under reduced pressure to afford the title compound (1.0 g, 3.3 mmol) as a pale brown oil (yield 59%).
1H-NMR (500 MHz, CDCl3) δ: 7.86 (2H, d, J=8 Hz), 7.79 (1H, s), 7.48 (2H, d, J=8 Hz), 4.76 (2H, s), 1.36 (9H, s).

### (4) [2-(4-tert-Butylphenyl)-1,3-thiazol-5-yl]acetonitrile

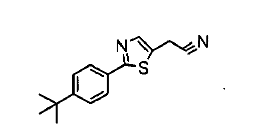

In accordance with Example 178-(5), but using 5-(bromomethyl)-2-(4-tert-butylphenyl)-1,3-thiazole (1.0 g, 3.3 mmol) instead of 3-(bromomethyl)-6-(4-tert-butylphenyl)-2-methylpyridine, the title compound (0.60 g, 2.3 mmol) was afforded as a pale yellow oil (yield 70%).
1H-NMR (500 MHz, CDCl3) δ: 7.84 (2H, d, J=8 Hz), 7.73 (1H, s), 7.48 (2H, d, J=8 Hz), 3.97 (2H, s), 1.36 (9H, s).

### (5) {[(2-{[2-(4-tert-Butylphenyl)-1,3-thiazol-5-yl]methyl}-5-hydroxy-6-methylpyrimidin-4-yl)carbonyl]amino} acetic acid

In accordance with Examples 173-(2) to (7), but using [2-(4-tert-butylphenyl)-1,3-thiazol-5-yl]acetonitrile (0.60 g, 2.3 mmol) instead of [6-(4-tert-butylphenyl)pyridin-3-yl]acetonitrile, the title compound (0.23 g, 0.52 mmol) was afforded as a white solid (yield 23%).
MS m/z: 441 (M+H)⁺;
1H-NMR (500 MHz, DMSO-d6) δ: 12.93 (1H, brs), 12.02 (1H, brs), 9.40 (1H, t, J=6 Hz), 7.82 (2H, d, J=7 Hz), 7.75 (1H, s), 7.50 (2H, d, J=7 Hz), 4.43 (2H, s), 4.02 (2H, d, J=6 Hz), 2.46 (3H, s), 1.30 (9H, s).

### Example 185

### {[(2-{[3-(4-Fluorophenyl)-1H-pyrrol-1-yl]methyl}-5-hydroxy-6-methylpyrimidin-4-yl)carbonyl]amino} acetic acid

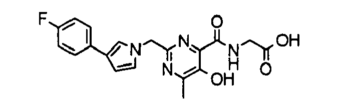

### (1) tert-Butyl 5-(benzyloxy)-2-(chloromethyl)-6-hydroxypyrimidine-4-carboxylate

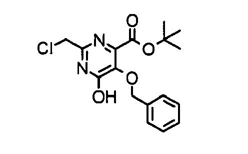

In accordance with Example 1-(4), but using 2-chloroethanimidamide hydrochloride (4.0 g, 31 mmol) instead of tert-butyl 4-(2-amino-2-iminoethyl)piperidine-1-carboxylate acetic acid salt, the title compound (3.8 g, 11 mmol) was afforded as a yellow solid (yield 34%).
MS m/z: 351 (M+H)⁺.

### (2) tert-Butyl 5-(benzyloxy)-2-(chloromethyl)-6-(methoxymethoxy)pyrimidine-4-carboxylate

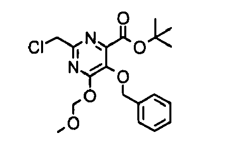

tert-Butyl 5-(benzyloxy)-2-(chloromethyl)-6-hydroxypyrimidine-4-carboxylate (7.0 g, 20 mmol) was dissolved in tetrahydrofuran (100 mL), and at 0°C chloromethyl methyl ether (2.3 mL, 30 mmol) and diisopropylethylamine (7.2 mL, 40 mmol) were added, followed by stirring at the same temperature for 30 minutes. After the reaction solution was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=4/1) by thin layer chromatography was concentrated under reduced pressure to afford the title compound (4.1 g, 10 mmol) as a colorless oil (yield 52%).
1H-NMR (500 MHz, CDCl3) δ: 7.48-7.44 (2H, m), 7.41-7.32 (3H, m), 5.70 (2H, s), 5.15 (2H, s), 4.60 (2H, s), 3.57 (3H, s), 1.54 (9H, s).

### (3) tert-Butyl 5-(benzyloxy)-2-{[3-(4-fluorophenyl)-1H-pyrrol-1-yl]methyl}-6-(methoxymethoxy)pyrimidine-4-carboxylate

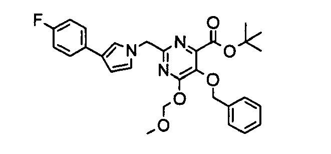

3-(4-Fluorophenyl)-1H-pyrrole (1.5 g, 9.4 mmol) was dissolved in N,N-dimethylformamide (70 mL), and sodium hydride (55%, 0.50 g, 11 mmol) was added, followed by stirring at room temperature for 1 hour. To the reaction solution was added a solution of tert-butyl [5-(benzyloxy)-2-(chloromethyl)-6-(methoxymethoxy)pyrimidine-4-carboxylate (4.1 g, 10 mmol) in N,N-dimethylformamide (35 mL), followed by stirring for 2.5 hours. Saturated aqueous ammonium chloride solution was added to the reaction solution, followed by extraction with ethyl acetate, and the organic layer was washed with water. After the solvent was distilled off under reduced pressure, the resulting residue was purified by silica gel column chromatography (Moritex Corporation, elution solvent: hexane/ethyl acetate), and a fraction corresponding to the Rf value=0.55 (hexane/ethyl acetate=4/1) by thin layer chromatography was concentrated under reduced pressure to afford the title compound (0.60 g, 1.2 mmol) as a yellow oil (yield 12%).
1H-NMR (500 MHz, CDCl3) δ: 7.48-7.40 (3H, m), 7.40-7.32 (4H, m), 7.07-7.03 (1H, m), 7.02-6.96 (2H, m), 6.86-6.80 (1H, m), 6.42-6.35 (1H, m), 5.56 (2H, s), 5.13 (2H, s), 5.11 (2H, s), 3.47 (3H, s), 1.54 (9H, s).

### (4) tert-Butyl 5-(benzyloxy)-2-{[3-(4-fluorophenyl)-1H-pyrrol-1-yl]methyl}-6-hydroxypyrimidine-4-carboxylate

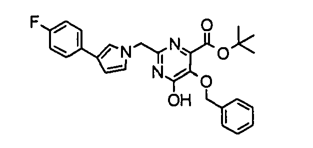

tert-Butyl 5-(benzyloxy)-2-{[3-(4-fluorophenyl)-1H-pyrrol-1-yl]methyl}-6-(methoxymethoxy)pyrimidine-4-carboxylate (0.027 g, 0.052 mmol) was dissolved in ethyl acetate (2.7 mL), and a solution of hydrogen chloride in dioxane (4 M, 0.30 mL, 1.2 mmol) was added, followed by stirring at room temperature for 30 minutes. After the reaction solution was concentrated under reduced pressure, the resulting residue was purified by silica gel column chromatography (Moritex Corporation, elution solvent: hexane/ethyl acetate), and a fraction corresponding to the Rf value=0.35 (hexane/ethyl acetate=1/1) by thin layer chromatography was concentrated under reduced pressure to afford the title compound (0.011 g, 0.023 mmol) as a colorless oil (yield 45%).
1H-NMR (500 MHz, CDCl3) δ: 7.48-7.40 (2H, m), 7.40-7.32 (5H, m), 7.00 (1H, s), 7.00-6.93 (2H, m), 6.82-6.77 (1H, m), 6.45-6.41 (1H, m), 5.26 (2H, s), 5.01 (2H, s), 1.53 (9H, s).

### (5) Ethyl ({[5-(benzyloxy)-2-{[3-(4-fluorophenyl)-1H-pyrrol-1-yl]methyl}-6-methylpyrimidin-4-yl]carbonyl}amino)acetate

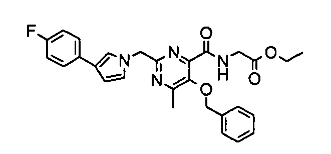

In accordance with Examples 1-(9), 1-(5) and 1-(6), but using tert-butyl 5-(benzyloxy)-2-{[3-(4-fluorophenyl)-1H-pyrrol-1-yl]methyl}-6-hydroxypyrimidine-4-carboxylate (0.31 g, 0.64 mmol) instead of tert-butyl 5-(benzyloxy)-2-{[1-(4-tert-butylphenyl)piperidin-4-yl]methyl}-6-methylpyrimidine-4-carboxylate, the title compound (0.095 g, 0.19 mmol) was afforded as a yellow oil (yield 30%).
1H-NMR (500 MHz, CDCl3) δ: 8.20 (1H, t, J=7 Hz), 7.48-7.43 (4H, m), 7.40-7.32 (3H, m), 7.10-7.05 (1H, m), 7.04-6.95 (2H, m), 6.88-6.82 (1H, m), 6.47-6.41 (1H, m), 5.22 (2H, s), 5.11 (2H, s), 4.24 (2H, q, J=8 Hz), 4.16 (2H, d, J=6 Hz), 2.46 (3H, s), 1.30 (3H, t, J=8 Hz)

### (6) {[(2-{[3-(4-Fluorophenyl)-1H-pyrrol-1-yl]methyl}-5-hydroxy-6-methylpyrimidin-4-yl)carbonyl]amino}acetic acid

In accordance with Examples 1-(10) and 1-(11), but using ethyl ({[5-(benzyloxy)-2-{[3-(4-fluorophenyl)-1H-pyrrol-1-yl]methyl }-6-methylpyrimidin-4-yl]carbonyl}amino)acetate (0.095 g, 0.19 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.050 g, 0.13 mmol) was afforded as a white solid (yield 69%).
MS m/z: 385 (M+H)⁺;
1H-NMR (500 MHz, DMSO-d6) δ: 12.90 (1H, brs), 12.07 (1H, brs), 9.48 (1H, t, J=6 Hz), 7.51 (2H, dd, J=5 Hz, 2 Hz), 7.30 (1H, s), 7.10 (2H, t, J=9 Hz), 6.93 (1H, s), 6.40 (1H, s), 5.19 (2H, s), 4.02 (2H, d, J=6 Hz), 2.44 (3H, s).

### Example 186

### {[(2-{[1-(4-Fluorophenyl)-4-methylpiperidin-4-yl]methyl}-5-hydroxy-6-methylpyrimidin-4-yl)carbonyl]amino}acetic acid

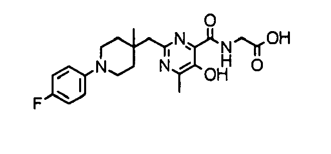

### (1) Ethyl cyano [1-(4-fluorophenyl)piperidin-4-ylidene]acetate

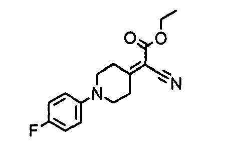

1-(4-Fluorophenyl)piperidin-4-one (3.7 g, 19 mmol) obtained in Example 5-(1), ethyl cyanoacetate (3.1 mL, 29 mmol) and ammonium acetate (0.15 g, 1.9 mmol) were dissolved in toluene (40 mL), followed by heating to reflux for 3 hours. Ethyl cyanoacetate (0.50 mL) was added to the reaction solution, followed by heating to reflux for a further 2 hours. After the reaction solution was concentrated under reduced pressure, the resulting residue was purified by silica gel column chromatography (Moritex Corporation, elution solvent: hexane/ethyl acetate), and a fraction corresponding to the Rf value=0.56 (hexane/ethyl acetate=5/1) by thin layer chromatography was concentrated under reduced pressure to afford the title compound (4.7 g, 16 mmol) as a yellow solid (yield 85%).
1H-NMR (500 MHz, CDCl3) δ: 6.98 (2H, t, J=8 Hz), 6.90-6.88 (2H, m), 4.30 (2H, q, J=7 Hz), 3.38 (2H, t, J=6 Hz), 3.33-3.27 (4H, m), 2.91 (2H, t, J=6 Hz), 1.36 (3H, t, J=7 Hz).

### (2) Ethyl cyano[1-(4-fluorophenyl)-4-methylpiperidin-4-yl]acetate

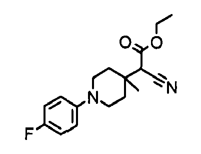

Copper(I) cyanide (2.9 g, 33 mmol) was dissolved in tetrahydrofuran (130 mL), and at -78°C a solution of (methyl)magnesium iodide in tetrahydrofuran (3.0 M, 22 mL, 66 mmol) was added dropwise over 15 minutes, followed by stirring while raising the temperature to 0°C over 1 hour. The reaction solution was cooled to -50°C, and a solution (15 mL) of ethyl cyano[1-(4-fluorophenyl)piperidin-4-ylidene]acetate (4.7 g, 16 mmol) in tetrahydrofuran was added dropwise over 20 minutes, followed by stirring for 1 hour. To the reaction solution were added aqueous ammonium chloride solution and ethyl acetate, followed by being filtered with celite. After the filtrate was extracted with ethyl acetate, the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.43 (hexane/ethyl acetate=5/1) by thin layer chromatography was concentrated under reduced pressure to afford the title compound (4.0 g, 13 mmol) as an oil (yield 80%).
1H-NMR (500 MHz, CDCl3) δ: 6.96 (2H, t, J=8 Hz), 6.90-6.87 (2H, m), 4.30-4.25 (2H, m), 3.50 (1H, s), 3.33-3.24 (2H, m), 3.05-2.96 (2H, m), 1.96-1.89 (2H, m), 1.84-1.79 (1H, m), 1.71-1.67 (1H, m), 1.34 (3H, t, J=7 Hz), 1.24 (3H, s).

### (3) [1-(4-Fluorophenyl)-4-methylpiperidin-4-yl]acetonitrile

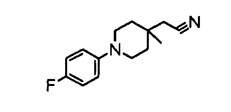

A solution of ethyl cyano[1-(4-fluorophenyl)-4-methylpiperidin-4-yl]acetate (4.0 g, 13 mmol) and lithium chloride (0.78 g, 18 mmol) in a mixture of dimethyl sulfoxide (39 mL) and water (0.46 mL) was stirred at 160°C for 2 hours. Water was poured into the reaction solution and the mixture was extracted twice with diethyl ether, and the organic layer was washed twice with aqueous sodium chloride solution, followed by being dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.43 (hexane/ethyl acetate=5/1) by thin layer chromatography was concentrated under reduced pressure to afford the title compound (2.6 g, 11 mmol) as an oil (yield 86%).
1H-NMR (500 MHz, CDCl3) δ: 6.96 (2H, t, J=8 Hz), 6.90-6.87 (2H, m), 3.18-3.14 (2H, m), 3.07-3.02 (2H, m), 2.35 (2H, s), 1.77-1.72 (2H, m), 1.70-1.65 (2H, m), 1.19 (3H, s).

### (4) tert-Butyl 5-(benzyloxy)-2-{[1-(4-fluorophenyl)-4-methylpiperidin-4-yl]methyl}-6-hydroxypyrimidine-4-carboxylate

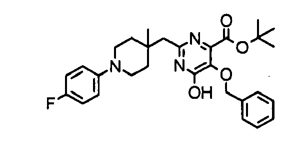

In accordance with Examples 1-(3) and 1-(4), but using [1-(4-fluorophenyl)-4-methylpiperidin-4-yl]acetonitrile (2.6 g, 11 mmol) instead of tert-butyl 4-(cyanomethyl)piperidine-1-carboxylate, the title compound (2.2 g, 4.3 mmol) was afforded as a solid (yield 39%).
1H-NMR (500 MHz, CDCl3) δ: 7.46 (2H, d, J=6 Hz), 7.36-7.31 (3H, m), 6.93 (2H, t, J=8 Hz), 6.87-6.85 (2H, m), 5.24 (2H, s), 3.27-3.23 (2H, m), 3.00-2.95 (2H, m), 2.67 (2H, d, J=2 Hz), 1.86-1.77 (2H, m), 1.61-1.55 (2H, m), 1.53 (9H, s), 1.09 (3H, s).

### (5) tert-Butyl 5-(benzyloxy)-2-{[1-(4-fluorophenyl)-4-methylpiperidin-4-yl]methyl}-6-{[(trifluoromethyl)sulfonyl]oxy}pyrimidine-4-carboxylate

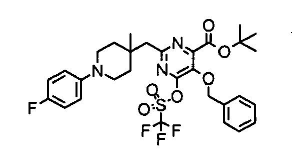

In accordance with Example 1-(5), but using tert-butyl 5-(benzyloxy)-2-{[1-(4-fluorophenyl)-4-methylpiperidin-4-yl]methyl}-6-hydroxypyrimidine-4-carboxylate (2.2 g, 4.3 mmol) instead of tert-butyl 5-(benzyloxy)-2-{[1-(tert-butoxycarbonyl)piperidin-4-yl]methyl}-6-hydroxypyrimidine-4-carboxylate, the title compound (2.6 g, 4.0 mmol) was afforded as a white solid (yield 93%).
1H-NMR (500 MHz, CDCl3) δ: 7.42-7.36 (5H, m), 6.96-6.88 (4H, m), 5.01 (2H, s), 3.30-3.25 (2H, m), 3.07-3.02 (2H, m), 2.95 (2H, s), 1.82-1.77 (2H, m), 1.61-1.57 (2H, m), 1.59 (9H, s), 1.03 (3H, s).

### (6) Ethyl {[(2-{[1-(4-fluorophenyl)-4-methylpiperidin-4-yl]methyl}-5-hydroxy-6-methylpyrimidin-4-yl)carbonyl]amino} acetate

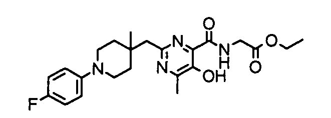

tert-Butyl 5-(benzyloxy)-2-{[1-(4-fluorophenyl)-4-methylpiperidin-4-yl]methyl}-6-{[(trifluoromethyl)sulfonyl]oxy}pyrimidine-4-carboxylate (2.6 g, 4.0 mmol) was dissolved in tetrahydrofuran (90 mL), and at room temperature methylboronic acid (0.36 g, 6.0 mmol), silver oxide (2.3 g, 10 mmol), potassium carbonate (1.7 g, 12 mmol) and [1,1'-bis(diphenylphosphino)ferrocene]palladium dichloride-dichloromethane adduct (0.33 g, 0.40 mmol) were added, followed by heating to reflux for 3 hours under a nitrogen atmosphere. The reaction solution was cooled to room temperature, followed by addition of water and ethyl acetate, and the insoluble materials were filtered off. After the filtrate was extracted with ethyl acetate, the organic layer was dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.53 (hexane/ethyl acetate=3/1) by thin layer chromatography was concentrated under reduced pressure to afford tert-butyl 5-(benzyloxy)-2-{[1-(4-fluorophenyl)-4-methylpiperidin-4-yl]methyl}-6-methylpyrimidine-4-carboxylate (2.1 g) as an amorphous solid.

This was dissolved in a mixture of tetrahydrofuran (8 mL) and methanol (12 mL), and aqueous sodium hydroxide solution (5 M, 4.9 mL, 25 mmol) was added, followed by stirring at 60°C for 2 hours. The reaction solution was cooled to room temperature and concentrated under reduced pressure, and water and hydrochloric acid (5 M, 4.9 mL) were added to the resulting residue, followed by extraction with ethyl acetate. The organic layer was concentrated under reduced pressure, and toluene was added to the resulting residue, followed by concentration under reduced pressure.

The resulting residue was dissolved in N,N-dimethylformamide (20 mL), and glycine ethyl ester hydrochloride (0.85 g, 6.1 mmol), (1H-benzotriazol-1-yloxy)(tripyrrolidin-1-yl)phosphonium hexafluorophosphate (PyBOP) (3.2 g; 6.1 mmol) and diisopropylethylamine (2.1 mL, 12 mmol) were added, followed by stirring at room temperature for 15 hours. Water was added to the reaction solution, followed by extraction with ethyl acetate, and the organic layer was dried over magnesium sulfate. After the solvent was distilled off under reduced pressure, the resulting residue was purified by silica gel column chromatography (Moritex Corporation, elution solvent: hexane/ethyl acetate), and a fraction corresponding to the Rf value=0.58 (hexane/ethyl acetate=2/1) by thin layer chromatography was concentrated under reduced pressure to afford the title compound (1.3 g, 3.0 mmol) as a white solid (yield 73%).
1H-NMR (500 MHz, CDCl3) δ: 11.33 (1H, s), 8.48 (1H, t, J=5 Hz), 6.97-6.90 (4H, m), 4.29 (2H, q, J=7 Hz), 4.22 (2H, d, J=5 Hz), 3.29-3.25 (2H, m), 3.09-3.05 (2H, m), 2.89 (2H, s), 2.52 (3H, s), 1.79-1.74 (2H, m), 1.59-1.55 (2H, m), 1.33 (3H, t, J=7 Hz), 1.02 (3H, s).

### (7) {[(2-{[1-(4-Fluorophenyl)-4-methylpiperidin-4-yl]methyl}-5-hydroxy-6-methylpyrimidin-4-yl)carbonyl]amino}acetic acid

In accordance with Example 1-(11), but using ethyl {[(2-{[1-(4-fluorophenyl)-4-methylpiperidin-4-yl]methyl}-5-hydroxy-6-methylpyrimidin-4-yl)carbonyl]amino}acetate (1.3 g, 3.0 mmol) instead of ethyl {[(2-{[1-(4-tert-butylphenyl)piperidin-4-yl]methyl} -5-hydroxy-6-methylpyrimidin-4-yl)carbonyl]amino} acetate, the title compound (1.1 g, 2.7 mmol) was afforded as a white solid (yield 89%).
mp 140-141 °C;
MS m/z: 417 (M+H)⁺;
Anal Calcd for C21H25FN4O4: C, 60.57; H, 6.05; N, 13.45. Found: C, 60.41; H, 6.13; N, 13.28;
1H-NMR (500 MHz, DMSO-d6) δ:12.88 (1H, s), 11.88 (1H, s), 9.28 (1H, t, J=5 Hz), 7.02 (2H, t, J=8 Hz), 6.96-6.93 (2H, m), 4.02 (2H, d, J=6 Hz), 3.30-3.25 (2H, m), 3.04-3.00 (2H, m), 2.84 (2H, s), 2.43 (3H, s), 1.68-1.64 (2H, m), 1.49-1.44 (2H, m), 0.97 (3H, s).

### Example 187

### {[(2-{2-[1-(4-tert-Butylphenyl)piperidin-4-yl]ethyl}-5-hydroxy-6-methylpyrimidin-4-yl)carbonyl]amino}acetic acid

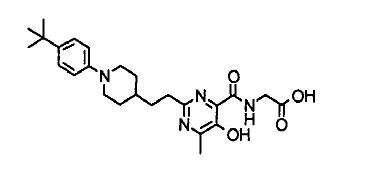

### (1) tert-Butyl 4-(2-cyanoethyl)piperidine-1-carboxylate

In accordance with Example 1-(1), but using tert-butyl 4-formylpiperidine-1-carboxylate (8.8 g, 41 mmol) instead of tert-butyl 4-oxopiperidine-1-carboxylate, a mixture (8.7 g, 37 mmol) of tert-butyl 4-[(E)-2-cyanovinyl]piperidine-1-carboxylate and tert-butyl 4-[(Z)-2-cyanovinyl]piperidine-1-carboxylate was afforded as a colorless oil (yield 75%).

In accordance with Example 1-(2), but using a mixture (4.4 g, 19 mmol) of tert-butyl 4-[(E)-2-cyanovinyl]piperidine-1-carboxylate and tert-butyl 4-[(Z)-2-cyanovinyl]piperidine-1-carboxylate instead of tert-butyl 4-(cyanomethylene)piperidine-1-carboxylate, the title compound (4.4 g, 18 mmol) was afforded as a colorless oil (yield 99%).
1H-NMR (500 MHz, CDCl3) δ: 4.20-4.05 (2H, m), 2.78-2.64 (2H, m), 2.39 (2H, t, J=6 Hz), 1.89-1.75 (5H, m), 1.46 (9H, s), 1.32-1.21 (2H, m).

### (2) tert-Butyl 4-(3-amino-3-iminopropyl)piperidine-1-carboxylate acetic acid salt

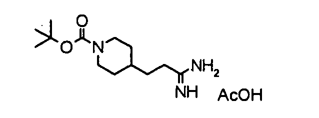

In accordance with Example 1-(3), but using tert-butyl 4-(2-cyanoethyl)piperidine-1-carboxylate (4.4 g, 18 mmol) instead of tert-butyl 4-(cyanomethyl)piperidine-1-carboxylate, the title compound (4.4 g, 14 mmol) was afforded as a white solid (yield 76%).
MS m/z: 256 (M+H)⁺.

### (3) tert-ButylS-(benzyloxy)-2-{2-[1-(tert-butoxycarbonyl)piperidin-4-yl]ethyl}-6-hydroxypyrimidine-4-carboxylate

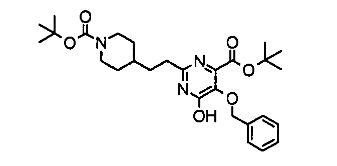

In accordance with Example 1-(4), but using tert-butyl 4-(3-amino-3-iminopropyl)piperidine-1-carboxylate acetic acid salt (4.4 g, 14 mmol) instead of tert-butyl 4-(2-amino-2-iminoethyl)piperidine-1-carboxylate acetic acid salt, the title compound (6.6 g, 13 mmol) was afforded as a yellow solid (yield 94%).
1H-NMR (500 MHz, CDCl3) δ: 7.48-7.44 (2H, m), 7.40-7.31 (3H, m), 5.24 (2H, s), 4.24-3.95 (2H, m), 2.72-2.65 (2H, m), 2.65-2.54 (2H, m), 1.80-1.71 (2H, m), 1.69-1.60 (3H, m), 1.52 (9H, s), 1.45 (9H, s), 1.28-1.16 (2H, m).

### (4) tert-Butyl 5-(benzyloxy)-2-{2-[1-(tert-butoxycarbonyl)piperidin-4-yl]ethyl}-6-methylpyrimidine-4-carboxylate

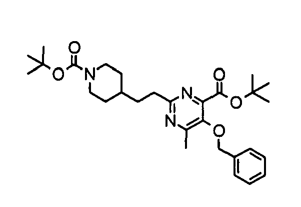

In accordance with Examples 1-(5) and 1-(6), but using tert-butyl 5-(benzyloxy)-2-{2-[1-(tert-butoxycarbonyl)piperidin-4-yl]ethyl}-6-hydroxypyrimidine-4-carboxylate (6.6 g, 13 mmol) instead of tert-butyl 5-(benzyloxy)-2-{[1-(tert-butoxycarbonyl)piperidin-4-yl]methyl}-6-hydroxypyrimidine-4-carboxylate, the title compound (3.5 g, 6.8 mmol) was afforded as a yellow oil (yield 53%).
1H-NMR (500 MHz, CDCl3) δ: 7.44-7.34 (5H, m), 4.99 (2H, s), 4.17-3.98 (2H, m), 2.93 (2H, t, J=7 Hz), 2.77-2.64 (2H, m), 2.44 (3H, s), 1.81-1.69 (5H, m), 1.58 (9H, s), 1.46 (9H, s), 1.30-1.19 (2H, m).

### (5) tert-Butyl 5-(benzyloxy)-6-methyl-2-[2-(piperidin-4-yl)ethyl]pyrimidine-4-carboxylate hydrochloride

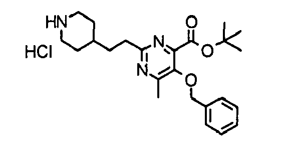

In accordance with Example 1-(7), but using tert-butyl 5-(benzyloxy)-2-{2-[1-(tert-butoxycarbonyl)piperidin-4-yl]ethyl} -6-methylpyrimidine-4-carboxylate (3.5 g, 6.8 mmol) instead of tert-butyl 5-(benzyloxy)-2-{[1-(tert-butoxycarbonyl)piperidin-4-yl]methyl}-6-methylpyrimidine-4-carboxylate, a crude product (3.1 g, 6.8 mmol) of the title compound was afforded (quantitative yield).
MS m/z: 412 (M+H)⁺.

### (6) {[(2-{2-[1-(4-tert-Butylphenyl)piperidin-4-yl]ethyl}-5-hydroxy-6-methylpyrimidin-4-yl)carbonyl]amino}acetic acid

In accordance with Examples 1-(8), 1-(9), 6-(2) and 1-(11), but using tert-butyl 5-(benzyloxy)-6-methyl-2-[2-(piperidin-4-yl)ethyl]pyrimidine-4-carboxylate hydrochloride (3.1 g, 6.8 mmol) instead of tert-butyl 5-(benzyloxy)-6-methyl-2-[(piperidin-4-yl)methyl]pyrimidine-4-carboxylate hydrochloride, the title compound (0.51 g, 1.1 mmol) was afforded as a white solid (yield 16%).
MS m/z: 455 (M+H)⁺;
1H-NMR (500 MHz, DMSO-d6) δ: 12.88 (1H, s), 11.90 (1H, s), 9.38 (1H, t, J=6 Hz), 7.28-7.16 (2H, m), 6.91-6.81 (2H, m), 4.00 (2H, d, J=6 Hz), 3.66-3.56 (2H, m), 2.91-2.81 (2H, m), 2.65-2.54 (2H, m), 2.44 (3H, s), 2.06-2.01 (1H, m), 1.88-1.70 (4H, m), 1.40-1.30 (2H, m), 1.25 (9H, s).

### Example 188

### {[(2-{3-[1-(4-tert-Butylphenyl)piperidin-4-yl]propyl}-5-hydroxy-6-methylpyrimidin-4-yl)carbonyl]amino}acetic acid

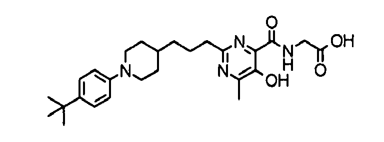

### (1) tert-Butyl 4-(3-cyanopropyl)piperidine-l-carboxylate

In accordance with Example 1-(1), but using tert-butyl 4-(2-oxoethyl)piperidine-1-carboxylate (20 g, 88 mmol) instead of tert-butyl 4-oxopiperidine-1-carboxylate, a mixture (13 g, 51 mmol) of tert-butyl 4-[(2E)-3-cyanoprop-2-en-1-yl]piperidine-1-carboxylate and tert-butyl 4-[(2Z)-3-cyanoprop-2-en-1-yl]piperidine-1-carboxylate was afforded as a colorless oil (yield 58%).

In accordance with Example 1-(2), but using a mixture (6.4 g, 26 mmol) of tert-butyl 4-[(2E)-3 -cyanoprop-2-en-1-yl]piperidine-1-carboxylate and tert-butyl 4-[(2Z)-3-cyanoprop-2-en-1-yl]piperidine-1-carboxylate instead of tert-butyl 4-(cyanomethylene)piperidine-1-carboxylate, the title compound (6.4 g, 26 mmol) was afforded as a colorless oil (yield 99%).
1H-NMR (500 MHz, CDCl3) δ: 4.20-4.05 (2H, m), 2.78-2.64 (2H, m), 2.35 (2H, t, J=6 Hz), 1.76-1.64 (5H, m), 1.46 (9H, s), 1.45-1.35 (2H, m), 1.17-1.07 (2H, m).

### (2) tert-Butyl 4-(4-amino-4-iminobutyl)piperidine-1-carboxylate acetic acid salt

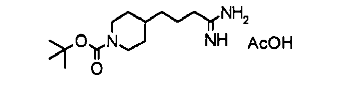

In accordance with Example 1-(3), but using tert-butyl 4-(3-cyanopropyl)piperidine-1-carboxylate (6.4 g, 26 mmol) instead of tert-butyl 4-(cyanomethyl)piperidine-1-carboxylate, the title compound (6.3 g, 19 mmol) was afforded as a white solid (yield 74%).
MS m/z: 270 (M+H)⁺.

### (3) tert-Butyl 5-(benzyloxy)-2-{3-[1-(tert-butoxycarbonyl)piperidin-4-yl]propyl}-6-hydroxypyrimidine-4-carboxylate

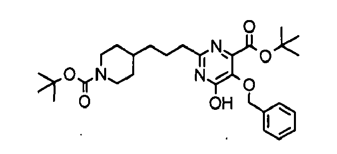

In accordance with Example 1-(4), but using tert-butyl 4-(4-amino-4-iminobutyl)piperidine-1-carboxylate acetic acid salt (6.3 g, 19 mmol) instead of tert-butyl 4-(2-amino-2-iminoethyl)piperidine-1-carboxylate acetic acid salt, the title compound (7.8 g, 15 mmol) was afforded as a yellow solid (yield 78%).
1H-NMR (500 MHz, CDCl3) δ: 7.48-7.44 (2H, m), 7.40-7.31 (3H, m), 5.24 (2H, s), 4.15-3.95 (2H, m), 2.70-2.55 (4H, m), 1.80-1.71 (2H, m), 1.69-1.60 (3H, m), 1.52 (9H, s), 1.45 (9H, s), 1.41-1.28 (2H, m), 1.28-1.16 (2H, m).

### (4) tert-Butyl 5-(benzyloxy)-2-{3-[1-(tert-butoxycarbonyl)piperidin-4-yl]propyl}-6-methylpyrimidine-4-carboxylate

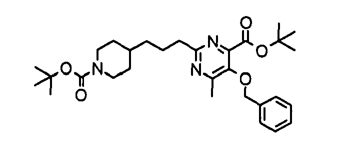

In accordance with Examples 1-(5) and 1-(6), but using tert-butyl 5-(benzyloxy)-2- {3-[1-(tert-butoxycarbonyl)piperidin-4-yl]propyl}-6-hydroxypyrimidine-4-carboxylate (7.8 g, 15 mmol) instead of tert-butyl 5-(benzyloxy)-2-{[1-(tert-butoxycarbonyl)piperidin-4-yl]methyl}-6-hydroxypyrimidine-4-carboxylate, the title compound (3.4 g, 6.5 mmol) was afforded as a yellow oil (yield 44%).
1H-NMR (500 MHz, CDC13) δ: 7.44-7.34 (5H, m), 4.99 (2H, s), 4.13-3.94 (2H, m), 2.89 (2H, t, J=7 Hz), 2.75-2.61 (2H, m), 2.44 (3H, s), 1.87-1.77 (2H, m), 1.72-1.62 (3H, m), 1.58 (9H, s), 1.46 (9H, s), 1.40-1.29 (2H, m), 1.13-1.04 (2H, m).

### (5) tert-Butyl 5-(benzyloxy)-6-methyl-2-[3-(piperidin-4-yl)propyl]pyrimidine-4-carboxylate hydrochloride

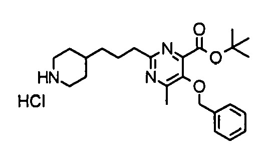

In accordance with Example 1-(7), but using tert-butyl 5-(benzyloxy)-2-{3-[1-(tert-butoxycarbonyl)piperidin-4-yl]propyl}-6-methylpyrimidine-4-carboxylate (3.4 g, 6.5 mmol) instead of tert-butyl 5-(benzyloxy)-2-{[1-(tert-butoxycarbonyl)piperidin-4-yl]methyl}-6-methylpyrimidine-4-carboxylate, a crude product (3.0 g, 6.5 mmol) of the title compound was afforded (quantitative yield).
MS m/z: 426 (M+H)⁺.

### (6) {[(2-{3-[1-(4-tert-Butylphenyl)piperidin-4-yl]propyl}-5-hydroxy-6-methylpyrimidin-4-yl)carbonyl]amino}acetic acid

In accordance with Examples 1-(8), 1-(9), 6-(2) and 1-(11), but using tert-butyl 5-(benzyloxy)-6-methyl-2-[3-(piperidin-4-yl)propyl]pyrimidine-4-carboxylate hydrochloride (3.0 g, 6.5 mmol) instead of tert-butyl 5-(benzyloxy)-6-methyl-2-[(piperidin-4-yl)methyl]pyrimidine-4-carboxylate hydrochloride, the title compound (1.2 g, 2.5 mmol) was afforded as a white solid (yield 39%).
MS m/z: 469 (M+H)⁺;
1H-NMR (500 MHz, DMSO-d6) δ: 9.23 (1H, t, J=5 Hz), 7.20 (2H, d, J=9 Hz), 6.84 (2H, d, J=9 Hz), 3.87 (2H, d, J=5 Hz), 3.60 (2H, d, J=12 Hz), 2.80 (2H, t, J=7 Hz), 2.56 (2H, t, J=12 Hz), 2.43 (3H, s), 1.85-1.72 (2H, m), 1.44-1.33 (1H, m), 1.32 (2H, dq, J=12 Hz, 3 Hz), 1.28-1.16 (4H, m), 1.23 (9H, s).

### Example 189

### {[(2-{4-[1-(4-tert-Butylphenyl)piperidin-4-yl]butyl}-5-hydroxy-6-methylpyrimidin-4-yl)carbonyl]amino} acetic acid

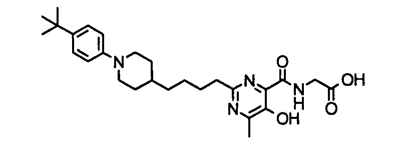

### (1) tert-Butyl 4-(4-cyanobutyl)piperidine-1-carboxylate

In accordance with Example 1-(1), but using tert-butyl 4-(3-oxopropyl)piperidine-1-carboxylate (5.2 g, 21 mmol) instead of tert-butyl 4-oxopiperidine-1-carboxylate, a mixture (4.5 g, 17 mmol) of tert-butyl 4-[(3E)-4-cyanobut-3-en-1-yl]piperidine-1-carboxylate and tert-butyl 4-[(3Z)-4-cyanobut-3-en-l-yl]piperidine-1-carboxylate was afforded as a colorless oil (yield 80%).

In accordance with Example 1-(2), but using a mixture (2.3 g, 8.6 mmol) of tert-butyl 4-[(3E)-4-cyanobut-3-en-1-yl]piperidine-1-carboxylate and tert-butyl 4-[(3Z)-4-cyanobut-3-en-1-yl]piperidine-1-carboxylate instead of tert-butyl 4-(cyanomethylene)piperidine-1-carboxylate, the title compound (2.3 g, 8.6 mmol) was afforded as a colorless oil (yield 99%).
1H-NMR (500 MHz, CDCl3) δ: 4.20-4.05 (2H, m), 2.78-2.64 (2H, m), 2.36 (2H, t, J=6 Hz), 1.70-1.61 (5H, m), 1.51-1.42 (2H, m), 1.46 (9H, s), 1.45-1.35 (2H, m), 1.14-1.02 (2H, m).

### (2) tert-Butyl 4-(5-amino-5-iminopentyl)piperidine-l-carboxylate acetic acid salt

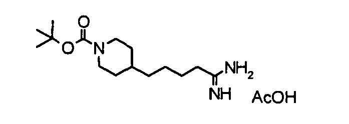

In accordance with Example 1-(3), but using tert-butyl 4-(4-cyanobutyl)piperidine-1-carboxylate (2.3 g, 8.6 mmol) instead of tert-butyl 4-(cyanomethyl)piperidine-1-carboxylate, the title compound (2.1 g, 6.0 mmol) was afforded as a white solid (yield 70%).
MS m/z: 284 (M+H)⁺.

### (3) tert-Butyl 5-(benzyloxy)-2-{4-[1-(tert-butoxycarbonyl)piperidin-4-yl]butyl}-6-hydroxypyrimidine-4-carboxylate

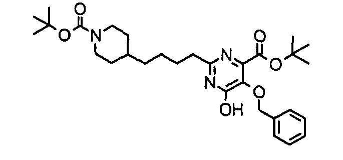

In accordance with Example 1-(4), but using tert-butyl 4-(5-amino-5-iminopentyl)piperidine-1-carboxylate acetic acid salt (2.1 g, 6.0 mmol) instead of tert-butyl 4-(2-amino-2-iminoethyl)piperidine-1-carboxylate acetic acid salt, the title compound (2.8 g, 5.2 mmol) was afforded as a yellow solid (yield 87%).
1H-NMR (500 MHz, CDCl3) δ: 7.48-7.44 (2H, m), 7.40-7.31 (3H, m), 5.24 (2H, s), 4.15-3.95 (2H, m), 2.70-2.55 (2H, m), 2.66 (2H, t, J=8 Hz), 1.80-1.71 (2H, m), 1.69-1.60 (3H, m), 1.52 (9H, s), 1.45 (9H, s), 1.41-1.28 (2H, m), 1.28-1.16 (2H, m), 1.15-1.00 (2H, m).

### (4) tert-Butyl 5-(benzyloxy)-2-{4-[1-(tert-butoxycarbonyl)piperidm-4-yl]butyl}-6-methylpyrimidine-4-carboxylate

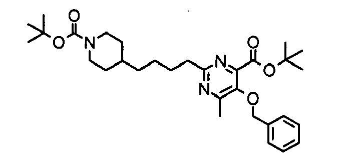

In accordance with Examples 1-(5) and 1-(6), but using tert-butyl 5-(benzyloxy)-2-{4-[1-(tert-butoxycarbonyl)piperidin-4-yl]butyl}-6-hydroxypyrimidine-4-carboxylate (2.8 g, 5.2 mmol) instead of tert-butyl 5-(benzyloxy)-2-{[1-(tert-butoxycarbonyl)piperidin-4-yl]methyl}-6-hydroxypyrimidine-4-carboxylate, the title compound (1.7 g, 3.1 mmol) was afforded as a yellow oil (yield 60%).
1H-NMR (500 MHz, CDC13) δ: 7.44-7.34 (5H, m), 4.99 (2H, s), 4.13-3.94 (2H, m), 2.91 (2H, t, J=7 Hz), 2.75-2.61 (2H, m), 2.45 (3H, s), 1.84-1.73 (2H, m), 1.70-1.58 (3H, m), 1.58 (9H, s), 1.46 (9H, s), 1.44-1.33 (2H, m), 1.33-1.22 (2H, m), 1.13-1.04 (2H, m).

### (5) tert-Butyl 5-(benzyloxy)-6-methyl-2-[4-(pipendin-4-yl)butyl]pyrimidine-4-carboxylate hydrochloride

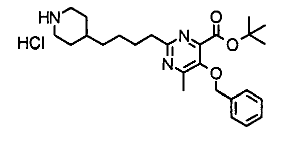

In accordance with Example 1-(7), but using tert-butyl 5-(benzyloxy)-2-{4-[1-(tert-butoxycarbonyl)piperidin-4-yl]butyl}-6-methylpyrimidine-4-carboxylate (1.7 g, 3.1 mmol) instead of tert-butyl 5-(benzyloxy)-2-{[1-(tert-butoxycarbonyl)piperidin-4-yl]methyl}-6-methylpyrimidine-4-carboxylate, a crude product (1.5 g, 3.1 mmol) of the title compound was afforded (quantitative yield).
MS m/z: 440 (M+H)⁺.

### (6) {[(2-{4-[1-(4-tert-Butylphenyl)piperidin-4-yl]buty}-5-hydroxy-6-methylpyrimidin-4-yl)carbonyl]amino}acetic acid

In accordance with Examples 1-(8), 1-(9), 6-(2) and 1-(11), but using tert-butyl 5-(benzyloxy)-6-methyl-2-[4-(piperidin-4-yl)butyl]pyrimidine-4-carboxylate hydrochloride (1.5 g, 3.1 mmol) instead of tert-butyl 5-(benzyloxy)-6-methyl-2-[(piperidin-4-yl)methyl]pyrimidine-4-carboxylate hydrochloride, the title compound (0.11 g, 0.22 mmol) was afforded as a white solid (yield 7.1%).
MS m/z: 483 (M+H)⁺;
1H-NMR (500 MHz, DMSO-d6) δ: 9.25 (1H, t, J=3 Hz), 7.19 (2H, d, J=8 Hz), 6.84 (2H, d, J=8 Hz), 3.87 (2H, d, J=3 Hz), 3.59 (2H, d, J=12 Hz), 2.80 (2H, t, J=7 Hz), 2.56 (2H, t, J=12 Hz), 2.42 (3H, s), 1.80-1.65 (3H, m), 1.40-1.31 (2H, m), 1.30-1.25 (2H, m), 1.24-1.13 (4H, m), 1.23 (9H, s).

### Example 190

### (S)-2- {[(2-{[1-(4-tert-butylphenyl)piperidin-4-yl]methyl}-5-hydroxy-6-methylpyrimidin-4-yl)carbonyl]amino} propionic acid

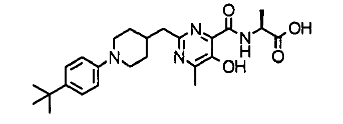

In accordance with Examples 1-(9) to 1-(11), but usingt L-alanine ethyl ester hydrochloride (0.25 g, 1.6 mmol) instead of glycine ethyl ester hydrochloride (0.27 g, 1.9 mmol), the title compound (0.20 g, 0.45 mmol) was afforded as a pale yellowish white solid (yield 42%).
MS m/z: 455 (M+H)⁺;
1H-NMR (500 MHz, DMSO-d6) δ: 9.26 (1H, d, J=7 Hz), 7.20 (2H, dd, J=9 Hz, 1 Hz), 6.84 (2H, dd, J=9 Hz, 1 Hz), 4.44-4.36 (1H, m), 3.60 (2H, d, J=12 Hz), 2.78 (2H, d, J=7 Hz), 2.59 (2H, t, J=12 Hz), 2.43 (3H, s), 2.07-1.96 (1H, m), 1.67 (2H, d, J=12 Hz), 1.44 (3H, d, J=7 Hz), 1.36 (2H, dq, J=12 Hz, 3 Hz), 1.23 (9H, s).

### Example 191

### [({5-Hydroxy-6-(methylsulfanyl)-2-[(1-phenylpiperidin-4-yl)methyl]pyrimidin-4-yl}carbonyl)amino] acetic acid

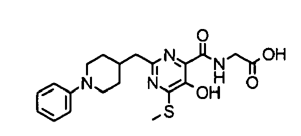

### (1) Ethyl ({[5-(benzyloxy)-2-[(1-phenylpiperidin-4-yl)methyl]-6-{[(trifluoromethyl)sulfonyl]oxy}pyrimidin-4-yl]carbonyl}amino)acetate

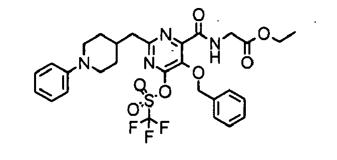

tert-Butyl 5-(benzyloxy)-6-hydroxy-2-[(1-phenylpiperidin-4-yl)methyl]pyrimidine-4-carboxylate (1.9 g, 4.0 mmol) obtained in Example 2-(3) was dissolved in a mixture of tetrahydrofuran (20 mL) and methanol (20 mL), and aqueous sodium hydroxide solution (8 M, 6.0 mL, 48 mmol) was added, followed by stirring at 60°C for 4 hours. After the reaction solution was cooled to room temperature and concentrated under reduced pressure, hydrochloric acid was added to the resulting residue, and subsequently the precipitated solid was collected by filtration to afford 5-(benzyloxy)-6-hydroxy-2-[(1-phenylpiperidin-4-yl)methyl]pyrimidine-4-carboxylic acid (1.6 g, 3.9 mmol) as a white solid (yield 97%).

This was dissolved in N,N-dimethylformamide (50 mL), and 1,1'-carbonylbis(1H-imidazole) (0.76 g, 4.7 mmol) and diisopropylethylamine (2.0 mL, 12 mmol) were added, followed by stirring at room temperature for 30 minutes under a nitrogen atmosphere. Glycine ethyl ester hydrochloride (0.81 g, 5.8 mmol) was added to the reaction solution, followed by stirring at room temperature for 17.5 hours. After the reaction solution was concentrated under reduced pressure, ethyl acetate was added to the resulting residue, and subsequently the organic layer was washed sequentially with hydrochloric acid and saturated aqueous sodium hydrogencarbonate solution, followed by being dried over sodium sulfate. After the solvent was distilled off under reduced pressure, the resulting residue was purified by silica gel column chromatography (Biotage, Inc., elution solvent: hexane/ethyl acetate), and a fraction corresponding to the Rf value=0.50 (ethyl acetate) by thin layer chromatography was concentrated under reduced pressure to afford ethyl [({5-(benzyloxy)-6-hydroxy-2-[(1-phenylpiperidin-4-yl)methyl]pyrimidin-4-yl}carbonyl)amino]acetate (1.5 g, 3.1 mmol) (yield 79%).

This was dissolved in methylene chloride (50 mL), and at -78°C trifluoromethanesulfonic anhydride (0.62 mL, 3.7 mmol) and triethylamine (0.64 mL, 4.6 mmol) were added, followed by stirring at the same temperature for 30 minutes. Water was poured into the reaction solution, followed by extraction with methylene chloride, and subsequently the organic layer was dried over sodium sulfate. After the solvent was distilled off under reduced pressure, the resulting residue was purified by silica gel column chromatography (Biotage, Inc., elution solvent: hexane/ethyl acetate), and a fraction corresponding to the Rf value=0.50 (hexane/ethyl acetate=1/1) by thin layer chromatography was concentrated under reduced pressure to afford the title compound (1.6 g, 2.5 mmol) (yield 81 %).
1H-NMR (500 MHz, CDCl3) δ: 8.14 (1H, t, J=4 Hz), 7.50 (2H, d, J=8 Hz), 7.39-7.34 (3H, m), 7.26-7.24 (2H, m), 6.95 (2H, d, J=8 Hz), 6.83 (1H, t, J=8 Hz), 5.25 (2H, s), 4.28 (2H, q, J=8 Hz), 4.23 (2H, d, J=4 Hz), 3.67 (2H, d, J=12 Hz), 2.92 (2H, d, J=4 Hz), 2.72 (2H, dt, J=12 Hz, 4 Hz), 2.11-2.05 (1H, m), 1.77 (2H, d, J=12 Hz), 1.58-1.47 (2H, m), 1.33 (3H, t, J=8 Hz).

### (2) Ethyl [({5-(benzyloxy)-6-(methylsulfanyl)-2-[(1-phenylpiperidin-4-yl)methyl]pyrimidin-4-yl}carbonyl)amino]acetate

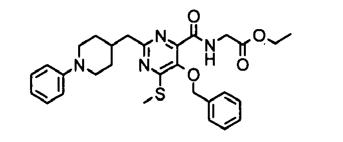

Ethyl ({[5-(benzyloxy)-2-[(1-phenylpiperidin-4-yl)methyl]-6-{[(trifluoromethyl)sulfonyl]oxy}pyrimidin-4-yl]carbonyl}amino)acetate (0.32 g, 0.50 mmol) and sodium methanethiolate (0.053 g, 0.75 mmol) were dissolved in N,N-dimethylformamide (5 mL), followed by stirring at room temperature for 17.5 hours. After the reaction solution was concentrated under reduced pressure, water (15 mL) was added to the resulting residue, followed by extraction with methylene chloride, and the organic layer was dried over sodium sulfate. After the solvent was distilled off under reduced pressure, the resulting residue was purified by silica gel column chromatography (Biotage, Inc., elution solvent: hexane/ethyl acetate), and a fraction corresponding to the Rf value=0.50 (hexane/ethyl acetate=2/1) by thin layer chromatography was concentrated under reduced pressure to afford the title compound (0.19 g, 0.35 mmol) (yield 69%).
1H-NMR (500 MHz, CDCl3) δ: 8.37 (1H, t, J=4 Hz), 7.60 (2H, d, J=4 Hz), 7.41-7.34 (3H, m), 7.27-7.24 (2H, m), 6.95 (2H, d, J=4 Hz), 6.83 (1H, t, J=4 Hz), 5.17 (2H, s), 4.26 (2H, q, J=8 Hz), 4.23 (2H, d, J=4 Hz), 3.68 (2H, d, J=8 Hz), 2.88 (2H, d, J=4 Hz), 2.75 (2H, dt, J=12 Hz, 4 Hz), 2.53 (3H, s), 2.17-2.05 (1H, m), 1.82 (2H, d, J=12 Hz), 1.60-1.49 (2H, m), 1.31 (3H, t, J=8 Hz).

### (3) Ethyl [({5-hydroxy-6-(methylsulfanyl)-2-[(1-phenylpiperidin-4-yl)methyl]pyrimidin-4-yl } carbonyl)amino]acetate

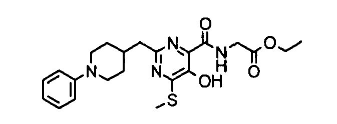

In accordance with Example 8-(2), but using ethyl [({5-(benzyloxy)-6-(methylsulfanyl)-2-[(1-phenylpiperidin-4-yl)methyl]pyrimidin-4-yl}carbonyl)amino]acetate (0.19 g, 0.35 mmol) instead of ethyl ({[5-(benzyloxy)-2-{[1-(4-chlorophenyl)piperidin-4-yl]methyl}-6-methylpyrimidin-4-yl]carbonyl}amino)acetate, the title compound (0.11 g, 0.25 mmol) was afforded (yield 73%).
1H-NMR (500 MHz, CDC13) δ: 11.66 (1H, s), 8.44 (1H, t, J=8 Hz), 7.26-7.23 (2H, m), 6.95 (2H, d, J=8 Hz), 6.83 (1H, t, J=8 Hz), 4.28 (2H, q, J=8 Hz), 4.21 (2H, d; J=8 Hz), 3.68 (2H, d, J=12 Hz), 2.81 (2H, d, J=4 Hz), 2.72 (2H, dt, J=12 Hz, 4 Hz), 2.57 (3H, s), 2.10-2.02 (1H, m), 1.79 (2H, d, J=8 Hz), 1.54-1.43 (2H, m), 1.32 (3H, t, J=8 Hz).

### (4) [({5-Hydroxy-6-(methylsulfanyl)-2-[(1-phenylpiperidin-4-yl)methyl]pyrimidin-4-yl}carbonyl)amino]acetic acid

In accordance with Example 1-(11), but using ethyl [({5-hydroxy-6-(methylsulfanyl)-2-[(1-phenylpiperidin-4-yl)methyl]pyrimidin-4-yl}carbonyl)amino]acetate (0.11 g, 0.25 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.020 g, 0.048 mmol) was afforded as a white solid (yield 19%).
mp 156-157°C;
1H-NMR (400 MHz, DMSO-d6) δ: 9.39 (1H, s), 7.20-7.17 (2H, m), 6.91 (2H, d, J=4 Hz), 6.73 (1H, t, J=4 Hz), 4.00 (2H, d, J=4 Hz), 3.67 (2H, d, J=12 Hz), 2.80 (2H, d, J=8 Hz), 2.66 (2H, dt, J=8 Hz, 4 Hz), 2.53 (3H, s), 2.15-2.05 (1H, m), 1.70 (2H, d, J =12 Hz), 1.38-1.34 (2H, m).

### Example 192

### ({[2-{[1-(4-Fluorophenyl)piperidin-4-yl]methyl}-5-hydroxy-6-(methylsulfanyl)pyrimidin-4-yl]carbonyl}amino)acetic acid

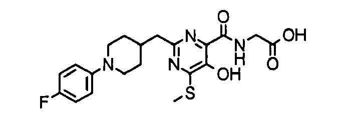

### (1) Ethyl({[5-(benzyloxy)-2-{[1-(4-fluorophenyl)piperidin-4-yl]methyl}-6-(methylsulfanyl)pyrimidin-4-yl]carbonyl}amino)acetate

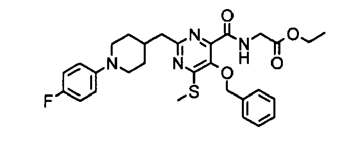

In accordance with Example 191-(2), but using ethyl ({[5-(benzyloxy)-2-{[1-(4-fluorophenyl)piperidin-4-yl]methyl}-6-{ [(trifluoromethyl)sulfonyl]oxy}pyrimidin-4-yl]carbonyl}amino)acetate (1.3 g, 2.0 mmol) obtained in Example 5-(3) instead of ethyl ({[5-(benzyloxy)-2-[(1-phenylpiperidin-4-yl)methyl]-6-{[(trifluoromethyl)sulfonyl]oxy}pyrimidin-4-yl]carbonyl}amino)acetate, the title compound (0.87 g, 1.6 mmol) was afforded (yield 79%).
1H-NMR (500 MHz, CDCl3) δ: 8.36 (1H, t, J=4 Hz), 7.61 (2H, d, J=8 Hz), 7.41-7.33 (3H, m), 6.99-6.88 (4H, m), 5.17 (2H, s), 4.26 (2H, q, J=8 Hz), 4.23 (2H, d, J=4 Hz), 3.68 (2H, d, J=8 Hz), 2.89 (2H, d, J=4 Hz), 2.70 (2H, dt, J=12 Hz, 4 Hz), 2.53 (3H, s), 2.17-2.05 (1H, m), 1.83 (2H, d, J=12 Hz), 1.60-1.49 (2H, m), 1.31 (3H, t, J=8 Hz).

### (2) Ethyl ({[2-{[1-(4-fluorophenyl)piperidin-4-yl]methyl}-5-hydroxy-6-(methylsulfanyl)pyrimidin-4-yl]carbonyl}amino)acetate

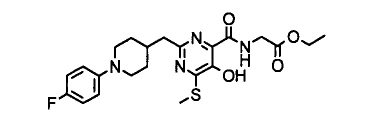

In accordance with Example 8-(2), but using ethyl ({[5-(benzyloxy)-2-{[1-(4-fluorophenyl)piperidin-4-yl]methyl}-6-(methylsulfanyl)pyrimidin-4-yl]carbonyl}amino)acetate (0.87 g, 1.6 mmol) instead of ethyl ({[5-(benzyloxy)-2-{[1-(4-chlorophenyl)piperidin-4-yl]methyl}-6-methylpyrimidin-4-yl]carbonyl}amino)acetate, the title compound (0.58 g, 1.3 mmol) was afforded (yield 80%).
1H-NMR (500 MHz, CDCl3) δ: 11.66 (1H, s), 8.41 (1H, t, J=8 Hz), 6.96-6.87 (4H, m), 4.28 (2H, q, J=8 Hz), 4.21 (2H, d, J=8 Hz), 3.54 (2H, d, J=12 Hz), 2.82 (2H, d, J=8 Hz), 2.67 (2H, dt, J=12 Hz, 4 Hz), 2.57 (3H, s), 2.08-2.01 (1H, m), 1.79 (2H, d, J=12 Hz), 1.54-1.43 (2H, m), 1.32 (3H, t, J=8 Hz).

### (3) ({[2-{[1-(4-Fluorophenyl)piperidin-4-yl]methyl}-5-hydroxy-6-(methylsulfanyl)pyrimidin-4-yl]carbonyl}amino)acetic acid

In accordance with Example 1-(11), but using ethyl ({[2-{[1-(4-fluorophenyl)piperidin-4-yl]methyl}-5-hydroxy-6-(methylsulfanyl)pyrimidin-4-yl]carbonyl}amino)acetate (0.58 g, 1.3 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.41 g, 0.94 mmol) was afforded as a white solid (yield 76%).
mp 228-230°C;
1H-NMR (500 MHz, DMSO-d6) δ: 7.03-6.99 (2H, m), 6.94-6.91 (2H, m), 3:88 (2H, t, J=6 Hz), 3.53 (2H, d, J=12 Hz), 2.74 (2H, d, J=8 Hz), 2.60 (2H, dt, J=8 Hz, 4 Hz), 2.46 (3H, s), 2.09-1.94 (1H, m), 1.71 (2H, d, J=12 Hz), 1.38-1.34 (2H, m).

### Example 193

### ({[2-{[1-(4-tert-Butylphenyl)piperidin-4-yl]methyl}-5-hydroxyl-6-(methylsulfanyl)pyrimidin-4-yl]carbonyl}amino)acetic acid

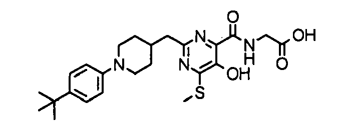

### (1) [1-(4-tert-Butylphenyl)piperidin-4-yl]acetonitrile

In accordance with Examples 1-(1) and (2), but using 1-(4-tert-butylphenyl)piperidin-4-one (18 g, 78 mmol) obtained in Example 167-(1) instead of tert-butyl 4-oxopiperidine-1-carboxylate, the title compound (15 g, 60 mmol) was afforded (yield 77%).
1H-NMR (500 MHz, CDCl3) δ: 7.28 (2H, d, J=9 Hz), 6.88 (2H, d, J=9 Hz), 3.67 (2H, d, J=13 Hz), 2.70 (2H, d, J=12 Hz), 2.35 (2H, d, J=7 Hz), 1.93 (2H, d, J=13 Hz), 1.87-1.80 (2H, m), 1.54-1.51 (1H, m), 1.29 (9H, s).

### (2) tert-Butyl 5-(benzyloxy)-2-{[1-(4-tert-butylphenyl)piperidin-4-yl]methyl}-6-hydroxypyrimidine-4-carboxylate

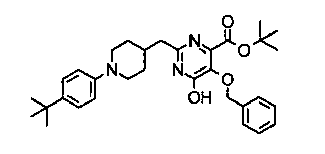

In accordance with Examples 1-(3) and 1-(4), but using [1-(4-tert-butylphenyl)piperidin-4-yl]acetonitrile (15 g, 60 mmol) instead of tert-butyl 4-(cyanomethyl)piperidine-1-carboxylate, the title compound (6.1 g, 11 mmol) was afforded as a solid (yield 22%).
1H-NMR (500 MHz, CDCl3) δ: 7.46 (2H, d, J=7 Hz), 7.39-7.30 (3H, m), 7.27-7.25 (2H, m), 6.87 (2H, brs), 5.25 (2H, s), 3.61 (2H, d, J=12 Hz), 2.66 (2H, brs), 2.66 (2H, d, J=7 Hz), 2.02 (2H, brs), 1.79 (2H, d, J=12 Hz), 1.52 (9H, s), 1.54-1.51 (1H, m), 1.28 (9H, s).

### (3) Ethyl ({[5-(benzyloxy)-2-{[1-(4-tert-butylphenyl)piperidin-4-yl]methyl}-6-{[(trifluoromethyl)sulfonyl] oxy}pyrimidin-4-yl]carbonyl}amino)acetate

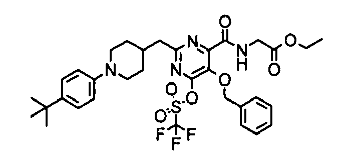

In accordance with Example 191-(1), but using tert-butyl 5-(benzyloxy)-2-{[1-(4-tert-butylphenyl)piperidin-4-yl]methyl}-6-hydroxypyrimidine-4-carboxylate (1.6 g, 3.0 mmol) instead of tert-butyl 5-(benzyloxy)-6-hydroxy-2-[(1-phenylpiperidin-4-yl)methyl]pyrimidine-4-carboxylate, the title compound (1.1 g, 1.6 mmol) was afforded (yield 53%).
1H-NMR (500 MHz, CDCl3) δ: 8.15 (1H, t, J=4 Hz), 7.50 (2H, d, J=4 Hz), 7.40-7.36 (3H, m), 7.29-7.27 (2H, m), 6.90 (2H, d, J=8 Hz), 5.26 (2H, s), 4.28 (2H, q, J=8 Hz), 4.24 (2H, d, J=4 Hz), 3.64 (2H, d, J=8 Hz), 2.92 (2H, d, J=4 Hz), 2.69 (2H, dt, J=8 Hz, 4 Hz), 2.11-2.05 (1H, m), 1.77 (2H, d, J=12 Hz), 1.58-1.47 (2H, m), 1.33 (3H, t, J=8 Hz),1.29(9H,s).

### (4) Ethyl ({[5-(benzyloxy)-2-{[1-(4-tert-butylphenyl)piperidin-4-yl]methyl}-6-(methylsulfanyl)pyrimidin-4-yl]carbonyl} amino)acetate

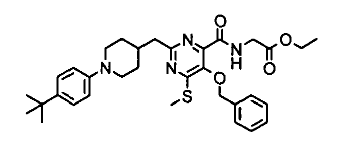

In accordance with Example 191-(2), but using ethyl ({[5-(benzyloxy)-2-{[1-(4-tert-butylphenyl)piperidin-4-yl]methyl}-6-{[(trifluoromethyl)sulfonyl]oxy}pyrimidin-4-yl]carbonyl}amino)acetate (1.1 g, 1.6 mmol) instead of ethyl ({[5-(benzyloxy)-2-[(1-phenylpiperidin-4-yl)methyl]-6-{[(trifluoromethyl)sulfonyl]oxy}pyrimidin-4-yl]carbonyl}amino)acetate, the title compound (0.59 g, 0.99 mmol) was afforded (yield 62%).
1H-NMR (400 MHz, CDCl3) δ: 8.35 (1H, t, J=4 Hz), 7.60 (2H, d, J=4 Hz), 7.41-7.32 (3H, m), 7.27 (2H, d, J=8 Hz), 6.96 (2H, d, J=8 Hz), 5.17 (2H, s), 4.26 (2H., q, J=8 Hz), 4.23 (2H, d, J=4 Hz), 3.64 (2H, d, J=12 Hz), 2.88 (2H, d, J=8 Hz), 2.70 (2H, dt, J=12 Hz, 4 Hz), 2.53 (3H, s), 2.17-2.05 (1H, m), 1.81 (2H, d, J=8 Hz), 1.60-1.49 (2H, m), 1.31 (3H, t, J=8 Hz), 1.29 (9H, s).

### (5) Ethyl ({[2-{[1-(4-tert-butylphenyl)piperidin-4-yl]methyl}-5-hydroxy-6-(methylsulfanyl)pynmidin-4-yl]carbonyl}amino)acetate

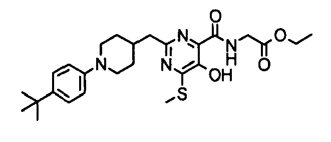

In accordance with Example 8-(2), but using ethyl ({[5-(benzyloxy)-2-{[1-(4-tert-butylphenyl)piperidin-4-yl]methyl}-6-(methylsulfanyl)pyrimidin-4-yl]carbonyl}amino)acetate (0.59 g, 0.99 mmol) instead of ethyl ({[5-(benzyloxy)-2-{[1-(4-chlorophenyl)piperidin-4-yl]methyl}-6-methylpyrimidin-4-yl]carbonyl}amino)acetate, the title compound (0.39 g, 0.77 mmol) was afforded (yield 78%).
1H-NMR (500 MHz, CDCl3) δ:11.65 (1H, s), 8.43 (1H, t, J=4 Hz), 7.27 (2H, d, J=8 Hz), 6.96 (2H, d, J=8 Hz), 4.27 (2H, q, J=8 Hz), 4.21 (2H, d, J=8 Hz), 3.63 (2H, d, J=12 Hz), 2.81 (2H, d, J=8 Hz), 2.68 (2H, dt, J=8 Hz, 4 Hz), 2.57 (3H, s), 2.08-2.01 (1H, m), 1.78 (2H, d, J=8 Hz), 1.54-1.41 (2H, m), 1.32 (3H, t, J=8 Hz), 1.29 (9H, s).

### (6) ({[2-{[1-(4-tert-Butylphenyl)piperidin-4-yl]methyl}-5-hydroxy-6-(methylsulfanyl)pyrimidin-4-yl]carbonyl}amino)acetic acid

In accordance with Example 1-(11), but using ethyl ({[2-{[1-(4-tert-butylphenyl)piperidin-4-yl]methyl}-5-hydroxy-6-(methylsulfanyl)pyrimidin-4-yl]carbonyl}amino)acetate (0.39 g, 0.77 mmol) instead of ethyl {[(2-{[1-(4-tert-butylphenyl)piperidin-4-yl]methyl}-5-hydroxy-6-methylpyrimidin-4-yl)carbonyl]amino} acetate, the title compound (0.34 g, 0.71 mmol) was afforded as a white solid (yield 92%).
mp 135-136°C;
1H-NMR (400 MHz, DMSOd6) δ:12.88 (1H, brs), 12.21 (1H, s), 9.37 (1H, s), 7.20 (2H, d, J=8 Hz), 6.84 (2H, d, J=8 Hz), 4.03-3.96 (2H, m), 3.60 (2H, d, J=12 Hz), 2.80 (2H, d, J=8 Hz), 2.62 (2H, dt, J=8 Hz, 4 Hz), 2.54 (3H, s), 2.14-2.02 (1H, m), 1.70 (2H, d, J=12 Hz), 1.41-1.32 (2H, m), 1.23 (9H, s).

### Example 194

### ({[2-{[6-(4-tert-Butylphenyl)pyridin-3-yl]methyl}-5-hydroxy-6-(methylsulfanyl)pyrimidin-4-yl]carbonyl}amino)acetic acid

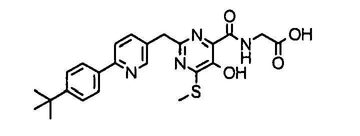

### (1) tert-Butyl 5-(benzyloxy)-2-{[6-(4-tert-butylphenyl)pyridin-3-yl]methyl}-6-(methylsulfanyl)pyrimidine-4-carboxylate

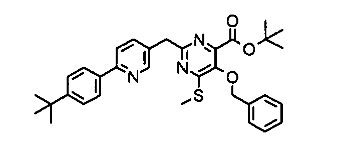

tert-Butyl 5-(benzyloxy)-2-{[6-(4-tert-butylphenyl)pyridm-3-yl]methyl}-6-{[(trifluoromethyl)sulfonyl]oxy}pyrimidine-4-carboxylate (0.73 g, 1.1 mmol) obtained in Example 173-(4) was dissolved in N,N-dimethylformamide (15 mL), and at room temperature sodium thiomethoxide (0.12 g, 1.7 mmol) was added, followed by stirring for 50 minutes. Aqueous sodium chloride solution was added to the reaction solution, followed by extraction with ethyl acetate. After the extract was concentrated under reduced pressure, the resulting residue was purified by silica gel column chromatography (Moritex Corporation, elution solvent: hexane/ethyl acetate), and a fraction corresponding to the Rf value=0.55 (hexane/ethyl acetate=2/1) by thin layer chromatography was concentrated under reduced pressure to afford the title compound (0.40 g, 0.72 mmol) as a pale orange amorphous solid (yield 64%).
1H-NMR (500 MHz, CDCl3) δ: 8.73 (1H, d, J=2 Hz), 7.91 (2H, d, J=8 Hz), 7.79 (1H, dd, J=8 Hz, 2 Hz), 7.67 (1H, d, J=8 Hz), 7.52-7.46 (5H, m), 7.39 (2H, d, J=8 Hz), 5.04 (2H, s), 4.28 (2H, s), 2.46 (3H, s), 1.58 (9H, s), 1.36 (9H, s).

### (2) ({[2-{[6-(4-tert-Butylphenyl)pyridin-3-yl]methyl}-5-hydroxy-6-(methylsulfanyl)pyrimidin-4-yl]carbonyl}amino)acetic acid

In accordance with Examples 1-(9), 6-(2) and 1-(11), but using tert-butyl 5-(benzyloxy)-2-{ [6-(4-tert-butylphenyl)pyridin-3-yl]methyl }-6-(methylsulfanyl)pyrimidine-4-carboxylate (0.40 g, 0.72 mmol) instead of tert-butyl 5-(benzyloxy)-2- {[1-(4-tert-butylphenyl)piperidin-4-yl]methyl}-6-methylpyrimidine-4-carboxylate, the title compound (0.090 g, 0.19 mmol) was afforded as a pale yellowish white solid (yield 27%).
MS m/z: 467 (M+H)⁺;
1H-NMR (500 MHz, DMSO-d6) δ: 9.68 (1H, brs), 8.67 (1H, s), 7.98 (2H, d, J=8 Hz), 7.87-7.84 (2H, m), 7.49 (2H, d, J=8 Hz), 4.20 (2H, s), 4.00 (2H, d, J=6 Hz), 2.46 (3H, s), 1.31 (9H, s).

### Example 195

### ({[2-{[1-(4-tert-Butylphenyl)piperidin-4-yl]methyl}-6-(difluoromethyl)-5-hydroxypyrimidin-4-yl]carbonyl}amino)acetic acid

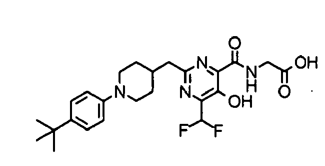

### (1) tert-Butyl 5-(benzyloxy)-2-{[1-(4-tert-butylphenyl)piperidin-4-yl]methyl}-6-(difluoromethyl)pyrimidine-4-carboxylate

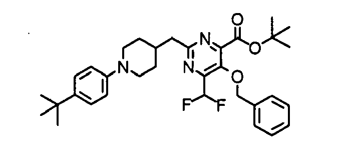

tert-Butyl 5-(benzyloxy)-2- {[1-(4-tert-butylphenyl)piperidin-4-yl]methyl}-6-methylpyrimidine-4-carboxylate (0.72 g, 1.4 mmol) obtained in Example 1-(8) was dissolved in tetrahydrofuran (20 mL), and a solution of lithium hexamethyldisilazide in tetrahydrofuran (1 M, 2.7 mL, 2.7 mmol) was added at -78°C under a nitrogen atmosphere, followed by stirring at the same temperature for 20 minutes. N-Fluorobenzenesulfonimide (0.86 g, 2.7 mmol) was added to the reaction solution at - 78°C, followed by stirring at the same temperature for 20 minutes. A solution of lithium hexamethyldisilazide in tetrahydrofuran (1 M, 2.7 mL, 2.7 mmol) was further added to the reaction solution followed by stirring for 20 minutes, and subsequently N-fluorobenzenesulfonimide (0.86 g, 2.7 mmol) was added, followed by stirring for a further 2 hours. Saturated aqueous ammonium chloride solution was added to the reaction solution, followed by extraction with ethyl acetate. After the extract was concentrated under reduced pressure, the resulting residue was purified by silica gel column chromatography (Moritex Corporation, elution solvent: hexane/ethyl acetate), and a fraction corresponding to the Rf value=0.50 (hexane/ethyl acetate=4/1) by thin layer chromatography was concentrated under reduced pressure to afford the title compound (0.16 g, 0.28 mmol) as a pale yellow oil (yield 21 %).
1H-NMR (500 MHz, CDCl3) δ: 7.44-7.38 (5H, m), 7.26 (2H, d, J=9 Hz), 6.88 (2H, d, J=9 Hz), 6.69 (1H, t, J=54 Hz), 5.12 (2H, s), 3.63 (2H, d, J=13 Hz), 3.00 (2H, d, J=7 Hz), 2.68 (2H, t, J=13 Hz), 2.15-2.04 (1H, m), 1.78 (2H, d, J=13 Hz), 1.61 (9H, s), 1.51 (2H, d, J=13 Hz), 1.28 (9H, s).

### (2) Ethyl ({[5-(benzyloxy)-2-{[1-(4-tert-butylphenyl)piperidin-4-yl]methyl}-6-(difluoromethyl)pyrimidin-4-yl]carbonyl}amino)acetate

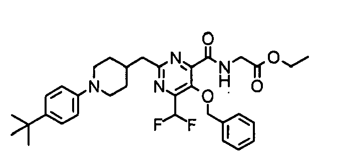

In accordance with Example 1-(9), but using tert-butyl 5-(benzyloxy)-2-{[1-(4-tert-butylphenyl)piperidin-4-yl]methyl}-6-(difluoromethyl)pyrimidine-4-carboxylate (0.16 g, 0.28 mmol) instead of tert-butyl 5-(benzyloxy)-2-{[1-(4-tert-butylphenyl)piperidin-4-yl]methyl}-6-methylpyrimidine-4-carboxylate, the title compound (0.15 g, 0.25 mmol) was afforded as a pale yellow oil (yield 88%).
1H-NMR (500 MHz, CDCl3) δ: 8.32 (1H, t, J=5 Hz), 7.49 (2H, d, J=6 Hz), 7.42-7.37 (3H, m), 7.27 (2H, d, J=9 Hz), 6.89 (2H, d, J=9 Hz), 6.79 (1H, t, J=54 Hz), 5.23 (2H, s), 4.28 (2H, q, J=7 Hz), 4.27 (2H, d, J=5 Hz), 3.65 (2H, d, J=12 Hz), 3.01 (2H, d, J=7 Hz), 2.70 (2H, t, J=12 Hz), 2.11-2.04 (1H, m), 1.77 (2H, d, J=12 Hz), 1.56-1.53 (2H, m), 1.32 (3H, t, J=7 Hz), 1.29 (9H, s).

### (3) Ethyl ({[2-{[1-(4-tert-butylphenyl)piperidin-4-yl]methyl}-6-(difluoromethyl)-5-hydroxypyrimidin-4-yl]carbonyl} amino)acetate

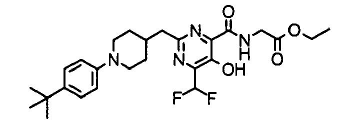

In accordance with Example 1-(10), but using ethyl ({[5-(benzyloxy)-2-{[1-(4-tert-butylphenyl)piperidin-4-yl]methyl}-6-(difluoromethyl)pyrimidin-4-yl]carbonyl}amino)acetate (0.80 g, 1.3 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.42 g, 0.83 mmol) was afforded as a pale yellow solid (yield 64%).
1H-NMR (500 MHz, CDCl3) δ: 11.73 (1H, s), 8.46 (1H, t, J=6 Hz), 7.27 (2H, d, J=9 Hz), 6.88 (2H, d, J=9 Hz), 6.87 (1H, t, J=54 Hz), 4.30 (2H, q, J=7 Hz), 4.23 (2H, d, J=6 Hz), 3.63 (2H, d, J=13 Hz), 2.93 (2H, d, J=7 Hz), 2.68-2.64 (2H, m), 2.05-2.01 (1H, m), 1.78-1.73 (2H, m), 1.54-1.48 (2H, m), 1.34 (3H, t, J=7 Hz), 1.28 (9H, s).

### (4) ({[2-{[1-(4-tert-Butylphenyl)piperidin-4-yl]methyl}-6-(difluoromethyl)-5-hydroxypyrimidin-4-yl]carbonyl}amino)acetic acid

In accordance with Example 1-(11), but using ethyl ({[2-{[1-(4-tert-butylphenyl)piperidin-4-yl]methyl}-6-(difluoromethyl)-5-hydroxypyrimidin-4-yl]carbonyl}amino)acetate (0.42 g, 0.83 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.35 g, 0.73 mmol) was afforded as a pale yellow solid (yield 88%).
MS m/z: 477 (M+H)⁺;
1H-NMR (500 MHz, DMSO-d6) δ:12.29 (1H, s), 9.66 (1H, t, J=6 Hz), 7.85-7.30 (4H, m), 7.17 (1H, t, J=53 Hz), 4.03 (2H, d, J=6 Hz), 3.56-3.50 (2H, m), 3.48-3.20 (4H, m), 2.40-2.15 (1H, m), 2.98-2.90 (2H, m), 1.90-1.73 (2H, m), 1.27 (9H, s).

### Example 196

### {[(2-{[trans-5-(4-Fluorophenyl)-1,3-dioxan-2-yl]methyl -5-hydroxyl-6-methylpyrimidin-4-yl)carbonyl]amino}acetic acid

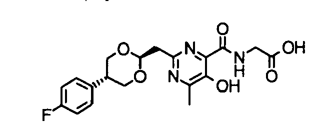

### (1) 2-(4-Fluorophenyl)propane-1,3-diol

1-Fluoro-4-iodobenzene (6.7 g, 30 mmol), diethyl malonate (5.5 mL, 36 mmol), cesium carbonate (39 g, 0.12 mol), L-proline (1.4 g, 12 mmol) and copper iodide (1.1 g, 6.0 mmol) were suspended in dimethyl sulfoxide (60 mL), followed by vigorously stirring at room temperature for 3 days under a nitrogen atmosphere. Water was added to the reaction solution, followed by extraction with ethyl acetate, and subsequently the organic layer was dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.64 (hexane/ethyl acetate=2/1) by thin layer chromatography was concentrated under reduced pressure to afford diethyl (4-fluorophenyl)malonate (3.5 g) as an oil. Under a nitrogen atmosphere, lithium aluminium hydride (1.2 g, 32 mmol) was suspended in tetrahydrofuran (26 mL), and a solution of diethyl (4-fluorophenyl)malonate (3.3 g, 13 mmol) in tetrahydrofuran (10 mL) was added dropwise at 0°C over 5 minutes, followed by stirring at room temperature for 4 hours. The reaction solution was added dropwise to hydrochloric acid (1 M) cooled to 0°C, followed by extraction with ethyl acetate, and the extract was washed with saturated aqueous sodium hydrogencarbonate solution and dried over sodium sulfate. After the solvent was distilled off under reduced pressure, the resulting residue was purified by silica gel column chromatography (Moritex Corporation, elution solvent: hexane/ethyl acetate), and a fraction corresponding to the Rf value=0.24 (ethyl acetate) by thin layer chromatography was concentrated under reduced pressure to afford the title compound (1.7 g, 9.8 mmol) as a colorless oil (yield 35%).
1H-NMR (500 MHz, CDCl3) δ: 7.22 (2H, dd, J=9 Hz, 5 Hz), 7.03 (2H, t, J=9 Hz), 4.01-3.90 (4H, m), 3.09 (1H, tt, J=7 Hz, 5 Hz), 2.02 (2H, t, J=5 Hz).

### (2) tert-Butyl 5-(benzyloxy)-2-(2,2-dimethoxyethyl)-6-hydroxypyrimidine-4-carboxylate

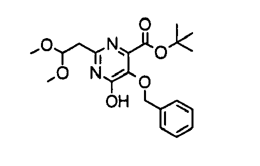

In accordance with Examples 1-(3) and 1-(4), but using 3,3-dimethoxypropanenitrile (3.5 g, 30 mmol) instead of tert-butyl 4-(cyanomethyl)piperidine-1-carboxylate, the title compound (1.4 g, 3.5 mmol) was afforded as a solid (yield 58%).
1H-NMR (500 MHz, CDCl3) δ: 10.35 (1H, brs), 7.47 (2H, d, J=7 Hz), 7.37-7.30 (3H, m), 5.28 (2H, s), 4.66 (1H, t, J=5 Hz), 3.44 (6H, s), 3.00 (2H, d, J=5 Hz), 1.50 (9H, s).

### (3) Ethyl ({[5-(benzyloxy)-2-(2,2-dimethoxyethyl)-6-methylpyrimidin-4-yl]carbonyl}amino)acetate

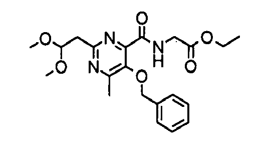

In accordance with Examples 1-(5), 1-(6) and 1-(9), but using tert-butyl 5-(benzyloxy)-2-(2,2-dimethoxyethyl)-6-hydroxypyrimidine-4-carboxylate (0.62 g, 1.6 mmol) instead of tert-butyl 5-(benzyloxy)-2-{[1-(tert-butoxycarbonyl)piperidin-4-yl]methyl}-6-hydroxypyrimidine-4-carboxylate, the title compound (0.64 g, 1.5 mmol) was afforded as a white solid (yield 94%).
1H-NMR (500 MHz, CDCl3) δ: 8.31 (1H, t, J=5 Hz), 7.48 (2H, d, J=7 Hz), 7.40-7.32 (3H, m), 5.11 (2H, s), 5.08 (1H, t, J=6 Hz), 4.26 (2H, q, J=7 Hz), 4.24 (2H, d, J=5 Hz), 3.39 (6H, s), 3.26 (2H, d, J=6 Hz), 2.45 (3H, s), 1.31 (3H, t, J=7 Hz).

### (4) Ethyl {[(5-(benzyloxy)-2-{[trans-5-(4-fluorophenyl)-1,3-dioxan-2-yl]methyl}-6-methylpyrimidin-4-yl)carbonyl]amino}acetate

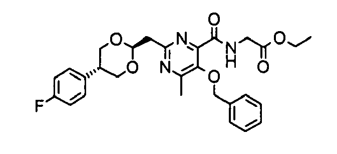

Ethyl ({[5-(benzyloxy)-2-(2,2-dimethoxyethyl)-6-methylpyrimidin-4-yl]carbonyl}amino)acetate (1.2 g, 2.8 mmol) was dissolved in toluene (10 mL), and 2-(4-fluorophenyl)propane-1,3-diol (0.57 g, 3.4 mmol) and p-toluenesulfonic acid monohydrate (0.016 g, 0.080 mmol) were added, followed by heating to reflux for 1 hour under a nitrogen atmosphere. Saturated aqueous sodium hydrogencarbonate solution was poured into the reaction solution, followed by extraction with ethyl acetate, and subsequently the extract was washed with saturated aqueous sodium chloride solution, and the organic layer was dried over sodium sulfate. After the solvent was distilled off under reduced pressure, the resulting residue was purified by silica gel column chromatography (Moritex Corporation, elution solvent: hexane/ethyl acetate), and fractions corresponding to the Rf value=0.44 and 0.34 (hexane/ethyl acetate=1/2) by thin layer chromatography were respectively concentrated under reduced pressure to afford ethyl {[(5-(benzyloxy)-2-{[trans-5-(4-fluorophenyl)-1,3-dioxan-2-yl]methyl}-6-methylpyrimidin-4-yl)carbonyl]amino}acetate (0.95 g, 1.8 mmol, yield 64%) and ethyl {[(5-(benzyloxy)-2-{[cis-5-(4-fluorophenyl)-1,3-dioxan-2-yl]methyl}-6-methylpyrimidin-4-yl)carbonyl]amino}acetate (0.44 g, 0.84 mmol, yield 30%) respectively as colorless oils.
trans-form:
Rf value=0.44 (hexane/ethyl acetate=1/2);
1H-NMR (400 MHz, CDCl3) δ: 8.37 (1H, t, J=5 Hz), 7.50 (2H, d, J=8 Hz), 7.42-7.35 (3H, m), 7.17 (2H, dd, J=9 Hz, 5 Hz), 7.02 (2H, t, J=9 Hz), 5.28 (1H, t, J=5 Hz), 5.12 (2H, s), 4.28 (2H, q, J=7 Hz), 4.25 (2H, d, J=5 Hz), 4.18 (2H, dd, J=12 Hz, 5 Hz), 3.84 (2H, t, J=12 Hz), 3.32 (2H, d, J=5 Hz), 3.31-3.22 (1H, m), 2.48 (3H, s), 1.33 (3H, t, J=7 Hz).
cis-form:

Rf value=0.34 (hexane/ethyl acetate=1/2);
1H-NMR (400 MHz, CDC13) δ: 8.35 (1H, t, J=5 Hz), 7.54 (2H, dd, J=9 Hz, 6 Hz), 7.49 (2H, d, J=7 Hz), 7.42-7.34 (3H, m), 7.01 (2H, t, J=9 Hz), 5.35 (1H, t, J=5 Hz), 5.11 (2H, s), 4.30-4.21 (4H, m), 4.18 (2H, d, J=12 Hz), 3.96 (2H, d, J=5 Hz), 3.33 (2H, d, J=5 Hz), 2.65 (1H, brs), 2.48 (3H, s), 1.32 (3H, t, J=7 Hz).

### (5) Ethyl {[(2-{[trans-5-(4-fluorophenyl)-1,3-dioxan-2-yl]methyl}-5-hydroxy-6-methylpyrimidin-4-yl)carbonyl]amino}acetate

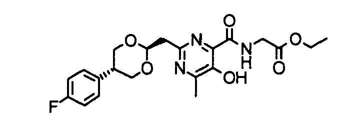

In accordance with Example 1-(10), but using ethyl {[(5-(benzyloxy)-2-{[trans-5-(4-fluorophenyl)-1,3-dioxan-2-yl]methyl}-6-methylpyrimidin-4-yl)carbonyl]amino}acetate (0.95 g, 1.8 mmol) instead of ethyl ({[5-(benzyloxy)-2-{[1-(4-tert-butylphenyl)piperidin-4-yl]methyl}-6-methylpyrimidin-4-yl]carbonyl}amino)acetate, the title compound (0.72 g, 1.7 mmol) was afforded as an amorphous solid (yield 90%).
1H-NMR (500 MHz, CDCl3) δ: 11.43 (1H, s), 8.49 (1H, t, J=6 Hz), 7.15 (2H, dd, J=8 Hz, 5 Hz), 7.01 (2H, t, J=8 Hz), 5.20 (1H, t, J=5 Hz), 4.29 (2H, q, J=7 Hz), 4.21 (2H, d, J=6 Hz), 4.17 (2H, dd, J=12 Hz, 4 Hz), 3.80 (2H, t, J=11 Hz), 3.28-3.21 (1H, m), 3.25 (2H, d, J=5 Hz), 2.55 (3H, s), 1.33 (3H, t, J=7 Hz).

### (6) {[(2-{[trans-5-(4-Fluorophenyl)-1,3-dioxan-2-yl]methyl}-5-hydroxy-6-methylpyrimidin-4-yl)carbonyl]amino}acetic acid

In accordance with Example 1-(11), but using ethyl {[(2-{[trans-5-(4-fluorophenyl)-1,3-dioxan-2-yl]methyl}-5-hydroxy-6-methylpyrimidin-4-yl)carbonyl]amino}acetate (0.72 g, 1.7 mmol) instead of ethyl {[(2-{[1-(4-tert-butylphenyl)piperidin-4-yl]methyl}-5-hydroxy-6-methylpyrimidin-4-yl)carbonyl]amino}acetate, the title compound (0.53 g, 1.3 mmol) was afforded as a white solid (yield 78%).
mp 209°C;
MS m/z: 406 (M+H)⁺;
Anal Calcd for C19H20FN3O6: C, 56.29; H, 4.97; N, 10.37; F, 4.69. Found: C, 56.24; H, 5.05; N, 10.42; F, 4.71;
1H-NMR (500 MHz, CDCl3) δ: 11.34 (1H, s), 8.49 (1H, t, J=6 Hz), 7.14 (2H, dd, J=8 Hz, 6 Hz), 7.01 (2H, t, J=8 Hz), 5.20 (1H, t, J=5 Hz), 4.30 (2H, d, J=6 Hz), 4.18 (2H, dd, J=11 Hz, 4 Hz), 3.80 (2H, t, J=11 Hz), 3.26-3.21 (3H, m), 2.56 (3H, s).

### Example 197

### {[(2-{[trans-5-(4-tert-Butylphenyl)-1,3-dioxan-2-yl]methyl}-5-hydroxy-6-methylpyrimidin-4-yl)carbonyl]amino}acetic acid

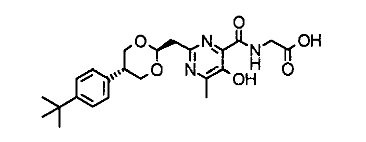

### (1) 2-(4-tert-Butylphenyl)propane-1,3-diol

In accordance with Example 196-(1), but using 1-tert-butyl-4-iodobenzene (0.89 mL, 5.0 mmol) instead of 1-fluoro-4-iodobenzene, the title compound (0.47 g, 2.3 mmol) was afforded as a white solid (yield 45%).
1H-NMR (500 MHz, CDCl3) δ: 7.36 (2H, d, J=8 Hz), 7.17 (2H, d, J=8 Hz), 4.03-3.98 (2H, m), 3.96-3.92 (2H, m), 3.12-3.07 (1H, m), 1.95 (2H, t, J=6 Hz), 1.31 (9H, s).

### (2) {[(2-{[trans-5-(4-tert-Butylphenyl)-1,3-dioxan-2-yl]methyl}-5-hydroxy-6-methylpyrimidin-4-yl)carbonyl]amino}acetic acid

In accordance with Examples 196-(4) to 196-(6), but using 2-(4-tert-butylphenyl)propane-1,3-diol (0.20 g, 0.97 mmol) instead of 2-(4-fluorophenyl)propane-1,3-diol, the title compound (0.23 g, 0.51 mmol) was afforded as a white solid (yield 53%).
MS m/z: 444 (M+H)⁺;
1H-NMR (500 MHz, CDCl3) δ:11.36 (1H, s), 8.51 (1H, t, J=6 Hz), 7.34 (2H, d, J=8 Hz), 7.11 (2H, d, J=8 Hz), 5.20 (1H, t, J=6 Hz), 4.30 (2H, d, J=6 Hz), 4.19 (2H, dd, J=12 Hz, 4 Hz), 3.83 (2H, t, J=12 Hz), 3.26 (2H, d, J=6 Hz), 3.26-3.20 (1H, m), 2.56 (3H, s), 1.30 (9H, s).

### Example 198

### {[(2-{([trans-5-(Biphenyl-4-yl)-1,3-dioxan-2-yl]methyl)-5-hydroxy-6-methylpyrimidin-4-yl)carbonyl]amino}acetic acid

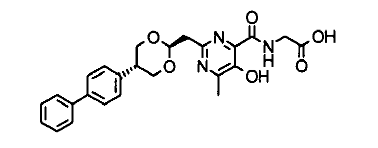

### (1) 2-(Biphenyl-4-yl)propane-1,3-diol

In accordance with Example 196-(1), but using 4-iodobiphenyl (1.4 g, 5.0 mmol) instead of 1-fluoro-4-iodobenzene, the title compound (0.37 g, 1.6 mmol) was afforded as a white solid (yield 33%).
1H-NMR (500 MHz, CDCl3) δ: 7.57 (4H, d, J=8 Hz), 7.44 (2H, t, J=8 Hz), 7.34 (1H, t, J=8 Hz), 7.32 (2H, d, J=8 Hz), 4.08-4.03 (2H, m), 4.01-3.97 (2H, m), 3.19-3.14 (1H, m), 2.03 (2H, brs).

### (2) {[(2-{[trans-5-(Biphenyl-4-yl)-1,3-dioxan-2-yl]methyl}-5-hydroxy-6-methylpyrimidin-4-yl)carbonyl] amino }acetic acid

In accordance with Examples 196-(4) to 196-(6), but using 2-(biphenyl-4-yl)propane-1,3-diol (0.11 g, 0.49 mmol) instead of 2-(4-fluorophenyl)propane-1,3-diol, the title compound (0.082 g, 0.18 mmol) was afforded as a white solid (yield 36%). MS m/z: 464 (M+H)⁺;
1H-NMR (500 MHz, DMSO-d6) 8: 9.21 (1H, brs), 7.63 (2H, d, J=8 Hz), 7.60 (2H, d, J=8 Hz), 7.45 (2H, t, J=8 Hz), 7.39 (2H, d, J=8 Hz), 7.35 (1H, t, J=8 Hz), 5.27 (1H, t, J=5 Hz), 4.09 (2H, dd, J=11, 4 Hz), 3.88 (2H, t, J=11 Hz), 3.72 (2H, brs), 3.24-3.17 (1H, m), 3.11 (2H, d, J=5 Hz), 2.43 (3H, s).

### Example 199

### {[(5-Hydroxy-6-methyl-2-{[trans-5-(4-pyridin-2-yl)phenyl-1,3-dioxan-2-yl]methyl}pyrimidin-4-yl)carbonyl]amino}acetic acid

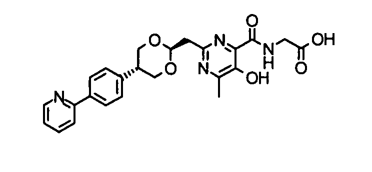

### (1) Diethyl [(4-pyridin-2-yl)phenyl]malonate

Diethyl (4-bromophenyl)malonate (1.7 g, 5.4 mmol) was dissolved in 1,4-dioxane (30 mL), and 4,4,4',4',5,5,5',5'-octamethyl-2,2'-bi-1,3,2-dioxaborolane (1.6 g, 6.4 mmol), potassium acetate (1.3 g, 13 mol) and [1,1'-bis(diphenylphosphino)ferrocene]palladium dichloride-dichloromethane adduct (0.22 g, 0.27 mmol) were added, followed by heating to reflux for 5 hours under a nitrogen atmosphere. The reaction solution was cooled to room temperature, and subsequently filtered with celite. After the filtrate was concentrated under reduced pressure, the resulting residue was purified by silica gel column chromatography (Moritex Corporation, elution solvent: hexane/ethyl acetate), and a fraction corresponding to the Rf value=0.19 (hexane/ethyl acetate=10/1) by thin layer chromatography was concentrated under reduced pressure to afford diethyl [4-(4,4,5,5-tetramethyl-1,3,2-dioxaborolan-2-yl)phenyl]malonate as an oil. This was dissolved in 1,2-dimethoxyethane (15 mL), and 2-bromopyridine (0.53 mL, 5.6 mmol), [1,1'-bis(diphenylphosphino)ferrocene]palladium dichloride-dichloromethane adduct (0.11 g, 0.14 mmol), potassium phosphate hydrate (1.8 g, 8.4 mmol) and water (1.5 mL) were added, followed by heating to reflux for 3 hours under a nitrogen atmosphere. After the reaction solution was cooled to room temperature, water was added, followed by extraction with ethyl acetate. After the extract was concentrated under reduced pressure, the resulting residue was purified by silica gel column chromatography (Moritex Corporation, elution solvent: hexane/ethyl acetate), and a fraction corresponding to the Rf value=0.23 (hexane/ethyl acetate=4/1) by thin layer chromatography was concentrated under reduced pressure to afford the title compound (0.50 g, 1.6 mmol) as a pale red solid (yield 30%).
1H-NMR (500 MHz, CDCl3) δ: 8.70 (1H, d, J=5 Hz), 8.00 (2H, d, J=8 Hz), 7.78-7.72 (2H, m), 7.52 (2H, d, J=8 Hz), 7.24 (1H, t, J=5 Hz), 4.68 (1H, s), 4.27-4.20 (4H, m), 1.27 (6H, t, J=7 Hz).

### (2) 2-[(4-Pyridin-2-yl)phenyl]propane-1,3-diol

Under a nitrogen atmosphere, lithium aluminum hydride (0.21 g, 5.6 mmol) was suspended in tetrahydrofuran (10 mL), and a solution of diethyl [(4-pyridin-2-yl)phenyl]malonate (0.58 g, 1.9 mmol) in tetrahydrofuran (10 mL) was added dropwise at 0°C over 5 minutes, followed by stirring at the same temperature for 1.5 hours. After the reaction solution was added dropwise to water; the resulting suspension was filtered with celite, and the filtrate was extracted with ethyl acetate. After the extract was concentrated under reduced pressure, the resulting residue was purified by silica gel column chromatography (Moritex Corporation, elution solvent: hexane/ethyl acetate), and a fraction corresponding to the Rf value=0.27 (ethyl acetate) by thin layer chromatography was concentrated under reduced pressure to afford the title compound (0.25 g, 1.1 mmol) as a pale yellow oil (yield 59%).
1H-NMR (500 MHz, CDCl3) δ: 8.68 (1H, d, J=5 Hz), 7.94 (2H, d, J=8 Hz), 7.78-7.74 (1H, m), 7.71 (1H, d, J=8 Hz), 7.35 (2H, d, J=8 Hz), 7.24 (1H, dd, J=7 Hz, 5 Hz), 4.04 (2H, dd, J=11 Hz, 7 Hz), 3.97 (2H, dd, J=11 Hz, 5 Hz), 3.16 (1H, tt, J=7 Hz, 5 Hz), 2.32 (2H, brs).

### (3) {[(5-Hydroxy-6-methyl-2-{[trans-5-(4-pyridin-2-yl)phenyl-1,3-dioxan-2-yl]methyl}pyrimidin-4-yl)carbonyl]amino}acetic acid

In accordance with Examples 196-(4) to 196-(6), but using 2-[(4-pyridin-2-yl)phenyl]propane-1,3-diol (0.13 g, 0.55 mmol) instead of 2-(4-fluorophenyl)propane-1,3-diol, the title compound (0.056 g, 0.12 mmol) was afforded as a white solid (yield 22%).
MS m/z: 465 (M+H)⁺;
1H-NMR (500 MHz, CDCl3) δ:11.47 (1H, s), 8.77 (1H, d, J=5 Hz), 8.59 (1H, t, J=5 Hz), 7.89 (2H, d, J=8 Hz), 7.86 (1H, t, J=7 Hz), 7.72 (1H, d, J=7 Hz), 7.36-7.32 (3H, m), 5.28 (1H, t, J=5 Hz), 4.25 (2H, d, J=5 Hz), 4.25-4.22 (2H, m), 3.92 (2H, t, J=11 Hz), 3.36 (1H, tt, J=11 Hz, 5 Hz), 3.38 (2H, d, J=5 Hz), 2.55 (3H, s).

### Example 200

### ({[5-Hydroxy-6-methyl-2-({trans-5-[4-(N-methyl-N-phenylamino)phenyl]-1,3-dioxan-2-yl}methyl)pyrimidin-4-yl]carbonyl}amino)acetic acid

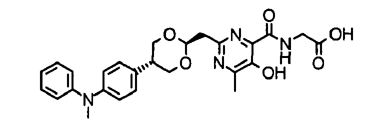

### (1) 2-[4-(N-Methyl-N-phenylamino)phenyl]propane-1,3-diol

Diethyl (4-bromophenyl)malonate (1.6 g, 5.0 mmol), N-methylaniline (1.1 mL, 10 mmol), dicyclohexyl(2',4',6'-triisopropylbiphenyl-2-yl)phosphine (X-PHOS) (0.24 g, 0.50 mmol), potassium carbonate (1.7 g, 13 mol) and tris(dibenzylideneacetone)dipalladium (0.23 g, 0.25 mmol) were suspended in tert-butanol (30 mL), followed by heating to reflux for 3.5 hours under a nitrogen atmosphere. The reaction solution was cooled to room temperature, and subsequently filtered with celite. After the filtrate was concentrated under reduced pressure, the resulting residue was purified by silica gel column chromatography (Moritex Corporation, elution solvent: hexane/ethyl acetate), and a fraction corresponding to the Rf value=0.21 (hexane/ethyl acetate=10/1) by thin layer chromatography was concentrated under reduced pressure to afford diethyl [4-(N-methyl-N-phenylamino)phenyl]malonate (1.4 g, 4.0 mmol) as a brown oil (yield 81%). Under a nitrogen atmosphere, lithium aluminum hydride (0.49 g, 13 mmol) was suspended in tetrahydrofuran (40 mL), and a solution of diethyl [4-(N-methyl-N-phenylamino)phenyl]malonate (1.5 g, 4.3 mmol) in tetrahydrofuran (10 mL) was added dropwise at 0°C over 5 minutes, followed by stirring at the same temperature for 2 hours. After the reaction solution was added dropwise to water, the resulting suspension was filtered with celite, the filtrate was extracted with ethyl acetate, and the organic layer was dried over anhydrous sodium sulfate. After the solvent was distilled off under reduced pressure, the resulting residue was washed with a small amount of ethyl acetate to afford the title compound (0.78 g, 3.0 mmol) as a white solid (yield 71%).
1H-NMR (500 MHz, CDCl3) δ: 7.29 (2H, d, J=7 Hz), 7.13 (2H, t, J=8 Hz), 7.04 (2H, d, J=7 Hz), 7.00-6.96 (3H, m), 3.98 (2H, dd, J=11 Hz, 7 Hz), 3.93 (2H, dd, J=11 Hz, 5 Hz), 3.30 (3H, s), 3.10-3.04 (1H, m), 1.95 (2H, brs).

### (2) ({[5-Hydroxy-6-methyl-2-({trans-5-[4-(N-methyl-N-phenylamino)phenyl]-1,3-dioxan-2-yl}methyl)pyrimidin-4-yl]carbonyl}amino)acetic acid

In accordance with Examples 196-(4) to 196-(6), but using 2-[4-(N-methyl-N-phenylamino)phenyl]propane-1,3-diol (0.11 g, 0.44 mmol) instead of 2-(4-fluorophenyl)propane-1,3-diol, the title compound (0.11 g, 0.23 mmol) was afforded as a pale yellow solid (yield 53%).
MS m/z: 493 (M+H)⁺;
1H-NMR (500 MHz, DMSO-d6) δ: 9.14 (1H, brs), 7.25 (2H, t, J=7 Hz), 7.20 (2H, d, J=8 Hz), 6.96 (2H, d, J=7 Hz), 6.94 (2H, d, J=8 Hz), 6.91 (1H, t, J=7 Hz), 5.23 (1H, t, J=5 Hz), 4.03 (2H, dd, J=11 Hz, 4 Hz), 3.79 (2H, t, J=11 Hz), 3.64 (2H, brs), 3.22 (3H, s), 3.10 (2H, d, J=5 Hz), 3.12-3.04 (1H, m), 2.42 (3H, s).

### Example 201

### [({5-Hydroxy-6-methyl-2-[(5-phenyl-1,3-dioxepan-2-yl)methyl]pynmidin-4-yl}carbonyl)amino]acetic acid

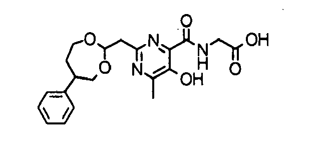

In accordance with Examples 196-(4) to 196-(6), but using 2-phenylbutane-1,4-diol (0.12 g, 0.72 mmol) instead of 2-(4-fluorophenyl)propane-1,3-diol, the title compound (0.17 g, 0.41 mmol) was afforded as a white solid (yield 57%).
MS m/z: 402 (M+H)⁺;
1H-NMR (500 MHz, CDCl3) δ:11.34 (1H, s), 8.49 and 8.47 (total 1H, each t, J=6 Hz), 7.32-7.28 (2H, m), 7.24-7.18 (3H, m), 5.48 and 5.46 (total 1H, each t, J=6 Hz), 4.27 and 4.26 (total 2H, d, J=6 Hz), 4.12-4.01 and 3.96-3.93 (total 2H, each m), 3.83-3.68 (2H, m), 3.25-3.17 (2H, m), 3.06-2.94 (1H, m), 2.54 and 2.53 (total 3H, each s), 2.09-1.90 (2H, m).

### (Test Example 1)

The utility (pharmacological activity) of compounds of the present invention was confirmed by the testing indicated below.

The in vitro erythropoietin (EPO) induction activity of test compounds was evaluated using human liver cancer-derived cell line Hep3B (ATCC, Manassas, VA). Hep3B cells were cultured overnight at 37°C in Dulbecco's Modified Eagle's Medium (DMEM) in the presence of 10% fetal bovine serum (FBS) (24-well plate, 1.0 x 10⁵ cells/well). After replacing with fresh DMEM (+10% FBS) containing a test compound dissolved in 0.5% dimethyl sulfoxide (DMSO) (prepared to a concentration of 12.5 µM) or a solvent control (0.5% DMSO), the cells were cultured for 24 hours at 37°C. After recovering the culture supernatant, EPO concentration in the culture supernatant was quantified using a human EPO ELISA kit (StemCell Technologies). The compounds of Examples 1, 2, 5, 20, 43, 56, 100, 103, 109, 167, 173, 181, 193 and 198 were used for the test compounds. The EPO concentration of each Example was expressed as a multiple against the EPO concentration in the control. The results are shown in Table 1. The compounds of the present invention or pharmacologically acceptable salts thereof, demonstrated superior EPO production activity.

**[Table 1]**

| Test Compound | EPO Concentration (multiple) |
|---|---|
| Control (0.5% DMSO) | 1 |
| Example 1 | 98 |
| Example 2 | 37 |
| Example 5 | 29 |
| Example 20 | 127 |
| Example 43 | 262 |
| Example 56 | 158 |
| Example 100 | 104 |
| Example 103 | 100 |
| Example 109 | 142 |
| Example 167 | 225 |
| Example 173 | 184 |
| Example 181 | 155 |
| Example 193 | 147 |
| Example 198 | 108 |

### Industrial applicability

A novel compound having a 5-hydroxypyrimidine-4-carboxamide structure of the present invention has superior EPO production activity and is useful for diseases caused by decreased levels of erythropoietin. More specifically, a compound of the present invention is useful as a medicament for prophylaxis and/or treatment of anemia, particularly nephrogenic anemia (dialysis stage, conservation stage), anemia of prematurity, anemia incidental to chronic diseases, anemia incidental to cancer chemotherapy and cancerous anemia, and further can also be used as a medicament for prophylaxis and /or treatment of ischemic brain diseases and the like.

## Claims

1. A compound represented by the following formula (1) or a pharmacologically acceptable salt thereof: 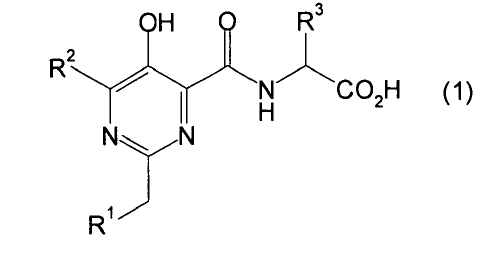 [wherein,
R¹ represents a group -X-Q¹, X-Q¹-Y-Q² or -X-Q¹-Y-Q²-Z-Q³,
X represents a single bond, -CH₂-, -(CH₂)₂-, -(CH₂)₃-, -CH₂O-, -OCH₂-, -O-, - S- or -CO-,
Q¹ represents a monocyclic or bicyclic heterocyclic group which may have 1 or 2 substituents selected from substituent group α (wherein the heterocyclic group includes 4- to 10-membered aromatic heterocycles and non-aromatic heterocycles, and contains 1 or 2 atoms selected from the group consisting of a nitrogen atom, a sulfur atom and an oxygen atom),
substituent group α represents a group consisting of a halogen atom; an oxo group, a hydroxyl group, a sulfanyl group, an amino group, a cyano group, a C₁-C₆ alkyl group and a halogenated C₁-C₆ alkyl group,
Y represents a single bond, -CH₂-, -(CH₂)₂-, -(CH₂)₃-, -CH₂O-, -OCH₂-, -O-, - S-, -NH-, -NCH₃- or -CO-,
Q² represents a monocyclic or bicyclic hydrocarbon ring group which may have 1 or 2 substituents selected from substituent group β (wherein the hydrocarbon ring group includes 3- to 10-membered aromatic hydrocarbon rings and non-aromatic hydrocarbon rings), or represents a monocyclic or bicyclic heterocyclic group which may have 1 or 2 substituents selected from substituent group β (wherein the heterocyclic group includes 4- to 10-membered aromatic heterocycles and non-aromatic heterocycles, and contains 1 or 2 atoms selected from the group consisting of a nitrogen atom, a sulfur atom and an oxygen atom),
substituent group β represents a group consisting of a halogen atom, an oxo group, a hydroxyl group, a sulfanyl group, an amino group, a cyano group, a nitro group, a formyl group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a C₁-C₆ alkylamino group, a (C₁-C₆ alkyl)(C₁-C₆ alkyl)amino group and a C₂-C₇ alkanoyl group,
Z represents a single bond, -CR¹¹R¹²-, -(CH₂)₂-, -(CH₂)₃-, -CH₂O-, -OCH₂-, -O--S-, -NH-, -NCH₃- or -CO-,
R¹¹ and R¹² each independently represents a hydrogen atom, a halogen atom, a hydroxyl group, a cyano group, a C₁-C₆ alkyl group, a halogenated C₁-C₆ alkyl group, a C₁-C₆ alkoxy group or a halogenated C₁-C₆ alkoxy group,
Q³ represents a phenyl group which may have 1 or 2 substituents selected from substituent group γ, a C₃-C₇ cycloalkyl group which may have 1 or 2 substituents selected from substituent group γ, a C₃-C₇ cycloalkenyl group which may have 1 or 2 substituents selected from substituent group γ, or a monocyclic or bicyclic heterocyclic group which may have 1 or 2 substituents selected from substituent group γ (wherein the heterocyclic group includes 4- to 10-membered aromatic heterocycles and non-aromatic heterocycles, and contains 1 or 2 atoms selected from the group consisting of a nitrogen atom, a sulfur atom and an oxygen atom),
substituent group γ represents a group consisting of a halogen atom, an oxo group, a hydroxyl group, a nitro group, a sulfanyl group, an amino group, a cyano group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a C₁-C₆ alkylamino group, a (C₁-C₆ alkyl)(C₁-C₆ alkyl)amino group and a C₂-C₇ alkanoyl group,
R² represents a C₁-C₃ alkyl group, a methylsulfanyl group, a fluoromethyl group or a difluoromethyl group, and
R³ represents a hydrogen atom or a methyl group].

2. The compound or pharmacologically acceptable salt thereof according to claim 1, wherein X represents a single bond, -CH₂-, -(CH₂)₂- or -(CH₂)₃-.

3. The compound or pharmacologically acceptable salt thereof according to claim 1, wherein X represents a single bond or -CH₂-.

4. The compound or pharmacologically acceptable salt thereof according to any one of claims 1 to 3, wherein Q¹ represents a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a piperazinyl group, a dihydrooxazolyl group, a dihydrothiazolyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a tropinyl group, a chromanyl group, an isochromanyl group, a quinolyl group, a tetrahydroquinolyl group, an isoquinolyl group or a tetrahydroisoquinolyl group, each of which may have 1 or 2 substituents selected from substituent group α.

5. The compound or pharmacologically acceptable salt thereof according to any one of claims 1 to 3, wherein Q¹ represents a dioxanyl group, a dioxepanyl group, a pyrrolidinyl group, a piperidyl group, an azepanyl group, a pyrrolyl group, a pyridyl group, a dihydropyridyl group, a tetrahydropyridyl group, a pyrimidinyl group, a thiazolyl group, a tropinyl group or a tetrahydroisoquinolyl group, each of which may have 1 or 2 substituents selected from substituent group α.

6. The compound or pharmacologically acceptable salt thereof according to any one of claims 1 to 3, wherein Q¹ represents a piperidyl group or a pyridyl group, each of which may have 1 or 2 substituents selected from substituent group α.

7. The compound or pharmacologically acceptable salt thereof according to any one of claims 1 to 6, wherein substituent group α represents a group consisting of a methyl group and an oxo group.

8. The compound or pharmacologically acceptable salt thereof according to any one of claims 1 to 7, wherein R¹ represents a group X-Q¹-Y-Q² or -X-Q¹-Y-Q²-Z-Q³, and Y represents a single bond, -CH₂-, -(CH₂)₂- or -(-CH₂)₃-.

9. The compound or pharmacologically acceptable salt thereof according to claim 8, wherein Y represents a single bond.

10. The compound or pharmacologically acceptable salt thereof according to any one of claims 1 to 9, wherein R¹ represents a group X-Q¹-Y-Q² or -X-Q¹-Y-Q²-Z-Q³, and Q² represents a cyclopentyl group, a cyclohexyl group, a cycloheptyl group, a cyclopentenyl group, a cyclohexenyl group, a cycloheptenyl group, a phenyl group, a naphthalenyl group, a tetrahydronaphthalenyl group, a decahydronaphthalenyl group, an indanyl group, an indenyl group, a norboranyl group, a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a piperazinyl group, a dihydrooxazolyl group, a dihydrothiazolyl group, a morpholinyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a tetrahydroquinolyl group, an isoquinolyl group, a tetrahydroisoquinolyl group, a benzoxazolyl group, a quinoxalyl group, a benzothiazolyl group, a benzodioxanyl group, an indolidinyl group, a thienopyridyl group, a dihydrothienopyridyl group, a furopyridyl group or a dihydrofuropyridyl group, each of which may have 1 or 2 substituents selected from substituent group β.

11. The compound or pharmacologically acceptable salt thereof according to claim 10, wherein Q² represents a phenyl group, a naphthalenyl group, a pyridyl group, a thienyl group, a pyrimidinyl group, a pyridazinyl group, a pyrazinyl group, a thiazolyl group, an oxazolyl group, a dihydrobenzofuranyl group, a benzothiophenyl group, an indolyl group, a quinolyl group, an isoquinolyl group, a benzoxazolyl group, a quinoxalyl group or a benzothiazolyl group, each of which may have 1 or 2 substituents selected from substituent group β.

12. The compound or pharmacologically acceptable salt thereof according to claim 10, wherein Q² represents a phenyl group, a pyridyl group, a thienyl group, a pyrimidinyl group, a pyrazinyl group or a thiazolyl group, each of which may have 1 or 2 substituents selected from substituent group β.

13. The compound or pharmacologically acceptable salt thereof according to any one of claims 1 to 12, wherein R¹ represents a group X-Q¹-Y-Q² or -X-Q¹-Y-Q²-Z-Q³, and substituent group β represents a group consisting of a halogen atom, a hydroxyl group, a cyano group, a nitro group, a formyl group, a C₁-C₆ alkyl group, a halogenated C₁-C₆ alkyl group, a C₁-C₆ alkoxy group, a halogenated C₁-C₆ alkoxy group, a C₁-C₆ alkylsulfanyl group, a (C₁-C₆ alkyl)(C₁-C₆ alkyl)amino group and a C₂-C₇ alkanoyl group.

14. The compound or pharmacologically acceptable salt thereof according to claim 13, wherein substituent group β represents a group consisting of a fluorine atom, a methyl group, a tert-butyl group and a trifluoromethyl group.

15. The compound or pharmacologically acceptable salt thereof according to any one of claims 1 to 14, wherein R¹ represents a group -X-Q¹-Y-Q²-Z-Q³, Z represents a single bond, -CR¹¹R¹²-, -(-CH₂)₂-, -CH₂O-, -OCH₂-, -O-, -CO- or -NCH₃-, and R¹¹ and R¹² each independently represents a hydrogen atom, a methyl group or a hydroxyl group.

16. The compound or pharmacologically acceptable salt thereof according to claim 15, wherein Z represents a single bond or -CR¹¹R¹²-, and R¹¹ and R¹² each independently represents a hydrogen atom, a methyl group or a hydroxyl group.

17. The compound or pharmacologically acceptable salt thereof according to any one of claims 1 to 16, wherein R¹ represents a group -X-Q¹-Y-Q²-Z-Q³, and Q³ represents a cyclopropyl group, a cyclobutyl group, a cyclopentyl group, a cyclohexyl group, a cycloheptyl group, a cyclopentenyl group, a cyclohexenyl group, a cycloheptenyl group, a phenyl group, a tetrahydrofuranyl group, a tetrahydropyranyl group, a dioxolanyl group, a dioxanyl group, a dioxepanyl group, an azetidinyl group, a pyrrolidinyl group, a piperidyl group, an azepanyl group, a dihydrooxazolyl group, a dihydrothiazolyl group, a morpholinyl group, a pyrrolyl group, a dihydropyrrolyl group, a pyridyl group, a dihydropyridyl group, a tetrahydropyridyl group, a thienyl group, a furyl group, a pyrimidinyl group, a pyridazinyl group, a pyrazinyl group, a pyrazolyl group, a thiazolyl group, an isothiazolyl group, an oxazolyl group, an isoxazolyl group, a chromanyl group, an isochromanyl group, a benzofuranyl group, a dihydrobenzofuranyl group, a benzothiophenyl group, a dihydrobenzothiophenyl group, an indolyl group, an isoindolyl group, a quinolyl group, a tetrahydroquinolyl group, an isoquinolyl group, a tetrahydroisoquinolyl group, an imidazopyridyl group, a benzoxazolyl group, a quinoxalyl group, a benzothiazolyl group, a benzodioxanyl group, an indolidinyl group, a thienopyridyl group, a dihydrothienopyridyl group, a furopyridyl group or a dihydrofuropyridyl group, each of which may have 1 or 2 substituents selected from substituent group γ.

18. The compound or pharmacologically acceptable salt thereof according to claim 17, wherein Q³ represents a cyclopropyl group, a cyclohexyl group, a cyclohexenyl group, a phenyl group, a dioxanyl group, a piperidyl group, a pyrrolyl group, a pyridyl group, a thienyl group, a pyrazolyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, a quinolyl group, a tetrahydroquinolyl group, an isoquinolyl group, an imidazopyridyl group or a benzodioxanyl group, each of which may have 1 or 2 substituents selected from substituent group γ.

19. The compound or pharmacologically acceptable salt thereof according to claim 17, wherein Q³ represents a cyclopropyl group, a phenyl group, a pyridyl group, a thienyl group or a thiazolyl group, each of which may have 1 or 2 substituents selected from substituent group γ.

20. The compound or pharmacologically acceptable salt thereof according to any one of claims 1 to 19, wherein R¹ represents a group -X-Q¹-Y-Q²-Z-Q³, and substituent group γ represents a group consisting of a halogen atom, a hydroxyl group, a nitro group, an amino group, a cyano group, a C₁-C₆ alkyl group, a halogenated C₁-C₆ alkyl group and a C₁-C₆ alkoxy group.

21. The compound or pharmacologically acceptable salt thereof according to claim 20, wherein substituent group γ represents a group consisting of a fluorine atom, a methyl group and a methoxy group.

22. The compound or pharmacologically acceptable salt thereof according to any one of claims 1 to 21, wherein R² represents a methyl group, a methylsulfanyl group or a difluoromethyl group.

23. The compound or pharmacologically acceptable salt thereof according to any one of claims 1 to 21, wherein R² represents a methyl group or a methylsulfanyl group.

24. The compound or pharmacologically acceptable salt thereof according to any one of claims 1 to 23, wherein R³ represents a hydrogen atom.

25. The compound or pharmacologically acceptable salt thereof according to claim 1, wherein
R¹ represents a group X-Q¹-Y-Q²,
X represents a single bond, -CH₂-, -(CH₂)₂- or -(CH₂)₃-,
Q¹ represents a piperidyl group or a pyridyl group, each of which may have 1 or 2 substituents selected from substituent group α,
substituent group α represents a group consisting of a methyl group,
Y represents a single bond,
Q² represents a phenyl group, a naphthalenyl group, a pyridyl group, a thienyl group, a thiazolyl group, a benzothiophenyl group, a quinolyl group, an isoquinolyl group, a benzoxazolyl group, a dihydrobenzofuranyl group, a benzothiazolyl group or a quinoxalyl group, each of which may have 1 or 2 substituents selected from substituent group β, and
substituent group β represents a group consisting of a fluorine atom, a methyl group, a tert-butyl group and a trifluoromethyl group.

26. The compound or pharmacologically acceptable salt thereof according to claim 25, wherein Q² represents a phenyl group, a pyridyl group, a thienyl group or a thiazolyl group, each of which may have 1 or 2 substituents selected from substituent group β.

27. The compound or pharmacologically acceptable salt thereof according to claim 1, wherein
R¹ represents a group -X-Q¹-Y-Q²-Z-Q³,
X represents a single bond,
Q¹ represents a piperidyl group or a pyridyl group, each of which may have 1 or 2 substituents selected from substituent group α,
substituent group α represents a group consisting of a methyl group,
Y represents a single bond,
Q² represents a phenyl group, a pyridyl group, a thienyl group, a pyrimidinyl group, a pyridazinyl group, a pyrazinyl group, a thiazolyl group, an oxazolyl group or an indolyl group, each of which may have 1 or 2 substituents selected from substituent group β,
substituent group β represents a group consisting of a fluorine atom or a methyl group,
Z represents a single bond, -CR¹¹R¹²-, -(CH₂)₂-, -CH₂O-, -OCH₂-, -O-, -CO- or -NCH₃-,
R¹¹ and R¹² each represents a hydrogen atom, a methyl group or a hydroxyl group,
Q³ represents a cyclopropyl group, a cyclohexyl group, a cyclohexenyl group, a phenyl group, a dioxanyl group, a piperidyl group, a pyrrolyl group, a pyrazolyl group, a pyridyl group, a thienyl group, a pyrimidinyl group, a pyridazinyl group, a pyrazinyl group, a thiazolyl group, an oxazolyl group, an isoxazolyl group, a benzofuranyl group, a benzothiophenyl group, a benzothiazolyl group, an imidazopyridyl group, a quinolyl group, an isoquinolyl group, a tetrahydroquinolyl group or a benzodioxanyl group, each of which may have 1 or 2 substituents selected from substituent group γ, and
substituent group γ represents a group consisting of a fluorine atom, a methyl group and a methoxy group.

28. The compound or pharmacologically acceptable salt thereof according to claim 27, wherein Q² represents a phenyl group, a pyridyl group, a thienyl group, a pyrimidinyl group, a pyrazinyl group or a thiazolyl group, each of which may have 1 or 2 substituents selected from substituent group β, and Q³ represents a cyclopropyl group, a phenyl group, a pyridyl group, a thienyl group or a thiazolyl group, each of which may have 1 or 2 substituents selected from substituent group γ.

29. The compound or a pharmacologically acceptable salt thereof according to claim 1 that is selected from the following:
{[(2-{[1-(4-tert-butylphenyl)piperidin-4-yl]methyl}-5-hydroxy-6-methylpyrimidin-4-yl)carbonyl]amino}acetic acid,
[({5-hydroxy-6-methyl-2-[(1-phenylpiperidin-4-yl)methyl]pyrimidin-4-yl}carbonyl)amino]acetic acid,
{[(2-{[1-(4-fluorophenyl)piperidin-4-yl]methyl}-5-hydroxy-6-methylpyrimidin-4-yl)carbonyl]amino}acetic acid,
({[5-hydroxy-6-methyl-2-({1-[4-(trifluoromethyl)phenyl]piperidin-4-yl}methyl)pyrimidin-4-yl]carbonyl}amino)acetic acid,
{[(2-{[1-(biphenyl-4-yl)piperidin-4-yl]methyl}-5-hydroxy-6-methylpyrimidin-4-yl)carbonyl]amino} acetic acid,
{[(2-{[1-(2'-fluorobiphenyl-4-yl)piperidin-4-yl]methyl}-5-hydroxy-6-methylpyrimidin-4-yl)carbonyl]amino}acetic acid,
{[(2-{[1-(3'-fluorobiphenyl-4-yl)piperidin-4-yl]methyl}-5-hydroxy-6-methylpyrimidin-4-yl)carbonyl]amino}acetic acid,
{[(2-{[1-(4'-fluorobiphenyl-4-yl)piperidin-4-yl]methyl}-5-hydroxy-6-methylpyrimidin-4-yl)carbonyl]amino} acetic acid,
{[(2-{[1-(4'-cyanobiphenyl-4-yl)piperidin-4-yl]methyl}-5-hydroxy-6-methylpyrimidin-4-yl)carbonyl]amino}acetic acid,
{[(5-hydroxy-2-{[1-(4'-methoxybiphenyl-4-yl)piperidin-4-yl]methyl} -6-methylpyrimidin-4-yl)carbonyl]amino} acetic acid,
{[(2-{[1-(2',4'-difluorobiphenyl-4-yl)piperidin-4-yl]methyl}-5-hydroxy-6-methylpyrimidin-4-yl)carbonyl]amino} acetic acid,
{[(2-{[1-(4'-fluoro-2-methylbiphenyl-4-yl)piperidin-4-yl]methyl} -5-hydroxy-6-methylpyrimidin-4-yl)carbonyl]amino}acetic acid,
{[(2-{[1-(4-benzylphenyl)piperidin-4-yl]methyl}-5-hydroxy-6-methylpyrimidin-4-yl)carbonyl]amino} acetic acid,
({[2-({1-[4-(4-fluorobenzyl)phenyl]piperidin-4-yl}methyl)-5-hydroxy-6-methylpyrimidin-4-yl]carbonyl}amino)acetic acid,
({[5-hydroxy-6-methyl-2-({1-[4-(3-methylbenzyl)phenyl]piperidin-4-yl}methyl)pyrimidin-4-yl]carbonyl}amino)acetic acid,
({[5-hydroxy-6-methyl-2-({1-[4-(4-methylbenzyl)phenyl]piperidin-4-yl}methyl)pyrimidin-4-yl]carbonyl}amino)acetic acid,
({[5-hydroxy-2-({1-[4-(4-methoxybenzyl)phenyl]piperidin-4-yl}methyl)-6-methylpyrimidin-4-yl]carbonyl}amino)acetic acid,
{[(2-{[1-(4-benzyl-3-fluorophenyl)piperidin-4-yl]methyl}-5-hydroxy-6-methylpyrimidin-4-yl)carbonyl]amino} acetic acid,
({[5-hydroxy-6-methyl-2-({1-[5-(4-methylphenyl)pyrimidin-2-yl]piperidin-4-yl}methyl)pyrimidin-4-yl]carbonyl}amino)acetic acid,
({[5-hydroxy-6-methyl-2-({1-[5-(4-methylphenyl)pyrazin-2-yl]piperidin-4-yl}methyl)pyrimidin-4-yl]carbonyl}amino)acetic acid,
{[(5-hydroxy-6-methyl-2-{[1-(5-phenylpyridin-2-yl)piperidin-4-yl]methyl}pyrimidin-4-yl)carbonyl]amino} acetic acid,
({[5-hydroxy-6-methyl-2-({1-[6-(4-methylphenyl)pyridin-3-yl]piperidin-4-yl}methyl)pyrimidin-4-yl]carbonyl}amino)acetic acid,
({[5-hydroxy-6-methyl-2-({1-[5-(4-methylphenyl)thiophen-2-yl]piperidin-4-yl}methyl)pyrimidin-4-yl]carbonyl}amino)acetic acid,
({[5-hydroxy-6-methyl-2-({1-[5-(4-methylphenyl)-1,3-thiazol-2-yl]piperidin-4-yl}methyl)pyrimidin-4-yl]carbonyl}amino)acetic acid,
({[5-hydroxy-6-methyl-2-({1-[4-(pyridin-2-yl)phenyl]piperidin-4-yl}methyl)pyrimidin-4-yl]carbonyl}amino)acetic acid,
({[5-hydroxy-6-methyl-2-({1-[4-(pyridin-3-yl)phenyl]piperidin-4-yl}methyl)pyrimidin-4-yl]carbonyl}amino)acetic acid,
({[5-hydroxy-6-methyl-2-({1-[4-(thiophen-3-yl)phenyl]piperidin-4-yl}methyl)pyrimidin-4-yl]carbonyl}amino)acetic acid,
({[5-hydroxy-6-methyl-2-({1-[4-(1,3-thiazol-2-yl)phenyl]piperidin-4-yl}methyl)pyrimidin-4-yl]carbonyl}amino)acetic acid,
({[2-({1-[4-(1-benzothiophen-3-yl)phenyl]piperidin-4-yl}methyl)-5-hydroxy-6-methylpyrimidin-4-yl]carbonyl}amino)acetic acid,
({[5-hydroxy-2-({1-[4-(isoquinolin-5-yl)phenyl]piperidin-4-yl}methyl)-6-methylpyrimidin-4-yl]carbonyl}amino)acetic acid,
({[2-({1-[4-(1,4-benzodioxan-5-yl)phenyl]piperidin-4-yl}methyl)-5-hydroxy-6-methylpyrimidin-4-yl]carbonyl}amino)acetic acid,
[({5-hydroxy-6-methyl-2-[(1-{4-[(pyridin-3-yl)methyl]phenyl}piperidin-4-yl)methyl]pyrimidin-4-yl}carbonyl)amino]acetic acid,
[({5-hydroxy-6-methyl-2-[(1-{4-[(6-methylpyridin-3-yl)methyl]phenyl}piperidin-4-yl)methyl]pyrimidin-4-yl}carbonyl)amino] acetic acid,
[({5-hydroxy-6-methyl-2-[(1-{4-[(4-methylpyridin-3-yl)methyl]phenyl}piperidin-4-yl)methyl]pyrimidin-4-yl}carbonyl)amino]acetic acid,
[({5-hydroxy-6-methyl-2-[(1-{4-[(6-methoxypyridin-3-yl)methyl]phenyl}piperidin-4-yl)methyl]pyrimidin-4-yl}carbonyl)amino]acetic acid,
[({5-hydroxy-6-methyl-2-[(1-{[3-methyl-4-(pyridin-3-yl)methyl]phenyl}piperidin-4-yl)methyl]pyrimidin-4-yl}carbonyl)amino]acetic acid,
[({2-[(1-{[3-fluoro-4-(pyridin-3-yl)methyl]phenyl}piperidin-4-yl)methyl]-5-hydroxy-6-methylpyrimidin-4-yl}carbonyl)amino]acetic acid,
[({5-hydroxy-6-methyl-2-[(1-{4-[(quinolin-3-yl)methyl]phenyl}piperidin-4-yl)methyl]pyrimidin-4-yl} carbonyl)amino]acetic acid,
({[2-({1-[4-(cyclopropylmethyl)phenyl]piperidin-4-yl}methyl)-5-hydroxy-6-methylpyrimidin-4-yl]carbonyl}amino)acetic acid,
({[5-hydroxy-6-methyl-2-({1-[4-(1-methyl-1-phenylethyl)phenyl]piperidin-4-yl}methyl)-pyrimidin-4-yl]carbonyl}amino)acetic acid,
{[(2-{[1-(biphenyl-4-yl)-3,3-dimethylpiperidin-4-yl]methyl}-5-hydroxy-6-methylpyrimidin-4-yl)carbonyl]amino}acetic acid,
{[(2-{[trans-1-(4-tert-butylphenyl)-2-methylpiperidin-4-yl]methyl} -5-hydroxy-6-methylpyrimidin-4-yl)carbonyl]amino} acetic acid,
{[(2-{[1-(4-tert-butylphenyl)-1,2,3,6-tetrahydropyridin-4-yl]methyl}-5-hydroxy-6-methylpyrimidin-4-yl)carbonyl]amino}acetic acid,
{[(2-{[6-(4-tert-butylphenyl)pyridin-3-yl]methyl} -5-hydroxy-6-methylpyrimidin-4-yl)carbonyl]amino}acetic acid,
{[(2-{[6-(biphenyl-4-yl)pyridin-3-yl]methyl}-5-hydroxy-6-methylpyrimidin-4-yl)carbonyl]amino}acetic acid,
{[(2-{[2-(4-tert-butylphenyl)pyrimidin-5-yl]methyl}-5-hydroxy-6-methylpyrimidin-4-yl)carbonyl]amino}acetic acid,
{[(2-{[5-(4-tert-butylphenyl)pyridin-2-yl]methyl}-5-hydroxy-6-methylpyrimidin-4-yl)carbonyl]amino}acetic acid,
({[2-[1-(4-tert-butylphenyl)piperidin-4-yl]methyl-5-hydroxy-6-(methylsulfanyl)pyrimidin-4-yl]carbonyl}amino)acetic acid,
({[2-{[6-(4-tert-butylphenyl)pyridin-3-yl]methyl} -5-hydroxy-6-(methylsulfanyl)pyrimidin-4-yl]carbonyl}amino)acetic acid, and
{[(2-{[trans-5-(biphenyl-4-yl)-1,3-dioxan-2-yl]methyl}-5-hydroxy-6-methylpyrimidin-4-yl)carbonyl]amino}acetic acid.

30. A medicament comprising as an active ingredient a compound or a pharmacologically acceptable salt thereof according to any one of claims 1 to 29.

31. The medicament according to claim 30, for prophylaxis and/or treatment of anemia.

32. The medicament according to claim 31, wherein the anemia is nephrogenic anemia, anemia of prematurity, anemia incidental to chronic diseases, anemia incidental to cancer chemotherapy or cancerous anemia.

33. The medicament according to claim 30, for producing erythropoietin.

34. A use of a compound or a pharmacologically acceptable salt thereof according to any one of claims 1 to 29 for manufacturing a medicament.

35. The use according to claim 34, wherein the medicament is a medicament for prophylaxis and/or treatment of anemia.

36. The use according to claim 35, wherein the anemia is nephrogenic anemia, anemia of prematurity, anemia incidental to chronic diseases, anemia incidental to cancer chemotherapy or cancerous anemia.

37. A method for producing erythropoietin, comprising administering an effective amount of a compound or a pharmacologically acceptable salt thereof according to any one of claims 1 to 29 to a mammal or bird.

38. The method according to claim 37 for prophylaxis and/or treatment of anemia.

39. The method according to claim 38, wherein the anemia is nephrogenic anemia, anemia of prematurity, anemia incidental to chronic diseases, anemia incidental to cancer chemotherapy or cancerous anemia.

40. The method according to claim 37, wherein the mammal is a human, horse, cow or pig, and the bird is a chicken.
